# EUROPÄISCHE PATENTANMELDUNG

(11) **EP 2 206 501 A2**
(43) Veröffentlichungstag der Anmeldung: **14.07.2010**
(21) Anmeldenummer: 09016125.8
(22) Anmeldetag: 04.05.2006
(51) Int. Cl.: A61K 31/7088, G01N 33/50, A61P 35/00, A61P 31/00, A61P 19/02, C12N 15/11

(54) **HMGA bindende Nukleinsäuren**

(30) Priorität: 04.05.2005 DE 102005020874
(62) Teilanmeldung aus: 06742799.7
(71) Anmelder: Noxxon Pharma AG, 10589 Berlin (DE)
(72) Erfinder: Jarosch, Florian, 13469 Berlin (DE); Maasch, Christian, 13509 Berlin (DE); Klussmann, Sven, 10709 Berlin (DE); Vater, Axel, 13467 Berlin (DE); Eulberg, Dirk, 10437 Berlin (DE); Purscheke, Werner, 13359 Berlin (DE); Buchner, Klaus, 14197 Berlin (DE)
(74) Vertreter: Bohmann, Armin K.

(57) **Zusammenfassung**

Die vorliegende Erfindung betrifft HMGA bindende Nukleinsäure, die dadurch gekennzeichnet sind, dass die Nukleinsäure einen Abschnitt Box A1 und einen Abschnitt Box A2 umfasst, wobei der Abschnitt Box A1 und der Abschnitt Box A2 durch einen Zwischenabschnitt miteinander verbunden sind und wobei Box A1 und Box A2 einzeln und unabhängig voneinander aus der Gruppe ausgewählt sind, die GGGCG, GGGUG und GGGAG umfasst.

SEQUENCE LISTING

<110>  NOXXON Pharma AG

<120>  HMGA bindende Nukleinsäuren

<130>  N 10048 EP/D-I

<150>  DE 10 2005 020 874.6
<151>  2005-05-04

<160>  50

<170>  PatentIn version 3.1

<210>  1
<211>  47
<212>  RNA
<213>  Artificial

<220>
<221>  misc_feature
<223>  synthetic

<400>  1
gcugcugcaa auugacgggg gcgugguugg ggcgggucga uugcagc                47

<210>  2
<211>  48
<212>  RNA
<213>  Artificial

<220>
<221>  misc_feature
<223>  synthetic

<400>  2
gcugaaugag gaucgcaggg gcguggcugg ggugggcgac cguucagc               48

<210>  3
<211>  48
<212>  RNA
<213>  Artificial

<220>
<221>  misc_feature
<223>  synthetic

<400>  3
gcugcgcaag gagaggggcg cgguugggga ggcucuaagc gcugcagc               48

<210>  4
<211>  47
<212>  RNA
<213>  Artificial

<220>

<221>  misc_feature
<223>  synthetic

<400>  4
gcuggcgcua uaggacaggg gugcgguugg ggcgguccgc ugucagc                    47

<210>  5
<211>  48
<212>  RNA
<213>  Artificial

<220>
<221>  misc_feature
<223>  synthetic

<400>  5
gcuggauaga acgcaggggu gcgguuuggg gugggcguga uaugcagc                   48

<210>  6
<211>  46
<212>  RNA
<213>  Artificial

<220>
<221>  misc_feature
<223>  synthetic

<400>  6
gcugccguaa agagggguga gguuggggag gcuuuacggu uucagc                     46

<210>  7
<211>  48
<212>  RNA
<213>  Artificial

<220>
<221>  misc_feature
<223>  synthetic

<400>  7
gcugcaugcc gcgaucaggg gagcgguugg ggcgggaucc ggcucagc                   48

<210>  8
<211>  48
<212>  RNA
<213>  Artificial

<220>
<221>  misc_feature
<223>  synthetic

<400>  8
gcugcgaggg agguagcggc ucugcgccgu gacgugggug gaugcagc                   48

<210> 9
<211> 50
<212> RNA
<213> Artificial

<220>
<221> misc_feature
<223> synthetic


<400> 9
ggcugauacg uggguggaua uggggcaguu ccaugugggu gguuucagcc 50

<210> 10
<211> 50
<212> RNA
<213> Artificial

<220>
<221> misc_feature
<223> synthetic


<400> 10
ggcugauacg ugggugaaua uggggcaguu ccaugugggu gguuucagcc 50


<210> 11
<211> 49
<212> RNA
<213> Artificial

<220>
<221> misc_feature
<223> synthetic


<400> 11
ggcugauacg ugggaggaaa gguguaacua ccugugggag guuucagcc 49


<210> 12
<211> 50
<212> RNA
<213> Artificial

<220>
<221> misc_feature
<223> synthetic


<400> 12
ggcuggcacu cgcaggggug aagugaugau uggggugggc gagaccagcc 50


<210> 13
<211> 50
<212> RNA
<213> Artificial

<220>

<221>  misc_feature
<223>  synthetic


<400>  13
ggcugccgag ugguugggug guguaaggga gguggaaucc gcgggcagcc                50


<210>  14
<211>  50
<212>  RNA
<213>  Artificial

<220>
<221>  misc_feature
<223>  synthetic


<400>  14
ggcuguucgu gggaggaagg cucuuggaua gagucguggg ugguucagcc                50


<210>  15
<211>  32
<212>  RNA
<213>  Artificial

<220>
<221>  misc_feature
<223>  synthetic


<400>  15
ggaucgcagg ggcguggcug gggugggcga cc                                   32


<210>  16
<211>  33
<212>  RNA
<213>  Artificial

<220>
<221>  misc_feature
<223>  synthetic


<400>  16
ggaucgcagg ggcguggcug gggugggcga ucc                                  33


<210>  17
<211>  21
<212>  PRT
<213>  Artificial

<220>
<221>  MISC_FEATURE
<223>  synthetic


<400>  17

Glu Pro Ser Glu Val Pro Thr Pro Lys Arg Pro Arg Gly Arg Pro Lys

1               5                   10                  15

Gly Ser Lys Asn Lys
            20

<210>  18
<211>  106
<212>  PRT
<213>  Homo sapiens

<220>
<221>  MISC_FEATURE
<223>  HMGA1a

<400>  18

Ser Glu Ser Ser Ser Lys Ser Ser Gln Pro Leu Ala Ser Lys Gln Glu
1               5                   10                  15

Lys Asp Gly Thr Glu Lys Arg Gly Arg Gly Arg Pro Arg Lys Gln Pro
            20                  25                  30

Pro Val Ser Pro Gly Thr Ala Leu Val Gly Ser Gln Lys Glu Pro Ser
        35                  40                  45

Glu Val Pro Thr Pro Lys Arg Pro Arg Gly Arg Pro Lys Gly Ser Lys
    50                  55                  60

Asn Lys Gly Ala Ala Lys Thr Arg Lys Thr Thr Thr Thr Pro Gly Arg
65                  70                  75                  80

Lys Pro Arg Gly Arg Pro Lys Lys Leu Glu Lys Glu Glu Glu Glu Gly
                85                  90                  95

Ile Ser Gln Glu Ser Ser Glu Glu Glu Gln
            100                 105

<210>  19
<211>  95
<212>  PRT
<213>  Homo sapiens

<220>
<221>  MISC_FEATURE
<223>  HMGA1b

<400>  19

Ser Glu Ser Ser Ser Lys Ser Ser Gln Pro Leu Ala Ser Lys Gln Glu
1               5                   10                  15

Lys Asp Gly Thr Glu Lys Arg Gly Arg Gly Arg Pro Arg Lys Gln Pro
            20                  25                  30

Pro Lys Glu Pro Ser Glu Val Pro Thr Pro Lys Arg Pro Arg Gly Arg
        35                  40                  45

Pro Lys Gly Ser Lys Asn Lys Gly Ala Ala Lys Thr Arg Lys Thr Thr
    50                  55                  60

Thr Thr Pro Gly Arg Lys Pro Arg Gly Arg Pro Lys Lys Leu Glu Lys
65                  70                  75                  80

Glu Glu Glu Glu Gly Ile Ser Gln Glu Ser Ser Glu Glu Glu Gln
                85                  90                  95

<210>  20
<211>  108
<212>  PRT
<213>  Homo sapiens

<220>
<221>  MISC_FEATURE
<223>  HMGA2

<400>  20

Ser Ala Arg Gly Glu Gly Ala Gly Gln Pro Ser Thr Ser Ala Gln Gly
1               5                   10                  15

Gln Pro Ala Ala Pro Ala Pro Gln Lys Arg Gly Arg Gly Arg Pro Arg
            20                  25                  30

Lys Gln Gln Gln Glu Pro Thr Gly Glu Pro Ser Pro Lys Arg Pro Arg
        35                  40                  45

Gly Arg Pro Lys Gly Ser Lys Asn Lys Ser Pro Ser Lys Ala Ala Gln
    50                  55                  60

Lys Lys Ala Glu Ala Thr Gly Glu Lys Arg Pro Arg Gly Arg Pro Arg
65                  70                  75                  80

Lys Trp Pro Gln Gln Val Val Gln Lys Lys Pro Ala Gln Glu Glu Thr
                85                  90                  95

Glu Glu Thr Ser Ser Gln Glu Ser Ala Glu Glu Asp
            100                 105

<210>  21
<211>  34
<212>  DNA

<213>  Artificial

<220>
<221>  misc_feature
<223>  synthetic


<400>  21
tcgaaaaaag caaaaaaaaa aaaaaaaaac tggc                                    34


<210>  22
<211>  50
<212>  RNA
<213>  Artificial

<220>
<221>  misc_feature
<223>  synthetic


<400>  22
ggcugauacg uggguggaua uggggcaguu ccaugugggu gguuucagcc                  50


<210>  23
<211>  50
<212>  RNA
<213>  Artificial

<220>
<221>  misc_feature
<223>  synthetic


<400>  23
ccgacuuugg uggguguacc uugacggggu auaggugggu gcauagucgg                  50


<210>  24
<211>  50
<212>  RNA
<213>  Artificial

<220>
<221>  misc_feature
<223>  synthetic


<400>  24
ggcugauacg uggguggaua uggggcaguu ccaugugggu gguuucagcc                  50


<210>  25
<211>  50
<212>  RNA
<213>  Artificial

<220>
<221>  misc_feature
<223>  synthetic

<400>  25
ccgacuuugg uggguguacc uugacggggu auaggugggu gcauagucgg                50

<210>  26
<211>  50
<212>  RNA
<213>  Artificial

<220>
<221>  misc_feature
<223>  synthetic

<400>  26
ccgacuuugg uggguguacc uugacggggu auaggugggu gcauagucgg                50

<210>  27
<211>  40
<212>  RNA
<213>  Artificial

<220>
<221>  misc_feature
<223>  synthetic

<400>  27
uaaggaaacu cggucugaug cgguagcgcu gugcagagcu                            40

<210>  28
<211>  32
<212>  DNA
<213>  Artificial

<220>
<221>  misc_feature
<223>  synthetic

<400>  28
cccatatcca cccacgtatc agcctttttt tt                                    32

<210>  29
<211>  28
<212>  DNA
<213>  Artificial

<220>
<221>  misc_feature
<223>  synthetic

<400>  29
ttttttttgg ctgaaaccac ccacatgg                                         28

<210>  30
<211>  25
<212>  DNA

<213>  Artificial

<220>
<221>  misc_feature
<223>  synthetic


<400>  30
ttttttttta gctctgcaca gcgct                                          25


<210>  31
<211>  31
<212>  DNA
<213>  Artificial

<220>
<221>  misc_feature
<223>  synthetic


<400>  31
ccgcatcaga ccgagtttcc ttattttttt t                                   31


<210>  32
<211>  19
<212>  DNA
<213>  Artificial

<220>
<221>  misc_feature
<223>  synthetic


<400>  32
tcgacaccat gggtgagtc                                                 19


<210>  33
<211>  21
<212>  DNA
<213>  Artificial

<220>
<221>  misc_feature
<223>  synthetic


<400>  33
gtctagaaag cttcccaact g                                              21


<210>  34
<211>  47
<212>  RNA
<213>  Artificial

<220>
<221>  misc_feature
<223>  synthetic

<400> 34
gcugcugcaa auugacgggg gcgugguugg ggcgggucga uugcagc 47

<210> 35
<211> 48
<212> RNA
<213> Artificial

<220>
<221> misc_feature
<223> synthetic

<400> 35
gcugaaugag gaucgcaggg gcguggcugg ggugggcgac cguucagc 48

<210> 36
<211> 48
<212> RNA
<213> Artificial

<220>
<221> misc_feature
<223> synthetic

<400> 36
gcugcgcaag gagaggggcg cgguugggga ggcucuaagc gcugcagc 48

<210> 37
<211> 47
<212> RNA
<213> Artificial

<220>
<221> misc_feature
<223> synthetic

<400> 37
gcuggcgcua uaggacaggg gugcgguugg ggcgguccgc ugucagc 47

<210> 38
<211> 48
<212> RNA
<213> Artificial

<220>
<221> misc_feature
<223> synthetic

<400> 38
gcuggauaga acgcaggggu gcgguuuggg gugggcguga uaugcagc 48

<210> 39
<211> 46
<212> RNA

<213>  Artificial

<220>
<221>  misc_feature
<223>  synthetic


<400>  39
gcugccguaa agagggguga gguuggggag gcuuuacggu uucagc                    46


<210>  40
<211>  48
<212>  RNA
<213>  Artificial

<220>
<221>  misc_feature
<223>  synthetic


<400>  40
gcugcaugcc gcgaucaggg gagcgguugg ggcgggaucc ggcucagc                   48


<210>  41
<211>  48
<212>  RNA
<213>  Artificial

<220>
<221>  misc_feature
<223>  synthetic


<400>  41
gcugcgaggg agguagcggc ucugcgccgu gacgugggug gaugcagc                   48


<210>  42
<211>  50
<212>  RNA
<213>  Artificial

<220>
<221>  misc_feature
<223>  synthetic


<400>  42
ggcugauacg uggguggaua uggggcaguu ccaugugggu gguuucagcc                50


<210>  43
<211>  50
<212>  RNA
<213>  Artificial

<220>
<221>  misc_feature
<223>  synthetic

<400> 43
ggcugauacg ugggugaaua uggggcaguu ccaugugggu gguuucagcc 50

<210> 44
<211> 49
<212> RNA
<213> Artificial

<220>
<221> misc_feature
<223> synthetic

<400> 44
ggcugauacg ugggaggaaa gguguaacua ccugugggag guuucagcc 49

<210> 45
<211> 50
<212> RNA
<213> Artificial

<220>
<221> misc_feature
<223> synthetic

<400> 45
ggcuggcacu cgcaggggug aagugaugau uggggugggc gagaccagcc 50

<210> 46
<211> 50
<212> RNA
<213> Artificial

<220>
<221> misc_feature
<223> synthetic

<400> 46
ggcugccgag ugguugggug guguaaggga gguggaaucc gcgggcagcc 50

<210> 47
<211> 50
<212> RNA
<213> Artificial

<220>
<221> misc_feature
<223> synthetic

<400> 47
ggcuguucgu gggaggaagg cucuuggaua gagucguggg ugguucagcc 50

<210> 48
<211> 32
<212> RNA

<213>  Artificial

<220>
<221>  misc_feature
<223>  synthetic


<400>  48
ggaucgcagg ggcguggcug gggugggcga cc                                    32


<210>  49
<211>  33
<212>  RNA
<213>  Artificial

<220>
<221>  misc_feature
<223>  synthetic


<400>  49
ggaucgcagg ggcguggcug gggugggcga ucc                                   33


<210>  50
<211>  31
<212>  DNA
<213>  Artificial

<220>
<221>  misc_feature
<223>  synthetic


<400>  50
gccagttttt tttttttttt tttgcttttt t                                     31

## Beschreibung

Die vorliegende Erfindung betrifft in einem Aspekt eine neue Verwendung von Spiegelmeren. In einem anderen Aspekt betrifft die vorliegende Erfindung HMG-Proteine bindende Spiegelmere.

Mit den Fortschritten in der molekularen Medizin ist es möglich geworden, an einer Erkrankung oder einem Krankheitszustand beteiligte Zielmoleküle zu identifizieren und auf diese spezifisch einzuwirken, um dadurch die Erkrankung oder den Krankheitszustand zu behandeln, zu verhindern oder zumindest die damit verbundenen Symptome zu lindern. Die Zielmoleküle können grundsätzlich in zwei Gruppen eingeteilt werden. Die erste Gruppe umfasst Zielmoleküle, die extrazellulär vorhanden sind und somit grundsätzlich durch Gabe eines Wirkstoffes in eine Körperflüssigkeit oder eine Körperkavität, die das Zielmolekül enthält, mit diesem in Kontakt gebracht werden. Die erste Gruppe von Zielmolekülen werden hierin auch als extrazelluläre Zielmoleküle bezeichnet. Die zweite Gruppe von Zielmolekülen umfasst Zielmoleküle, die in Zellen vorhanden sind, wobei die Zellen mit der zu behandelnden Erkrankung oder der Disposition für die Erkrankung im Zusammenhang stehen. Es ist dabei nicht erforderlich, dass das Zielmolekül unmittelbar für den Krankheitszustand verantwortlich ist oder unmittelbar mit der Disposition für die Erkrankung im Zusammenhang steht. Vielmehr ist es ausreichend, dass das jeweilige Zielmolekül an einer Wirkkaskade beteiligt ist, die in ihrem Ablauf durch den Wirkstoff beeinflusst wird, mit der Folge, dass der Wirkstoff für die Behandlung oder Prävention der Erkrankung geeignet ist. Die zweite Gruppe von Zielmolekülen wird hierin auch als intrazelluläre Zielmoleküle bezeichnet.

Die Art des Zielmoleküls, d. h. extrazelluläres oder intrazelluläres Zielmolekül, bestimmt grundsätzlich die Verbindungsklasse, mit der versucht werden kann, die für die therapeutische oder präventive Wirkung erforderliche Wechselwirkung zwischen dem Wirkstoff, typischerweise dem pharmazeutischen Wirkstoff, und dem Zielmolekül bereitzustellen. Nahezu ubiquitär lassen sich sog. small molecules einsetzen, d. h. chemische Verbindungen mit einem Molekulargewicht von typischerweise 1000 oder weniger Dalton. Diese Moleküle können direkt mit extrazellulären, aber auch mit intrazellulären Zielmolekülen in erwünschter Weise wechselwirken.

Vor diesem Hintergrund wurden von der biotechnologischen Industrie neue Wirkstoffklassen entwickelt, wie beispielsweise Antikörper, insbesondere monoklonale Antikörper, Antisense-Moleküle, siRNA-Moleküle, Aptamere und Spiegelmere. Obgleich einige dieser Molekülklassen noch am Beginn von klinischen Untersuchungen stehen, existieren zumindest im Falle von Antikörpern und Antisense-Molekülen bereits in der Klinik eingesetzte Produkte. Gleichwohl bestehen auch mit diesen neuen Substanzklassen erhebliche Probleme, wenn es gilt, intrazelluläre Zielmoleküle zu adressieren. So ist beispielsweise die intrazelluläre Verwendung von Antikörpern derzeit immer noch nicht möglich, zumindest nicht in einem Umfang oder in einer Art und Weise, die eine routinemäßige Anwendung von gegen intrazelluläre Zielmoleküle gerichteten Antikörpern am Patienten zur Therapie und/oder Prophylaxe erlaubt. Auch die anderen neuen Wirkstoffklassen, insbesondere Antisense-Moleküle und siRNA-Moleküle, müssen infolge ihres Wirkmechanismus' in die jeweilige, das Zielmolekül oder das dafür codierende Gen enthaltende Zelle eingebracht werden. Die zielgerichtete Abgabe des Wirkstoffes, auch als Delivery bezeichnet, ist auch für diese Substanzklassen der derzeit limitierende Faktor für eine klinische Anwendung.

Gleiches gilt auch für Aptamere und Spiegelmere, d. h. funktionale Nukleinsäuren mit einer definierten dreidimensionalen Struktur, die die spezifische Wechselwirkung mit den jeweiligen Zielmolekülen ermöglicht. Die Anwendung von Aptameren, um intrazellulär vorliegende Zielmoleküle zu adressieren, bedient sich dabei Methoden der Gentechnologie, genauer der Gentherapie. Die gegen ein intrazelluläres Zielmolekül gerichteten, auch als Intramere bezeichneten Aptamere, werden vermittels gentechnologischer Verfahren in die jeweilige Zielzelle eingeschleust. Ein derartiger Ansatz ist jedoch ebenfalls mit erheblichen Beschränkungen verbunden, nicht zuletzt wegen der fehlenden Akzeptanz von Behandlungsansätzen auf der Grundlage der Gentherapie. Speziell der bei Intrameren beschrittene Weg der intrazellulären Expression einer für das jeweilige Aptamer intrazellulär codierenden Nukleinsäure ist den Spiegelmeren grundsätzlich verschlossen, da kein biologisches System existiert, welches Spiegelmere, d. h. aus L-Nukleotiden bestehende Aptamere, aufzubauen in der Lage wäre.

Aufgabe der vorliegenden Erfindung ist somit, eine Substanzklasse bereitzustellen, die in der Lage ist, spezifisch mit intrazellulären Zielmolekülen, d. h. Zielmolekülen, die in einer Zelle vorhanden sind, zu wechselwirken.

Erfindungsgemäß wird diese Aufgabe gelöst durch den Gegenstand der beigefügten unabhängigen Ansprüche. Bevorzugte Ausführungsformen ergeben sich aus den Unteransprüchen.

Gemäß der vorliegenden Erfindung wird die zugrundeliegende Aufgabe durch den Gegenstand der unabhängigen Ansprüche gelöst. Bevorzugte Ausführungsformen ergeben sich aus den Unteransprüchen.

Erfindungsgemäß wird die Aufgabe gelöst in einem ersten Aspekt durch die Verwendung einer L-Nukleinsäure als intrazelluläres aktives Agens.

In einer ersten Ausführungsform des ersten Aspektes ist die L-Nukleinsäure ein Spiegelmer.

In einer zweiten Ausführungsform des ersten Aspektes, die auch eine Ausführungsform der ersten Ausführungsform ist, tritt die L-Nukleinsäure in Wechselwirkung mit einem intrazellulären Rezeptor.

In einer dritten Ausführungsform des ersten Aspektes, die auch eine Ausführungsform der zweiten Ausführungsform ist, ist der intrazelluläre Rezeptor ausgewählt aus der Gruppe umfassend molekulare Rezeptoren, Enzyme, Chaperon-Moleküle, Signalpeptide, intrazelluläre Strukturen und Stoffwechselintermediate.

In einer vierten Ausführungsform des ersten Aspektes, die auch eine Ausführungsform der zweiten Ausführungsform ist, ist der intrazelluläre Rezeptor ausgewählt aus der Gruppe umfassend Polypeptide, Kohlenhydrate, Nukleinsäuren, Lipide und Kombinationen davon.

In einer fünften Ausführungsform des ersten Aspektes, die auch eine Ausführungsform der zweiten, dritten und vierten Ausführungsform ist, tritt die L-Nukleinsäure mit einem intrazellulären Rezeptor innerhalb einer Zelle in Wechselwirkung.

In einer sechsten Ausführungsform des ersten Aspektes, die auch eine Ausführungsform der zweiten, dritten, vierten und fünften Ausführungsform ist, ist der intrazelluläre Rezeptor ausgewählt aus der Gruppe umfassend Transkriptionsfaktoren und einen AT-Haken bindende DNA-bindende Proteine.

In einer siebten Ausführungsform des ersten Aspektes, die auch eine Ausführungsform der sechsten Ausführungsform ist, ist der intrazelluläre Rezeptor ausgewählt aus der Gruppe umfassend die HMG-Proteine, bevorzugterweise aus der Gruppe umfassend HMGA1, HMGA1a, HMGA1b, und HMGA2.

Erfindungsgemäß wird die Aufgabe gelöst in einem zweiten Aspekt durch ein Verfahren zum Binden eines intrazellulären Rezeptors umfassend:
- Bereitstellen einer Zelle enthaltend mindestens den einen intrazellulären Rezeptor,
- Bereitstellen einer L-Nukleinsäure, und
- Inkubieren der Zelle mit der L-Nukleinsäure.

In einer ersten Ausführungsform des zweiten Aspekts erfolgt das Inkubieren unter Bedingungen, so dass die L-Nukleinsäure an den intrazellulären Rezeptor in der Zelle bindet.

In einer zweiten Ausführungsform des zweiten Aspekts, die auch eine Ausführungsform der ersten Ausführungsform ist, ist die L-Nukleinsäure ein Spiegelmer.

In einer dritten Ausführungsform des zweiten Aspekts, die auch eine Ausführungsform der ersten und zweiten Ausführungsform ist, wird nach der Inkubation der Zelle mit der L-Nukleinsäure bestimmt, ob eine Bindung, insbesondere eine intrazelluläre Bindung, der L-Nukleinsäure an dem intrazellulären Rezeptor erfolgt ist.

In einer vierten Ausführungsform des zweiten Aspekts, die auch eine Ausftihrungsform der ersten, zweiten und dritten Ausführungsform ist, ist der intrazelluläre Rezeptor ausgewählt aus der Gruppe umfassend molekulare Rezeptoren, Stoffwechselintermediate und Enzyme.

In einer fünften Ausführungsform des zweiten Aspekts, die auch eine Ausführungsform der ersten, zweiten, dritten und vierten Ausführungsform ist, ist der intrazelluläre Rezeptor ausgewählt aus der Gruppe umfassend Polypeptide, Kohlenhydrate, Nukleinsäure, Lipide und Kombinationen davon.

In einer sechsten Ausführungsform des zweiten Aspekts, die auch eine Ausführungsform der ersten, zweiten, dritten, vierten und fünften Ausführungsform ist, ist der intrazelluläre Rezeptor ausgewählt aus der Gruppe umfassend Transkriptionsfaktoren und einen AT-Haken bindende DNA-bindende Proteine.

In einer siebten Ausführungsform des zweiten Aspekts, die auch eine Ausführungsform der sechsten Ausführungsform ist, ist der intrazelluläre Rezeptor ausgewählt aus der Gruppe umfassend HMG-Proteine, bevorzugterweise ausgewählt aus der Gruppe umfassend HMGA1, HMGA1a, HMGA1b und HMGA2.

Erfindungsgemäß wird die Aufgabe gelöst in einem dritten Aspekt durch die Verwendung einer L-Nukleinsäure zur Herstellung eines Medikamentes zur Behandlung und/oder Prävention einer Erkrankung, wobei das Zielmolekül des Medikamentes ein intrazelluläres Zielmolekül ist.

In einer ersten Ausführungsform des dritten Aspekts ist der intrazelluläre Rezeptor ausgewählt aus der Gruppe umfassend molekulare Rezeptoren, Enzyme, Chaperon-Moleküle, Signalpeptide, intrazelluläre Strukturen und Stoffwechselintermediate.

In einer zweiten Ausführungsform des dritten Aspekts, die auch eine Ausführungsform der ersten Ausführungsform ist, ist der intrazelluläre Rezeptor ausgewählt aus der Gruppe umfassend Polypeptide, Kohlenhydrate, Nukleinsäuren, Lipide und Kombinationen davon.

In einer dritten Ausführungsform des dritten Aspekts, die auch eine Ausführungsform der ersten und zweiten Ausführungsform ist, ist das Zielmolekül ausgewählt aus der Gruppe, die Transkriptionsfaktoren und einen AT-Haken bindende DNA-bindende Proteine umfasst.

In einer vierten Ausführungsform des dritten Aspekts, die auch eine Ausführungsform der dritten Ausführungsform ist, ist das Zielmolekül ausgewählt aus der Gruppe umfassend HMG-Proteine, bevorzugterweise ausgewählt aus der Gruppe umfassend HMGA1, HMGA1a, HMGA1b und HMGA2.

In einer fünften Ausführungsform des dritten Aspekts, die auch eine Ausführungsform der dritten und vierten Ausführungsform ist, ist die Erkrankung ausgewählt aus der Gruppe umfassend Tumorerkrankungen, Virusinfektionen und Arteriosklerose.

In einer sechsten Ausführungsform des dritten Aspekts, die auch eine Ausführungsform der fünften Ausführungsform ist, ist die Tumorerkrankung ausgewählt aus der Gruppe umfassend mesenchymale Tumoren, epitheliale Tumoren, benigne Tumoren, maligne Tumoren und metastasierende Tumoren.

In einer siebten Ausführungsform des dritten Aspekts, die auch eine Ausführungsform der dritten, vierten, fünften und sechsten Ausführungsform ist, sind das Zielmolekül HMGA und die Erkrankung ausgewählt aus der Gruppe umfassend Karzinome von Prostata, Pankreas, Schilddrüse, Cervix, Magen, Brust, Colon/Rektum, Ovarien; Neuroblastome; Lymphome, Uterusleiomyome; Lipome; Endometriumpolypen; chondroide Hamartome der Lunge; pleomorphe Adenome der Kopfspeicheldrüsen; Hämangiopericytome; chondromatöse Tumore; aggressive Angiomyxome; diffuse Astrocytome; Osteoklastome; Hautkrebs; Burkitts Lymphom; Lewis-Lungenkrebs; Leukämie; nicht-kleinzelliges Lungenkarzinom; sowie jeweils Metastasen und/oder metastasierende Formen davon.

In einer achten Ausführungsform des dritten Aspekts, die auch eine Ausführungsform der fünften Ausführungsform ist, ist die Arteriosklerose durch HMGA1, HMGA1a, HMG1b und/oder HMGA2 vermittelte Bildung von artierosklerotischen Plaques ausgelöst oder bedingt.

In einer neunten Ausführungsform des dritten Aspekts, die auch eine Ausführungsform der ersten, zweiten, dritten, vierten, fünften, sechsten, siebten und achten Ausführungsform ist, liegt das intrazelluläre Zielmolekül intrazellulär vor.

Erfindungsgemäß wird die Aufgabe gelöst in einem vierten Aspekt durch die Verwendung einer L-Nukleinsäure zur Herstellung eines diagnostischen Mittels zur Diagnose, wobei das Zielmolekül des diagnostischen Mittels ein intrazelluläres Zielmolekül ist.

In einer ersten Ausführungsform des vierten Aspekts ist der intrazelluläre Rezeptor ausgewählt aus der Gruppe umfassend molekulare Rezeptoren, Enzyme, Chaperon-Moleküle, Signalpeptide, intrazelluläre Strukturen und Stoffwechselintermediate.

In einer zweiten Ausführungsform des vierten Aspekts, die auch eine Ausführungsform der ersten Ausführungsform ist, ist der intrazelluläre Rezeptor ausgewählt aus der Gruppe umfassend Polypeptide, Kohlenhydrate, Nukleinsäuren, Lipide und Kombinationen davon.

In einer dritten Ausführungsform des vierten Aspekts, die auch eine Ausführungsform der ersten und zweiten Ausführungsform ist, ist das Zielmolekül ausgewählt aus der Gruppe, die Transkriptionsfaktoren und einen AT-Haken bindende DNA-bindende Proteine umfasst.

In einer vierten Ausführungsform des vierten Aspekts, die auch eine Ausführungsform der dritten Ausführungsform ist, ist das Zielmolekül ausgewählt aus der Gruppe umfassend HMG-Proteine, bevorzugterweise ausgewählt aus der Gruppe umfassend HMGA, HMGA1a, HMGA1b und HMGA2.

In einer fünften Ausführungsform des vierten Aspekts, die auch eine Ausführungsform der dritten und vierten Ausführungsform ist, ist die Erkrankung ausgewählt aus der Gruppe umfassend Tumorerkrankungen, Virusinfektionen und Arteriosklerose.

In einer sechsten Ausführungsform des vierten Aspekts, die auch eine Ausführungsform der fünften Ausführungsform ist, ist die Tumorerkrankung ausgewählt aus der Gruppe umfassend mesenchymale Tumoren, epitheliale Tumoren, benigne Tumoren, maligne Tumoren und metastatisierende Tumoren.

In einer siebten Ausführungsform des vierten Aspekts, die auch eine Ausführungsform der dritten, vierten, fünften und sechsten Ausführungsform ist, ist das Zielmolekül HMGA und die Erkrankung ist ausgewählt aus der Gruppe umfassend Karzinome von Prostata, Pankreas, Schilddrüse, Cervix, Magen, Brust, Colon/Rektum, Ovarien; Neuroblastome; Lymphome, Uterusleiomyome; Lipome; Endometriumpolypen; chondroide Hamartome der Lunge; pleomorphe Adenome der Kopfspeicheldrüsen; Hämangiopericytome; chondromatöse Tumore; aggressive Angiomyxome; diffuse Astrocytome; Osteoklastome; Hautkrebs; Burkitts Lymphom; Lewis-Lungenkrebs; Leukämie; nicht-kleinzelliges Lungenkarzinom; sowie jeweils Metastasen und/oder metastasierende Formen davon.

In einer achten Ausführungsform des vierten Aspekts, die auch eine Ausführungsform der fünften Ausführungsform ist, wird die Arteriosklerose durch HMGA1, HMGA1a, HMG1b und/oder HMGA2 vermittelte Bildung von artierosklerotischen Plaques ausgelöst.

In einer neunten Ausführungsform des vierten Aspekts, die auch eine Ausführungsform der ersten, zweiten, dritten, vierten, fünften, sechsten und siebten Ausführungsform ist, liegt das intrazelluläre Zielmolekül intrazellulär vor.

Erfindungsgemäß wird die Aufgabe gelöst in einem fünften Aspekt durch eine Zusammensetzung umfassend eine an ein intrazelluläres Zielmolekül bindende L-Nukleinsäure und ein Abgabevehikel.

In einer ersten Ausführungsform des fünften Aspekts ist das Abgabevehikel ein für die intrazelluläre Abgabe der L-Nukleinsäure geeignetes Abgabevehikel.

In einer zweiten Ausführungsform des fünften Aspekts, die auch eine Ausführungsform der ersten Ausführungsform ist, ist das Abgabevehikel ausgewählt aus der Gruppe umfassend Vehikel, Konjugate und physikalische Maßnahmen.

In einer dritten Ausführungsform des fünften Aspekts, die auch eine Ausführungsform der zweiten Ausführungsform ist, ist das Abgabevehikel ein Vehikel, wobei das Vehikel ausgewählt ist aus der Gruppe umfassend Liposome, Nanopartikel, Mikropartikel, Cyclodextrine oder Dendrimere, oder ein Vesikel, das aus Polypeptiden, Polyethylenimin und/oder amphipathischen Molekülen besteht.

In einer vierten Ausführungsform des fünften Aspekts, die auch eine Ausführungsform der zweiten Ausführungsform ist, ist das Abgabevehikel ein Konjugat, wobei das Konjugat ein Konjugat für die Rezeptor-vermittelte Endozytose, ein Konjugat mit einem fusogenen Peptid, ein Konjugat mit einem Signalpeptid, ein Konjugat mit einer Nukleinsäure, bevorzugterweise ein Konjugat mit einem Spiegelmer oder ein lipophiles Konjugat ist.

In einer fünften Ausführungsform des fünften Aspekts, die auch eine Ausführungsform der zweiten Ausführungsform ist, ist das Abgabevehikel eine physikalische Maßnahme, wobei die Maßnahme bevorzugterweise ausgewählt ist aus der Gruppe umfassend Elektroporation, Iontophorese, Druck, Ultraschall und Schockwellen.

In einer sechsten Ausführungsform des fünften Aspekts, die auch eine Ausführungsform der dritten Ausführungsform ist, umfasst das Abgabevehikel Polyethylenimin.

In einer siebten Ausführungsform des fünften Aspekts, die auch eine Ausführungsform der sechsten Ausführungsform ist, ist das Polyethylenimin ein verzweigtes Polyethylenimin mit einem Molekulargewicht von etwa 25 kDa.
In einer achten Ausführungsform des fünften Aspekts, die auch eine Ausführungsform der sechsten und siebten Ausführungsform ist, bildet das Polyethylenimin eine Mizelle oder eine mizellenartige Struktur aus.

In einer neunten Ausführungsform des fünften Aspekts, die auch eine Ausführungsform der ersten, zweiten, dritten, vierten, fünften, sechsten, siebten und achten Ausführungsform ist, ist die L-Nukleinsäure ein Spiegelmer.

In einer zehnten Ausführungsform des fünften Aspekts, die auch eine Ausführungsform der neunten Ausführungsform ist, trägt das Spiegelmer eine Modifikation, wobei die Modifikation ausgewählt ist aus der Gruppe umfassend PEG-Reste.

In einer elften Ausführungsform des fünften Aspekts, die auch eine Ausführungsform der zehnten Ausführungsform ist, weist der PEG-Rest ein Molekulargewicht von etwa 1.000 bis 10.000 Da auf, bevorzugterweise ein Molekulargewicht von etwa 1.500 bis 2.500 Da und am bevorzugtesten ein Molekulargewicht von etwa 2.000 Da.

In einer zwölften Ausführungsform des fünften Aspekts, die auch eine Ausführungsform der zehnten und elften Ausführungsform ist, ist die Modifikation am 5'-Terminus oder am 3'-Terminus der L-Nukleinsäure gebunden.

In einer dreizehnten Ausführungsform des fünften Aspekts, die auch eine Ausführungsform der neunten, zehnten, elften und zwölften Ausführungsform ist, beträgt in der Zusammensetzung das Verhältnis aus Gesamtanzahl der Stickstoffgruppen des Polyethylenimins und Gesamtanzahl der Phosphatgruppen der in der Zusammensetzung enthaltenen Nukleinsäure etwa 1 bis 20, bevorzugterweise etwa 1,5 bis 10, bevorzugtererweise etwa 2 bis 5 und am bevorzugtesten etwa 2 bis 3.

In einer vierzehnten Ausführungsform des fünften Aspekts, die auch eine Ausführungsform der ersten, zweiten, dritten, vierten, fünften, sechsten, siebten achten, neunten, zehnten, elften, zwölften und dreizehnten Ausführungsform ist, stellt die Zusammensetzung die L-Nukleinsäure intrazellulär bereit.

Erfindungsgemäß wird die Aufgabe gelöst in einem sechsten Aspekt durch die pharmazeutische Zusammensetzung umfassend eine Zusammensetzung nach dem fünften Aspekt, und einen pharmazeutisch akzeptablen Träger.

In einer Ausführungsform der Verwendung nach dem ersten Aspekt ist die L-Nukleinsäure eine Zusammensetzung nach dem fünften Aspekt.

In einer Ausführungsform des Verfahrens nach dem zweiten Aspekt ist die L-Nukleinsäure eine Zusammensetzung nach dem fünften Aspekt.

In einer Ausführungsform der Verwendung nach dem dritten Aspekt ist die L-Nukleinsäure eine Zusammensetzung nach dem fünften Aspekt.

In einer Ausführungsform der Verwendung nach dem vierten Aspekt ist die L-Nukleinsäure eine Zusammensetzung nach dem fünften Aspekt.

Erfindungsgemäß wird die Aufgabe gelöst in einem siebten Aspekt durch eine HMGA bindende Nukleinsäure, **dadurch gekennzeichnet, dass** die Nukleinsäure einen Abschnitt Box A1 und einen Abschnitt Box A2 umfasst, wobei der Abschnitt Box A1 und der Abschnitt Box A2 durch einen Zwischenabschnitt miteinander verbunden sind und wobei Box A1 und Box A2 einzeln und unabhängig voneinander aus der Gruppe ausgewählt sind, die GGGCG, GGGUG und GGGAG umfasst.

In einer ersten Ausführungsform des siebten Aspekts besteht der Zwischenabschnitt entweder aus einem Zwischenabschnitt Z1 umfassend sechs oder sieben Nukleotide oder aus einem Zwischenabschnitt Z2 umfassend 12 bis 25 Nukleotide.

In einer zweiten Ausführungsform des siebten Aspekts, die auch eine Ausführungsform der ersten Ausführungsform ist, weist die Nukleinsäure am 5'-Ende des Abschnittes Box A1 einen ersten Abschnitt und am 3'-Ende des Abschnittes Box A2 einen zweiten Abschnitt auf, wobei bevorzugterweise beide Abschnitte unabhängig voneinander je vier bis acht Nukleotide umfassen.

In einer dritten Ausführungsform des siebten Aspekts, die auch eine Ausführungsform der zweiten Ausführungsform ist, sind die beiden Abschnitte wenigstens teilweise oder vollständig miteinander hybridisiert, wobei sich die Hybridisierung über vier bis acht Nukleotidpaare erstreckt.

In einer vierten Ausführungsform des siebten Aspekts, die auch eine Ausführungsform der zweiten und dritten Ausführungsform ist, weist die Nukleinsäure am 5'-Ende des Abschnittes Box A1 einen Abschnitt Helix A1 und am 3'-Ende des Abschnittes Box A2 einen Abschnitt Helix A2 auf, wobei bevorzugterweise der Abschnitt Helix A1 vier bis acht Nukleotide umfasst und bevorzugterweise der Abschnitt Helix A2 vier bis acht Nukleotide umfasst, und wobei bevorzugterweise der Abschnitt Helix A1 den ersten Abschnitt am 5'-Ende des Abschnittes Box A1 oder einen Teil davon darstellt, und wobei bevorzugterweise der Abschnitt Helix A2 den zweiten Abschnitt am 3'-Ende des Abschnittes Box A2 oder einen Teil davon darstellt, wobei die Länge des Abschnittes Helix A1 unabhängig von der Länge des Abschnittes Helix A2 ist.

In einer fünften Ausführungsform des siebten Aspekts, die auch eine Ausführungsform der vierten Ausführungsform ist, sind die Abschnitte Helix A1 und Helix A2 wenigstens teilweise oder vollständig miteinander hybridisiert, wobei sich die Hybridisierung über vier bis acht Nukleotidpaare erstreckt.

In einer sechsten Ausführungsform des siebten Aspekts, die auch eine Ausführungsform der vierten und fünften Ausführungsform ist, ist zwischen dem 3'-Ende des Abschnittes Helix A1 und dem 5'-Ende des Abschnittes Box A1 ein Abschnitt Helix B1 und zwischen dem 3'-Ende des Abschnittes Box A2 und dem 5'-Ende des Abschnittes Helix A2 ein Abschnitt Helix B2 angeordnet, wobei bevorzugterweise die Länge des Abschnittes Helix B1 und Helix B2 jeweils einzeln und unabhängig eine Länge von vier bis acht Nukleotiden umfasst.

In einer siebten Ausführungsform des siebten Aspekts, die auch eine Ausführungsform der sechsten Ausführungsform ist, sind die Abschnitte Helix B1 und Helix B2 wenigstens teilweise oder vollständig miteinander hybridisiert, wobei sich die Hybridisierung über vier bis acht Nukleotidpaare erstreckt.

In einer achten Ausführungsform des siebten Aspekts, die auch eine Ausführungsform der sechsten und siebten Ausführungsform ist, sind zwischen dem 3'-Ende des Abschnittes Helix A1 und dem 5'-Ende des Abschnittes Helix B1 null bis fünf Nukleotide angeordnet.

In einer neunten Ausführungsform des siebten Aspekts, die auch eine Ausführungsform der achten Ausführungsform ist, sind zwischen dem 3'-Ende des Abschnittes Helix A1 und dem 5'-Ende des Abschnittes Helix B1 zwei Nukleotide angeordnet.

In einer zehnten Ausführungsform des siebten Aspekts, die auch eine Ausführungsform der sechsten, siebten, achten und neunten Ausführungsform ist, sind zwischen dem 3'-Ende des Abschnittes Helix B2 und dem 5'-Ende des Abschnittes Helix A2 null bis sechs Nukleotide angeordnet.

In einer elften Ausführungsform des siebten Aspekts, die auch eine Ausführungsform der zehnten Ausführungsform ist, bevorzugterweise insoweit diese eine Ausführungsform der neunten Ausführungsform ist, ist zwischen dem 3'-Ende des Abschnittes Helix B2 und dem 5'-Ende des Abschnittes Helix A2 ein Nukleotid angeordnet.

In einer zwölften Ausführungsform des siebten Aspekts, die auch eine Ausführungsform der sechsten, siebten, achten, neunten, zehnten und elften Ausführungsform ist, beträgt die Summe der Nukleotide von Abschnitt Helix A1 und von Abschnitt Helix B1 10 bis 12 Nukleotide und die Summe der Nukleotide von Abschnitt Helix A2 und von Abschnitt Helix B2 10 bis 12 Nukleotide.

In einer dreizehnten Ausführungsform des siebten Aspekts, die auch eine Ausführungsform der zwölften Ausführungsform ist, beträgt die Summe der hybridisierten Nukleotide aus der Hybridisierung von Abschnitt Helix A1 mit Abschnitt Helix A2 und von Abschnitt Helix B1 mit Abschnitt Helix B2 10 bis 12 Nukleotidpaare.

In einer vierzehnten Ausführungsform des siebten Aspekts, die auch eine Ausführungsform der sechsten, siebten, achten, neunten, zehnten, elften, zwölften und dreizehnten Ausführungsform ist, bevorzugterweise der sechsten oder siebten, umfasst die Nukleinsäure keinen Abschnitt Helix A1 und Helix A2, wodurch am 5'-Ende der Nukleinsäure der Abschnitt Helix B1 und am 3'-Ende die Helix B2 angeordnet ist, wobei bevorzugterweise die Länge des Abschnittes Helix B1 und Helix B2 jeweils einzeln und unabhängig eine Länge von vier bis acht Nukleotiden umfasst.

In einer fünfzehnten Ausführungsform des siebten Aspekts, die auch eine Ausführungsform der vierzehnten Ausführungsform ist, sind die Abschnitte Helix B1 und Helix B2 wenigstens teilweise oder vollständig miteinander hybridisiert, wobei sich die Hybridisierung über vier bis acht Nukleotidpaare erstreckt.

In einer sechzehnten Ausführungsform des siebten Aspekts, die auch eine Ausführungsform der vierten und fünften Ausführungsform ist, sind zwischen dem 3'-Ende des Abschnittes Helix A1 und dem 5'-Ende des Abschnittes Box A1 ein bis fünf Nukleotide und zwischen dem 3'-Ende des Abschnittes Box A2 und dem 5'-Ende des Abschnittes Helix A2 ein bis drei Nukleotide angeordnet.

In einer siebzehnten Ausführungsform des siebten Aspekts, die auch eine Ausführungsform der sechsten, siebten, achten, neunten, zehnten, elften, zwölften, dreizehnten, vierzehnten und fünfzehnten Ausführungsform ist, sind zwischen dem 3'-Ende des Abschnittes Helix B1 und dem 5'-Ende des Abschnittes Box A1 zwei Nukleotide und zwischen dem 3'-Ende des Abschnittes Box A2 und dem 5'-Ende des Abschnittes Helix B2 eins bis sieben Nukleotide angeordnet.

In einer achtzehnten Ausführungsform des siebten Aspekts, die auch eine Ausführungsform der ersten, zweiten, dritten, vierten, fünften, sechsten, siebten, achten und zehnten Ausführungsform ist, soweit diese eine Ausführungsform der sechsten, siebten und achten Ausführungsform ist, der zwölften und dreizehnten Ausführungsform, soweit diese Ausführungsformen der sechsten, siebten, achten und zehnten Ausführungsform sind, der vierzehnten und fünfzehnten Ausführungsform, soweit diese Ausführungsformen der sechsten, siebten, achten, zehnten, zwölften und dreizehnten Ausführungsform sind, oder der siebzehnten Ausführungsform, soweit diese Ausführungsformen der sechsten, achten, zehnten, zwölften, dreizehnten und fünfzehnten Ausführungsform sind, jeweils in dem hierin eingeschränkten Umfang, umfasst der Zwischenabschnitt Z1 sechs oder sieben Nukleotide.

In einer neunzehnten Ausführungsform des siebten Aspekts, die auch eine Ausführungsform der achtzehnten Ausführungsform ist, weist der Zwischenabschnitt Z1 die Sequenz N₁N₂GN₈N₃N₄N₅ auf, wobei
N₁ = U, C, A oder G ist;
N₂ = G oder U ist;
N₃ = U oder C ist;
N₄ = U oder A ist;
N₅ = G oder A ist; und
N₈ = U ist oder fehlt.

In einer zwanzigsten Ausführungsform des siebten Aspekts, die auch eine Ausführungsform der neunzehnten Ausführungsform ist, umfasst die Nukleinsäure einen Abschnitt Box A1 und einen Abschnitt Box A2, wobei das 3'-Ende des Abschnittes Box A1 direkt mit dem 5'-Ende des Zwischenabschnittes Z1 verbunden ist, und das 3'-Ende des Zwischenabschnittes Z1 direkt mit dem 5'-Ende des Abschnittes Box A2 verbunden ist.

In einer einundzwanzigsten Ausführungsform des siebten Aspekts, die auch eine Ausführungsform der achtzehnten, neunzehnten und zwanzigsten Ausführungsform ist, insbesondere der zwanzigsten, umfasst die Nukleinsäure einen Abschnitt Helix B1 und einen Abschnitt Helix B2.

In einer zweiundzwanzigsten Ausführungsform des siebten Aspekts, die auch eine Ausführungsform der einundzwanzigsten Ausführungsform ist, umfassen die Abschnitte Helix B1 und Helix B2 jeweils einzeln und unabhängig voneinander vier bis acht Nukleotide, die bevorzugterweise vollständig oder teilweise miteinander hybridisiert sind.

In einer dreiundzwanzigsten Ausführungsform des siebten Aspekts, die auch eine Ausführungsform der einundzwanzigsten und zweiundzwanzigsten Ausführungsform ist, sind zwischen dem 3'-Ende des Abschnittes Helix B1 und dem 5'-Ende des Abschnittes Box A1 in 5'-3'-Richtung zwei Nukleotide N₆, N₇ angeordnet, wobei N₆ G, A oder U ist, und N₇ = G oder U ist.

In einer vierundzwanzigsten Ausführungsform des siebten Aspekts, die auch eine Ausführungsform der einundzwanzigsten, zweiundzwanzigsten und dreiundzwanzigsten Ausführungsform ist, ist zwischen dem 3'-Ende des Abschnittes Box A2 und dem 5'-Ende des Abschnittes Helix B2 kein Nukleotid, oder in 5'-3'-Richtung die Nukleotidsequenz GN_{y} angeordnet, wobei N_{y} null bis sechs Nukleotide umfasst, bevorzugterweise 0 oder 6 Nukleotide.

In einer fünfundzwanzigsten Ausführungsform des siebten Aspekts, die auch eine Ausführungsform der achtzehnten, neunzehnten, zwanzigsten, einundzwanzigsten, zweiundzwanzigsten, dreiundzwanzigsten und vierundzwanzigsten Ausführungsform ist, umfasst die Nukleinsäure einen Abschnitt Helix A1 und Helix A2.

In einer sechsundzwanzigsten Ausführungsform des siebten Aspekts, die auch eine Ausführungsform der funfundzwanzigsten Ausführungsform ist, umfassen die Abschnitte Helix A1 und Helix A2 jeweils und unabhängig voneinander vier bis acht Nukleotide, wobei bevorzugterweise die Abschnitte Helix A1 und Helix A2 vollständig oder teilweise miteinander hybridisiert sind.

In einer siebenundzwanzigsten Ausführungsform des siebten Aspekts, die auch eine Ausführungsform der fünfundzwanzigsten und sechsundzwanzigsten Ausführungsform ist, umfasst zwischen dem 3'-Ende des Abschnittes Helix A1 und dem 5'-Ende des Abschnittes Helix B1 eine Nukleotidsequenz Nₓ angeordnet ist, wobei Nₓ null bis fünf Nukleotide.

In einer achtundzwanzigsten Ausführungsform des siebten Aspekts, die auch eine Ausführungsform der fünfundzwanzigsten, sechsundzwanzigsten und siebenundzwanzigsten Ausführungsform ist, umfasst zwischen dem 3'-Ende des Abschnittes Helix B2 und dem 5'-Ende des Abschnittes Helix A2 eine Nukleotidsequenz N_{z} angeordnet ist, wobei N_{z} null bis sechs Nukleotide.

In einer neunundzwanzigsten Ausführungsform des siebten Aspekts, die auch eine Ausführungsform der einundzwanzigsten, zweiundzwanzigsten, dreiundzwanzigsten, vierundzwanzigsten, fünfundzwanzigsten, sechsundzwanzigsten, siebenundzwanzigsten und achtundzwanzigsten Ausführungsform ist, beträgt die Summe der hybridisierten Nukleotide aus der Hybridisierung von Abschnitt Helix A1 mit Abschnitt Helix A2 und von Abschnitt Helix B1 mit Abschnitt Helix B2 10 bis 12 Nukleotidpaare.

In einer dreißigsten Ausführungsform des siebten Aspekts, die auch eine Ausführungsform der vierundzwanzigsten, fünfundzwanzigsten, sechsundzwanzigsten, siebenundzwanzigsten, achtundzwanzigsten und neunundzwanzigsten Ausführungsform ist, ist zwischen dem 3'-Ende des Abschnittes Box A2 und dem 5'-Ende des Abschnittes Helix B2 in 5'-3'-Richtung die Nukleotidsequenz GN_{y} angeordnet, wobei N_{y} null bis sechs Nukleotide umfasst, bevorzugterweise 0 oder 6 Nukleotide.

In einer einunddreißigsten Ausführungsform des siebten Aspekts, die auch eine Ausführungsform der dreißigsten Ausführungsform ist, umfasst die HMGA bindende Nukleinsäure die folgende Struktur Helix A1-Nₓ-**Helix B1**-N₆N₇[Box A1]N₁N₂GN₈N₃N₄N₅[BOX A2]G-N_{Y}-**Helix B2**-N_{z}-Helix A2 wobei
N₁ = U, C, A oder G ist;
N₂ = G oder U ist;
N₃ = U oder C ist;
N₄ = U oder A ist;
N₅ = G oder A ist;
N₆ = G, A oder U ist;
N₇ = G oder U ist;
N₈ = U oder kein Nukleotid ist;
Nₓ = null bis fünf Nukleotide ist;
N_{y} = null oder sechs Nukleotide ist; und
N_{z} = null bis sechs Nukleotide ist;
der Abschnitt Box A1 und Abschnitt Box A2 jeweils einzeln und unabhängig voneinander ausgewählt sind aus der Gruppe von Nukleotidsequenzen umfassend GGGCG, GGGUG und GGGAG;
der Abschnitt Helix A1 und der Abschnitt Helix A2 jeweils einzeln und unabhängig voneinander vier bis acht Nukleotide umfassen, wobei bevorzugterweise die Abschnitte Helix A1 und Helix A2 vollständig oder teilweise miteinander hybridisiert sind, und
die Abschnitte Helix B1 und Helix B2 jeweils einzeln und unabhängig voneinander vier bis acht Nukleotide umfassen, wobei bevorzugterweise die Abschnitte Helix B1 und Helix B2 vollständig oder teilweise miteinander hybridisiert sind und der hybridisierende Bereich vier bis acht Nukleotide umfasst, und wobei die Summe der hybridisierten Nukleotide aus der Hybridisierung von Abschnitt Helix A1 mit Abschnitt Helix A2 und von Abschnitt Helix B1 mit Abschnitt Helix B2 10 bis 12 Nukleotidpaare beträgt.

In einer zweiunddreißigsten Ausführungsform des siebten Aspekts, die auch eine Ausführungsform der dreißigsten und einunddreißigsten Ausführungsform ist, umfasst die HMGA bindende Nukleinsäure eine Sequenz ausgewählt aus der Gruppe umfassend SEQ. ID. No. 1, SEQ. ID. No. 2, SEQ. ID. No. 3, SEQ. ID. No. 5, SEQ. ID. No. 6, SEQ. ID. No. 7 und SEQ. ID. No. 13.

In einer dreiunddreißigsten Ausführungsform des siebten Aspekts, die auch eine Ausführungsform der vierundzwanzigsten, fünfundzwanzigsten, sechsundzwanzigsten, siebenundzwanzigsten, achtundzwanzigsten und neunundzwanzigsten Ausführungsform ist, schließt sich das 3'-Ende des Abschnittes Box A2 unmittelbar an das 5'-Ende des Abschnittes Helix B2 an.

In einer vierunddreißigsten Ausführungsform des siebten Aspekts, die auch eine Ausführungsform der dreiunddreißigsten Ausführungsform ist, umfasst die HMGA bindende Nukleinsäure die folgende Struktur Helix A1-Nₓ-Helix B1-N₆N₇Box A1 N₁N₂GN₈N₃N₄N₅BOX A2 Helix B2-N_{z}-Helix A2 wobei
N₁ = U, C, A oder G ist;
N₂ = G oder U ist;
N₃ = U oder C ist;
N₄ = U oder A ist;
N₅ = G oder A ist;
N₆ = G, A oder U ist;
N₇ = G oder U ist;
N₈ = U oder kein Nukleotid ist;
Nₓ = null bis fünf Nukleotide ist; und
N_{z} = null bis sechs Nukleotide ist;
der Abschnitt Box A1 und Abschnitt Box A2 jeweils einzeln und unabhängig voneinander ausgewählt sind aus der Gruppe von Nukleotidsequenzen umfassend GGGCG, GGGUG und GGGAG;
der Abschnitt Helix A1 und der Abschnitt Helix A2 jeweils einzeln und unabhängig voneinander vier bis acht Nukleotide umfassen, wobei bevorzugterweise die Abschnitte Helix A1 und Helix A2 vollständig oder teilweise miteinander hybridisiert sind, und
die Abschnitte Helix B1 und Helix B2 jeweils einzeln und unabhängig voneinander vier bis acht Nukleotide umfassen, wobei bevorzugterweise die Abschnitte Helix B1 und Helix B2 vollständig oder teilweise miteinander hybridisiert sind und der hybridisierende Bereich vier bis acht Nukleotide umfasst, und wobei die Summe der hybridisierten Nukleotide aus der Hybridisierung von Abschnitt Helix A1 mit Abschnitt Helix A2 und von Abschnitt Helix B1 mit Abschnitt Helix B2 10 bis 12 Nukleotidpaare beträgt.

In einer fünfunddreißigsten Ausführungsform des siebten Aspekts, die auch eine Ausführungsform der dreiunddreißigsten und vierunddreißigsten Ausführungsform ist, umfasst die HMGA bindende Nukleinsäure eine Sequenz umfassend SEQ. ID. No. 3.

In einer sechsunddreißigsten Ausführungsform des siebten Aspekts, die auch eine Ausführungsform der einunddreißigsten Ausführungsform ist, umfasst die HMGA bindende Nukleinsäure die folgende Struktur

Helix B1-N6N7Box A1N1N2GN8N3N4N5BOX A2G-NY-Helix B2

In einer siebenunddreißigsten Ausführungsform des siebten Aspekts, die auch eine Ausführungsform der vierunddreißigsten Ausführungsform ist, umfasst die HMGA bindende Nukleinsäure die folgende Struktur

Helix B1-N6N7Box A1N1N2GN8N3N4N5BOX A2Helix B2

In einer achtunddreißigsten Ausführungsform des siebten Aspekts, die auch eine Ausführungsform der sechsunddreißigsten Ausführungsform ist, umfasst die HMGA bindende Nukleinsäure eine Sequenz, die ausgewählt ist aus der Gruppe umfassend SEQ. ID. No. 15 und SEQ. ID. No. 16.

In einer neununddreißigsten Ausführungsform des siebten Aspekts, die auch eine Ausführungsform der ersten, zweiten, dritten, vierten, fünften, sechsten, siebten, achten, neunten, zehnten, elften, zwölften, dreizehnten und sechzehnten oder siebzehnten Ausführungsform des siebten Aspekts ist, umfasst die HGMA bindende Nukleinsäure einen Zwischenabschnitt Z₂ der 12 bis 25 Nukleotide umfasst.

In einer vierzigsten Ausführungsform des siebten Aspekts, die auch eine Ausführungsform der neununddreißigsten Ausführungsform ist, umfassend die HGMA bindende Nukleinsäure einen Zwischenabschnitt Z2 einen Abschnitt Helix C1 und einen Abschnitt Helix C2.

In einer einundvierzigsten Ausführungsform des siebten Aspekts, die auch eine Ausführungsform der vierzigsten Ausführungsform ist, ist zwischen dem Abschnitt Helix C1 und dem Abschnitt Helix C2 der HMGA bindenden Nukleinsäure ein zentraler Abschnitt N_{c} angeordnet.

In einer zweiundvierzigsten Ausführungsform des siebten Aspekts, die auch eine Ausführungsform der vierzigsten oder einundvierzigsten Ausführungsform ist, ist die Länge des Abschnittes Helix C1 und Helix C2 der HGMA bindenden Nukleinsäure gleich lang.

In einer dreiundvierzigsten Ausführungsform des siebten Aspekts, die auch eine Ausführungsform der vierzigsten, einundvierzigsten und zweiundvierzigsten Ausführungsform ist, ist die Länge des Abschnittes Helix C1 und Helix C2 der HGMA bindenden Nukleinsäure einzeln und unabhängig drei bis sechs Nukleotide.

In einer vierundvierzigsten Ausführungsform des siebten Aspekts, die auch eine Ausführungsform der vierzigsten, einundvierzigsten, zweiundvierzigsten und dreiundvierzigsten Ausführungsform ist, sind die Abschnitte Helix C1 und Helix C2 der HMGA bindenden Nukleinsäure vollständig oder teilweise miteinander hybridisiert.

In einer fünfundvierzigsten Ausführungsform des siebten Aspekts, die auch eine Ausführungsform der neununddreißigsten, vierzigsten, einundvierzigsten, zweiundvierzigsten, dreiundvierzigsten und vierundvierzigsten Ausführungsform ist, umfasst der zentrale Abschnitt N_{c} der HMGA bindenden Nukleinsäure drei bis fünf Nukleotide.

In einer sechsundvierzigsten Ausführungsform des siebten Aspekts, die auch eine Ausführungsform der neununddreißigsten, vierzigsten, einundvierzigsten, zweiundvierzigsten, dreiundvierzigsten, vierundvierzigsten und fünfundvierzigsten Ausführungsform ist, umfasst die HMGA bindende Nukleinsäure einen Abschnitt Box A1 und einen Abschnitt Box A2, wobei zwischen dem 3'-Ende des Abschnittes Box A1 und dem 5'-Ende des Abschnittes Helix C1 eine Nukleotidsequenz N_{b} angeordnet ist und drei Nukleotide umfasst.

In einer siebenundvierzigsten Ausführungsform des siebten Aspekts, die auch eine Ausführungsform der neununddreißigsten, vierzigsten, einundvierzigsten, zweiundvierzigsten, dreiundvierzigsten, vierundvierzigsten, fünfundvierzigsten und sechsundvierzigsten Ausführungsform ist, umfasst die HMGA bindende Nukleinsäure einen Abschnitt Box A1 und einen Abschnitt Box A2, wobei zwischen dem 3'-Ende des Abschnittes Helix C2 und dem 5'-Ende des Abschnittes Box A2 eine Nukleotidsequenz N_{d} angeordnet ist und zwei bis fünf Nukleotide umfasst.

In einer achtundvierzigsten Ausführungsform des siebten Aspekts, die auch eine Ausführungsform der neununddreißigsten, vierzigsten, einundvierzigsten, zweiundvierzigsten, dreiundvierzigsten, vierundvierzigsten, fünfundvierzigsten, sechsundvierzigsten und siebenundvierzigsten Ausführungsform ist, umfasst die HMGA bindende Nukleinsäure einen Abschnitt Helix A1 und einen Abschnitt Helix A2.

In einer neunundvierzigsten Ausführungsform des siebten Aspekts, die auch eine Ausführungsform der achtundvierzigsten Ausführungsform ist, umfassen die Abschnitte Helix A1 und Helix A2 der HMGA bindenden Nukleinsäure jeweils einzeln und unabhängig voneinander fünf bis sechs Nukleotide, wobei bevorzugterweise der Abschnitt Helix A1 und der Abschnitt Helix A2 vollständig oder teilweise miteinander hybridisiert sind.

In einer fünfzigsten Ausführungsform des siebten Aspekts, die auch eine Ausführungsform der achtundvierzigsten und neunundvierzigsten Ausführungsform ist, ist zwischen dem 3'-Ende des Abschnittes Helix A1 und dem 5'-Ende des Abschnittes Box A1 der HMGA bindenden Nukleinsäure eine Nukleotidsequenz Nₐ angeordnet, wobei Nₐ ein bis fünf Nukleotide umfasst.

In einer einundfünfzigsten Ausführungsform des siebten Aspekts, die auch eine Ausführungsform der achtundvierzigsten, neunundvierzigsten und fünfzigsten Ausführungsform ist, ist zwischen dem 3'-Ende des Abschnittes Box A2 und dem 5'-Ende des Abschnittes Helix A2 der HMGA bindenden Nukleinsäure in 5'-3'-Richtung eine Nukleotidsequenz GNₑ angeordnet, wobei Nₑ eins bis zwei Nukleotide, bevorzugterweise A oder UU umfasst.

In einer zweiundfünfzigsten Ausführungsform des siebten Aspekts, die auch eine Ausführungsform der achtundvierzigsten, neunundvierzigsten, fünfzigsten und einundfünfzigsten Ausführungsform ist, weisen der Abschnitt Helix C1 und der Abschnitt Helix C2 der HMGA bindenden Nukleinsäure jeweils einzeln und unabhängig voneinander eine Länge von fünf oder sechs Nukleotiden auf, wobei bevorzugterweise die Abschnitte Helix C1 und Helix C2 vollständig oder teilweise miteinander hybridisiert sind.

In einer dreiundfünfzigsten Ausführungsform des siebten Aspekts, die auch eine Ausführungsform der zweiundfünfzigsten Ausführungsform ist, umfasst die HMGA bindende Nukleinsäure die folgende Struktur: Helix A1-Nₐ-Box A1-N_{b}-Helix C1-N_{c}-Helix C2-N_{d}-Box A2-G-Nₑ-Helix A2 (III) wobei
Nₐ = ein bis fünf Nukleotide ist;
N_{b} = drei Nukleotide ist;
N_{c} = drei bis fünf Nukleotide ist;
N_{d} = zwei bis fünf Nukleotide ist; und
Nₑ = eins bis zwei Nukleotide, bevorzugterweise A oder UU ist;
der Abschnitt Box A1 und der Abschnitt Box A2 jeweils einzeln und unabhängig voneinander ausgewählt sind aus der Gruppe, die GGGCG, GGGUG und GGGAG umfasst,
die Abschnitte Helix A1 und Helix A2 jeweils einzeln und unabhängig voneinander fünf oder sechs Nukleotide umfassen, und
die Abschnitte Helix C1 und Helix C2 jeweils fünf oder sechs Nukleotide umfasst, die bevorzugterweise vollständig oder teilweise miteinander hybridisiert sind.

In einer vierundfünfzigsten Ausführungsform des siebten Aspekts, die auch eine Ausführungsform der dreiundfünfzigsten Ausführungsform ist, umfasst die HMGA bindende Nukleinsäure eine Sequenz, die ausgewählt ist aus der Gruppe umfassend SEQ. ID. No. 8, SEQ. ID. No. 9, SEQ. ID. No. 10, SEQ. ID. No. 11, SEQ. ID. No. 14, SEQ. ID. No. 22 und SEQ. ID. No. 24.

In einer fünfundfünfzigsten Ausführungsform des siebten Aspekts, die auch eine Ausführungsform der neununddreißigsten, vierzigsten, einundvierzigsten, zweiundvierzigsten, dreiundvierzigsten und vierundvierzigsten Ausführungsform ist, umfasst die Nukleinsäure einen Abschnitt Box A1 und einen Abschnitt Helix C1 der HMGA bindenden Nukleinsäure, wobei zwischen dem 3'-Ende des Abschnittes Box A1 und dem 5'-Ende des Abschnittes Helix C1 ein Nukleotid A angeordnet ist.

In einer sechsundfünfzigsten Ausführungsform des siebten Aspekts, die auch eine Ausführungsform der fünfundfünfzigsten Ausführungsform ist, umfasst die HMGA bindende Nukleinsäure einen Abschnitt Helix C2 und einen Abschnitt Box A2, wobei zwischen dem 3'-Ende des Abschnittes Helix C2 und dem 5'-Ende des Abschnittes Box A2 ein Nukleotid G angeordnet ist.

In einer siebenundfünfzigsten Ausführungsform des siebten Aspekts, die auch eine Ausführungsform der fünfundfünfzigsten oder sechsundfünfzigsten Ausführungsform ist, umfasst der zentrale Abschnitt N_{c} der HMGA bindenden Nukleinsäure vier Nukleotide, wobei N_{c} bevorzugterweise GAUG ist.

In einer achtundfünfzigsten Ausführungsform des siebten Aspekts, die auch eine Ausführungsform der fünfundfünfzigsten, sechsundfünfzigsten und siebenundfünfzigsten Ausführungsform ist, umfasst die HMGA bindende Nukleinsäure einen Abschnitt Helix B1 und einen Abschnitt Helix B2.

In einer neunundfünfzigsten Ausführungsform des siebten Aspekts, die auch eine Ausführungsform der achtundfünfzigsten Ausführungsform ist, umfassen die Abschnitte Helix B1 und Helix B2 der HMGA bindenden Nukleinsäure einzeln und unabhängig voneinander jeweils fünf Nukleotide, wobei bevorzugterweise der Abschnitt Helix B1 mit dem Abschnitt Helix B2 hybridisiert ist.

In einer sechzigsten Ausführungsform des siebten Aspekts, die auch eine Ausführungsform der achtundfünfzigsten oder neunundfünfzigsten Ausführungsform ist, sind zwischen dem 3'-Ende des Abschnittes Helix B1 und dem 5'-Ende des Abschnittes Box A1 der HMGA bindenden Nukleinsäure eine Nukleotidsequenz umfassend zwei Nukleotide Nⱼ angeordnet, wobei Nⱼ bevorzugterweise AG ist.

In einer einundsechzigsten Ausführungsform des siebten Aspekts, die auch eine Ausführungsform der achtundfünfzigsten, neunundfünfzigsten und sechzigsten Ausführungsform ist, ist zwischen dem 3'-Ende des Abschnittes Box A2 und dem 5'-Ende von Helix B2 der HMGA bindenden Nukleinsäure ein Nukleotid G angeordnet.

In einer zweiundsechzigsten Ausführungsform des siebten Aspekts, die auch eine Ausführungsform der fünfundfünfzigsten, sechsundfünfzigsten, siebenundfünfzigsten, achtundfünfzigsten, neunundfünfzigsten, sechzigsten und einundsechzigsten Ausführungsform ist, umfasst die HGMA bindende Nukleinsäure einen Abschnitt Helix A1 und einen Abschnitt Helix A2.

In einer dreiundsechzigsten Ausführungsform des siebten Aspekts, die auch eine Ausführungsform der zweiundsechzigsten Ausführungsform ist, umfassen die Abschnitte Helix A1 und Helix A2 der HMGA bindenden Nukleinsäure einzeln und unabhängig voneinander jeweils sechs Nukleotide und sind bevorzugterweise der Abschnitt Helix A1 und der Abschnitt Helix A2 miteinander hybridisiert.

In einer vierundsechzigsten Ausführungsform des siebten Aspekts, die auch eine Ausführungsform der zweiundsechzigsten und dreiundsechzigsten Ausführungsform ist, ist zwischen dem 3'-Ende des Abschnittes Helix A1 und dem 5'-Ende des Abschnittes Helix B1 eine Nukleotidsequenz umfassend zwei Nukleotide Nᵢ angeordnet, wobei Nᵢ bevorzugterweise CA ist.

In einer fünfundsechzigsten Ausführungsform des siebten Aspekts, die auch eine Ausführungsform der zweiundsechzigsten, dreiundsechzigsten und vierundsechzigsten Ausführungsform ist, ist zwischen dem 3'-Ende des Abschnittes Helix B2 und dem 5'-Ende des Abschnittes Helix A2 ein Nukleotid A angeordnet.

In einer sechsundsechzigsten Ausführungsform des siebten Aspekts, die auch eine Ausführungsform der fünfundfünfzigsten bis fünfundsechzigsten Ausführungsform ist, umfassen die Abschnitte Helix C1 und Helix C2 jeweils drei Nukleotide, wobei bevorzugterweise die Abschnitte Helix C1 und Helix C2 miteinander hybridisiert sind.

In einer siebenundsechzigsten Ausführungsform des siebten Aspekts, die auch eine Ausführungsform der sechsundsechzigsten Ausführungsform ist, umfasst die HMGA bindende Nukleinsäure die folgende Struktur: Helix A1-Nᵢ-Helix B1-Nⱼ-Box A1-A-Helix C1-N_{c}-Helix C2-G-Box A2-G-Helix B2-A -Helix A2 wobei
Nᵢ = zwei Nukleotide umfasst, bevorzugterweise CA ist;
Nj = zwei Nukleotide umfasst, bevorzugterweise AG ist;
N_{c} = vier Nukleotide umfasst, bevorzugterweise GAUG ist;
die Abschnitte Box A1 und Box A2 jeweils einzeln und unabhängig voneinander ausgewählt sind aus der Gruppe umfassend die Sequenzen GGGCG, GGGUG und GGGAG;
die Abschnitte Helix A1 und Helix A2 jeweils einzeln und unabhängig sechs Nukleotide umfassen, die bevorzugterweise miteinander hybridisiert sind;
die Abschnitte Helix B1 und Helix B2 jeweils einzeln und unabhängig fünf Nukleotide umfassen, wobei bevorzugterweise der Abschnitt Helix B1 und der Abschnitt Helix B2 miteinander hybridisiert ist; und
die Abschnitte Helix C1 und Helix C2 jeweils einzeln und unabhängig drei Nukleotide umfassen, wobei bevorzugterweise die Abschnitte Helix C1 und Helix C2 miteinander hybridisiert sind.

In einer achtundsechzigsten Ausführungsform des siebten Aspekts, die auch eine Ausführungsform der siebenundsechzigsten Ausführungsform ist, umfasst die HMGA bindende Nukleinsäure eine Sequenz, die ausgewählt ist aus der Gruppe umfassend SEQ. ID. No. 12.

In einer neunundsechzigsten Ausführungsform des siebten Aspekts, die auch eine Ausführungsform der zweiten bis siebenundsechzigsten Ausführungsform ist, ist die Nukleinsäure eine solche, die an Transkriptionsfaktoren bindet, insbesondere Transkriptionsfaktoren, die einen AT-Haken aufweisen.

Erfindungsgemäß wird die Aufgabe gelöst in einem achten Aspekt durch eine Nukleinsäure , die an einen Transkriptionsfaktor umfassend einen AT-Haken bindet, wobei die Nukleinsäure einen Aufbau gemäß dem siebten Aspekt umfasst.

In einer Ausführungsform der Zusammensetzung nach dem sechsten Aspekt ist die L-Nukleinsäure eine Nukleinsäure nach dem siebten Aspekt.

In einer Ausführungsform der Verwendung nach dem ersten Aspekt ist die L-Nukleinsäure eine Nukleinsäure nach dem siebten Aspekt.

In einer Ausführungsform des Verfahrens nach dem zweiten Aspekt ist die L-Nukleinsäure eine Nukleinsäure nach dem siebten Aspekt.

In einer Ausführungsform der Verwendung nach dem dritten Aspekt ist die L-Nukleinsäure eine Nukleinsäure nach dem siebten Aspekt.

In einer Ausführungsform des Verfahrens nach dem vierten Aspekt ist die L-Nukleinsäure eine Nukleinsäure nach dem siebten Aspekt.

Erfindungsgemäß wird die Aufgabe gelöst in einem neunten Aspekt durch ein Verfahren zum Screenen eines HMGA-Antagonisten oder HMGA-Agonisten umfassend die folgenden Schritte:
- Bereitstellen eines Kandidaten-HMGA-Antagonisten und/oder eines Kandidaten-HMGA-Agonisten,
- Bereitstellen einer Nukleinsäure nach dem siebten Aspekt,
- Bereitstellen eines Testsystems, welches ein Signal in Gegenwart eines HMGA-Antagonisten und/oder eines HMGA-Agonisten liefert, und
- Bestimmen, ob der Kandidaten-HMGA-Antagonist ein HMGA-Antagonist ist und/oder ob der Kandidaten-HMGA-Agonist ein HMGA-Agonist ist.

Erfindungsgemäß wird die Aufgabe gelöst in einem zehnten Aspekt durch ein Verfahren zum Screenen eines HMGA-Agonisten und/oder eines HMGA-Antagonisten umfassend die folgenden Schritte:
- Bereitstellen eines an einer Phase, bevorzugterweise einer Festphase, imobilisierten HMGA,
- Bereitstellen einer Nukleinsäure gemäß dem siebten Aspekt, bevorzugterweise eine Nukleinsäure nach dem siebten Aspekt, die markiert ist,
- Zugeben eines Kandidaten-HMGA-Agonisten und/oder eines Kandidaten-HMGA-Antagonisten, und
- Bestimmen, ob der Kandidaten-HMGA-Agonist ein HMGA-Agonist ist und/oder ob der Kandidaten-HMGA-Antagonist ein HMGA-Antagonist ist.

In einer Ausführungsform des zehnten Aspekts ist vorgesehen, dass die Bestimmung so durchgeführt wird, dass getestet wird, ob die Nukleinsäure durch den Kandidaten-HMGA-Agonisten oder durch den Kandidaten-HMGA-Antagonisten ersetzt wird.

Erfindungsgemäß wird die Aufgabe gelöst in einem elften Aspekt durch einen Kit für den Nachweis von HMGA, umfassend eine Nukleinsäure nach dem siebten Aspekt.

Erfindungsgemäß wird die Aufgabe gelöst in einem zwölften Aspekt durch einen HMGA-Antagonisten, der durch ein Verfahren nach dem zehnten Aspekt erhältlich ist.

Erfindungsgemäß wird die Aufgabe gelöst in einem dreizehnten Aspekt durch einen HMGA-Agonisten, der durch ein Verfahren nach dem zehnten Aspekt erhältlich ist.

Erfindungsgemäß wird die Aufgabe gelöst in einem vierzehnten Aspekt durch einen Komplex umfassend ein HMGA-Protein und eine Nukleinsäure nach dem siebten Aspekt.

Der vorliegenden Erfindung liegt die überraschende Erkenntnis zugrunde, dass es entgegen der Auffassung im Stand der Technik möglich ist, L-Nukleinsäuren und insbesondere Spiegelmere zu verwenden, um intrazelluläre Zielmoleküle zu adressieren. Bei den intrazellulären Zielmolekülen handelt es sich bevorzugterweise um Zielmoleküle, die in einer Zelle vorliegen. Die den funktionalen L-Nukleinsäuren durch ihren Aufbau aus L-Nukleotiden inhärenten Eigenschaften wie hohe Spezifität der Wechselwirkung mit ihren Zielmolekülen bei gleichzeitig hoher Stabilität und Fehlen von toxischen oder immunologisch aktiven Abbauprodukten bei Anwendung derselben in biologischen Systemen und insbesondere im tierischen und menschlichen Körper erlaubt es nicht, die zellulären Mechanismen zu nutzen, um, wie im Falle der Intramere, L-Nukleinsäuren von einem Plasmid oder allgemein einem Vektor codieren zu lassen und damit die eigentlich funktionale Nukleinsäure durch den intrazellulär ablaufenden Prozess der Transkription bereitzustellen.

Dieses unentrinnbare Dilemma wird mit der vorliegenden Erfindung gelöst: Funktionale L-Nukleinsäuren und insbesondere Spiegelmere können unter Beibehaltung ihrer Spezifität was ihre Bindung an ihr Zielmolekül anbelangt, und ihrer Aktivität über eine Cytoplasmamembran transportiert werden. Diese Durchgängigkeit der funktionalen L-Nukleinsäuren ist den Spiegelmeren inhärent und kann durch die Verwendung von Abgabevehikeln oder Abgabetechniken noch verstärkt werden. Ohne im folgenden darauf festgelegt sein zu wollen, gehen die vorliegenden Erfinder davon aus, dass funktionale L-Nukleinsäuren an sich die Cytoplasmamembran überwinden können, bzw. bei Beteiligung von endosomalen Transportmechanismen bei der Überwindung der Cytoplasmamembran, diese aus den sich dabei ausbildenden Vesikelstrukturen unter Einnahme einer zwei- bzw. dreidimensionalen Struktur zu befreien in der Lage sind, die die spezifische Wechselwirkung der funktionalen Nukleinsäure mit ihrem Zielmolekül erlaubt. Mit der hierin gegebenen technischen Lehre wendet man sich bewusst von dem für Aptamere entwickelten Prinzip der Nutzung von intrazellulären Transkriptionsmechanismen zur Erzeugung von Aptameren in der Zelle ab und stellt erstmalig Mittel für die Anwendung von funktionalen L-Nukleinsäuren und insbesondere von Spiegelmeren in Zellen bereit.

Wie hierin in einer bevorzugten Ausführungsform verwendet, bezeichnet der Begriff der funktionalen Nukleinsäuren solche Nukleinsäuren, die verschieden sind von strukturellen, insbesondere natürlich vorkommenden strukturellen Nukleinsäuren wie rRNAs oder von codierenden Nukleinsäuren wie mRNAs. Insbesondere sind funktionale Nukleinsäuren Nukleinsäuren, die aufgrund ihrer zwei- und/oder dreidimensionalen Struktur in der Lage sind, an Zielmoleküle zu binden. In einer besonders bevorzugten Ausführungsform erfolgt die Bindung an das Zielmolekül nicht durch Hybridisierung oder Basenpaarung auf der Grundlage von Watson-Crick-Basenpaarungen oder einer Hoogsteen-Basenpaarung. Besonders bevorzugte funktionale Nukleinsäuren sind Aptamere und Spiegelmere.

Eine L-Nukleinsäure ist in einer bevorzugten Ausführungsform eine Nukleinsäure, die vollständig, im wesentlichen oder teilweise aus L-Nukleotiden aufgebaut ist. Besonders bevorzugt ist, wenn die L-Nukleinsäure vollständig aus L-Nukleotiden besteht. Der Begriff "im wesentlichen" bezeichnet dabei eine Ausführungsform, bei der derjenige Teil der L-Nukleinsäure, der für die Wechselwirkung mit dem Zielmolekül verantwortlich ist bzw. derjenige Teil, der die Bindung an das Zielmolekül vermittelt, aus L-Nukleotiden besteht oder aus diesen aufgebaut ist.

Wie hierin verwendet ist eine funktionale L-Nukleinsäure eine funktionale Nukleinsäure, die vollständig, im wesentlichen oder teilweise aus L-Nukleotiden aufgebaut ist.

Die Synthese von L-Nukleinsäuren ist den Fachleuten auf dem Gebiet bekannt und beispielsweise beschrieben in Nolte et al., Nat. Biotech, 14, 1116-1119, 1996.; und Klussmann et al., Nat. Biotechnol, 14, 1112-1115, 1996.

Das grundsätzliche Verfahren zur Erzeugung von Aptameren ist beispielsweise beschrieben in Tuerck et al. Science, 248, 505-510, 1990; oder Ellington et al. Nature, 346, 818-822, 1990, das grundsätzliche Verfahren zur Erzeugung von Spiegelmeren ist beispielsweise beschrieben in Nolte et al., Nat. Biotech, 14, 1116-1119, 1996.; oder Klussmann et al., Nat. Biotechnol, 14, 1112-1115, 1996 Spielgelmere sind somit Aptamere, die aus L-Nukleotiden statt aus D-Nukleotiden bestehen. Im Zusammenhang mit der Erzeugung von Aptameren bzw. Spiegelmeren bezeichnet der Begriff Zielmolekül dasjenige Molekül, das im Selektionsprozess zur Erzeugung der Aptamere bzw. Spiegelmere verwendet wird bzw. dasjenige Molekül, das letztlich durch das Aptamer bzw. das Spiegelmer gebunden wird.

In einer bevorzugten Ausführungsform ist ein intrazellulär aktives Agens eine chemische Verbindung, die in einer Zelle an ein Molekül zu binden in der Lage ist. Dabei ist besonders bevorzugt, wenn die Zelle eine Zelle ist, die isoliert in einem Gewebe oder einem Organ vorliegt, aber bevorzugtererweise nicht in einem menschlichen oder tierischen Körper. Ist das intrazellulär aktive Agens ein Spiegelmer, so ist es bevorzugterweise dann ein intrazellulär aktives Agens, wenn es in der Lage ist, an ein intrazelluläres Zielmolekül zu binden. Alternativ ist das Spiegelmer dann ein intrazellulär aktives Agens, wenn es in der Lage ist, an sein Zielmolekül unter Bedingungen zu binden, wie sie in einer Zelle vorliegen. Tests, um diese Eigenschaften zu bestimmen, sind den Fachleuten auf dem Gebiet bekannt und umfassen z.B. Gleichgewichtsanbindungsassays unter Pufferbedingungen, wie sie intrazellulär vorliegen (Ionenstärke und Solutzusammensetzung, pH, Temperatur) wie in Beispiel 1 offenbart.

In einer bevorzugten Ausführungsform ist das Zielmolekül der L-Nukleinsäure, insbesondere der funktionalen L-Nukleinsäure ein intrazellulärer Rezeptor. Ein intrazellulärer Rezeptor, wie hierin verwendet, ist bevorzugterweise eine chemische Verbindung oder eine chemische Struktur oder jeweils ein Teil davon, mit der/dem die funktionale L-Nukleinsäure in Wechselwirkung tritt und bevorzugterweise eine solche, an die die funktionale L-Nukleinsäure bindet, wobei der intrazelluläre Rezeptor, d.h. die chemische Verbindung oder die chemische Struktur oder jeweils ein Teil davon, intrazellulär, d. h. in einer Zelle vorliegt, wie sie bevorzugt in dem vorstehenden Absatz beschrieben ist, vorliegt. Dabei ist es im Rahmen der vorliegenden Erfindung, dass der intrazelluläre Rezeptor das Zielmolekül bei der Erzeugung der funktionalen Nukleinsäure, insbesondere der funktionalen L-Nukleinsäure, ist.

In einer Ausführungsform bezeichnet der Begriff des Rezeptors einen jeglichen Wechselwirkungspartner, bevorzugterweise einen spezifisch bindenden Wechselwirkungspartner der funktionalen Nukleinsäure, d.h. einen mit der funktionalen Nukleinsäure interagierenden Wechselwirkungspartner, der eine bestimmte räumliche Struktur, Ladungsverteilung, Hydrophobizitätsverteilung etc. aufweisen. In einer besonders bevorzugten Ausführungsform entspricht der Wechselwirkungspartner dem Zielmolekül der funktionalen Nukleinsäure, wie es bei der Erzeugung der funktionalen Nukleinsäure verwendet wurde. Dabei ist es im Rahmen der vorliegenden Erfindung, dass ein Rezeptor auch von dem bei der Erzeugung der funktionalen Nukleinsäure verwendeten Zielmolekül verschieden sein kann, jedoch die spezifische Wechselwirkung durch eine Kreuzreaktivität der funktionalen Nukleinsäure zwischen dem Wechselwirkungspartner und dem bei der Erzeugung der funktionalen Nukleinsäure verwendeten Zielmolekül bedingt wird.

In einer bevorzugten Ausführungsform bezeichnet der Begriff "intrazellulärer Rezeptor" einen Rezeptor, der in einer Zelle vorliegt, oder einen Rezeptor, der in einer Zelle vorliegen kann, unter natürlichen Umständen in einer Zelle vorkommt oder unter solchen in einer Zelle vorhanden ist. Dabei ist besonders bevorzugt, wenn die Zelle eine Zelle ist, die isoliert in einem Gewebe oder einem Organ vorliegt, aber bevorzugtererweise nicht in einem menschlichen oder tierischen Körper. Wie hierin verwendet bezeichnet das Begriff "intrazellulärer Rezeptor" aber auch einen Rezeptor, der unter Bedingungen vorliegt, wie sie in einer Zelle vorliegen.

In einer bevorzugten Ausführungsform bezeichnet der Begriff der Zelle eine Zelle, die ausgewählt ist aus der Gruppe, die prokaryotische und eukaryotische Zellen umfasst. Bevorzugterweise ist die eukaryotische Zelle ausgewählt aus der Gruppe, die Pilzzellen, pflanzliche Zellen, tierische Zellen und menschliche Zellen umfasst. In einer alternativen Ausführungsform bezeichnet der Begriff der Zelle hierin allgemein ein durch eine Phospholipiddoppelmembran, die in einer bevorzugten Ausführungsform einer Cytoplasmamembran entspricht, begrenztes Kompartiment, das durch die Membran von der Umgebung abgetrennt ist. Die Abtrennung von der Umgebung ist dabei nicht eine vollständige Abtrennung, sondern erlaubt einen Energieaustausch und einen Stoffaustausch zwischen der Zelle und der Umgebung. Bevorzugterweise ist der Stoffaustausch beschränkt. Die Beschränkung des Stoffaustausches wird im Falle der Abgrenzung der Zelle gegenüber der Umgebung durch eine Cytoplasmamembran oder durch eine einer Cytoplasmamembran ähnlichen Membran durch die Transporteigenschaften der Membran definiert. In einer Ausführungsform umfasst der Begriff der Zelle hierin somit auch Vesikel und/oder Kompartimente von einer prokaryotischen oder eukaryotischen Zellen wie hierin definiert, die sowohl wiederum innerhalb einer prokaryotischen oder eukaryotischen Zelle als auch ausserhalb einer derartigen prokaryotischen oder eukaryotischen Zelle vorliegen oder vorliegen können, beispielsweise als Vesikel oder von einer Cytoplasmamembran umgebene Teile einer prokaryotischen oder eukaryotischen Zelle, die in einer Ausführungsform in einer Körperflüssigkeit vorliegen kann. In einer bevorzugten Ausführungsform ist in einer Zelle gemäß der zweiten alternativen Ausführungsform vorgesehen, dass die Bedingungen innerhalb einer solchen Zelle im wesentlichen jenen entsprechen, wie sie in einer prokaryotischen oder eukaryotischen Zelle vorliegen, insbesondere hinischtlich der Faktoren, die die Bindung der funktionalen Nukleinsäure an ihr Zielmolekül beeinflussen.

In einer Bevorzugten Ausführungsform ist die Körperflüssigkeit aus der Gruppe ausgewählt, die Blut, Urin, Liquor, Lymphflüssigkeit, Serum, Plasma, Vaginalsekret, Speichel und Sperma umfasst.

In einer Ausführungsform ist der Rezeptor über seine Funktion in einer Zelle definiert. Entsprechend kann der Rezeptor aus der Gruppe ausgewählt sein, die molekulare Rezeptoren, Enzyme, Stoffwechselintermediate, Signalpeptide, Chaperon-Moleküle und intrazelluläre Strukturen wie beispielsweise Ribosomen, Mitochondrien, Elemente des Zytoskeletts wie z.B. Tubulin- und Aktinfilamente, endosomale Partikel, Lysosomen, sonstige intrazelluläre Strukturen wie Vesikel, insbesondere intrazelluläre Vesikel umfasst. Wie hierin verwendet bezeichnet der Begriff des molekularen Rezeptors in einer bevorzugten Ausführungsform ein Molekül, welches Information(en) aufnimmt und innerhalb einer Zelle, einem Gewebe, einem Organ oder einem Organismus weiterleitet. Die Information wird typischerweise durch ein Molekül vermittelt, das mit dem molekularen Rezeptor in Wechselwirkung tritt. Infolge der Wechselwirkung ist der molekulare Rezeptor in der Lage, ein Signal zu erzeugen. Ein derartiges Signal kann in der Änderung der Konformation und/oder der Aktivität des Rezeptors begründet sein oder sich darin manifestieren. Das Signal selbst ist in der Lage, die aufgenommene oder von ihm dargestellte Information in einer anderen Form weiterzuleiten. Infolge der Änderung der Konformation bzw. der Aktivität des molekularen Rezeptors kann das Signal bevorzugterweise ein chemisches, biochemisches oder ein elektrisches Signal sein. Bevorzugterweise ist der molekulare Rezeptor Teil einer Reaktionskaskade, noch bevorzugtererweise einer Signalkaskade. Die von einem molekularen Rezeptor übertragene Information kann eine quantitative und/oder eine qualitative Information sein, beispielsweise betreffend die Anwesenheit einer Verbindung und/oder deren Konzentration.

In einer bevorzugten Ausführungsform bezeichnet der Begriff der Stoffwechselintermediate all jene Verbindungen, die durch metabolische Aktivitäten in einer Zelle als Bestandteile des Katabolismus ebenso wie des Anabolismus auftreten.

In einer weiteren Ausführungsform ist der Rezeptor über seine chemische Natur definiert. Bevorzugterweise ist der Rezeptor aus der Gruppe ausgewählt, die Polypeptide, Kohlenhydrate, Nukleinsäure, Lipide und Kombinationen davon umfasst. Wie hierin verwendet bezeichnet der Begriff Polypeptid bevorzugterweise ein jegliches Polymer aus zwei oder mehr Aminosäuren. Bevorzugterweise handelt es sich bei den Aminosäuren um L-Aminosäuren, wobei jedoch auch D-Aminosäuren im Rahmen der Ausführungsform verwendet werden können. Wie hierin verwendet bezeichnet der Begriff Nukleinsäuren bevorzugterweise ein Polymer aus zwei oder mehreren Nukleotiden oder Nukleotidanaloga, die den Fachleuten auf dem Gebiet bekannt sind, wobei es sich um entweder D-Nukleotide oder um L-Nukleotide oder Mischungen davon handelt. Bevorzugte Kombinationen umfassen glykosilierte Polypeptide und glykosilierte Lipide

Eine besondere Gruppe von intrazellulären Rezeptoren sind Transkriptionsfaktoren und DNA-bindende Proteine, die an einen AT-Haken binden. Beispielhafte Transkriptionsfaktoren sind in der folgenden Tabelle 1 angebeben:

**Tabelle 1: Transkriptionsfaktoren**

| | | |
|---|---|---|
| gamma)OBP | ALL-1 | AP-2beta |
| (STAT5A)4 | alpha-CBF | AP-2gamma |
| 120-kDa CRE- | alpha-CP2b | AP-2rep |
| Bindungsprotein | alphaH0 | AP-3 (1) |
| 14-3-3 epsilon | alphaH2-alphaH3 | AP-3 (2) |
| 14-3-3 zeta | ALX3 | AP-4 |
| 50-55K Protein | Alx-4 | AP-5 |
| 53BP1 | aMEF-2 | APC |
| 70-75K Protein | AML1 | AR |
| 80-90K Protein | AML1a | Arnt |
| AAF | AML1b | Arnt (774 AA Form) |
| ABF-1 | AML1c | ARNT2 |
| ADA2 | AML1DeltaN | ASC-2 |
| ADA3 | AML2 | ASPP1 |
| ADA-NF1 | AML3 | ASPP2 |
| AFP1 | AML3a | ATBF1-A |
| AhR | AML3b | ATBF1-B |
| AhR:Arnt | AMY-1L | ATF |
| AllN3 | A-Myb | ATF-1 |
| Aiolos | ANF | ATF2 |
| AIRE | AP-1 | ATF-2 |
| AKNA | AP-2alphaA | ATF-2:c-Jun |
| ALF | AP-2alphaB | ATF3 |
| ATF3 deltaZIP | BRIP1 | CDX2 |
| ATF4 | Brm | Cdx-4 |
| ATF5 | BTEB1 | c-Ets-1 |
| ATF6 | BTEB2 | c-Ets-2 |
| ATF-a | BTEB3 | CFF |
| ATF-adelta | BTEB4 | c-Fos |
| ATOH1 | B-TFIID | ChCh |
| ATPF1 | C/EBPalpha | CHOP-10 |
| Bach1 | C/EBPbeta | Chx10 |
| Bach1t | C/EBPdelta | CIITA |
| Bach2 | C/EBPepsilon | c-Jun |
| BAF155 | C/EBPgamma | c-Jun:JunD |
| BAF47 | CA150 | CL1M1 |
| BAF53a | c-abl | CLIM2 |
| BAF60A | CACCC-Bindungsfaktor | CLOCK |
| Barhl1 | CAR | c-Myb |
| Barhl2 | CAR:RXR-alpha | c-Myc |
| Barx1 | Cart-1 | C-Myc 1 |
| Barx2 | CBAF | c-Myc:Max |
| Bcl-3 | CBF (4) | CNBP |
| BCL-6 | CBF (5) | CoS |
| beta-catenin | CBP | COUP-TF1 |
| Bin1 | CCAAT- Bindungsfa- | COUP-TF2 |
| BMAL2 | ktor | CP1A |
| B-Myb | CCF | CP1C |
| BNC | CCG1 | CP2 |
| BP1 | CCK-1a | CPBP |
| BP2 | CCK-1b | CPE Bindungsprotein |
| BR140 | CD28RC | CREB |
| Brachyury | Cdc5 | CRE-BPa |
| BRCA1 | cdk2 | c-Rel |
| BRCA2 | cdk9 | c-Rel:RelA |
| BRG1 | Cdx-1 | CREMalpha |
| CREST | DbpB | DSIF-p14 |
| CRF | DCoHm | DSIF-p160 |
| Crx | DDB | DTF |
| CSA | DDB-1 | DUX1 |
| CSB | DDB-2 | DUX2 |
| CSBP-1 | DEC1 | DUX3 |
| CSEN | DEC2 | DUX4 |
| c-Ski | DEF | E |
| CtBP1 | deltaCREB | E12 |
| CtBP2 | deltaFosB | E2F |
| CTCF | deltaMax | E2F+E4 |
| CTF | DeltaN p63beta | E2F+p107 |
| CTF-1 | DeltaN p73alpha | E2F-1 |
| CTF-2 | DeltaN p73beta | E2F-1:DP-1 |
| CTF-3 | DeltaN p73gamma | E2F-1:DP-2 |
| CTF-5 | DeltaNp63alpha | E2F-2 |
| CTF-7 | DeltaNp63gamma | E2F-3a |
| CUP | Dermo-1 | E2F-4 |
| CUTL1 | DF-1 | E2F-4:DP-1 |
| CUTL2 | DF-2 | E2F-4: DP-2 |
| Cx | DF-3 | E2F-5 |
| cyclin A | Dlx-1 | E2F-6 |
| cyclin T1 | Dlx-2 | E2F-7 |
| cyclin T2 | Dlx-3 | E47 |
| cyclin T2a | Dlx-4 (lange Isoform) | E4BP4 |
| cyclin T2b | Dlx-4 (kurze Isoform) | E4F |
| DAP | Dlx-5 | E4F1 |
| DAX1 | Dlx-6 | E4TF2 |
| DB1 | DP-1 | E7; HPV-16, Papilloma |
| DBF4 | DP-2 | Virus Typ 16 |
| DBP | DPBF | EAR2 |
| DbpA | DRIL1 | EBF |
| DbpAv | DSIF | EBP-80 |
| EC2 | ER-beta5 | f-EBP |
| EF1 | ERF | FEV |
| Egr-1 | Erg-1 | Fgf3 |
| Egr-2 | Erg-2 | FKBP59 |
| Egr-3 | ERM | FKHL18 |
| Egr-4 | ERR1 | FKHRL1P2 |
| EllaE-A | ERR2 | FKLF |
| EllaE-B | ERR3 | Fli-1 |
| EllaE-Calpha | ERR3-1 | FosB |
| EllaE-Cbeta | ERR3-2 | FOXB1 |
| EivF | ERR3-3 | FOXC1 |
| EKLF | ERRalpha1 | FOXC2 |
| ELF-1 | ESE-1 | FOXD1 |
| ELFR | ESE-1a | FOXD2 |
| elios | ESE-1b | FOXD3 |
| Elk-1 | ESE-2 | FOXD4 |
| Emx-1 | ESE-2a | FOXE1 |
| Emx-2 | ESE-2b | FOXE3 |
| En-1 | ESE-3 | FOXF1 |
| En-2 | ESE-3a | FOXF2 |
| ENH-Bindungsprotein | ESE-3b | FOXG1a |
| ENKTF-1 | ESXR1 | FOXG1b |
| EP400 | ETF | FOXG1c |
| EPAS1 | Ets-1 deltaVII | FOXH1 |
| Epicardin | Evi-1 | FOXI1 |
| epsilonF1 | EVX1 | FOXJ1a |
| ER-alpha | EZF-2 | FOXJ1b |
| ER-alpha:ER-beta | EZH1 | FOXJ2 (lange Isoform) |
| ER-beta | EZH2 | FOXJ2 (kurze Isoform) |
| ER-beta1 | F2F | FOXJ3 |
| ER-beta2 | FAC1 | FOXK1 |
| ER-beta3 | factor 2 | FOXK2a |
| ER-beta4 | FBP | FOXK2b |
| FOXK2c | GABPB | GT-IC |
| FOXL1 | GABP-beta1 | GT-IIA |
| FOXL2 | GABP-beta2 | GT-IIBalpha |
| FOXM1a | GAF | GT-IIBbeta |
| FOXM1b | gammaCAAT | H1TF1 |
| FOXM1c | gammaCAC1 | H1TF2 |
| FOXN1 | gammaCAC2 | H1TF2A |
| FOXN2 | GATA-1 | H4TF-1 |
| FOXN3 | GATA-2 | H4TF-2 |
| FOXO1a | GATA-3 | HAF |
| FOXO1b | GATA-4 | HAND1 |
| FOXO2 | GATA-5 | HAND2 |
| FOXO3a | GATA-6 | HB9 |
| FOXO3b | Gbx1 | HDAC1 |
| FOXO4 | Gbx2 | HDAC2 |
| FOXP1 | GCF | HDAC3 |
| FOXP2 | GCMa | HDAC4 |
| FOXP3 | GCN5 | HDAC5 |
| FOXP4 | GCNF-1 | hDaxx |
| Fra-1 | GCNF-2 | HDBP1 |
| Fra-2 | GF1 | HDBP2 |
| FTF | GKLF | Hitze-induzierter Faktor |
| FTS | GLI1 | HEB |
| FXR | GLI2 | HEB1-p67 |
| FXR:RXR-alpha | GLI3 | HEB1-p94 |
| FXR-alpha | GLIS2 | HEF-1B |
| FXR-beta1 | GMEB-1 | HEF-1T |
| FXR-beta2 | GR | HEF-4C |
| G factor | GR-alpha | HEN1 |
| G6 factor | GR-beta | HEN2 |
| GAAP-1 | GRF-1 | HES-1 |
| GABP | Gsc | HES-2 |
| GABP-alpha | Gscl | Hesx1 |
| Hex | HNF-1beta-A | HOXB2 |
| Hey1 | HNF-1beta-B | HOXB3 |
| Hey2 | HNF-1beta-C | HOXB4 |
| HeyL | HNF-3 | HOXB5 |
| HFH-1 | HNF-3alpha | HOXB6 |
| HIC-1 | HNF-3beta | HOXB7 |
| Hic-5 | HNF-3gamma | HOXB8 |
| HIF-1 | HNF-4 | HOXB9 |
| HIF-1alpha | HNF-4alpha | HOXC10 |
| HiNF-A | HNF-4alpha1 | HOXC11 |
| HiNF-B | HNF-4alpha2 | HOXC12 |
| HiNF-C | HNF-4alpha3 | HOXC13 |
| HiNF-D | HNF-4alpha4 | HOXC4 |
| HiNF-D3 | HNF-4alpha7 | HOXC5 |
| HiNF-E | HNF-4gamma | HOXC6 |
| HiNF-P | HNF-6alpha | HOXC8 |
| HIP1 | hnRNP K | HOXC9 |
| HIV-EP2 | HOX11 | HOXD10 |
| Hlf | HOXA1 | HOXD11 |
| HLTF | HOXA10 | HOXD12 |
| HLTF (Met123) | HOXA10 PL2 | HOXD13 |
| HLX | HOXA11 | HOXD3 |
| HMBP | HOXA13 | HOXD4 |
| HMG I | HOXA2 | HOXD8 |
| HMG I(Y) | HOXA3 | HOXD9 |
| HMG Y | HOXA4 | Hp55 |
| HMGB1 | HOXA5 | Hp65 |
| HMGB2 | HOXA6 | HPX42B |
| HMGI-C | HOXA7 | HrpF |
| HMX1 | HOXA9A | HSBP1 |
| HNF-1alpha-A | HOXA9B | HSF |
| HNF-1alpha-B | HOXB1 | HSF1 (lang) |
| HNF-1alpha-C | HOXB13 | HSF1 (kurz) |
| HSF2 | ING1b | JunD |
| HSF4a | INSAF | JunD:Fra-2 |
| HSF4b | IPCS-BF | kappaY FaKtor |
| HSF4c | IPF1 | KBP-1 |
| hsp56 | IPF1:Pbx | KER1 |
| Hsp90 | IRF-1 | KER-1 |
| IA-1 | IRF-1:C/EBPbeta | KLF15 |
| iASPP | IRF-2 | KLF7 |
| IASPP-RAI | IRF-3 | Kox1 |
| IB1 | IRF-4 | KR3 |
| IBP-1 | IRF-5 | KRF-1 |
| ICER-II | IRF-6 | KRN |
| ICER-Ilgamma | IRF-7A | KSR-1 |
| Id1 | IRF-7B | Ku Autoantigen |
| Id1H | IRF-7H | Ku70 |
| Id2 | IRF-8 | Ku80 |
| Id3 | IRF-9 | KUP |
| Id3 / Heir-1 | irlB | LAF-4 |
| IF1 | IRX-1 | LANA; KSHV, Kaposi's |
| IFI-16 | IRX2a | sarcoma-associated |
| IgPE-1 | Irx-3 | herpesvirus (herpesvi- |
| IgPE-2 | Irx-4 | rus 8) |
| IgPE-3 | ISGF-1 | LBP-1 |
| Ik-1 | ISGF-3 | LBP-1a |
| IkappaB | ISGF-3alpha | LBP-1d |
| IkappaB-alpha | Isl-1alpha | LBP-32 |
| IkappaB-beta | ITF | LBP-9 |
| IkappaBR | ITF-1 | LBX1 |
| II-1 RF | ITF-2 | LCR-F1 |
| IL-10E1 | JRF | LEF-1 |
| IL-6 RE-BP | JunB | LEF-1B |
| II-6 RF | JunB:Fra-1 | LF-A1 |
| ING1 | JunB:Fra-2 | LHX1 |
| LHX2 | MASH-1 | Meis-3 |
| LHX3a | Max | Mel-18 |
| LHX3b | Max1 | Meox1 |
| LHX5 | Max2 | Meox1a |
| LHX6.1a | MAZ | Meox2 |
| LHX6.1b | MAZi | MHox (K-2) |
| LIT-1 | MAZR | MIF-1 |
| LITAF | MBF1 | MITF |
| LKLF | MBF2 | MIXL1 |
| Lmo1 | MBF3 | Miz-1 |
| Lmo2 | MBP-1 (1) | MLX |
| LMO3 | MBP-1 (2) | MM-1 |
| LMX1A | MBP-2 | MondoA |
| LMX1B | MDBP | MOP3 |
| L-Myc-1 (lange Form) | MECP-2 | MR |
| L-Myc-1 (kurze Form) | MEF-2A | MRF-2 |
| L-Myc-2 | MEF-2B1 | Msx-1 |
| LUN-1 | MEF-2C | Msx-2 |
| LUN-2 | MEF-2C/delta32 | MTA1-L1 |
| LXR-alpha | MEF-2C/delta8 | MTB-Zf |
| LXR-alpha:RXR-alpha | MEF-2C/delta8,32 | MTF-1 |
| LXR-beta | MEF-2D00 | mtTFA |
| LXR-beta:RXR-alpha | MEF-2D0B | Mxi1 |
| Lyl-1 | MEF-2DA0 | Myf-3 |
| M factor | MEF-2DA'0 | Myf-4 |
| Mad1 | MEF-2DAB | Myf-5 |
| Maf | MEF-2DA'B | Myf-6 |
| MafB | Meis-1 | Myocardin, Splice Form |
| MafF | Meis-2a | 1 |
| MafG | Meis-2b | MyoD |
| MafG:MafG | Meis-2c | MyoD:E12 |
| MafK | Meis-2d | MyT1 |
| MAML1 | Meis-2e | MZF-1 |
| NC1 | NFdeltaE3A | NF-muE1 |
| NC2 | NFdeltaE3B | NF-muE2 |
| NCOR1 | NFdeltaE3C | NF-muE3 |
| NCOR2 | NFdeltaE4A | NF-S |
| NCX | NFdeltaE4B | NF-X |
| NELF | NFdeltaE4C | NF-X1 |
| NERF | NFe | NF-X2 |
| NERF-1a | NF-E | NF-X3 |
| NERF-1b | NF-E2 | NF-Xc |
| NERF-2 | NF-E2 p45 | NF-Y |
| Net | NF-E3 | NF-YA |
| NeuroD1 | NFE-6 | NF-Zc |
| NEUROD-2 | NF-GMa | NF-Zz |
| NEUROD-3 | NF-GMb | NGN3 |
| NF III-a | NFI/CTF | NHP-1 |
| NF III-c | NFIA | NHP-2 |
| NF III-e | NFIB | NHP3 |
| NF-1 | NF-IL-2A | NHP4 |
| NF-4FA | NF-IL-2B | Nkx2-1 |
| NF-4FB | NFIX | Nkx2-2 |
| NF-4FC | NF-jun | Nkx2-3 |
| NF-AB | NF-kappaB | Nkx2-5 |
| NF-AT1 | NF-kappaB(-ähnlich) | Nkx2-8 |
| NF-AT1 | NF-kappaB1 | Nkx3-1 |
| NF-AT2 | NF-kappaB1 Vorläufer | Nkx3-1 v1 |
| NF-AT2-alpha | NF-kappaB2 | Nkx3-1 v2 |
| NF-AT2-beta | NF-kappaB2 (p49) | Nkx3-1 v3 |
| NF-AT3 | NF-kappaB2 Vorläufer | Nkx3-1 v4 |
| NF-AT4 | NF-kappaE1 | Nkx3-2 |
| NF-AT5 | NF-kappaE2 | Nkx6-1 |
| NFbetaA | NF-kappaE3 | Nkx6-2 |
| NF-CLE0a | NF-MHCIIA | Nmi |
| NF-CLE0b | NF-MHCIIB | N-Myc |
| N-Oct-2alpha | Octamer- | p73alpha |
| N-Oct-2beta | Bindungsfaktor | p73beta |
| N-Oct-4 | Oct-B1 | p73delta |
| NOR1 | oct-B2 | p73epsilon |
| NOR1/MINOR | oct-B3 | p73eta |
| NPA3 | OLIG2 | p73gamma |
| NPAS1 | Oligo1 | p73kappa |
| NPAS2 | Otx1 | p73zeta |
| NP-TCII | Otx2 | Pax-1 |
| NRF | Otx3 | Pax-2 |
| Nrf1 | OZF | Pax-3 |
| NRF-1 | p107 | Pax-3A |
| Nrf1:MafG | p130 | Pax-3B |
| Nrf1:MafK | p160MBP | Pax-4a |
| Nrf2 | p28 Modulator | Pax-5 |
| Nrf2:MafG | p300 | Pax-6 |
| Nrf2:MafK | p38erg | Pax-6 / Pd-5a |
| NRF-2beta1 | p40x; HTLV-I, T-Zell | Pax-7 |
| NRF-2gamma1 | Lymphotropischer Virus | Pax-8 |
| Nrf3 | Typ I | Pax-8a |
| Nrf3:MafK | p45 | Pax-8b |
| N RL | p49erg | Pax-8c |
| NRSF | p50:c-Rel | Pax-8d |
| NRSF Form 1 | p53 | Pax-8e |
| NRSF Form 2 | p55 | Pax-8f |
| NTF | p55erg | Pax-9 |
| Nur77 | p63 | Pbx |
| NURR1 | p63alpha | Pbx1 |
| OAZ | p63beta | Pbx1:HoxB1 |
| OC-2 | p63delta | Pbx1:HoxB2 |
| OCA-B | p63gamma | Pbx1:HoxB3 |
| Octa-factor | p65delta | Pbx1:HoxB4 |
| | p73 | Pbx1:HoxB5 |
| Pbx1:HoxB6 | PEBP2beta | POU3F1 |
| Pbx1: HoxB8 | PGC-1 | POU3F2 |
| Pbx1:PKNOX1 | PITX1 | POU3F2 (N-Oct-5a) |
| Pbx1:Tcl3 | PITX2 | POU3F2 (N-Oct-5b) |
| Pbx1a | PITX2A | POU3F3 |
| Pbx1A:HoxA5 | PITX2A: Nkx2.5 | POU3F4 |
| Pbx1a:Hoxb7 | PITX2B | POU4F1(I) |
| Pbx1a:Hoxb8 | PITX2B:Nkx2.5 | POU4F1(s) |
| Pbx1a:Hoxc6 | PITX2C | POU4F2 |
| Pbx1A:HoxC8 | PITX2C:Nkx2.5 | POU4F3 |
| Pbx1A:HoxD4 | PITX3 | POU5F1 |
| Pbx1a:IPF1 | PKNOX1 | POU5F1A |
| Pbx1b | PKNOX2 | POU5F1B |
| Pbx1B:HoxA5 | PLAGL1 | POU5F1C |
| Pbx1B:HoxB7 | PLAGL2 | POU6F1 |
| Pbx1 B:HoxB8 | PLZF | PPAR-alpha |
| Pbx1B:HoxC8 | PML | PPAR-alpha:RXR- |
| Pbx1B:HoxD4 | PML-3 | alpha |
| Pbx1b:PKNOX1 | Pmx2a | PPAR-beta |
| Pbx2 | Pmx2b | PPAR-gamma1 |
| Pbx2:HoxB8 | PNR | PPAR-gamma2 |
| Pbx2:Hoxc6 | PO-B | PPAR-gamma3 |
| Pbx2:PKNOX1 | Pontin52 | PPAR-gamma4 |
| Pbx3a | POU1F1 | PPUR |
| Pbx3a:Hoxc6 | POU2F1 | PR |
| Pbx3b | POU2F2 | PRA |
| PC2 | POU2F2 (Oct-2.1) | PR B |
| PC4 | POU2F2B | pRb |
| PC5 | POU2F2C | PRDI-BF1 |
| PCAF | POU2F3 | PRDI-BFc |
| PDEF | POU2F3, Isoform a | Preb |
| PEA3 | POU2F3, Isoform d1 | Prop-1 |
| PEBP2alpha | POU2F3, Isoform d2 | PROX1 |
| PSE1 | RAR-gamma:RXR- | RSRFC4 |
| P-TEFb | alpha | RSRFC9 |
| PTF | RAR-gamma1 | RVF |
| PTFalpha | Rb:E2F-1:DP-1 | RX |
| PTFbeta | RBP60 | RXR-alpha |
| PTFdelta | RBP-Jkappa | RXR-beta |
| PTFgamma | Ref-1 | RXR-gamma |
| Pu box Bindungsfaktor | RelA | SAP-1a |
| Pu box Bindungsfaktor | RelB | SAP-1b |
| (BJA-B) | REVERB-alpha | SF-1 |
| PU.1 | REVERB-beta | SHOX2a |
| PuF | RFX1 | SHOX2b |
| Pur factor | RFX1:RFX2 | SHOXa |
| pX; HBV, Hepatitis B | RFX1:RFX3 | SHOXb |
| Virus | RFX2 | SHP |
| PXR-1 | RFX3 | SIII-p110 |
| PXR-1: RXR-alpha | RFX4 | SIII-p15 |
| PXR-1 :RXR-beta | RFX5 | SIII-p18 |
| PXR-2 | RFX5:RFXAP:RFXANK | SIM1 |
| R1 | RFXANK | SIM2 |
| R2 | RFXAP | SIP1 |
| RAR-alpha | RFX-B-delta5 | Six-1 |
| RAR-alpha:RXR-alpha | RF-Y | Six-2 |
| RAR-alpha:RXR-beta | RORalpha1 | Six-3 |
| RAR-alpha:RXR- | RORalpha2 | Six-4 |
| gamma | RORalpha3 | Six-5 |
| RAR-alpha1 | RORbeta | Six-6 |
| RAR-alpha2 | RORgamma | SKIP |
| RAR-beta | Rox | SLUG |
| RAR-beta:RXR-alpha | RP58 | Smad1 |
| RAR-beta2 | RPF1 | Smad2 |
| RAR-gamma | RPGalpha | Smad2 (437 Ami- |
| | RREB-1 | nosäuren) |
| Smad3 | Sp1 | SXR:RXR-alpha |
| Smad3:Smad4 | Sp2 | SYT |
| Smad4 | Sp3 | T3R-alpha |
| Smad4delta3 | Sp4 | T3R-alpha:RXR-alpha |
| Smad4delta4 | Spi-B | T3R-alpha1 |
| Smad4delta4-6 | SPT16 | T3R-alpha2 |
| Smad4delta4-7 | SRC-1 | T3R-beta1 |
| Smad4delta5-6 | SRC-3 | T3R-beta2 |
| Smad4delta6 | SRCAP | TAF(I)110 |
| Smad5 | SREBP-1a | TAF(I)48 |
| Smad6 | SREBP-1b | TAF(I)63 |
| Smad7 | SREBP-1c | TAF(II)100 |
| Smad8 | SREBP-2 | TAF(II)125 |
| SMIF | SRE-ZBP | TAF(II)135 |
| Sna | SRF | TAF(II)170 |
| SnoN | SRF:SRF | TAF(II)18 |
| Sox1 | SRY | TAF(II)20 |
| Sox10 | SSRP1 | TAF(II)250 |
| Sox11 | Staf-50 | TAF(II)250Delta |
| Sox12 | STAT1 | TAF(II)28 |
| Sox13 | STAT1:STAT1 | TAF(II)30 |
| Sox14 | STAT1:STAT3 | TAF(II)31 |
| Sox17 | STAT1alpha | TAF(II)55 |
| Sox18 | STAT1beta | TAF(II)70-alpha |
| Sox2 | STAT2 | TAF(I I)70-beta |
| Sox20 | STAT3 | TAF(II)70-gamma |
| Sox21 | STAT3:STAT3 | TAF-I |
| Sox3 | STAT4 | TAF-II |
| Sox4 | STAT5A | TAF-L |
| Sox5 | STAT5B | Tal-1 |
| Sox7 | STAT5B:STAT5B | Tal-1beta |
| Sox8 | STAT6 | Tal-2 |
| Sox9 | SXR | TAR factor |
| tat; HIV-1, Immunode- | TCF-1F | TFIIE-alpha |
| fizienz Virus Typ 1 | TCF-1G | TFIIE-beta |
| Tax; HTLV-I, T-Zell | TCF-2alpha | TFIIF |
| Lymphotropisches Vi- | TCF-3 | TFIIF-alpha |
| rus Typ I | TCF-4 | TFIIF-beta |
| T-bet | TCF-4(K) | TFIIH |
| TBP | TCF-4B | TFIIH* |
| Tbr-1 | TCF-4E | TFIIH-CAK |
| TBR2 | TEF | TFIIH-cyclin H |
| TBX18 | TEF-1 | TFIIH-MAT1 |
| TBX19 | TEF-2 | TFIIH-MO15 |
| TBX1A | TEF-3 | TFIIH-p34 |
| TBX1B | TEF-5 | TFIIH-p44 |
| TBX2 | TEL1 | TFIIH-p62 |
| TBX20 | Tel-2a | TFIIH-p80 |
| Tbx22 | Tel-2b | TFIIH-p80:CAK |
| TBX3 (722 Ami- | Tel-2c | TFIIH-p90 |
| nosäuren) | Tel-2d | TFII-I |
| TBX3 (742 Ami- | Tel-2e | TFIIIA |
| nosäuren) | Tel-2f | Tf-LF1 |
| TBX4 | TFE3 | Tf-LF2 |
| TBX5 (lange Isoform) | TFEB | TFP-95 |
| TBX5 (kurze Isoform) | TFEB-A | TGIF |
| Tbx5:Nkx2.5 | TFEC | TGIF2 |
| TBX6 | TFIIA | TGT3 |
| TCF | TFIIA-alpha/beta Vor- | TIEG-1 |
| TCF-1 | läufer (Hauptform) | TIF1a |
| TCF17 | TFIIA-alpha/beta Vor- | TIF1g |
| TCF-1A | läufer (Nebenform) | TIF2 |
| TCF-1B | TFIIA-gamma | TLE1 |
| TCF-1C | TFIIB | TLX |
| TCF-1D | TFIID | TLX3 |
| TCF-1E | TFIIE | TMF |
| TR2-11 | Vax-2 | ZER6 p52 |
| TR2-5 | VDR | ZER6 p71 |
| TR2-9 | VITF; Vacciniavirus/, | ZF1 |
| TR4 | Homo sapiens | ZF2 |
| TRAP | Vpr; HIV-1, Immunode- | ZFP-37 |
| TREB-1 | fizienz Virus Typ 1 | ZFX |
| TREB-2 | WBSCR14 | ZFY |
| TREB-3 | WSTF | ZHX1 |
| TREF1 | WT1 | ZIC2 |
| TREF2 | WT1 | ZID |
| TRF (2) | WT1 I-KTS | ZNF11a |
| TRRAP | WT1 I-del2 | ZNF124 |
| TWIST | WT1-KTS | ZNF133 |
| TxRE BP | WT1-del2 | ZNF143 |
| TxREF | XBP-1 | ZNF174 |
| UBF | XW,V | ZNF-20 |
| UBP-1 | YAF2 | ZNF-24 |
| UEF-1 | YB-1 | ZNF33a |
| UEF-2 | YEBP | ZNF35 |
| UEF-3 | YL-1 | ZNF43 |
| UEF-4 | YY1 | ZNF44 |
| USF1 | ZAC | ZNF45 |
| USF1:USF2 | ZBP89 | ZNF7 |
| USF2 | ZBP99 | ZNF76 |
| USF2b | ZEB (1124 AA) | ZNF83 |
| Vav | ZEB (1154 AA) | ZNF85 |

Eine weitere Gruppe von intrazellulären Rezeptoren sind die in der nachfolgenden Tabelle 2 dargestellten intrazellulären Zielmoleküle.

**Tabelle 2: Intrazelluläre Zielmoleküle**

| | | |
|---|---|---|
| "long-chain"-Fettsäuren- | Acetyl-CoA-Malat-Citrat- | Amyloid-Vorläuferprotein |
| CoA-Ligase | Synthase | Ankarin |
| "major basic"-Protein | Acetylglucosaminyl- | Arginase |
| "mixed function"- | Transferase | Argininosuccinate- |
| Oxygenase | Acetylspermin- | Synthetase |
| 11β-Hydroxylase (EC | Deacetylase | Argininosuccinat-Lyase |
| 1.14.15.4) | Acetyltransacylase | Aromatase |
| 18-Hydroxylase | Aconitase | Arylsulfatase |
| 1-Acylglycerol-3- | Actin | Aspartat- |
| phosphate Acyltransferase | Adenosin-Deaminase | Aminotransferase |
| 2,3- | Adenosylhomocysteine- | Aspartat- |
| Oxidosqualenlanosterol- | Hydrolase | Transcarbamoylase |
| Cyclase | Adenosylmethionine- | ATPase |
| 21-Steroid-Hydroxylase | Decarboxylase | ATP-Diphosphohydrolase |
| (EC 1.14.99.10) | Adenylatcyclase | *bcl*-2 Onkogen-Protein |
| 24,28-Sterolreduktase | Adenylatdeaminase | Bindegewebeaktivieren- |
| 3-Hydroxybutyrat- | Adenylatkinase | des Peptid |
| Dehydrogenase | Adenylsuccinate-Lyase | C5a-inaktivierender Fac- |
| 3-Ketothiolase | Adenylsuccinate-Synthase | tor |
| 3-β-Hydroxy-steroid- | Alanin-Aminotransferase | Calcitonin |
| Dehydrogenase | Aldolase | Calmodulin |
| (EC5.3.3.1) | Aldose-Reductase | Calpain I |
| 5'-Nucleotidase | Alkalische Phosphatase | Calreticulin |
| 8-Oxoguanosin- | Alkohol-Dehydrogenase | Carbamoylphosphat- |
| Deglykosylase | Amidophosphoribosyla- | Synthetase |
| *abl* Onkogen-Protein | min-Transferase | Carbonat-Anhydrase |
| Acetolactat-Synthase | AMP- | Caseinkinase 1 |
| Acetylcholinesterase | Phosphodiestererase | Caseinkinase 2 |
| Acetyl-CoA-Carboxylase | Amyloid β / A4-Protein | Catalase |
| Catechol- | Dihydrouracil- | Glycerinphosphat- |
| Methyltransferase | Dehydrogenase | Acyltransferase |
| Cathepsin | Dioxygenase | Glycerinphosphat- |
| Cathepsin B and L | Dopaminmonooxygenase | Dehydrogenase |
| cdc 10 | Dynenin | Glycinamide- |
| cdc 13 p60 | Elastase | Ribonucleotidtransfor- |
| cdc 2 p34 | Elastin | mylase |
| cdc 25 p80 | Elongation factor Tu | GTP bindendes Protein |
| Chaparonin | Endo-Rhamosidase | Hämoglobin A |
| Cholesterin-Esterase | Enolase | Hämoglobin A1 |
| Cholesterin- | Enoyl-ACP-Hydratase | Hämoglobin Barcelona |
| Monooxygenase | Enoyl-ACP-Reductase | Hämoglobin Barts |
| Citratsynthetase | *ets* Onkogen-Protein | Hämoglobin Beth Isreal |
| Clathrin | Ferritin | Hämoglobin Bunbury |
| Collagenase | Ferrodoxin | Hämoglobin Cochin-Port |
| Cortison-Dehydrogenase | Fettsäuresynthetase | Royal |
| *crk* Onkogen-Protein | fgr Onkogen-Protein | Hämoglobin Cowtown |
| Cyclin A and B | *fps* Onkogen-Protein | Hämoglobin Cranston |
| Cyclophilin | Fructosebisphosphat- | Hämoglobin Creteil |
| Cytidin-Deaminase | Aldolase | Hämoglobin D |
| Cytidylate-Deaminase | Fumarase | Hämoglobin D-Los Ange- |
| Cytochrom C-Peroxidase | GABA-Aminotransferase | les |
| Cytochrom P450 | Galactosidase | Hämoglobin D-Punjab |
| Cytosin- | Gelatinase | Hämoglobin F |
| Methyltransferase | Gelsolin | Hämoglobin Gower |
| *dbl* Onkogen-Protein | Glucophosphat-Isomerase | Hämoglobin Hammers- |
| Defensin | Glucosylceramid- | mith |
| Diacylglycerol- | Galactosyltransferase | Hämoglobin Hiroshima |
| Acyltransferase | Glutaminase | Hämoglobin Indianapolis |
| Dihydrofolate-Reductase | Glutamin- | Hämoglobin Kansas |
| Dihydroorotatase | Phosphoribosylpyropho- | Hämoglobin Kariya |
| Dihydroorotat- | sphat-Amidotransferase | Hämoglobin Kempsey |
| Dehydrogenase | | Hämoglobin Kenya |
| Hämoglobin Lepore | Hydroxymethylglutaryl- | Myeloperoxidase |
| Hämoglobin M | CoA-Reductase | Myofilament |
| Hämoglobin M Hyde | Hydroxymethylglutaryl- | Myristoyltransferase |
| Park | CoA-Sythetase | Na / K ATPase |
| Hämoglobin M Iwate | Hydroxysteroid- | N-Acetylglucuronidase |
| Hämoglobin M Saskatoon | Dehydrogenase | NAD-abhängige Sterol-4- |
| Hämoglobin Nancy | Hypoxanthine-Guanine- | carboxylase |
| Hämoglobin Philly | Phosphoribosyltransferase | NADase |
| Hämoglobin Quong Sze | IMP-Dehydrogenase | NADPH-abhängige 3- |
| Hämoglobin Raleigh | Indollyase | Oxosteroidreductase |
| Hämoglobin Ranier | Inositolphosphat- | Nexin |
| Hämoglobin S | Phosphatase | *N-ras* Onkogen-Protein |
| Hämoglobin Sealy | *int-1* Onkogen-Protein | Nukleolusprotein B23 |
| Hämoglobin Seattle | Isocitratlyase | Nukleosiddiphosphat- |
| Hämoglobin St. Louis | Kininbildendes Enzym | Kinase |
| Hämoglobin St. Mande | *Ki-ras* Onkogen-Protein | Omithin- |
| Hämoglobin Titusville | Lactat-Dehydrogenase | Aminotransferase |
| Hämoglobin Torino | Lactoferrin | Omithin- |
| Hämoglobin Wayne | Laminin | Carbamoyltransferase |
| Hämoglobin York | Leukozyten-Elastase | Ornithin-Decarboxylase |
| Hämoglobin Zurich | Lipocortin | Orotate-Decarboxylase |
| *Ha-ras* Onkogen-Protein | Lipoxygenase | Orotat- |
| Hexokinase | *L-myc* Onkogen-Protein | Phosphoribosyltransferase |
| Histaminase | Lysozym | p53 |
| Histidin-Decarboxylase | Malat-Dehydrogenase | Peptidylamidoglycolate- |
| HSP 27 | Malat-Synthase | Lyase |
| Hydropyrimidine- | Malonyltransacylase | Peptidyl-Prolyl-Isomerase |
| Hydrolase | Mannosidase | PF4 |
| Hydroxyacyl-CoA- | *met* Onkogen-Protein | Phenylalanine- |
| Dehydrogenase | Methämoglobin | Hydroxylase |
| Hydroxymethylglutaryl- | Methionin- | Phosphatidat-Phosphatase |
| CoA Spaltendes Enzyme | Adenosyltransferase | Phosphoenolpyruvate- |
| | mos Onkogen-Protein | Carboxykinase |
| Phosphofructokinase | *rel* Onkogen-Protein | tRNA-Synthetase |
| Phosphoglucokinase | Ribonucleotid-Reduktase | Tropomyosin |
| Phosphoglucomutase | Ribosephosphate- | Tryptophan-Synthase |
| Phosphoglycerate-Kinase | Pyrophosphat-Kinase | Tubulin |
| Phosphoglyceromutase | Ricintropoelastin | Tyrosinkinase |
| Phospholipase A2 | Saure Phosphatase | Ubioquinon-Reduktase |
| Phospholipase C | Saure Protease | UPA |
| Phospholipase CG1 | Schweres Meromyosin | Uridinmonophosphat- |
| Phospholipase D | Serine / Threonin-Kinase | Kinase |
| Phospholipase S | Spectrin | Vitamin K-Reduktase |
| Phosphoribomutase | Spermine-Synthase | *wee*-1 Genproduct |
| Phosphoribosylphosphat- | Squalene-Epoxidase | Xanthindehydrogenase |
| Transferase | Squalen-Monooxygenase | Xanthinoxidase |
| pim Onkogen-Protein | src Onkogen-Protein | Xylosyltransferase |
| Plasminogenaktivator- | Sterolmethyltransferase | *yes* Onkogen-Protein |
| Inhibitor | *suc* 1 p13 | α-Actin |
| Porin | Succinyl-CoA-Synthetase | α-Mannosidase |
| pRB (Retinoblastomgen- | Superoxid-Dismutase | α-Melogenin |
| Produkt) | Tartrat-Dehydrogenase | α-Tubulin |
| pRb Retinablastom- | Thioesterase | β-Actin |
| Genprodukt | Thioredoxin | β-Glucuronidase |
| Properdin | Thrombospondin | β-Glycerophosphatase |
| Prostaglandin-Synthase | Thromboxane-A2- | β-Ketoacyl-ACP- |
| Proteinkinase C | Synthetase | Reduktase |
| Purinnucleosid- | Thymidylat-synthetase | β-Ketoacyl-ACP- |
| Phosphorylase | Transacylase | Synthetase |
| PyruvateDehydrogenase | Triosephophat-Isomerase | β-Spectrin |
| Pyruvate-Kinase | Triosephosphate- | β-Tropomyosin |
| *raf* Onkogen-Protein | Dehyrogenase | β-Tubulin |

Eine weitere besonders bevorzugte Gruppe von intrazellulären Rezeptoren sind die HMG-Proteine wie beispielsweise in der internationalen Patentanmeldung PCT/EP96/00716 beschrieben und insbesondere die HMGA-Proteine. Wie hierin verwendet bezeichnet der Begriff der HMGA-Proteine bevorzugterweise summarisch die folgenden Proteine: HMGA1, HMGA1a, HMGA1b und HMGA2.

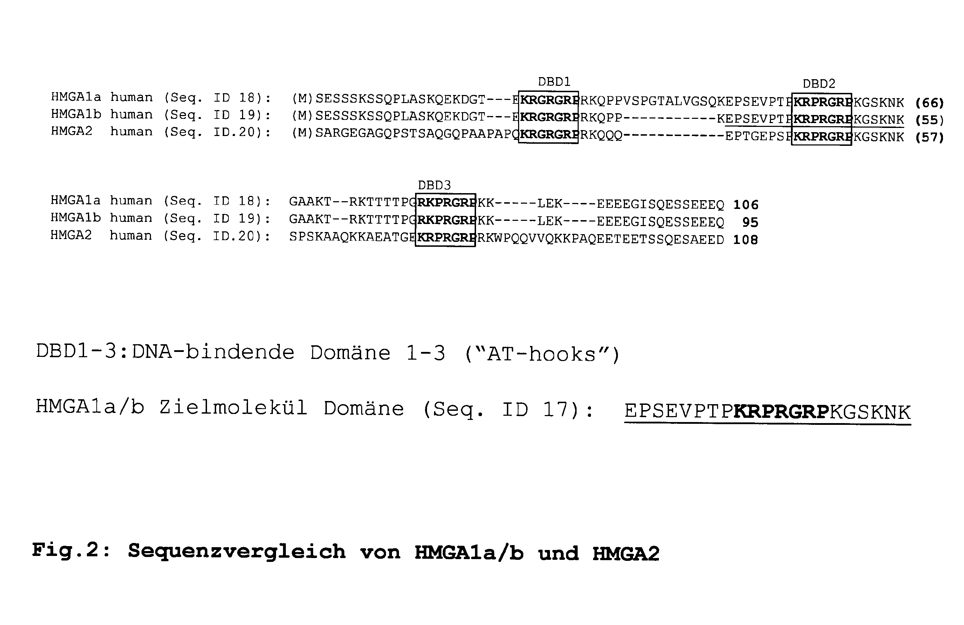

Die HMGA-Proteine sind modular aufgebaut und besitzen je drei DNA-bindende Domänen, die als "AT-hooks" oder als "AT-Haken" bezeichnet werden und als DBD1 bis DBD3 in Fig. 2 dargestellt sind, sowie einen sehr sauren C-terminalen Bereich. Es ist für den Fachmann offenkundig, dass Antagonisten, die an einen der "AT-hooks" binden, nicht nur die HMGA1-Proteine und dabei die beiden Splice-Varianten HMGA1a und HMGA1b erkennen (siehe Fig. 2), sondern auch Kreuzreaktivität mit ähnlichen DNA-bindenden Molekülen wie HMGA2 aufweisen. Außer HMGA2 besitzen noch viele weitere Proteine den "AT-hooks" ähnliche Sequenzen und stellen in jedem Falle weitere Rezeptoren dar. Solche Proteine sind unter anderen die in Tabelle 3 angegebenen:

**Tabelle 3:**

| Spalte 1: Proteindatenbank-Zugriffscodes; Spalte 2: Proteinbezeichnung | |
|---|---|
| Q9UKB0 | Humanes HMG-Protein-R |
| Q9UKY1 | ZHX1_Humanes Zinkfinger- und Homöobox-Protein 1 |
| P55198 | AF17_HUMAN AF-17 Protein [MLLT6] |
| Q59F28 | Humanes Trithorax Homologon (Fragment) |
| Q6PJQ2 | Humanes ZNF406 Protein (Fragment) |
| Q75PJ9 | Humanes ZFAT-1 Protein |
| Q75PJ7 | Humanes ZFAT-3 Protein |
| Q75PJ6 | Humanes TR-ZFAT Protein |
| Q9ULG1 | Humanes KIAA1259 Protein |
| Q9NUK2 | Humanes Hypothetisches Protein FLJ11314 |
| Q9NTG6 | Humanes Hypothetisches Protein DKFZp434B0616 |
| Q8IX01 | SFR14_HUMAN Mutmaßlicher Spleißfaktor |
| Q9H5J8 | Humanes Hypothetisches Protein FLJ23363 |
| Q6I9Y6 | Humanes MGC5306 Protein |
| Q8IX01-2 | Spleiß-Isoform 2 von Q8IX01 |
| Q8IX01-3 | Spleiß-Isoform 3 von Q8IX01 |
| Q8IX01-4 | Spleiß-Isoform 4 von Q8IX01 |
| Q15291 | RBBP5_HUMAN Retinoblastom-bindendes Protein 5 (RBBP-5) |
| P51608 | MECP2_HUMAN Methyl-CpG-bindendes Protein 2 |
| Q6IPE2 | Humanes FLJ12800 Protein |
| Q6QHH9 | Humanes Methyl-CpG-bindendes Protein 2, Isoform B |
| Q9H8H4 | Humanes Hypothetisches Protein FLJ13629 |
| Q7Z384 | Humanes Hypothetisches Protein DKFZp686A24160 |
| 042043 | ENK7_HUMAN HERV-K_1q23.3 Provirus |
| P61569 | ENK16_HUMAN HERV-K_10p14 Provirus |
| Q86VM3 | Humanes MYB bindendes Protein 1a [MYBBP1A] |
| Q9UNW3 | Humanes Hüllprotein RIC-2 |
| Q9BWE0 | Humanes REPIN1 Protein (Hypothetisches Protein ZNF464) |
| Q9ULL5 | Humanes KIAA1205 Protein |
| Q9NZH2 | Humanes Dhfr Oribeta-bindendes Protein RIP60 |
| Q9NZI3 | Humaner Linensepithel-enthaltener Wachstumsfaktor p52 |
| Q9NY27 | Humane regulatorische Untereinheit 2 der Proteinphosphatase-4 |
| Q86U91 | Humanes HMGA2/RAD51L1 Fusionsprotein |
| 095368 | Humaner Transkriptioneller Koaktivator p52 |
| Q9P015 | Humanes HSPC145 (Mitochondriales Ribosomenprotein L15) |
| Q5U071 | Humanes HMG Protein 'box 2' |
| Q9H0Y1 | Humanes Hypothetisches Protein DKFZp564I206 |
| Q6ZP45 | Humanes Hypothetisches Protein FLJ26517 |
| P17096-2 | Spleiß-Isoform HMG-Y von P17096 [HMGA1] |
| Q9Y6X0 | SETBP_HUMAN SET-bindendes Protein (SEB) [SETBP1] |
| Q8TEK3 | DOT1L_HUMAN Histon-Lysine N-Methyltransferase |
| Q8TEK3-2 | Spleiß-Isoform 1 von Q8TEK3 [DOT1L] |
| Q03164 | HRX_HUMAN Zinkfinger-Protein HRX (ALL-1) |
| Q86YP1 | Humaner Transkriptionsfaktor MLL UPN96240 |
| Q86YN9 | Humaner Transkriptionsfaktor MLL UPN95022 |
| Q03164-2 | Spleiß-Isoform 14P-18B von Q03164 [MLL] |
| P04920 | B3A2_HUMAN Anionenaustauscher-Protein 2 |
| Q59GF1 | Humane Anionenaustauscher-2 Typ a-Variante |
| Q8TAG3 | Humanes SLC4A2 Protein |
| Q6P391 | Humanes PSIP1 Protein |
| 075475 | Humaner Linensepithel-enthaltener Wachstumsfaktor p75 |
| Q9UEY6 | Humaner Anionenaustauscher-2 Typ a [SLC4A2] |
| Q9UEY5 | Humaner Anionenaustauscher-2 Typ b2 [SLC4A2] |
| Q9UEY4 | Humaner Anionenaustauscher-2 Typ b1[SLC4A2] |
| Q9UER6 | Humaner Transkriptioneller Koaktivator p75 |
| 000256 | Humanes DFS70 |
| P04920-2 | Spleiß-Isoform B1 von P04920 [SLC4A2] |
| Q9BTB1 | Humanes Hypothetisches Protein MGC10561 |
| Q9UKB0 | Humanes HMG Protein-R |
| 043167 | ZBT24_HUMAN Zinkfinger- und BTB-Domänen enthaltendes Protein |
| Q8N455 | Humanes ZBTB24 Protein [ZBTB24] |
| Q5TED5 | Humanes Zinkfinger-Protein 450 [ZNF450] |
| Q96CK0 | Humanes Zinkfinger-Protein 653 |
| Q96AS7 | Humanes Zinkfinger-Protein 653 |
| P51888 | PRELP_HUMAN Prolargin-Vorläufer |
| Q5JPC9 | Humanes Hypothetisches Protein DKFZp667H216 |
| Q6FHG6 | Humanes PRELP-Potein |
| Q6ZR44 | Humanes Hypothetisches Protein FLJ46672 |
| Q8NEZ4 | MLL3_HUMAN Myeloid/lymphoid-Leukämieprotein 3 Homologon |
| Q96AC6 | KIFC2_HUMAN Kinesin-ähnliches Protein KIFC2 |
| Q9C0H5 | K1688_HUMAN Protein KIAA1688 |
| P52926 | HMGIC_HUMAN HMG Protein I-C |
| Q9UKV3 | ACINU_HUMAN Induktor apoptotischer Chromatinkondensation |
| Q59F82 | Humane C21orf2-Proteinvariante |
| Q5VYT7 | Humanes OTTHUMP00000021181 |
| Q96M56 | Humanes Hypothetisches Protein FLJ32810 |
| Q69YJ6 | Humanes Hypothetisches Protein DKFZp667N107 |
| Q8NEY3 | SPAT4_HUMAN Spermatogenes-Assoziiertes Protein 4 |
| Q12809 | KCNH2_HUMAN Kalium Potential-gesteuerte Ionenkanal-Subfamilie |
| Q8IYY4 | Humanes dem DAZ-interagierenden Protein 1-ähnliches Protein [DZIP1L] |
| Q6ZN04 | Humanes Hypothetisches Protein FLJ16544 |
| Q5SXN7 | Humanes Serologisch definiertes Kolonkrebs-Antigen 3 |
| Q8IVG2 | Humanes KIAA2009 Protein (Fragment) [RKHD3] |
| Q75VX8 | Humanes KIAA2038 Protein (Fragment) [KIAA2038] |
| Q12809-2 | Spleiß-Isoform 2 von Q12809 [KCNH2] |

Vor diesem Hintergrund betrifft die vorliegende Erfindung auch L-Nukleinsäuren und insbesondere Spiegelmere, die gegen ein jegliches der in den Tabellen 1 bis 3 genannten Zielmoleküle gerichtet sind.

Indem die L-Nukleinsäure als intrazellulär aktives Agens insbesondere innerhalb einer Zelle verwendet wird, um dort an einen intrazellulären Rezeptor zu binden, können intrazellulär verschiedene Formen der Wechselwirkungen zwischen dem intrazellulären Rezeptor und seinen Wechselwirkungspartnern beeinflusst werden. In Abhängigkeit von der Art der Wechselwirkungspartner des intrazellulären Rezeptors erlaubt somit die intrazelluäre Verwendung von L-Nukleinsäuren die Beeinflussung von Wechselwirkungen von Proteinen, Nukleinsäuren, Lipiden, Kohlenhydraten, oder Kombinationen von Proteinen, Nukleinsäuren, Lipiden, Kohlenhydraten miteinander und untereinander.

Im Zusammenhang mit der erfindungsgemäßen Verwendung einer L-Nukleinsäure, insbesondere eines Spiegelmers, als intrazelluläres Agens bzw. dem Verfahren zum Binden eines intrazellulärem Rezeptors ist beachtlich, dass es sich dabei bevorzugterweise im eine in vitro Anwendung bzw. ein in vitro Verfahren handelt.

Im Zusammenhang mit der erfindungsgemäßem Verwendung einer L-Nukleinsäure, insbesondere eines Spiegelmers, zur Herstellung eines Medikamentes für die Behandlung und/oder Prävention einer Erkrankung und/oder zur Herstellung eines Mittels zur Diagnose ist das Zielmolekül ein intrazelluläres Zielmolekül. In diesem Zusammenhang ist das intrazelluläre Zielmolekül ein solches, das an der zu verhindernden, zu therapierenden oder zu diagnostizierenden Erkrankung oder Krankheit kausal oder nicht-kausal beteiligt ist, in einem jeden Fall aber dessen Bindung an eine daran spezifisch bindende L-Nukleinsäure dazu führt, dass im Falle eines Medikamentes die Erkrankung gelindert, verhindert, oder geheilt wird und/oder im Falle eines diagnostischen Mittels die Erkrankung oder eine Disposition festgestellt oder diagnostiziert werden kann. Wie hierin verwendet umfasst der Begriff der Diagnose eine Erstdiagnose ebenso wie nachfolgende Diagnosen, insbesondere Diagnosen oder Untersuchungen, um, bspw., den Verlauf der Erkrankung oder die Stadien der Erkrankung zu verfolgen oder zu bestimmen. Es ist im Rahmen der Erfindung, dass das Zielmolekül ein intrazellulärer Rezeptor wie hierin beschrieben ist, insbesondere ein Transkriptionsfaktor, ein intrazelluläres Zielmolekül oder ein HMG-Protein. Es ist im Rahmen der vorliegenden Erfindung ganz besonders bevorzugt, dass das Zielmolekül intrazellulär vorliegt, d.h. innerhalb einer Zelle und die auf die Erkrankung und/oder Diagnostik Einfluss nehmende Wechselwirkung zwischen der L-Nukleinsäure und insbesondere dem Spiegelmer und dem Zielmolekül, d. h. dem Rezeptor, intrazellulär erfolgt. Es ist jedoch auch im Rahmen der vorliegenden Erfindung, wenn das Zielmolekül außerhalb einer Zelle vorliegt und die Wechselwirkung zwischen der L-Nukleinsäure und insbesondere dem Spiegelmer und dem Zielmolekül, d. h. dem Rezeptor, extrazellulär erfolgt.

Die indikationsmäßige Anwendung des unter Verwendung einer L-Nukleinsäure hergestellten Medikamentes, wobei die Nukleinsäure gegen ein intrazelluläres Zielmolekül gerichtet ist, ergibt sich für den Fachmann aus der Beteiligung des intrazellulären Zielmoleküls an dem der jeweiligen Indikation zugrundeliegenden Pathogenitätsmechanismus. So ist bspw. für HMGA-Proteine bekannt, dass diese mit Karzinomen (u.a. von Brust, Lunge, Haut, Schilddrüse) sowie Leukämien und Lymphomen sowie anderen malignen Tumoren wie u.a. Sarkomen (Rhabdomyosarkom, Osteosarkom) assoziiert sind. Ausserdem werden sie in vielerlei mesenchymalen Tumoren, u.a. Hamartome (Brust und Lunge), Fettgewebstumoren (Lipome), pleomorphen Adenomen der Kopfspeicheldrüsen, Uterus-Leiomyomen, Angiomyxomen, Fibroadenomen der Brust, Polypen des Endometriums und atherosklerotischen Plaques exprimiert. HMGA ist ein interessantes therapeutisches Target. Blockade von HMGA könnte ein geeigneter Ansatzpunkt zur Kontrolle des Krebses sein und seine metastatische Verbreitung verhindern. Wie detaillierter hierin beschrieben sind gegen HMGA-Proteine gerichtete L-Nukleinsäuren auch für die Diagnose und/oder Therapie von Viruserkrankungen und Arteriosklerose geeignet infolge der Beteiligung der HMGA-Proteine an der Regulation der Transkription einer Vielzahl viraler Gene bzw. der starken Expression von HMGA und insbesondere HMGA1 in dem von Areteriosklerose betroffenen Gewebe, das mit neointimalen, vaskuläre glatten Muskelzellen, Makrophagen und neuen Blutgefäßen assoziiert ist

Obwohl - wie von den vorliegenden Erfindern überraschend festgestellt - Nukleinsäuren, bevorzugt L-Nukleinsäuren und bevorzugterweise Spiegelmere in der Lage sind, als solches eine Phospholipid-Doppelmembran wie eine Cytoplasmamembran zu durchdringen und dann intrazellulär funktional zu sein im Sinne der - spezifischen - Wechselwirkung mit dem intrazellulären Rezeptor, kann die Wirksamkeit der Einschleusung der L-Nukleinsäure durch die Verwendung verschiedener Techniken beeinflusst und insbesondere erhöht werden. Zu diesen Techniken gehört die Verwendung von chemischen Verbindungen oder Molekülen ebenso wie die Verwendung von physikalischen Maßnahmen. Unabhängig von der Art werden diese Techniken hierin allgemein als Abgabevehikel bezeichnet. Es ist im Rahmen der vorliegenden Erfindung, dass die Erfinder ebenfalls festgestellt haben, dass auch Aptamere diese Eigenschaft aufweisen und wie die somit Spiegelmere ebenfalls zusammen mit der erfindungsgemäßen Zusammensetzung zu den grundsätzlich gleichen Zwecken, Anwendungen und Verwendungen verwendet oder herangezogen werden können.

Bei der Verwendung von chemischen Verbindungen bzw. Molekülen kann weiter unterschieden werden, ob die Nukleinsäure für die Abgabe modifiziert werden muss, oder ob sie nicht modifiziert werden muss. Eine Modifikation zu Zwecken der Verwendung eines Abgabevehikels ist typischerweise nicht erforderlich, wenn das Abgabevehikel ein Vesikel darstellt oder umfasst, wie beispielsweise bei Liposomen, Polypeptid-Vehikeln, Cyclodextrinen, Dendrimeren, Nano- und Mikropartikeln sowie Polyethylenimin. Eine Modifikation zu Zwecken der Verwendung eines Abgabevehikels ist dagegen typischerweise erforderlich wenn das Abgabevehikel Rezeptor-vermittelte Endozytose, fusogene Peptide, Signalpeptide oder lipophile Konjugate verwendet. Die Gruppe der physikalischen Techniken umfasst insbesondere Elektroporation und Iontophorese. Es wird anerkannt werden, dass den Fachleuten auf dem Gebiet weitere Techniken bekannt sind, um eine Verbindung über eine Phospholipid-Doppelmembran wie eine Cytoplasmamembran zu transportieren, die grundsätzlich auch für den Transfer eine funktionalen Nukleinsäure wie beispielsweise eines Aptamers und/oder eines Spielgelmers geeignet sind.

Im folgenden sollen die einzelnen Abgabevehikel, die im Rahmen der verschiedenen Aspekte der vorliegenden Erfindung verwendet werden können, detaillierter beschrieben werden.

Liposomen bestehen aus künstlichen kationischen Lipiden wie N-[1-(2,3-dioleoyloxy) propyl]-N,N,N-trimethylammoniumchlorid (DOTMA) und N-[1-(2,3-dioleoyloxy) propyl]-N,N,N-trimethylammoniumsulfat (DOTAP), in denen die kationischen Gruppen mit den negativ geladenen Nukleinsäuren wechselwirken und deren anionischen Ladung neutralisieren.
Der Transport erfolgt über Endozytose (PNAS, 93:11493-11498, 1996). Allerdings sind kationische Liposomen zytotoxisch, insbesondere in höheren Konzentrationen, was ihre Anwendung *in vitro* und *in vivo* begrenzt (Biochem Biophys Res Commun, 197:818, 1993; Biochem Biophys Res Commun, 1372:55-68, 1998). Eine nicht toxische Formulierung stellt dagegen das amphiphile Pyridinium-basierte Lipid SAINT-2 dar (Nucleic Acids Res, 29:2079-2087, 2001). Eine Alternative stellen auch pH-sensitive Liposomen dar, die aus amphipatischen Molekülen wie Cholesterylhemisuccinat (CHEMS) und Dioleylphosphatidylethanolamin (DOPE) bestehen (J Pharmacol Exp Ther, 297:1129-1136, 2001). Verschiedenste Formulierungen von Liposomen finden sich in den Übersichtsartikeln von Dass und Torchili (Drug Delivery, 9:169-180, 2002; Nat Rev Drug Disc, 4:145-160, 2005).

Bei der Rezeptor-vermittelten Endozytose (RME) werden Transportmechanismen, die bereits in der Zellmembran vorhanden sind, genutzt. Dazu wird die Nukleinsäure beispielsweise über einen Poly-L-lysin (PPL)-Linker kovalent mit einem Transporter-Protein ("Carrier"-Protein) verknüpft. Die Auswahl des Transporter-Proteins ist dabei abhängig von der Fähigkeit an bestimmte Rezeptoren der Zellmembran zu binden und in der Zelle durch Endozytose zu akkumulieren. So kann ein zellspezifischer Transport realisiert werden. So konnte beispielsweise ein gegen c-*myc* gerichtetes Antisense-Phosphorothioat in M-14 humane Melanom-Zellen geschleust werden (Anticancer Res, 17:29-35, 1997). Allerdings hängt ein effektiver Transport mittels RME dabei nicht nur von der Affinität des Rezeptors für den Liganden sondern auch von der Limitierung des ausgewählten Rezeptors bezüglich der Zellen - speziell in vivo - ab. Des weiteren muss der ausgewählte Ligand inaktiv oder verstärkend bezogen auf das therapeutischen Ergebnis sein, um eine mögliche Toxizität des Transportvehikels zu vermeiden. So ist die Auswahl und die ubiquitäre Verbreitung des ausgewählten Rezeptors *in vivo* entscheidend für einen erfolgreichen RME-basierten Transport. Des weiteren konnte eine Sequestration von Nukleinsäuren in endosomalen Kompartments bei RME-basiertem Transport beobachtet werden, was diese Methode für einen intrazellulären Transport bzw. eine intrazelluläre Abgabe oder Delivery wenig vielversprechend erscheinen lässt. Zuallerletzt muss die Verknüpfung zwischen Rezeptor und Nukleinsäure so gewählt werden, dass sowohl die Funktion des einen oder des anderen nicht reduziert wird (J Pharmaceutical Science, 92 (8):1559-1573, 2003).

Fusogene Peptide sind verwendet worden, um Peptid-Oligonukleotid-Konjugaten die Fusion mit der Zellmembran zu ermöglichen und so den Transport in die Zelle zu realisieren (Bioconjug Chem, 9: 466-475, 1998; Bioconjug Chem, 6:43-53, 1995; Nucleic Acids Research, 25:2730-2736, 1997).

Der selektive Import von Kernproteinen aus dem Zytosol in den Nukleus wird durch kurze Peptidsequenzen, die "Nuclear localization signals" (NLS) genannt werden, vermittelt. So können verschiedene NLS-Peptidderivate verwendet werden, um Nukleinsäuren in den Nukleus zu transportieren (Bioconjug Chem, 10:1005-1012, 1999; Bioconjug Chem, 10:598-606, 1999; Bioconjug Chem, 6:101-108, 1995). Zudem gibt es auch sogenannte "Signal import peptides" (IP), die die zelluläre Aufnahme von Nukleinsäuren befördern können und beispielsweise aus dem Kaposi's Fibroblastenwachstumsfaktor (K-FGF) abgeleitet werden konnten (Adv Drug Deliv Rev, 44:35-49, 2000).

Durch Blöcke von Polypeptiden können Vesikel analog zu viralen Kapsiden ausgebildet werden, die als mögliche Transportvehikel für einen intrazellulären Transport dienen (Nat Materials, 3(4):244-8, 2004).

Der hydrophile Charakter von Oligonukleotiden und das anionische Phosphodiester-Rückgrat reduzieren die zelluläre Permeation. Daher stellen lipophile Konjugate eine Möglichkeit dar, die Fähigkeit von Oligonukleotiden zu erhöhen, an Lipoproteine zu binden und dadurch die intrazelluläre Delivery zu verbessern. Das am besten untersuchte Konjugat ist das Cholesterin (Antisense and Nucleic Acid Drug Development, 12:103-128, 2002).

Cyclodextrine sind zyklische Oligosaccharide, die eine zentrale hydrophobe Kavität und multiple Hydroxylgruppen an der äußeren Oberfläche aufweisen. Cyclodextrine konnten so bereits für den Transport von Antisense-Oligonukleotiden in humane T- Zelllinien (Antisense Res Dev, 5:185-192, 1995) und auch *in vivo* zum intrazellulären Transport bzw. zur intrazellulären Abgabe oder Delivery von immunogenen CpG-Sequenzen verwendet werden (Biochem Pharmacol, 52:1537-1544, 1996). Verschiedenste Formulierungen von Cycloodextrinen finden sich in dem Übersichtsartikel von Davis und Brewster (Nature Reviews Drug Discovery 3:1023-1035, 2004).

Dendrimere sind hochverzweigte Makromoleküle, die sich aus repetitiven Einheiten von - typischerweise - Polyamiden zusammensetzen. Die Moleküle tragen auf ihrer Oberfläche funktionelle Gruppen wie primäre Aminogruppen, die mit anderen Molekülen durch elektrostatische Interaktion wechselwirken. Dadurch findet schnell und hoch reproduzierbar eine komplexe Formbildung statt, die zu Komplexen mit schwacher Zytotoxizität führt (Nucleic Acids Research, 28:4225-4231, 2000; Clin Cancer Res, 7:3606-3612, 2001;)

Zyanacrylatnanopartikel sind seit Beginn der 1990er Jahre für die Abgabe oder Delivery von Oligonukleotiden getestet worden. Die Wechselwirkung von Oligonukleotiden zu den Nanopartikeln erfolgt durch Ionen-Paare der anionischen Ladung der Oligonukleotide mit verschiedenen hydrophoben Kationen vorrangig mit geladenden Nanopartikeln. Typischerweise werden zur Bildung von Nanopartiklen Polyisohexyl-(PIHCA), Polyisobutyl-(PIBCA) oder Polyhexycyanoacrylate (PHCA) verwendet, obwohl auch eine Vielzahl von lipophilen Kation-Oligonukleotid-Paaren getestet wurde (Pharm Res., 1:1370-1378, 1994; PNAS, 91:10460-10464, 1994; Pharm Res, 9:441-449, 1992). Auch zur *in vivo* Anwendung wurden Nanopartikel bereits eingesetzt (Biochem Biophys Res Commun, 279:401-406, 2000; Pharm Res, 13:38-43, 1996).

Mikropartikel oder sogenannte Mikrokugeln ("microspheres") sind typischerweise aus bioabbaubaren Polymeren Poly(d,l-laktid-co-glykolide [P(LA-GA)] und werden zum verzögerten Abgabe von Oligonukleotiden eingesetzt (J Pharm Sci, 91:790-799; 2000; J Controlled Release, 69:197-207, 2000; J Drug Traget, 5:291-302, 1998)

Elektroporation ist eine Transporttechnologie, die ein starkes elektrisches Feld benutzt, um die Lipiddoppelmembran zu destabilisieren, dadurch die Zellmembran zu permeabilisieren und so einen Transport der zu verabreichenden Substanz, die auch ionisiert vorliegen kann (Iontophorese), in die Zelle zu ermöglichen. Elektroporation ist bereits erfolgreich verwendet worden, um transdermalen Transport von Oligonukleotiden *ex vivo* sowie *in vivo* zu ermöglichen (Int J Pharm, 184:147-156, 1999; J Drug Traget, 5:275-289, 1998; Pharm Res, 15:1596-1602, 1998; Int J Cancer, 85:260-266, 2000; Biochem Biophys Res Commun, 212:286-292, 1995; Blood, 88:731-741, 1996).

Durch Aufwendung von hohem Druck kann die Aufnahme "nackter" Oligonukleotide in Zellen *in vitro* und *ex vivo* verbessert werden. Die Notwendigkeit geschlossener Systeme zur Verwendung dieser Technologie lässt nur eine Verwendung für *ex vivo* Anwendungen zu (PNAS, 96:6411-6416, 1999; Hum Gene Ther, 10:2355-2664, 1999).

Auch die Verwendung von Schockwellen, akustischen Hochdruckpulsen, ermöglicht den Transport von Oligonukleotiden in Zellen (J Mol Med, 79:306-313, 2001; Cancer Res, 58:219-221, 1998). Ultraschall ist ebenfalls eine akustische Technolgie vergleichbar zu den Schockwellen, benutzt allerdings höhere Frequenzbereiche (MHz statt Hz) und kürzere Verwendungszeiten (Sekunden bis Minuten) und wurde bereits unterstützend für gentherapeutische Ansätze verwende (Hum Gene Ther, 7:1339-1346, 1996; Invest Radiol, 32:723-727, 1997; Ultrasound Med Bio, 25:1451-1457, 1999).

In einem weiteren Aspekt der vorliegenden Erfindung wird ein neues Abgabevehikel bereitgestellt, das insbesondere für den Transport von funktionalen Nukleinsäuren wie Aptameren, bevorzugt funktionalen L-Nukleinsäuren und ganz besonders bevorzugt Spiegelmeren geeignet ist. Das Abgabevehikel ist dabei eine mizellenartige oder liposomenartige Struktur auf der Grundlage von Polyethylenimin. Ohne darauf im folgenden festgelegt sein zu wollen, gehen die vorliegenden Erfinder derzeit davon aus, dass die Nukleinsäure in der micellenartigen oder liposomenartigen Struktur eingebettet oder enthalten vorliegt. Polyethylenimin kann grundsätzlich als lineares oder verzweigtes Polyethylenimin vorliegen und auch verwendet werden, wobei verzweigtes Polyethylenimin in der verzweigten Form besonders bevorzugt ist. Des weiteren kann Polyethylenimin als hochmolekulares oder niedermolekulares Polyethylenimin vorliegen und auch verwendet werden. Bevorzugterweise weist hochmolekulares Polyethylenimin ein Molekulargewicht von etwa 800 kDa auf und niedermolekulares Polyethylenimin ein Molekulargewicht von etwa 3 kDa auf. Im Rahmen der vorliegenden Erfindung wird ein Polyethylenimin mit einem mittleren Molekulargewicht von etwa 25 kDa bevorzugt, wobei es sich dabei bevorzugtererweise um ein verzweigtes Polyethylenimin mit einem Molekulargewicht von etwa 25 kDa handelt.

Obgleich es für ein Funktionieren nicht obligatorisch, ist es doch bevorzugt, dass bei dem erfindungsgemäßen Abgabevehikel die abzugebende Nukleinsäure selbst auch eine Modifikation trägt. Dabei ist es bevorzugt, wenn die Modifikation aus der Gruppe ausgewählt ist, die PEG-Reste umfasst. Dabei ist weiter bevorzugt, wenn der PEG-Rest ein Molekulargewicht von etwa 1000 bis 10000 Da, bevorzugtererweise etwa von 1200 bis 5000 Da und weiter bevorzugtererweise etwa von 1500 bis 2500 Da und am bevorzugtesten von etwa 2000 Da aufweist.

Bei der Mischung der Nukleinsäure mit dem Abgabevehikel zur Herstellung einer erfindungsgemäßen Zusammensetzung wird ein Quotient aus Gesamtanzahl der Stickstoffgruppen des Polyethylenimins und Gesamtanzahl der Phoshpatgruppen der mit oder durch das Abgabevehikel abzugebenden oder zu verpackenden Nukleinsäure von etwa 1 bis 20, bevorzugterweise von etwa 1,5 bis 10, bevorzugtererweise von 2 bis 5 und am bevorzugtesten von etwa 2 bis 3 eingestellt bzw. realisiert.

Das erfindungsgemäße Abgabevehikel erlaubt somit, auch für funktionale Nukleinsäure wie Aptamere und insbesondere L-Nukleinsäuren wie Spiegelmere den Mechanismus des intrazellulären Transports von Nukleinsäuren über Kondensation bzw. Verpackung mit geladenen Partikeln oder Reagenzien und damit verbundene Änderung der Ladung des Gesamtkomplexes zu nutzen. Dieser Komplex wird leicht über Endozytose aufgenommen und gelangt somit in das Zytosol der Zelle. Ein Nachteil dieser Methode ist die Stabilität der DNA/ RNA bzw. das Freisetzen der Nukleinsäure aus dem endosomalen Kompartiment. Im Zytosol der Zelle entsteht aus dem eingeschnürten Endosom durch das Einbringen von Proteasen bzw. Nukleasen und durch Protonierung des Kompartiments schnell ein Lysosom. Nukleasen verdauen dort die Nukleinsäuren. Das gilt jedoch nicht für Spiegelmere, da diese durch ihre unnatürliche Konfiguration nukleasestabil sind. Zudem sind Nukleinsäuren im saurem Milieu des Lysosoms nicht stabil. Allerdings gilt dies eher für Nukleinsäuren aus DNA, weniger für Nukleinsäuren, die aus RNA aufgebaut sind. Über Exozytose bzw. den Abbau im Golgi-Apparat wird der gesamte Komplex schnell wieder aus der Zelle transportiert und somit gelangen nur wenige Nukleinsäuren in die Zelle. Eine der Herausforderung an ein geeignetes Transfektionssystem ist somit die Stabilisierung, sowie die Freisetzung der Nukleinsäure aus den Endosomen in das Zytosol. Hinsichtlich der Stabilität haben RNA-Spiegelmere ideale Eigenschaften für eine Transfektion von eukaryotischen Zellen, da sie als Enantiomere nicht von Enzymen gespalten werden.

Die erfindungsgemäße Verwendung der L-Nukleinsäuren und insbesondere im Zusammenhang mit der erfindungsgemäßen Zusammensetzung ist gerade für diese Wirkstoffklasse von Bedeutung, da deren Wirkmechanismus auf einem stöchiometrischen Ansatz und nicht auf einem katalytischen Ansatz beruht, bei dem bereits die intrazelluläre Abgabe von einigen wenigen Molekülen ausreichend ist, um die gewünschte Wirkung zu erzielen. Insoweit wird mit der vorliegenden Erfindung ein bisher durch die Techniken des Standes der Technik nicht befriedigter Bedarf befriedigt.

Das mit den erfindungsgemäßen Abgabevehikeln bereitgestellte bzw. ausgebildete erfindungsgemäße Transfektionssystem basiert auf der Bildung von Mizellen aus Nukleinsäuren und verzweigtem Polyethylenimin (PEI). Das Phosphodiesterrückgrat der Nukleinsäuren interagiert mit den freien Stickstoff-Positionen des PEI und bildet über Querverzweigung kleine Mizellen, welche aufgrund des PEI eine positive Ladung haben. Diese Mizellen werden leicht durch Einschnürung der Plasmamembran als Endosomen von einer Zelle aufgenommen. Das PEI puffert nun einströmende Protonen ab, woraufhin viele Chlorid-Ionen im Inneren des Endosoms zu einem Anschwellen des Kompartiments aufgrund des osmotischen Drucks führen. Dieser Effekt des PEI wird als Protonen-Schwamm-Effekt in der Literatur beschrieben und führt letztlich zum Platzen des Endosoms und zur Freisetzung der Spiegelmere in das Zytosol. (Pharm Res, 22 (3): 373-80, 2005; Eur J Cell Biol 83 (3): 97-111, 2004; Gene Ther 9(24):1700-7, 2002).

Es ist im Rahmen der vorliegenden Erfindung, dass die erfindungsgemäße Zusammensetzung als Aerosol verabreicht wird.

Des Weiteren können Spiegelmere mit Signalpeptiden für die intrazelluläre, sowie intranukleäre Abgabe (engl. "Delivery"), als auch für die Organ spezifische Delivery derivatisiert werden. Eine Kopplung von Signalpeptiden direkt an das Polyethylenimin kann für eine gerichte Lokalisation zu Organen oder innerhalb der Zelle verwendet werden.

In einem noch weiteren Aspekt betrifft die vorliegende Erfindung L-Nukleinsäuren, insbesondere Spiegelmere und noch bevorzugter RNA-Spiegelmere, die gegen HMGA-Proteine gerichtet sind. Die hierin offenbarten Spiegelmere gegen HMGA-Proteine stellen insbesondere Beispiele dar für die der vorliegenden Anmeldung ebenfalls zugrundeliegende Erkenntnis, dass L-Nukleinsäuren und insbesondere Spiegelmere in der Lage sind, eine Phospholipid-Doppelmembran bzw. eine Cytoplasmamembran einer Zelle zu überwinden und intrazellulär mit dem intrazellulären Rezeptor, auf dessen spezifische Bindung hin sie selektiert worden sind, zu binden. Hinsichtlich der Ausgestaltung der HMGA-Proteine und der dagegen gerichteten L-Nukleinsäuren gilt das hierin zu der intrazellulären Verwendung von L-Nukleinsäure offenbarte auch im Zusammenhang mit dem vorliegenden Aspekt der Erfindung (und vice versa) und wird zur Vermeidung unnötiger Wiederholungen an dieser Stelle durch Bezugnahme wiederholt.

Die HMG (high mobility group)-Familie von DNA-bindenden Phosphoproteinen sind als Nicht-Histon-Komponenten des Chromatins ubiquitär in Säugerzellen vorhanden (Grosschedl et al. 1994). Die basischen HMG-Proteine werden in drei verschiedene Familien eingeteilt - die HMGB- (früher: HMG-1/-2), die HMGN- (früher: HMG-14/-17), und die HMGA-Familie (früher: HMG-IY/C). Jede HMG-Familie besitzt ihr charakteristisches funktionelles Sequenzmotiv: die "HMG-box" (HMGB-Familie), die "nucleosomal binding domain" (HMGN-Familie), bzw. den "AT-hook" (HMGA-Familie).

Zum gegenwärtigen Stand umfasst die HMGA-Familie zwei Gene, *HMGA1* und *HMGA2.* Von *HMGA1* können durch alternatives *splicing* drei verschiedene Proteine exprimiert werden (HMGA1a [früher: HMG-I], HMGA1b [früher: HMG-Y], HMGA1c [früher: HMG-I/R]), von HMGA2 nur eines (HMGA2 [früher: HMGI-C]). HMGA1a, HMGA1b und HMGA2 sind Polypeptide von ungefähr 100 Aminosäuren Länge und weisen eine modulare Sequenzorganisation auf: sie besitzen drei stark basische Regionen ("AT-hook") die die schmale kleine Furche von doppelsträngiger AT-reicher DNA binden (Reeves & Nissen 1990). Der C-Terminus dagegen enthält viele saure Aminosäuren. Die Proteine besitzen frei in Lösung keine stabile Sekundärstruktur und nehmen erst eine definierte Konformation an, wenn sie im Komplex mit DNA oder anderen Proteinen vorliegen (Huth et al 1997). HMGA-Proteine gehören zu den am stärksten modifizierten Proteinen im Säugerzellkern und werden phosphoryliert, acetyliert, und methyliert (Reeves & Beckerbauer 2001).

Die HMGA-Proteine besitzen *per se* keine transkriptionelle Aktivität, organisieren aber als s.g. architektonische Transkriptionsfaktoren durch ihre Protein-Protein sowie Protein-DNA-Interaktionen die Ausbildung des Nukleoprotein-DNA-Transkriptionskomplexes (Wolffe 1994). Sie nehmen somit regulatorisch auf die Expression einer Vielzahl von Genen einen aktivierenden oder inhibitorischen Einfluß. Das prominenteste Beispiel einer positiven Regulation ist die Involvierung von HMGA1 in die Regulation des IFN-β (Thanos & Maniatis, 1992). So stimuliert beispielsweise im Falle des IFN-β-Promotors das HMGA1b die Bindung von NF-κB und ATF-2 an die DNA-Doppelhelix und verändert gleichzeitig die DNA-Struktur dergestalt, dass NF-κB und ATF-2 miteinander und vermutlich auch mit der restlichen Transkriptionsmaschinerie interagieren können (Thanos & Maniatis 1992, Du et al 1993). Ein weiterer transkriptionsaktivierender Effekt im Zusammenhang mit der arteriosklerotischen Pathogenese ist die durch HMGA1 induzierte *CD44*-Genregulation (Foster et al 1998). CD44 ist ein Zelloberflächen-Glykoprotein und in die Migration und Proliferation glatter Muskelzellen nach Endothelverletzung involviert (Jain et al 1996, Cuff et al 2001). Die transkriptionelle Regulation von *CD44* wird durch die Bindung von c-Fos und c-Jun an die AP-1 Bindungsstelle im *CD44*-Promotor induziert und durch die Bindung von HMGA1 verstärkt. Untersuchungen in Ratten zeigten, daß es durch CD44-Überexpression zu einer verstärkten Rekrutierung glatter Muskelzellen kommt und dies direkten Einfluß auf die Entstehung von arteriosklerotischen Läsionen hat (Pellacani et al 1999; Foster et al. 1998; 2000).

Untersuchungen zur Expression des in der chromosomalen Bande 6p21.3 lokalisierten HMGAI-Gens und des in der Region 12q14-15 lokalisierten *HMGA2-* Gens zeigten, dass diese hauptsächlich bei Prozessen zellulärer Differenzierung aktiv sind. Dementsprechend ist eine starke Expression dieser Gene während der Embryonalentwicklung und in undifferenzierten Zellen (Chiappetta et al 1996) sowie in Wachstumsfaktor-stimulierten Zellen zu finden (Friedman et al 1993; Johnson et al 1990; Ogram et al 1995; Holth et al 1997). Im adulten, differenzierten Gewebe ist lediglich in der Retina HMGA1 stark exprimiert, während in den übrigen Geweben HMGA2 gar nicht und HMGA1 nur noch in sehr geringen Konzentrationen zu finden ist (Bussemakers et al 1991; Chiappetta et al 1996; Rogalla et al 1996; Zhou et al 1995; Chau et al 2000). Eine reaktivierte Expression von HMGA-Proteinen in differenziertem Normalgewebe wird dabei mit dem Wachstum und der Differenzierung von Adipozyten (Zhou et al 1995; Anand & Chada 2000; Melillo et al 2001), der Proliferation glatter Muskelzellen in den Blutgefäßen nach Gefäßverletzungen (Chin et al 1999), der Immunantwort bei entzündlichen Reaktionen (Pellacani et al 1999) sowie mit apoptotischen Prozessen (Diana et al 2001; Sgarra et al 2003) assoziiert. Die Menge an HMGA1 variiert dabei in Abhängigkeit von der Proliferationsrate der Zellen (Johnson et al 1990).

Im Verlauf der Embryonalentwicklung konzentriert sich die HMGA1-Expression auf spezifische Organe ekto-, meso- oder endodermalen Ursprungs, während HMGA2 auf mesenchymale Gewebe beschränkt ist. Bislang liegen keine Informationen über den Phänotyp von *HMGA1*-knockout Mäusen vor, möglicherweise weil das Fehlen dieses Faktors die embryonale Entwicklung zu sehr schädigt. *HMGA2*-knockout Mäuse dagegen sind zwergwüchsig und weisen besonders wenig Fettgewebe auf (Zhou et al 1995) und sind darüber hinaus gegen diätinduzierte Fettleibigkeit resistent (Anand & Chada 2000).

Schließlich ist HMGA2- bzw. HMGA1b-Expression nicht nachweisbar im Fettgewebe von normalen Mäusen, jedoch dramatisch erhöht im Fett von fetten oder diabetischen Mäusen (Chada et al. 2004), was auf einen Zusammenhang von Adipositas und HMGA-Expression hindeutet

Überexpression von HMGA1 beeinflusst im Einzelnen (Reeves et al 2001):
- Regulatoren von Zellzyklus und Wachstum wie cdc25A,
- Intermediäre Filamentmarker wie Zytokeratin, Typ 1
- Apoptoseregulatoren wie TRAR15
- Onkogene und Tumorsuppressor-Gene wie *MET*
- Gene für DNA-Reparatur und -Rekombination wie *DNase X*
- Regulatoren von Zellschicksal und -entwicklung wie frizzled-5
- Rezeptoren wie FGFR1
- Zelladhäsions-, Motilitäts- und Invasionsgene wie *collagen type 1*
- Angiogenese-Regulatoren wie FGFR2
- Invasionsregulatoren wie MMP-16
- kleinen GTPasen der Rho-Familie und ihre Regulatoren wie RhoC
- Zell-Zellinteraktionsgene wie *cadherin 12*
- Wachstumsfaktoren und Zytokine wie IL-11

Abnormale Regulation von HMGA1 könnte daher zu generellen Alterationen der Genexpression führen und dadurch signifikant zur Bildung von transformierten und/oder metastatischen Phänotypen beitragen.

HMGA-Proteine scheinen in mesenchymalen bzw. epithelialen Tumoren unterschiedliche Rollen zu spielen: in malignen epithelialen Tumoren ist HMGA-Expression eher mit späteren Stadien der Karzinogenese assoziiert, während die benignen - eher selten konvertierenden mesenchymalen Tumoren - bereits in früher Hyperplasie HMGA exprimieren. Dies deutet darauf hin, dass HMGA-Proteine in Geweben von unterschiedlicher embryonaler Herkunft verschiedene Funktionen erfüllen, woraus sich auch unmittelbar die entsprechenden Anwendungen der erfindungsgemäßen L-Nukleinsäuren in der Diagnose und/oder Therapie von - entsprechenden - Erkrankungen ergeben, wie auch im Folgenden auch detaillierter dargstellt.

In Tiermodellen wurde die Expression von HMGA1 in verschiedenen menschlichen und tierischen Neoplasmen untersucht. Die Rolle von HMGA-1 konnte in Tiermodellen bezüglich Tumorigenese (Leman et al 2003; Ram et al 1993) sowie neoplastischer Progression gezeigt werden (Bussemakers et al 1991; Nestl et al 2001; Ram et al 1993).

Erhöhte Expression des *HMGA1*-Gens ist belegt für Karzinome von
- Prostata (Bussemaker et al 1991; Tamimi et al 1996, Leman et al 2003; Nestl et al 2001)
- Pankreas (Nestl et al 2001; Abe et al 2000, 2002; Tarbe et al 2001)
- Schilddrüse (Chiappetta et al 1998, 1995)
- Zervix (Bandiera et al 1998)
- Magen (Xiang et al 1997)
- Brust (Holth et al 1997; Baldassarre et al 2003; Reeves et al 2001; Nestl et al 2001; Ram et al 1993; Dolde et al 2002)
- Colon/Rektum (Fedele et al 1996; Abe et al 1999; Kim et al 1999; Chiapetta et al 2001)
- Ovarien (Masciullo et al 2003)
- und weiterhin für
- Neuroblastome (Giannini et al 2000; 1999) sowie
- Lymphome (Wood et al 2000a; b).

Die genaue Basis für die erhöhte Expression und die Rolle des *HMGAI-*Gens in der Pathogenese des Tumors und des Prozesses der Metastasierung ist zwar noch nicht ganz geklärt. Verschiedene Studien legen jedoch nahe, dass die Stärke der *HMGA1*-Expression des jeweiligen Tumors als prognostischer Marker mit dessen Metastasierungspotential korreliert und damit ein charakteristisches Merkmal für eine maligne transformierte Zelle darstellt (Giancotti et al 1987).

Weitere HMGA1-assozüerte - in diesem Falle benigne, mesenchymale Tumore - sind charakterisiert durch chromosomale Veränderungen in der chromosomalen *HMGA1*-Region 6p21.3. Derartige Aberrationen wurden bislang unter anderem für
- Uterus-Leiomyome (Mark et al 1988; Ozisik et al 1993)
- Lipome (Sreekantaiah et al 1990)
- Endometriumpolypen (Fletcher et al 1992; Dal Cin et al 1995) sowie
- chondroide Hamartome der Lunge (Fletcher et al 1991; Johansson et al 1992, 1993)
   beschrieben.

Aberrationen in den genetischen Mechanismen, die Wachstum und Proliferation kontrollieren, sind die primäre Ursache für Karzinogenese. Die Expression von HMGA-Proteinen ist stark mit Tumorentwicklung assoziiert, wie in einer Anzahl von Arbeiten gezeigt werden konnte (Giancotti et al. 1987, 1989, 1993). So wurde eine beträchtliche HMGA2-Expression in chemisch oder viral erzeugten wie auch in spontan erhaltenen Tumoren gefunden, während in nicht-transformierten Zellen bzw. gesundem Gewebe dieses Protein nicht nachgewiesen werden konnte (Giancotti et al. 1989). In Einklang damit fehlten mit onkogenen Retroviren infizierten Zellen, in denen die Synthese von HMGA2-Expression spezifisch blockiert worden war, verschiedene phänotypische Marker für Transformation (Berlingieri et al. 1995).

Die Schlüsselrolle der HMGA-Proteine für normales wie auch pathologisches Wachstum konnte in Mausmodellen herausgearbeitet werden: HMGA2 *knockout*-Mäuse zeigen Zwergwuchs, d. h. die Tiere sind ca. 60% kleiner als Wildtyp-Mäuse. Diese Zwergmäuse jedoch weisen eine hohe Resistenz gegen chemisch induzierte Hauttumoren auf.

Mit Hilfe zytogenetischer Untersuchungen wurden in den letzten Jahren für eine ganze Reihe von benignen Tumoren mesenchymalen Ursprungs - hierbei handelt es sich um die größte Gruppe gutartiger Neoplasien des Menschen - strukturelle Aberrationen der chromosomalen Region 12q14-15 das *HMGA2*-Gen betreffend gefunden. Trotz einer Vielzahl an Aberrationen (Schoenmakers et al 1995; Kottickal et al 1998; Klotzbüchel et al 1999) haben die veränderten Formen aber immer eine Gemeinsamkeit: sie beinhalten alle die drei DNA-bindenden Domänen, gleichzeitig verlieren sie aber sowohl die saure C-terminale Domäne als auch auf Ebene der RNA die Informationen des 3'UTRs.

Solche Veränderungen wurden bereits für viele (meist gutartige) mesenchymalen HMGAassoziierte Tumore gefunden:
- Leiomyome des Uterus, die häufigsten Unterleibstumoren bei Frauen und Indikation für über 200.000 jährliche Hysterektomien in den USA (Heim et al 1988; Turc-Carel et al 1986; Vanni et al 1988)
- Lipome (Heim et al 1988; Turc-Carel et al 1986; Mandahl et al 1987; Sreekantaiah et al 1991; Belge et al 1992)
- Endometriumpolypen (Walter et al 1989; Vanni et al 1993; Dal Cin et al 1995)
- chondroide Hamartome der Lunge (Fletcher et al 1991, 1995; Dal Cin et al 1993)
- pleomorphe Adenome der Kopfspeicheldrüsen (Mark et al 1980, 1986; Bullerdiek et al 1987)
- Hämangiopericytome (Mandahl et al 1993)
- chondromatöse Tumore (Mandahl et al 1989; Bridge et al 1992)
- benigne Tumore der Brust (Birdsal et al 1992; Rohen et al 1995; Staats et al 1996)
- aggressive Angiomyxome (Kazmierczak et al 1995)
- diffuse Astrocytome
- Osteoklastome (Nuguera et al 1989)

Die Hauptursache für Mortalität und Morbidität von Krebspatienten ist die metastatische Verbreitung des primären Neoplasmas im Körper. Metastasierung ist ein kein einfacher Vorgang, da eine erfolgreiche Kolonisierung von entfernten Organen durch ausgestreute neoplastische Zellen viele Stadien durchlaufen muss. Neoplastische Zellen müssen sich vom primären Neoplasma lösen, in den Blutkreislauf eintreten, an entfernten Orten wieder extravadieren, und schliesslich im Parenchym des entsprechenden Organs wieder proliferieren. Bei den verschiedenen Stadien dieser hochkomplexen metastatischen Kaskade sind viele Gene beteiligt die Proteine wie Proteasen, Adhäsionsmoleküle, Motilitätsfaktoren und angiogene Faktoren exprimieren.

Welches dieser Gene letztendlich das ausschlaggebende für die Metastasierung ist, ist nicht bekannt. Das *HMGA1*-Gen als einer der wichtigsten Faktoren, der diesen Prozess kontrolliert, ist jedoch ein valider Kandidat dafür. Die Genprodukte von *HMGA1* beeinflussen die Transkription vieler Gene, die für erfolgreiche Metastasierung wichtig sind. So wurde bereits gezeigt, dass andere Metastase-assoziierte Gene bei Suppression von HMGA1-Expression ihrerseits vermindert exprimiert werden (Battista 1998; Vallone 1997).

HMGA1 ist daher ein interessantes therapeutisches Zielmolekül. Die Blockade von HMGA1 ist somit grundsätzlich geeignet den Krebs zu kontrollieren und seine metastatische Verbreitung zu verhindern (Evans 2004; Sgarra 2004). So konnte beispielsweise durch Einsatz von gegen HMGA-Transkripte gerichtete Antisense-RNAs in Krebszellen in vitro die Zellproliferation verringert oder die Zellen sogar zur Apoptose gebracht werden (Masciullo 2003; Scala 2000; Chau 2003). Im Tiermodell wurde gezeigt, dass durch Gentherapie (adoenovirale Expression von gegen HMGA-Transkripte gerichtete Antisense-RNAs) das Wachstum von verschiedenen Pankreaskrebs-Xenografts drastisch reduziert wird (Trapasso et al 2004).

HMGA1 könnte weiterhin als prognostischer diagnostischer Marker verwendet werden, um zu bestimmen, welche Patienten von einer aggressiveren Krebstherapie profitieren würden. Es besteht eine enge Korrelation zwischen dem Grad der malignen Transformation und der Menge an exprimiertem HMGA1. Dies kann wiederum mit einer schlechten Prognose bei vielen menschlichen Krebstypen korreliert werden, wie Prostata- ( Tamimi 1996; Bussemakers 1991) und kolorektale Karzinome (Abe 1999) bzw. Neuroblastome (Giannini 2000).

HMGA-Proteine werden von vielen Viren als von der Wirtszelle bereitgestellte Kontrollfaktoren für die Expression viraler Gene oder als Cofaktoren, unter anderem von
- humanem Papovavirus JC (Leger et al 1995)
- Epstein-Barr-Virus (Schaefer et al 1997)
- Herpes-Simplex-Virus (Panagiotidis 1999 ; French et al 1996)
- HIV-1 Virus (Henderson et al 2000)
   verwendet.

Insbesondere werden HMGA Proteine mit der Regulation der Transkription einer Vielzahl viraler Gene in einer Wirtszelle in Verbindung gebracht. Beispiele hierfür sind die Regulation der Expression der frühen und spät expremierten Gene des humanen Papovavirus JC (Leger et al. 1995), Regulation des EBNA1 (Epstein-Barr virus nuclear antigen 1) Gens des Ebstein-Bar Virus (EBV), welches mitverantwortlich für die Kontrolle viraler Latenz ist, (Schaefer et al. 1997), Regulation des IE-3 (immediate-early) Gens des Herpes Simplex Virus-1 (HSV-1), welches das frühzeitig expremierte Protein ICP4 codiert (Panagiotidis et al. 1999), Regulation des während der Latenzphase aktiven Promotors 2 des HSV-1 (French et al. 1996) und Regulation des LTR (long terminal repeats) -Promotors des humanen HIV-1 Virus (Henderson et al 2000).

Die Requirierung von HMGA seitens der Wirtszelle im Rahmen viraler Erkrankungen ist nicht nur auf virale Gen-Regulation beschränkt. HMGA1 scheint auch eine entschiedene Rolle als architektonischer Kofaktor bei der Integration der viralen DNA des HIV-1 Virus, des Moloney murinen Leukämie Virus (MoMuLv) und sarkomen Geflügelgrippe Virus (ASV) in das menschliche Genom zu spielen und scheint somit ein interessanter therapeutischer Ansatz antiviraler Therapie zu sein (Van Maele et al. 2006, Li et al 1998, Hindmarsh et al. 1999)

Somit sind Inhibitoren von HMGA Proteinen auch zur Behandlung und der Diagnose von Virusinfektionen geeignet (Reeves & Beckerbauer 2002).

Infolge der vorstehend dargelegten Beteiligung von HMGA-Proteinen an verschiedenen Erkrankungen bzw. ihrer Eignung als diagnostische Marker, können dagegen gerichtete L-Nukleinsäure und insbesondere Spiegelmere für die Prävention, Therapie und Diagnose von eben diesen Erkrankungen verwendet werden. Besonders bevorzugte Spiegelmere sind dabei die hierin beschriebenen Spiegelmeren. In diesem Zusammenhang wird von den Fachleuten anerkannt werden, dass obwohl die einzelnen Spiegelmere für ein bestimmtes HMGA-Protein generiert worden sind, infolge des in Beispiel 2 dargestellten Domänen-Ansatzes diese auch eine Kreuzreaktivität mit anderen HMGA-Proteinen erlauben, wie sich auch aus dem in Fig. 2 dargestellten Alignment ergibt.

Weiterhin wird von den Fachleuten auf dem Gebiet anerkannt werden, dass die erfindungsgemäßen Nukleinsäuren eine Reihe von Strukturmotiven aufweisen, die eine Klasse von Spiegelmeren definieren, die gegen HMGA-Proteine als intrazelluläre Rezeptoren binden. Die verschiedenen Strukturmotive werden detaillierter in Beispiel 1 weiter dargestellt.

Die erfindungsgemäßen Nukleinsäuren umfassen in einer bevorzugten Ausführungsform auch solche Nukleinsäuren, die im Wesentlichen homolog zu den spezifisch hierin offenbarten Sequenzen sind. Der Begriff "im Wesentlichen homolog" soll hierin bevorzugterweise so verstanden werden, dass die Homologie wenigstens 75 %, bevorzugterweise 85 %, bevorzugtererweise 90 % und am bevorzugtesten mehr als 95, 96, 97, 98 oder 99 % beträgt.

Der Begriff der erfindungsgemäßen Nukleinsäuren oder Nukleinsäuren gemäß der vorliegenden Erfindung soll weiterhin auch jene Nukleinsäuren umfassen, die Nukleinsäuresequenzen umfassen, wie sie hierin offenbart sind oder Teile davon, bevorzugterweise in dem Umfang, dass die Nukleinsäuren oder die besagten Teile davon an der Bindung an HMGA-Proteine beteiligt sind. Eine derartige Nukleinsäure kann von den hierin offenbarten abgeleitet werden, z. B. durch Verkürzung oder Trunkierung. Eine Verkürzung kann entweder ein oder beide Enden der Nukleinsäuren, wie sie hierin offenbart sind, betreffen. Eine Verkürzung kann auch die innere Sequenz aus Nukleotiden betreffen, d. h. sie kann Nukleotid(e) zwischen dem 5'- bzw. dem 3'-terminalen Nukleotid betreffen. Darüber hinaus soll der Begriff Verkürzung auch die Deletion von so wenig wie einem einzelnen Nukleotid von der Sequenz der hierin offenbarten Nukleinsäuren betreffen. Verkürzung kann auch mehr als einen Bereich der erfindungsgemäßen Nukleinsäure(n) betreffen, wobei ein jeder dieser Bereiche so klein wie ein Nukleotid lang sein kann.

Die Nukleinsäuren gemäß der vorliegenden Erfindung können darüber hinaus entweder D-Nukleinsäuren oder L-Nukleinsäuren sein. Bevorzugterweise sind die erfindungsgemäßen Nukleinsäuren L-Nukleinsäuren. Zusätzlich ist es möglich, dass ein oder mehrere Teile der Nukleinsäure als D-Nukleinsäuren vorliegt, oder wenigstens ein oder mehrere Teile der Nukleinsäuren L-Nukleinsäuren sind. Der Begriff "Teil" der Nukleinsäuren soll sowenig wie ein Nukleotid bezeichnen. Derartige Nukleinsäuren werden allgemein hierin als D- bzw. L-Nukleinsäuren bezeichnet. Deshalb bestehen in einer bevorzugten Ausführungsform die Nukleinsäuren gemäß der vorliegenden Erfindung aus L-Nukleotiden und umfassen wenigstens ein D-Nukleotid. Ein derartiges D-Nukleotid ist bevorzugterweise an einem Teil befestigt, der von dem/den Bereich(en) verschieden ist, die die Nukleinsäuren gemäß der vorliegenden Erfindung definieren, bevorzugterweise jenen Teilen davon, mit denen eine Wechselwirkung mit anderen Teilen der Nukleinsäuren erfolgt. Bevorzugterweise ist ein derartiges D-Nukleotid am Ende eines jeden Bereiches bzw. einer jeden Nukleinsäure gemäß der vorliegenden Erfindung befestigt. In einer bevorzugten Ausführungsform können derartige D-Nukleotide als ein Spacer oder ein Linker fungieren, der bevorzugterweise Modifikationen wie PEG und HES an die Nukleinsäuren gemäß der vorliegenden Erfindung bindet.

Im Rahmen der vorliegenden Erfindung umfassen in einer Ausführungsform die erfindungsgemäßen Nukleinsäuren auch solche, die Teil einer längeren Nukleinsäure sind, wobei diese längeren Nukleinsäuren mehrere Teile umfassen können, wobei wenigstens ein Teil eine Nukleinsäure gemäß der vorliegenden Erfindung oder ein Teil davon ist. Der andere Teil oder die anderen Teile dieser längeren Nukleinsäuren können entweder eine D-Nukleinsäure oder eine L-Nukleinsäure sein. Eine jegliche Kombination kann in Verbindung mit der vorliegenden Erfindung und zu den Zwecken bzw. Verwendungen verwendet werden, wie sie für die erfindungsgemäßen Nukleinsäuren hierin offenbart sind. Dieser andere Teil bzw. diese anderen Teile der längeren Nukleinsäure können eine Funktion aufweisen, die vom Binden und insbesondere vom Binden an HMGA-Protein, verschieden ist. Eine mögliche Funktion besteht darin, eine Wechselwirkung mit anderen Molekülen, z. B. zu Zwecken der Immobilisierung, Kreuzvernetzung, des Nachweises, der Amplifikation oder Modifikation oder Molekulargewichtserhöhung zu erlauben.

Insbesondere in diesem Zusammenhang sind L-Nukleinsäuren, wie hierin verwendet, Nukleinsäuren, die aus L-Nukleotiden bestehen, bevorzugterweise vollständig aus L-Nukleotiden bestehen.

Entsprechend sind insbesondere D-Nukleinsäuren, wie hierin verwendet, Nukleinsäuren, die aus D-Nukleotiden bestehen, bevorzugterweise vollständig aus D-Nukleotiden bestehen.

Unabhängig davon, ob die erfindungsgemäße Nukleinsäure aus D-Nukleotiden, L-Nukleotiden oder einer Kombination davon aus beiden besteht, wobei die Kombination, z. B., eine zufällige Kombination oder eine definierte Sequenz aus Bereichen ist, die aus wenigstens einem L-Nukleotid und wenigstens einer D-Nukleinsäure bestehen, kann die Nukleinsäure aus einem oder mehreren Desoxyribonukleotiden, Ribonukleotiden und Kombinationen davon bestehen.

In einem weiteren Aspekt betrifft die vorliegende Erfindung eine pharmazeutische Zusammensetzung, die aus mindestens einer der erfindungsgemäßen Nukleinsäuren in Kombination mit einer oder mehreren anderen Nukleinsäuren besteht, wobei die andere(n) Nukleinsäuren bevorzugterweise an von HMGA-Protein verschiedene Zielmoleküle bindet/binden oder eine von den erfindungsgemäßen Nukleinsäuren verschiedene Funktion ausübt/ausüben.

Die Konstruktion der erfindungsgemäßen Nukleinsäuren als L-Nukleinsäure ist aus mehreren Gründen vorteilhaft. L-Nukleinsäuren sind Enantiomere von natürlicher Weise auftretenden Nukleinsäuren. D-Nukleinsäuren sind jedoch in wässrigen Lösungen und insbesondere in biologischen Systemen und in biologischen Proben infolge des umfänglichen Vorhandenseins von Nukleasen nicht sehr stabil. Natürlicherweise auftretende Nukleasen, insbesondere Nukleasen aus tierischen Zellen, sind nicht in der Lage, L-Nukleinsäuren abzubauen. Infolge dessen ist die biologische Halbwertszeit der L-Nukleinsäure in einem derartigen System, einschließlich des menschlichen und tierischen Körpers, signifikant erhöht. Infolge der fehlenden Abbaubarkeit von L-Nukleinsäuren werden keine Nuklease-Abbauprodukte erzeugt und somit werden keine dadurch bedingten Nebenwirkungen beobachtet. Dieser Aspekt grenzt die L-Nukleinsäure von faktisch allen anderen Verbindungen ab, die bei der Therapie von Erkrankungen und/oder Störungen verwendet werden und die Anwesenheit von HMGA oder dessen kausale Involvierung umfassen L-Nukleinsäuren, die spezifisch an ein Zielmolekül durch einen Mechanismus binden, der von dem Watson-Crick-Basenpaarung unterschiedlich ist, oder Aptamere, die teilweise oder vollständig aus L-Nukleotiden bestehen, insbesondere wobei jene Teile des Aptamers, die an der Bindung des Aptamers an das Zielmolekül beteiligt sind, werden als Spiegelmere bezeichnet.

Es ist auch im Rahmen der vorliegenden Erfindung, dass die erfindungsgemäßen Nukleinsäuren, unabhängig davon, ob sie als D-Nukleinsäuren, L-Nukleinsäuren oder D-L-Nukleinsäuren vorliegen, und ob sie DNA oder RNA sind, als einzelsträngige oder doppelsträngige Nukleinsäuren vorhanden sein können. Typischerweise sind die erfindungsgemäßen Nukleinsäuren einzelsträngige Nukleinsäuren, die infolge der Primärsequenz definierte Sekundärstrukturen aufweisen und somit auch Tertiärstrukturen ausbilden können. Die erfindungsgemäßen Nukleinsäuren können jedoch auch doppelsträngig sein in dem Sinne, dass zwei Stränge, die komplementär oder teilweise komplementär zueinander sind, miteinander hybridisiert sind. Dies verleiht den Nukleinsäuren Stabilität, was insbesondere vorteilhaft sein wird, wenn die Nukleinsäure in der natürlicherweise auftretenden D-Form statt der L-Form vorliegt.

Die erfindungsgemäßen Nukleinsäuren können modifiziert werden. Derartige Modifikationen können einzelne Nukleotide der Nukleinsäure betreffen und sind in der Technik gut bekannt. Beispiele für eine derartige Modifikation sind, unter anderem, beschrieben in Venkatesan N. et al. (2003) Curr Med Chem. Oct;10(19):1973-91; Kusser, W.(2000) J Biotechnol, 74: 27-38; Aurup, H. et al. (1994) Nucleic Acids Res, 22, 20-4; Cummins, L.L. et al, (1995) Nucleic Acids Res, 23, 2019-24; Eaton, B.E. et al. (1995) Chem Biol, 2, 633-8; Green, L.S. et al., (1995) Chem Biol, 2, 683-95; Kawasaki, A.M. et al., (1993) J Med Chem, 36, 831-41; Lesnik, E.A. et al., (1993) Biochemistry, 32, 7832-8; Miller, L.E. et al., (1993) J Physiol, 469, 213-43. Eine derartige Modifikation kann beispielhaft ein H-Atom, ein F-Atom oder eine O-CH₃-Gruppe oder NH₂-Gruppe an der 2'-Position eines einzelnen Nukleotids sein, das in der Nukleinsäure enthalten ist. Darüberhinaus kann die Nukleinsäure gemäß der vorliegenden Erfindung wenigstens ein LNA-Nukleotid umfassen. In einer Ausführungsform besteht die Nukleinsäure gemäß der vorliegenden Erfindung aus LNA-Nukleotiden, bevorzugterweise vollständig aus LNA-Nukleotiden.

In einer Ausführungsform können die Nukleinsäuren gemäß der vorliegenden Erfindung eine mehrteilige Nukleinsäure sein. Eine mehrteilige Nukleinsäure, wie hierin verwendet, ist eine Nukleinsäure, die aus wenigstens zwei Nukleinsäuresträngen besteht. Diese wenigstens zwei Nukleinsäurestränge bilden eine funktionale Einheit, wobei die funktionale Einheit ein Ligand für ein Zielmolekül ist. Die wenigstens zwei Nukleinsäurestränge können von einer der erfindungsgemäßen Nukleinsäuren abgeleitet werden entweder durch Spalten der Nukleinsäure, um zwei Stränge zu erzeugen, oder durch Synthetisieren von einer Nukleinsäure entsprechend einem ersten Teil der erfindungsgemäßen, d. h. Gesamtnukleinsäure, und einer weiteren Nukleinsäure entsprechend dem zweiten Teil der Gesamtnukleinsäure. Es wird anerkannt, dass sowohl die Spaltung als auch die Synthese verwendet werden kann, um eine mehrteilige Nukleinsäure zu erzeugen, wo mehr als die beiden beispielhaft oben beschriebenen Stränge vorhandensein können. Mit anderen Worten, die wenigstens zwei Nukleinsäurestränge sind bevorzugterweise verschieden von zwei Strängen, die komplementär zueinander sind und miteinander hybridisieren, obwohl eine Komplementarität in einem bestimmten Umfang zwischen den verschiedenen Nukleinsäureteilen bestehen kann.

Die vorliegenden Erfinder haben festgestellt, dass die Nukleinsäuren gemäß der vorliegenden Erfindung einen sehr vorteilhaften K_{D}-Wertebereich oder Dissoziationswertebereich und damit eine sehr vorteilhafte Bindungskonstante aufweisen. Ein Beispiel, um die Bindungskonstante zu bestimmen, ist die Verwendung eines Gleichgewichtsanbindungsassay , wie er in Beispiel 1 beschrieben ist.

Der K_{D}-Wert der erfindungsgemäßen Nukleinsäuren beträgt bevorzugterweise weniger als 1 µM. Ein K_{D}-Wert von etwa ein µM soll für eine nicht-spezifische Bindung einer Nukleinsäure an ein Ziel charakteristisch sein. Wie von den Fachleuten anerkannt werden wird bewegt sich der K_{D}-Wert einer Gruppe von Verbindungen wie beispielsweise der Nukleinsäuren gemäß der vorliegenden Erfindung, innerhalb eines bestimmten Bereiches. Der oben erwähnte K_{D} von etwa 1 µM ist ein bevorzugterer oberer Grenzwert für den K_{D}-Wert. Der bevorzugte untere Grenzwert für den K_{D} von das Zielmolekül bindenden Nukleinsäuren kann etwa pikomolar oder niedriger sein. Es ist im Rahmen der vorliegenden Erfindung, dass die K_{D}-Werte von einzelnen Nukleinsäuren, die an HMGA binden, bevorzugterweise innerhalb dieses Bereiches liegen. Bevorzugte Bereiche können ausgewählt werden, indem eine erste Zahl innerhalb dieses Bereiches ausgewählt wird, und eine zweite Zahl innerhalb dieses Bereiches. Bevorzugte obere Werte sind 0,25 µM, 0,1 µM, bevorzugte untere Werte sind 100 nM, 10 nM, 1 nM und 0,05 nM.

Die erfindungsgemäßen Nukleinsäuren binden bevorzugterweise an HMGA1b bei 37° C in Lösung mit einer Dissoziationskonstante K_{D}<20 nM , wie in Beispiel 2 dargestellt.

Die Nukleinsäuren gemäß der vorliegenden Erfindung können eine beliebige Länge aufweisen unter der Voraussetzung, dass sie noch in der Lage sind, an das Zielmolekül zu binden. Es wird in der Technik anerkannt werden, dass bestimmte Längen der Nukleinsäuren gemäß der vorliegenden Erfindung bevorzugt sind. Typischerweise beträgt die Länge zwischen 15 und 120 Nukleotiden. Es wird von den Fachleuten weiterhin anerkannt werden, dass irgendeine ganze Zahl zwischen 15 und 120 eine bevorzugte mögliche Länge für die Nukleinsäuren gemäß der vorliegenden Erfindung ist. Bevorzugterere Bereiche für die Länge der Nukleinsäuren gemäß der vorliegenden Erfindung sind Längen von etwa 20 bis 100 Nukleotiden, etwa 20 bis 80 Nukleotiden, etwa 20 bis 60 Nukleotiden, etwa 20 bis 50 Nukleotiden und etwa 30 bis 50 Nukleotiden.

In einer Ausführungsform können die erfindungsgemäßen Nukleinsäuren modifiziert vorliegen. Eine besonders bevorzugte Form der Modifizierung ist die PEGylierung. Dabei handelt es sich um die Modifizierung der erfindungsgemäßen Nukleinsäuren durch Kopplung mit Polyethylenglycol (PEG) oder anderer Gruppen.

Infolge der hohen Stabilität der erfindungsgemäßen Nukleinsäuren, insbesondere in der Ausführungsform, dass diese als L-Nukleinsäuren vorliegen, ist eine direkte Gabe der erfindungsgemäßen Nukleinsäuren zur Behandlung eines Patienten, der einer solchen Behandlung bedarf, möglich. Bevorzugterweise werden die erfindungsgemäßen Nukleinsäuren als physiologische Lösung zur lokalen oder systemischen Verabreichung bereitgestellt.

Neben der direkten Verwendung der erfindungsgemäßen Nukleinsäuren zur Behadnlung, Prävention und Diagnostik der hierin beschrieben Erkrankungen kann diese einzeln oder in Kombination mit anderen in einer pharmazeutischen Zusammensetzumng vorliegen bzw. verwendet werden. Die pharmazeutische Zusammensetzung gemäß der vorliegenden Erfindung umfasst somit wenigstens eine der Nukleinsäuren gemäß der vorliegenden Erfindung und bevorzugterweise einen pharmazeutisch akzeptablen Binder. Ein derartiger Binder kann ein jeglicher bekannter oder auf dem Gebiet bekannter Binder sein. Insbesondere ist ein derartiger Binder irgendein Binder, wie er im Zusammenhang mit der Herstellung des Medikaments beschrieben ist, wie hierin offenbart. In einer weiteren Ausführungsform umfasst die pharmazeutische Zusammensetzung ein weiteres pharmazeutisch aktives Agens. Es ist im Rahmen der vorliegenden Erfindung, dass das hierin beschriebene Medikament die pharmazeutische Zusammensetzung darstellt, wie sie hierin beschrieben ist.

Bevorzugterweise ist die pharmazeutische Zusammensetzung für die intravenöse Verabreichung. Es ist jedoch auch im Rahmen der vorliegenden Erfindung, dass derartige pharmazeutische Zusammensetzungen intramuskulär, intraperitoneal, oder subkutan verabreicht werden können. Andere Wege der Verabreichung sind per orum oder intranasal, wobei diejenige Verabreichungsart bevorzugt ist, die am wenigsten invasiv ist, unter gleichzeitiger Beibehaltung der Wirksamkeit der pharmazeutischen Zusammensetzung bzw. des pharmazeutisch aktiven Agens.

Die erfindungsgemäßen Nukleinsäuren sind bevorzugterweise als solches oder im Zusammenhang mit der erfindungsgemäßen pharmazeutischen Zusammensetzung in einem pharmazeutisch akzeptablen Lösungsmittel enthalten oder gelöst. Derartige Lösungsmittel sind insbesondere jene, die aus der Gruppe ausgewählt sind, Wasser, physiologische Kochsalzlösung, PBS, oder eine Glukoselösung, insbesondere eine 5% Glukose-Lösung umfasst. Ein derartiger Träger kann, z. B., Wasser, Puffer, PBS, Glucoselösung, bevorzugterweise eine 5% Glukoselösung (iso-osmotisch), Stärke, Zucker, Gelatine oder irgendeine andere akzeptable Trägersubstanz sein. Derartige Träger sind den Fachleuten auf dem Gebiet im allgemeinen bekannt.

Es ist im Rahmen der vorliegenden Erfindung, dass die pharmazeutische Zusammensetzung mindestens eine der erfindungsgemäßen Nukleinsäuren in ihren verschiedenen Ausführungsformen enthält, einschließlich, aber nicht darauf beschränkt, die Nukleinsäure als Konjugat, wie hierin beschrieben.

In einer weiteren Ausführungsform umfasst das Medikament ein weiteres pharmazeutisch aktives Agens. Derartige weitere pharmazeutische aktive Agenzien sind beispielsweise Proteasehemmer, Proliferationsinhibitoren und Angiogenesehemmer und/ oder Agenzien die zystostatsich wirken. Alternativ oder zusätzlich ist ein derartiges weiteres pharmazeutisch aktives Agens eine weitere Nukleinsäure gemäß der vorliegenden Erfindung. Alternativ umfasst das Medikament wenigstens eine oder mehrere Nukleinsäuren, die an ein Zielmolekül binden, das von HMGA verschieden ist oder eine Funktion aufweist, die verschieden ist von einer der Nukleinsäuren gemäß der vorliegenden Erfindung.

Die pharmazeutische Zusammensetzung gemäß der vorliegenden Erfindung kann für die Behandlung, Diagnose und/oder Prävention einer jeglichen der hierin beschriebenen Erkrankungen oder Störungen verwendet werden.

In einem weiteren Aspekt betrifft die vorliegende Erfindung ein Verfahren für die Behandlung eines Lebewesens, das einer derartiger Behandlung bedarf, wobei das Verfahren die Verabreichung einer pharmazeutisch aktiven Menge von wenigstens einer der Nukleinsäuren gemäß der vorliegenden Erfindung umfasst. In einer Ausführungsform leidet das Lebewesen unter einer Erkrankung oder es besteht das Risiko, dass es an einer derartigen Erkrankung erkrankt, wobei die Erkrankung eine jede der hierin offenbarten ist, insbesondere eine jede Erkrankung, die im Zusammenhang mit der Verwendung von einer der Nukleinsäuren gemäß der vorliegenden Erfindung für die Herstellung eines Medikaments beschrieben ist.

Obgleich sich die Verwendung der erfindungsgemäßen Nukleinsäuren bereits aus der oben dargestellten Involvierung der HMGA-Proteine bei den verschiedenen Erkrankungen und Zuständen ergibt, soll auf diesen Aspekt im folgenden zur Veranschaulichung weiter eingegangen werden.

HMGA Proteine und deren Gene wurden insbesondere vermehrt mit der Diagnose und Prognose neoplastischer Erkrankungen in Verbindung gebracht und als potentielle Biomarker vorgeschlagen. In gesundem Gewebe ist das Expressionsniveau von HMGA1a/b Proteinen sehr niedrig, wenn überhaupt detektierbar. Erhöhte HMGA1a/b Proteinexpression ist charakteristisch für den Phänotyp einer Vielzahl von Tumoren und Metastasen unterschiedlichster Krebserkrankungen (Sarhadi et al. 2006, Balcercak et al. 2005, Briese et al. 2006, Chang et al. 2005, Peters et al. 2005, Sato et al. 2005, Chiappetta et al.2004, Li et al. 2004 , Chuma et al. 2004 , Donato et al. 2004, Czyz et al. 2004, Kettunen et al. 2004, Lee et al. 2004, Chen et al. 2004, Abe et al. 2003, Blacerczak et al. 2003, Flohr et al. 2003 , Masciullo et al. 2003, Nam et al. 2003, Pierantoni et al. 2003). Hohe HMGA-Proteinexpression korreliert signifikant mit einer schlechten Prognose und der Entstehung von Metastasen. Die Detektion des HMGA1a/b Expressionsniveaus in Biopsien und deren histologischen Charakterisierung stellt einen diagnostischen Ansatz zur Früherkennung, Prognose und Identifizierung von neoplastischen Erkrankungen dar, insbesondere den vorstehend dargestellten Erkrankugnen und Zuständen.

Des Weiteren ist in der Literatur eine Assoziation von HMGA1 Proteinen mit arteriosklerotischen Plaques beschrieben (Schlueter et al. 2005.). HMGA1 reguliert CD44, eines der hauptsächlichen Zielgene für die Entstehung von Plaques. Dabei zeigte im Vergleich zum umliegenden Gewebe, dass die betroffene Regionen wie neointimale, vaskuläre glatte Muskelzellen, Makrophagen und neue Blutgefäßen eine starke Expression von HMGA1 aufweisen. HMGA1 scheint hierbei einer der Mediatoren bei der Entstehung von Plaques zu sein und stellt so ein Zielmolekül für die Diagnostik dar.

Die hier beschriebenen L-Nukleinsäuren und insbesondere die Spiegelmer, die HMGA1a/b binden, können im Rahmen der dem Fachmann bekannten Methoden ähnlich Antikörpern eingesetzt werden. Bisher sind nur sehr wenig spezifische (differenzierende) und affine Antikörper gegen HMGA1 identifiziert worden und kommerziell erhältlich. Dies scheint durch die nicht vorhandene Sekundärstruktur von HMGA1 bedingt zu sein, welche kein geeignetes Ziel für den MHC Komplex bei der Generierung von Antikörpern darstellt.

Vor diesem Hintergrund konnte hierin überraschenderweise gezeigt werden, dass das biotinylierte HMGA1a/b bindende Spiegelmer 5'-bio-NOX-A50 im Western Blot HMGA1a/b als einzelne Bande in Krebszelllinien erkennt. Des Weiteren konnte - wie in Beispiel 2 beschrieben - rekombinant expremiertes HMGA1b Protein detektiert werden. Die Detektion des biotinylierten Spiegelmers erfolgt beispielsweise über einen anti-Biotin Antikörper konjugiert mittels Horse Raddish Peroxidase (HRP).

Einen weiteren Ansatz stellt in die *in-vivo* Diagnostik vom HMGA1a/b dar, bei der die erfiundungsgemäßen Nukleinsäuren verwendet werden können. Tumore und Metastasen sind oft eingebettet von nekrotischen Tumorzellen, welche HMGA1a/b in das umliegende Gewebe abgeben. Die Detektion des extrazellulären HMGA1a/b- stellt einen Ansatz der Diagnose vom in gesunden Gewebe eingebetteten Tumoren und Metastasen dar.

Wie bevorzugterweise hierin verwendet ist ein Diagnostikum oder diagnostisches Agens oder diagnostisches Mittel in der Lage, entweder direkt oder indirekt ein HMGA-Protein, bevorzugterweise HMGA1a/b, wie hierin beschrieben, nachzuweisen und bevorzugtererweise HMGA1a/b, wie hierin beschrieben im Zusammenhang mit den verschiedenen Störungen und Erkrankungen. Das Diagnostikum ist geeignet für den Nachweis und/oder die Nachsorge für eine jegliche der hierin beschriebenen Krankheiten bzw. Erkrankungen. Ein derartiger Nachweis ist möglich durch die Bindung der Nukleinsäuren gemäß der vorliegenden Erfindung an HMGA1a/b. Ein derartiges Binden kann entweder direkt oder indirekt nachgewiesen werden. Die entsprechenden Verfahren und Mittel sind den Fachleuten auf dem Gebiet bekannt. Unter anderem können die Nukleinsäuren gemäß der vorliegenden Erfindung eine Markierung umfassen, die den Nachweis der Nukleinsäuren gemäß der vorliegenden Erfindung erlaubt, bevorzugterweise der Nukleinsäure, die an HMGA-Protein und bevorzugterweise HMGA1a/b gebunden ist oder binden kann. Eine derartige Markierung ist bevorzugterweise ausgewählt aus der Gruppe bestehend aus radioaktiven, enzymatischen und Fluoreszenz-Markierungen. Im Prinzip können alle bekannten Tests, die für Antikörper entwickelt worden sind, für die Nukleinsäuren gemäß der vorliegenden Erfindung angepasst werden, wobei der Zielmolekül-bindende Antikörper durch eine Zielmolekül-bindende Nukleinsäure ersetzt ist. In Antikörper-Tests, die unmarkierte Zielmolekül-bindende Antikörper verwenden, erfolgt der Nachweis bevorzugterweise durch einen Sekundärantikörper, der mit radioaktiven, enzymatischen oder Fluoreszenz-Markierungen modifiziert ist und an den Zielmolekül-bindenden Antikörper an seinem Fc-Fragment bindet. Im Falle einer Nukleinsäure, bevorzugterweise einer Nukleinsäure gemäß der vorliegenden Erfindung, ist die Nukleinsäure mit einer derartigen Markierung modifiziert, wobei bevorzugterweise eine derartige Markierung ausgewählt ist aus der Gruppe bestehend aus Biotin, CY-3 und CY-5, und eine derartige Markierung nachgewiesen wird durch einen Antikörper, der gegen eine derartige Markierung gerichtet ist, z. B. ein anti-Biotin-Antikörper, ein anti-CY-3-Antikörper oder ein anti-CY5-Antikörper oder in dem Fall, dass die Markierung Biotin ist, wird die Markierung durch Streptavidin oder Avidin nachgewiesen, das natürlicherweise an Biotin bindet. Ein derartiger Antikörper, Streptavidin oder Avidin ist wiederum bevorzugterweise mit einer entsprechenden Markierung modifiziert, z. B. einer radioaktiven, enzymatischen oder Fluoreszenz-Markierung, analog einem Sekundärantikörper.

In einer weiteren Ausführungsform werden die Nukleinsäuren gemäß der vorliegenden Erfindung nachgewiesen oder analysiert durch ein zweites Nachweismittel, wobei das Nachweismittel ein molekularer Leuchtturm (engl. "molecular beacon") ist. Die Technik der molekularen Leuchttürme ist den Fachleuten auf dem Gebiet bekannt. Kurz gesagt sind diese molekularen Leuchttürme Nukleinsäuresonden, die ein reverses Komplement zu der nachzuweisenden Nukleinsäureprobe sind, und hybridisieren deshalb mit einem Teil der nachzuweisenden Nukleinsäureprobe, die es nachzuweisen gilt. Nach Binden der Nukleinsäureprobe werden die Fluorophorgruppen des molekularen Leuchtturms voneinander getrennt, was zur Änderung des Fluoreszenzsignals führt, bevorzugterweise eine Änderung der Intensität. Diese Änderung korreliert mit der Menge an vorhandener Nukleinsäureprobe.

Es ist im Rahmen der vorliegenden Erfindung, dass die erfindungsgemäßen Nukleinsäuren im Rahmen der verschiedenen hierin offenbarten Aspekte entsprechend als L-Nukleinsäuren verwendet werden können.

Die erfindungsgemäßen Nukleinsäuren können weiterhin als Ausgangsmaterial für das Design von pharmazeutischen Wirkstoffen (engl. drug design) verwendet werden. Grundsätzlich gibt es hierzu zwei mögliche Ansätze. Ein Ansatz besteht in dem Screening von Bibliotheken von Verbindungen, wobei derartige Bibliotheken von Verbindungen bevorzugterweise Bibliotheken von niedermolekularen Verbindungen (engl. low oder small molecules) sind. Derartige Bibliotheken sind den Fachleuten auf diesem Gebiet bekannt. In einer Ausführungsform, ist das Screening ein Hochdurchsatzscreening. Bevorzugterweise ist Hochdruchsatzscreening schnell, effizient und wird als Versuch-und-Fehler-Evaluation ("Trial-and-Error-Evaluation") von Wirkstoffen in einem Zielmolekül-basierten Assay durchgeführt.

Alternativ können gemäß der vorliegenden Erfindung die Nukleinsäuren für das rationale Design von Wirkstoffen verwendet werden. Bevorzugterweise ist das rationale Design von Wirkstoffen das Design eines pharmazeutischen Wirkstoffkandidaten. Beginnend von der dreidimensionalen Struktur des Zielmoleküls, die üblicherweise durch Methoden wie Röngtenstrukturanalyse oder Magnetresonanzspektroskopie (NMR = nuclear magnetic resonance spectroscopy) bestimmt wird, werden Computerprogramme verwendet, um Datenbanken mit Strukturen einer Vielzahl verschiedener chemischer Verbindungen zu durchsuchen. Die Auswahl wird dabei von dem Computer durchgeführt. Die ausgewählten Verbindungen werden zusätzlich im Labor getestet.

Das rationale Design von Wirkstoffen kann dabei seinen Ausgangspunkt von irgendeiner der Nukleinsäuren gemäß der vorliegenden Erfindung nehmen und umfaßt eine Struktur, insbesondere eine dreidimensionale Struktur, die ähnlich der Struktur der erfindungsgemäßen Nukleinsäure(n) ist oder identisch ist zu dem Teil der Struktur der erfindungsgemäßen Nukleinsäure(n), die die Bindung an HMG-Proteine vermittelt. In einem jeden Fall zeigt eine derartige Struktur noch das gleiche oder ein zumindest ähnliches Bindungsverhalten, wie die erfindungsgemäße(n) Nukleinsäure(n). In entweder einem weiteren Schritt oder als ein alternativer Schritt wird bei dem rationalen Design von Wirkstoffen die bevorzugterweise dreidimensionale Struktur jener Teile der an HMG-Proteine bindenden Nukleinsäuren durch chemische Gruppen nachgeahmt, die bevorzugterweise verschieden sind von Nukleotiden und Nukleinsäuren. Durch dieses Nachahmen, auch als Mimikry bezeichnet, kann eine Verbindung konstruiert werden, die von der Nukleinsäure bzw. den Nukleinsäuren verschieden ist, die als Ausgangsmaterialien für das rationale Design des Wirkstoffes verwendet wurde. Eine derartige Verbindung oder Wirkstoff ist bevorzugterweise ein kleines Molekül (engl. small molecule) oder ein Peptid.

Im Falle des Screenings von Verbindungsbibliotheken unter Verwendung kompetitiver Tests, die den Fachleuten auf dem Gebiet bekannt sind, können geeignete HMG-Analoga, HMG-Agonisten und HMG-Antagonisten gefunden werden. Derartige kompetitive Assays können wie folgt aufgebaut sein. Die erfindungsgemäße Nukleinsäure, bevorzugterweise ein Spiegelmer, d. h. eine das Zielmolekül bindende L-Nukleinsäure, wird an eine bevorzugterweise feste Phase gekoppelt. Um HMG-Analoga zu identifizieren, wird mit einer Markierung versehenes HMG-Protein zu dem Testsystem hinzugegeben. Alternativ könnte auch das HMG-Protein an eine feste Phase gekoppelt werden und die erfindungsgemäße Nukleinsäure könnte markiert sein. Ein potentielles Analogon bzw. ein potentieller Agonist oder Antagonist würde mit den HMG-Molekülen, die an das Spiegelmer binden, kompetitieren, was mit einer Abnahme des Signals, das von der entsprechenden Markierung erhalten wird, einhergehen würde. Das Screenen auf Agonisten oder Antagonisten kann die Verwendung eines Zellkultur-Testsystems umfassen, das den Fachleuten auf dem Gebiet bekannt ist.

In einem weiteren Aspekt können die erfindungsgemäßen Nukleinsäuren infolge ihres charakteristischen Bindungsverhaltens an HMG-Protein für die Targetvalidierung ("Target" ist englisch für "Zielmolekül") verwendet werden. Die erfindungsgemäßen Nukleinsäuren können in einem ex vivo-Organmodell verwendet werden, um die Funktion von HMG-Protein zu studieren. Grundsätzlich existieren ex vivo Modelle, in denen HMG-Agonisten/Antagonisten getestet werden können.

Ein Kit gemäß der vorliegenden Erfindung kann wenigstens eine oder mehrere der erfindungsgemäßen Nukleinsäuren umfassen. Zusätzlich kann der Kit wenigstens eine oder mehrere Positiv- oder Negativkontrollen umfassen. Als Positivkontrollen kann z. B. HMG-Protein verwendet werden, gegen das die erfindungsgemäße Nukleinsäure gescreent wurde, oder an die diese bindet, bevorzugterweise in flüssiger Form. Als Negativkontrolle kann unter anderem ein Peptid verwendet werden, welches sich hinsichtlich seiner biophysikalischen Eigenschaften ähnlich wie HMG-Protein verhält, welches jedoch nicht durch die erfindungsgemäßen Nukleinsäuren erkannt wird oder ein Peptid mit gleicher Aminosäurezusammensetzung aber von HMG-Protein verschiedener Sequenz.

Weiterhin kann der Kit einen oder mehrere Puffer umfassen. Die verschiedenen Bestandteile können in dem Kit in trockener oder lyophilisierter Form, oder gelöst in einer Flüssigkeit vorliegen. Der Kit kann eine oder mehrere Behältnisse umfassen, die wiederum ein oder mehrere der Bestandteile des Kits enthalten können. Bevorzugterweise enthalten die Gefäße Reaktionsansätze, wie sie für eine einmalige Durchführung eines Experimentes unter Verwendung einer oder mehrerer Bestandteile des Kits erforderlich sind.

Es wird anerkannt werden, dass, sofern nicht gegenteiliges angegeben ist, die hierin angeführten Sequenzen in 5'-3'-Richtung angegeben sind. Weiterhin wird anerkannt werden, dass der Begriff, das zwei Abschnitt miteinander hybridisieren, hierin so verstanden wird, dass die Abschnitte in vitro aufgrund von allgemeinen Basenpaarungsregeln hybridisieren können, oder dass die Abschnitte unter den Bedingungen der Anwendung hybridisieren oder hybridisieren können, jedoch nicht notwendigerweise miteinander unter den Bedingungen der Anwendung hybridisiert sind bzw. hybridisiert vorliegen.

Die verschiendenen SEQ.ID., der chemische Aufbau der Nukleinsäuren wie hierin offenbart und das Zielmolekül HMGA1a/1b wie hierin verwendet, die tatsächlichen Sequenzen und die interne Referenz sind in der folgenden Tabelle zusammengefasst.

| **Seq. ID** | **Interne Referenz** | **RNA/Peptid** | **Sequenz** |
|---|---|---|---|
| 1 | 132-C3, NOX-h | L-RNA (Spiegelmer) | GCUGCUGCAAAUUGACGGGGGCGUGGUUGGGGCGGGUCGAUUGCAGC |
| 2 | 132-B3, NOX-f (48nt) | L-RNA (Spiegelmer) | GCUGAAUGAGGAUCGCAGGGGCGUGGCUGGGGUGGGCGACCGUUCAGC |
| 3 | 132-C4 | L-RNA (Spiegelmer) | GCUGCGCAAGGAGAGGGGCGCGGUUGGGGAGGCUCUAAGCGCUGCAGC |
| 4 | 132-E2 | L-RNA (Spiegelmer) | GCUGGCGCUAUAGGACAGGGGUGCGGUUGGGGCGGUCCGCUGUCAGC |
| 5 | 132-A2 | L-RNA (Spiegelmer) | GCUGGAUAGAACGCAGGGGUGCGGUUUGGGGUGGGCGUGAUAUGCAGC |
| 6 | 132-H1, NOX-i | L-RNA (Spiegelmer) | GCUGCCGUAAAGAGGGGUGAGGUUGGGGAGGCUUUACGGUUUCAGC |
| 7 | 132-F1 | L-RNA (Spiegelmer) | GCUGCAUGCCGCGAUCAGGGGAGCGGUUGGGGCGGGAUCCGGCUCAGC |
| 8 | 132-G2, NOX-g | L-RNA (Spiegelmer) | GCUGCGAGGGAGGUAGCGGCUCUGCGCCGUGACGUGGGUGGAUGCAGC |
| 9 | 122-A1, NOX-A | L-RNA (Spiegelmer) | GGCUGAUACGUGGGUGGAUAUGGGGCAGUUCCAUGUGGGUGGUUUCAGCC |
| 10 | 122-C1, NOX-B | L-RNA (Spiegelmer) | GGCUGAUACGUGGGUGAAUAUGGGGCAGUUCCAUGUGGGUGGUUUCAGCC |
| 11 | 122-B2 | L-RNA (Spiegelmer) | GGCUGAUACGUGGGAGGAAAGGUGUAACUACCUGUGGGAGGUUUCAGCC |
| 12 | 122-E2, NOX-C | L-RNA (Spiegelmer) | GGCUGGCACUCGCAGGGGUGAAGUGAUGAUUGGGGUGGGCGAGACCAGCC |
| 13 | 122-G2, NOX-E | L-RNA (Spiegelmer) | GGCUGCCGAGUGGUUGGGUGGUGUAAGGGAGGUGGAAUCCGCGGGCAGCC |
| 14 | 122-B4, NOX-D | L-RNA (Spiegelmer) | GGCUGUUCGUGGGAGGAAGGCUCUUGGAUAGAGUCGUGGGUGGUUCAGCC |
| 15 | 132-B3 32nt, NOX-f 32nt | L-RNA (Spiegelmer) | GGAUCGCAGGGGCGUGGCUGGGGUGGGCGACC |
| 16 | 132-B3 33nt, NOX-f 33nt | L-RNA (Spiegelmer) | GGAUCGCAGGGGCGUGGCUGGGGUGGGCGAUCC |
| 17 | HMGA1a/b Zielmolekül Domäne, Biotinyl-D-HMGA1a/b-21mer | D-Peptid | Biotin-EPSEVPTPKRPRGRPKGSKNK |
| 18 | HMGA1a (human) | L-Peptid | (M)SESSSKSSQPLASKQEKDGTEKRGRGRPRKQPPVSPGTALVGSQKEPSEVPTPKRPRGRPK GSKNKGAAKTRKTTTTPGRKPRGRPKKLEKEEEEGISQESSEEEQ |
| 19 | HMGA1b (human) | L-Peptid | (M)SESSSKSSQPLASKQEKDGTEKRGRGRPRKQPPKEPSEVPTPKRPRGRPKGSKNKGAAKT RKTTTTPGRKPRGRPKK-LEKEEEEGISQESSEEEQ |
| 20 | HMGA2 human | L-Peptid | (M)SARGEGAGQPSTSAQGQPAAPAPQKRGRGRPRKQQQEPTGEPSPKRPRGRPKGSKNKSPS KAAQKKAEATGEKRPRGRPRKWPQQVVQKKPAQEETEETSSQESAEED |
| 21 50 | bio-dsDNA (AT hook) | D-DNA | 5'biotin-TCGAAAAAAGCAAAAAAAAAAAAAAAAAACTGGC) und 5'GCCAGTTTTTTTTTTTTTTTTTTGCTTTTTT |
| 22 | NOX-A-3'PEG, NOX-A-3'PEG2000, NOX-A-2kDa PEG, NOX-A PEG | L-RNA | GGCUGAUACGUGGGUGGAUAUGGGGCAGUUCCAUGUGGGUGGUUUCAGCC-2kDA-PEG |
| 23 | INVERSE-3'-PEG INV 3'-PEG | L-RNA | CCGACUUUGGUGGGUGUACCUUGACGGGGUAUAGGUGGGUGCAUAGUCGG-2kDA-PEG |
| 24 | 5'-Biotin-NOX-A | L-RNA | Biotin-GGCUGAUACGUGGGUGGAUAUGGGGCAGUUCCAUGUGGGUGGUUUCAGCC |
| 25 | 5'-Biotin-NOX-A inverse | L-RNA | Biotin-CCGACUUUGGUGGGUGUACCUUGACGGGGUAUAGGUGGGUGCAUAGUCGG |
| 26 | INVERSE | L-RNA | CCGACUUUGGUGGGUGUACCUUGACGGGGUAUAGGUGGGUGCAUAGUCGG |
| 27 | POC-3'-PEG | L-RNA | UAAGGAAACUCGGUCUGAUGCGGUAGCGCUGUGCAGAGCU-2kDA-PEG |
| 28 | Capture-Sonde NOX-A | L-RNA | CCCATATCCACCCACGTATCAGCCTTTTTTTT-NH₂ |
| 29 | Detektor-Sonde NOX-A | L-RNA | Biotin-TTTTTTTTGGCTGAAACCACCCACATGG |
| 30 | Capture-Sonde POC | L-RNA | NH₂(C7)-TTTTTTTTTAGCTCTGCACAGCGCT |
| 31 | Detektor-Sonde POC | L-RNA | CCGCATCAGACCGAGTTTCCTTATTTTTTTT-Biotin |
| 32 | HMG_fwd1 Primer | D-DNA | TCGACACCATGGGTGAGTC |
| 33 | HMG_rev1 Primer | D-DNA | GTCTAGAAAGCTTCCCAACTG |
| 34 | 132-C3, NOX-h | D-RNA (Aptamer) | GCUGCUGCAAAUUGACGGGGGCGUGGUUGGGGCGGGUCGAUUGCAGC |
| 35 | 132-B3, NOX-f (48nt) | D-RNA (Aptamer) | GCUGAAUGAGGAUCGCAGGGGCGUGGCUGGGGUGGGCGACCGUUCAGC |
| 36 | 132-C4 | D-RNA (Aptamer) | GCUGCGCAAGGAGAGGGGCGCGGUUGGGGAGGCUCUAAGCGCUGCAGC |
| 37 | 132-E2 | D-RNA (Aptamer) | GCUGGCGCUAUAGGACAGGGGUGCGGUUGGGGCGGUCCGCUGUCAGC |
| 38 | 132-A2 | D-RNA (Aptamer) | GCUGGAUAGAACGCAGGGGUGCGGUUUGGGGUGGGCGUGAUAUGCAGC |
| 39 | 132-H1, NOX-i | D-RNA (Aptamer) | GCUGCCGUAAAGAGGGGUGAGGUUGGGGAGGCUUUACGGUUUCAGC |
| 40 | 132-F1 | D-RNA (Aptamer) | GCUGCAUGCCGCGAUCAGGGGAGCGGUUGGGGCGGGAUCCGGCUCAGC |
| 41 | 132-G2, NOX-g | D-RNA (Aptamer) | GCUGCGAGGGAGGUAGCGGCUCUGCGCCGUGACGUGGGUGGAUGCAGC |
| 42 | 122-A1, NOX-A | D-RNA (Aptamer) | GGCUGAUACGUGGGUGGAUAUGGGGCAGUUCCAUGUGGGUGGUUUCAGCC |
| 43 | 122-C1, NOX-B | D-RNA (Aptamer) | GGCUGAUACGUGGGUGAAUAUGGGGCAGUUCCAUGUGGGUGGUUUCAGCC |
| 44 | 122-B2 | D-RNA (Aptamer) | GGCUGAUACGUGGGAGGAAAGGUGUAACUACCUGUGGGAGGUUUCAGCC |
| 45 | 122-E2, NOX-C | D-RNA (Aptamer) | GGCUGGCACUCGCAGGGGUGAAGUGAUGAUUGGGGUGGGCGAGACCAGCC |
| 46 | 122-G2, NOX-E | D-RNA (Aptamer) | GGCUGCCGAGUGGUUGGGUGGUGUAAGGGAGGUGGAAUCCGCGGGCAGCC |
| 47 | 122-B4, NOX-D | D-RNA (Aptamer) | GGCUGUUCGUGGGAGGAAGGCUCUUGGAUAGAGUCGUGGGUGGUUCAGCC |
| 48 | 132-B3 32nt, NOX-f 32nt | D-RNA (Aptamer) | GGAUCGCAGGGGCGUGGCUGGGGUGGGCGACC |
| 49 | 132-B333nt,NOX-f33nt | D-RNA (Aptamer) | GGAUCGCAGGGGCGUGGCUGGGGUGGGCGAUCC |

Es ist im Rahmen der vorliegenden Erfindung, dass, sofern für die einzelnen Abschnitte der erfindungsgemäßen Nukleinsäuren keine Sequenzen explizit angegeben sind, diese gemäß der hierin offenbarten technischen Lehre frei gewählt werden können, d. h. so gewählt werden können, dass sie das erforderliche Bindungsverhalten an das jeweilige Zielmolekül zeigen und/oder die hierin beschriebenen Strukturen, insbesondere Sekundärstrukturen auszubilden in der Lage sind.

Weiterhin ist es im Rahmen von bevorzugten Ausführungsformen der vorliegenden Erfindung, dass für den Fall, dass in Sequenzen, die als RNA-Sequenzen bezeichnet werden, T statt U angegeben sind, T für U stehen soll.

Die vorliegende Erfindung wird weiter anhand der folgenden Figuren und Beispiele erläutert, aus denen sich weitere Merkmale, Ausführungsformen und Vorteile ergeben. Dabei zeigt
Fig. 1A durch *in vitro* Selektion gegen D-21AS-HMGA1a/b erzeugte Aptamere, die die 21AS-HMGA1a/b-Domäne binden;
Fig. 1B eine Darstellung der identifizierten, wiederholt auftretenden Sequenzbereiche der durch *in vitro* Selektion gegen D-21AS-HMGA1a/b erzeugten Aptamere, die die 21AS-HMGA1a/b-Domäne binden;
Fig. 2 einen Sequenzvergleich von HMGA1a/b und HMGA2;
Fig.3 eine Verkürzung von HMGA1a/b-bindendem Aptamer NOX-f;
Fig. 4 die Bindungseigenschaften von verkürzten HMGA1a/b-bindendem Aptamer NOX-f;
Fig. 5 einen Kompetitionsassay zur Messung der Bindung von HMGA an die doppelsträngige natürliche Ziel-DNA im Multiwellplatten-Assay; die Bindung des Spiegelmers kompetiert mit der Bindung des rekombinanten HMGA1b an die biotinylierte dsDNA (AT-hook Motiv). Die Detektion des gebundenen HMGA1b erfolgt über den His-Tag per Nickel-HRP, welche ein Substrat in fluoreszierendes Signal umsetzt;
Fig. 6 einen Vergleich von Spiegelmer NOX-A und Spiegelmmer NOX-f (48nt; 33nt) im kompetitiven Multiwellplatten-Assay; Spiegelmer NOX-A, sowie Spiegelmer NOX-f und dessen verkürzte Variante Spiegelmer NOX-f33 verhindern im Plattenassay die Bindung von rekombinaten HMGA1b an seinen natürlichen vorkommenden Bindungspartner im niedrig nanomolaren Bereich.
Fig. 7 die Aktivität von 2kDa-PEG-gekoppeltem Spiegelmer NOX-A sowie nichtfunktionalem Kontrollspiegelmer im kompetitiven Multiwellplatten-Assay; das PEGylierte Spiegelmer NOX-A kompetiert die Bindung von rekombinantem HMGA1b an das AT-Hook-Motiv der dsDNA mit einer IC50 von 15nM; das inverse Kontroll-Spiegelmer von NOX-A zeigt bei hohen Spiegelmerkonzentrationen eine unspezifische Interaktion mit HMGA1b;
Fig. 8 eine Western Blot; Nachweis von immobilisiertem HMGA1b durch biotinyliertes Spiegelmer; rekombinantes HMGA1b wandert im elektrophoretischen Feld wie ein 20kDa großes Protein und kann bei niedrigen Konzentrationen (3nM) durch das biotinylierte Spiegelmer (hier am Beispiel von NOX-A) erkannt werden; ein inverses Kontroll Spiegelmer konnte HGMGA1b nicht erkennen;
Fig. 9: die Aktivität von freiem und PEGyliertem Spiegelmer NOX-A im kompetitiven Multiwellplatten-Assay;
Fig. 10: eine Untersuchung der Verpackung von PEGyliertem Spiegelmer in Mizellen im "RiboGreen exclusion assay";
Fig. 11 die Stabilität von PEI-Spiegelmer-Mizellen im "RiboGreen exclusion assay";
Fig. 12 die effiziente Aufnahme von in PEI-Mizellen verpacktem Spiegelmer, genauer einen Vergleich der Transfektion von "nacktem" Spiegelmer im Vergleich zu in Mizellen verpackten Spiegelmeren am Beispiel von Spiegelmer NOX-A-3'PEG2kDa.; die Zellen die mit Spiegelmer-Mizellen transfiziert wurden, zeigten bei geringerer Einstellung der Sensitivität der Kamera (camera gain) eine stärkere Fluoreszenz im Zytosol verglichen mit Zellen die nur mit reinem Spiegelmer inkubiert wurden; die Effizienz lag bei beiden Transfektionsmethoden bei >95%;
Fig. 13 die Freisetzung von Spiegelmer aus dem endosomalen Kompartiment; Spiegelmer Mizellen zeigten eine deutlich höhere Fluoreszenz im Vergleich zu reinem Spiegelmer; Spiegelmer-Mizellen zeigten ein punktuelles perinukleäres, sowie zytoplasmatisches Verteilungsmuster; die punktuelle Verteilung weist auf eine Lokalisation in endosomalen Kompartimenten hin; die diffuse Verteilung im Zytosol und an der Plasmamembran deutet auf aus Endosomen freigesetztes Spiegelmer hin;
Fig. 14 einen Proliferationsassay mit "nacktem" Spiegelmer; dosisabhängige Hemmung der Proliferation von MCF-7 Zellen bei hohen Spiegelmerkonzentrationen nach 2 Tagen im Zellkulturmedium (Quantifizierung über Resazurin);
Fig. 15 die Proliferation von H1299-Zellen ("non-small cell lung cancer") nach Behandlung mit PEI-verpacktem NOX-A-2kDa PEG; Hemmung der Proliferation von H-1299 Zellen bei 1µM Spiegelmer, appliziert als PEI-Spiegelmer-Mizellen (N/P 2,5); NOX-A zeigte eine geringe Hemmung der Proliferation im Vergleich zum Kontroll-Spiegelmer ;
Fig. 16 die Inhibierung der HMGA1a/b-induzierten cdc25a-Genexpression, nachgewiesen durch quantitative RT-PCR; Bestimmung der spezifischen Hemmung der cdc25a mRNA Expression in H-1299 Zellen durch 1µM NOX-A Spiegelmer-Mizellen (N/P 2,5) mittels RT-PCR;
Fig. 17 Dosisabhängige Inhibition der cdc25a mRNA-Expression durch Spiegelmer NOX-A; Quantifizierung der Dosis-abhängigen Hemmung der cdc25a mRNA Expression in H1299 Zellen mittels RT-PCR; NOX-A Spiegelmer-Mizellen (N/P 2,5) zeigten ab 250nM eine spezifische Hemmung der cdc25a mRNA Expression; bei einer Konzentration >4µM zeigte sich ein unspezischer Effekt des Kontroll-Spiegelmers, sowie toxische Effekte des Polyethylenimins (PEI) bei > 10µM (Daten nicht gezeigt);
Fig. 18 die Hemmung des Tumorwachstums im Xenograft-Modell in Nacktmäusen durch das Spiegelmer NOX-A; Inhibition des Tumorwachstums nach subkutaner Injektion von PSN-1 Zellen durch 2mg/kg Spiegelmer-Mizellen (N/P 2,5). Spiegelmer NOX-A zeigte eine deutliche Reduktion des Tumorwachstums;
Fig. 19 die statistische Analyse der Daten aus dem Xenograft-Versuchs; Inhibition des Tumorwachstums nach subkutaner Injektion von PSN-1 Zellen durch 2mg/kg Spiegelmer-Mizellen (N/P 2,5); Endpunktanalyse und Darstellung als Box-andWhisker-plot. NOX-A bewirkte eine hoch signifikante Verringerung des Tumorwachstums (p=0,0098 gegenüber PBS und p=0,022 gegenüber inversem Kontroll-Spiegelmer);
Fig. 20: die Gewebeverteilung von Spiegelmer NOX-A im Xenograft-Versuch; quantitative Analyse der Verteilung von Spiegelmer NOX-A in Plasma und Geweben; Im Tumorgewebe war im Vergleich zu den anderen Geweben und Plasma eine hohe Konzentration an Spiegelmer NOX-A nachweisbar.
Fig. 21: Gewebeverteilung von in Mizellen verpacktem und unverpacktem Spiegelmer 24 und 96 Stunden nach der letzten Injektion im Xenograft-Versuch; quantitative Analyse der Verteilung eines unfunktionalen Spieglemers in Plasma und Geweben; im Tumorgewebe war im Falle eines in Mizellen verpackten Spiegelmers im Vergleich zu den anderen Geweben und Plasma eine deutlich erhöhte Konzentration an Spiegelmer sowohl nach 24 als auch nach 96 Stunden nachweisbar.

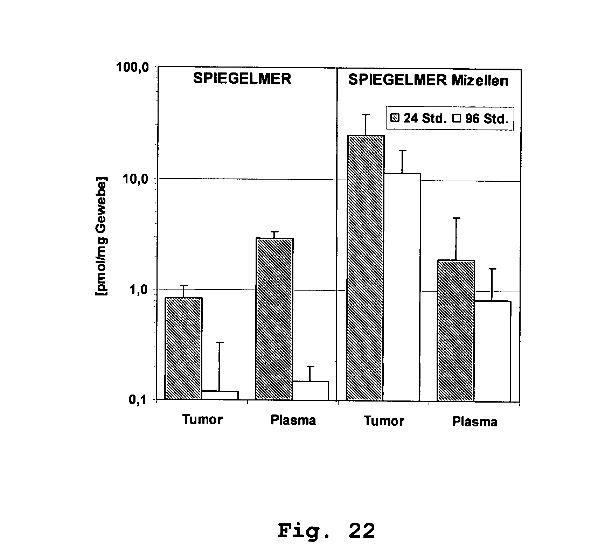

Fig. 22: Verteilung von in Mizellen verpacktem und unverpacktem Spiegelmer im Plasma und im Tumor 24 und 96 Stunden nach der letzten Injektion im Xenograft-Versuch; quantitative Analyse der Verteilung eines unfunktionalen Spieglemers in Plasma und Tumor; Im Tumorgewebe war im Falle eines in Mizellen verpackten Spiegelmers im Vergleich zu unverpackten Spiegelmers eine deutlich erhöhte Konzentration an Spiegelmer sowohl nach 24 als auch nach 96 Stunden nachweisbar.

### Beispiel 1: HMGA1a/b-bindende Spiegelmere

### 1.1 HMGA1a/b-bindende Sequenzen

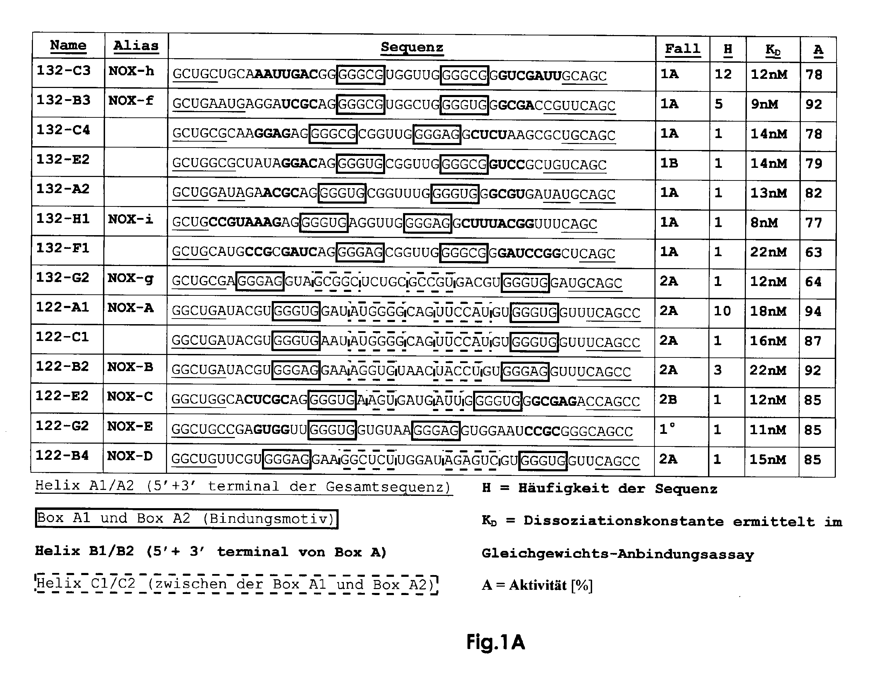

Die HMGA1a/b-bindenden RNA-Spiegelmere wurden durch *in vitro* Selektion gegen D-21AS-HMGA1a/b und nachfolgende Verkürzungschritte generiert. Die erzeugten Aptamere, die die 21AS-HMGA1a/b-Domäne binden, sind in Fig. 1A dargestellt.

### 1.1.1 Ranking auf Aptamerebene

Die unterschiedlichen Klone (siehe Fig . 1A) wurden als Aptamere (D-RNA) mittels Standard-Phosphoramidit-Synthese hergestellt und am 5'-Ende durch Kinasierung radioaktiv markiert (siehe unten). Die Klone wurden anschließend bei zwei Konzentrationen an D-bio-21aa HMGA1a/b mittels Gleichgewichtsanbindungsassay hinsichtlich Ihrer Affinität und Aktivität analysiert.

**Radioaktive Markierung durch Kinasierung:**

| **Substanz** | **[final]** |
|---|---|
| RNA | 5 µM |
| T4 Forward Reaction Buffer (Invitrogen) | 1x |
| T4 Polynucleotide Kinase (Invitrogen) | 10 U/10 µl_{Reaktionsansatz} |
| [γ-³²P]-ATP | 1 µl/10 µl_{Reaktionsansatz} |

Die Reaktion lief für eine Stunde bei 37°C und wurde dann durch Erhitzen (10 min bei 65°C) gestoppt. Die Abtrennung radioaktiver Nukleotide von markierten Oligonukleotiden erfolgte über eine analytische Polyacrylamid-Gelelektrophorese (PAGE) (siehe folgend). Danach erfolgte eine "Crush-and-Soak"-Gel-Elution mit Ammoniumacetat und eine Ethanolfällung (siehe folgend). Die Menge der aufgereinigten RNA wurde über die Radioaktivtät des Pellets (nach der Fällung) im Verhältnis zur Radiokativität der ausgeschnittenen Bande abgeschätzt.

### Polyacrylamid-Gelelektrophorese (PAGE)

Zur präparativen Reinigung von Oligonukleotiden wurden den Reaktionsansätzen ½ bis 2 Volumina konzentrierten Probenpuffers für denaturierende PAGE hinzugefügt. Zusätzlich wurden bei Bedarf Größenstandards vorbereitet (je 250 pmol) und in Probenpuffer aufgenommen. Die Ansätze wurden 5 min bei 95°C denaturiert und auf Eis abgekühlt. Ein präparatives, denaturierendes 7 %iges oder 10 %iges PAA-Gel (200 x 200 x 1,5 mm) wurde durch Anlegen der Spannung von maximal 600 V bei 40-50 W vortemperiert (ca. 1 h). Nach Spülen der Taschen mit 1x TBE wurden die Proben aufgetragen. Nach Abschluß der Trennung (50 min bei 50 W) wurde das Gel auf eine durch Klarsichtfolie geschützte, fluoreszierende Dünnschicht-Chromatographie-Platte (Farbstoff 60F₂₅₄) gelegt. Mittels UV-Licht (254 nm) konnten die Banden als Schatten visualisiert werden ("UV-Shadowing") und mit einem Skalpell ausgeschnitten werden. Danach erfolgte eine "Crush-and-Soak"-Gel-Elution mit Ammoniumacetat.

### "Crush-and-Soak"-Gel-Elution

Zur Elution von Oligonukleotiden aus PAA-Gelen wurde nach Zerkleinerung der ausgeschnittenen PAA-Gel-Banden mit einer Pipettenspitze oder einem Spatel 500 µl Ammoniumacetat (2 M) zugegeben. Die "Crush-and-Soak"-Elution wurde 2 x 1,5 h bei 68°C in einem Thermoschüttler (1000 U/min) durchgeführt. Die Überstände wurden durch "Ultrafree-MC"-Säulchen (Millipore/Amicon, Schwalbach, Germany) in einer Tischzentrifuge (16.100 x g) von Gelresten befreit. Die so eluierte RNA wurde dann mittels einer Ethanolfällung entsalzt.

### Ethanolfällung

Zur Ethanolfällung wurden 1-2 µl Glycogen als Fällungshilfsmittel eingesetzt. Nach Zugabe von 2,5 Volumen absoluten Ethanols und "Vortexen" wurden die Oligonukleotide 30 min bei -80°C gefällt und 30 min bei 16.100 x g, 4°C abzentrifugiert. Das Pellet wurde 1x mit 70 %igem Ethanol gewaschen und 5 min bei 16.100 x g, 4°C zentrifugiert.

### Aufnahme von Bindungsisothermen im Gleichgewichts-Anbindungs-Assay

Je 2 pmol der 5'-radioaktiv markierten Aptamere wurden in Biotinyl-D-HMGA1a/b-21mer (EPSEVPTPKRPRGRPKGSKNK [Seq. ID. 17]; siehe Fig. 2), wurde hergestellt von Bachem (Weil am Rhein, Deutschland). Lösungen im Konzentrationsbereich von 1 - 3000 nM (oder zur Zwei-Punkt-Mesung mit 300 nM und 30 nM oder 100 nM und 10 nM Peptid) für eine Stunde bei 37°C in Selektionspuffer (10mM Tris HCl pH7,4, 5mM KCI, 0,8mM MgCl2, 0,1% Tween) inkubiert. Als Hintergrundkontrolle diente eine Lösung ohne Biotinyliertes-D-HMGA1a/b-21mer. Peptid und Komplexe wurden anschließend mit 10 µl Streptavidin Ultra-Link Gel innerhalb von 30 min bei 37°C immobilisiert. Die Radioaktivität der Suspension wurde bestimmt. Der Überstand wurde abgenommen. Dann wurde die Matrix einmal mit 100 µl Selektionspuffer gewaschen und dann mit Selektionspuffer aufgefüllt. Durch Messung der Radioaktivität wurde für jede Peptidkonzentration der mit Biotinyl-D-HMGA1a/b-21mer im Komplex vorliegende Aptamer-Anteil ermittelt. Durch grafische Auftragung und Fit (GraFit, Version 4.0.10, Erithacus Software) konnten die Dissoziationskonstante der aktiven Spezies und der Anteil aktiver Moleküle ermittelt werden.

### Ergebnisse

Für alle als Aptamere (D-RNA) synthetisierten Klone wurde im Gleichgewichtsanbindungsassay eine Dissoziationskonstante für die Bindung an das 21 Aminosäure lange D-Fragment von HMGA1a/b (Biotinyl-D-HMGA1a/b-21mer) von 8 - 22 nM ermittelt (Fig. 1A).

### 1.1.2 Verkürzung am Beispiel 132-B3

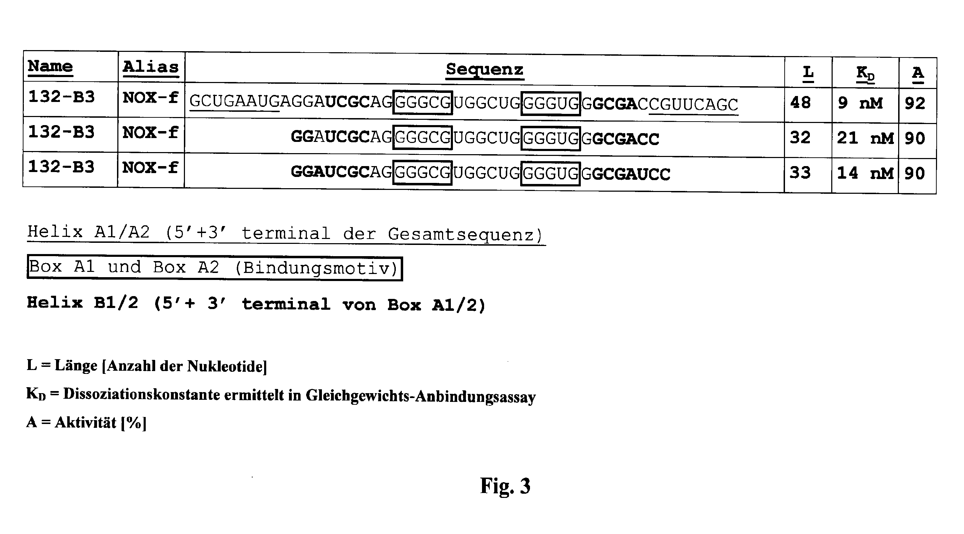

Alle Kandidaten der Selektionen zeigten ein repetitiv auftretendes Sequenzmotiv GGGCG bzw. GGGUG bzw. GGGAG, welches am 5'und am 3'-Ende von einem Helix/ Stem-Motiv stabilisiert wird (Fig. 3).

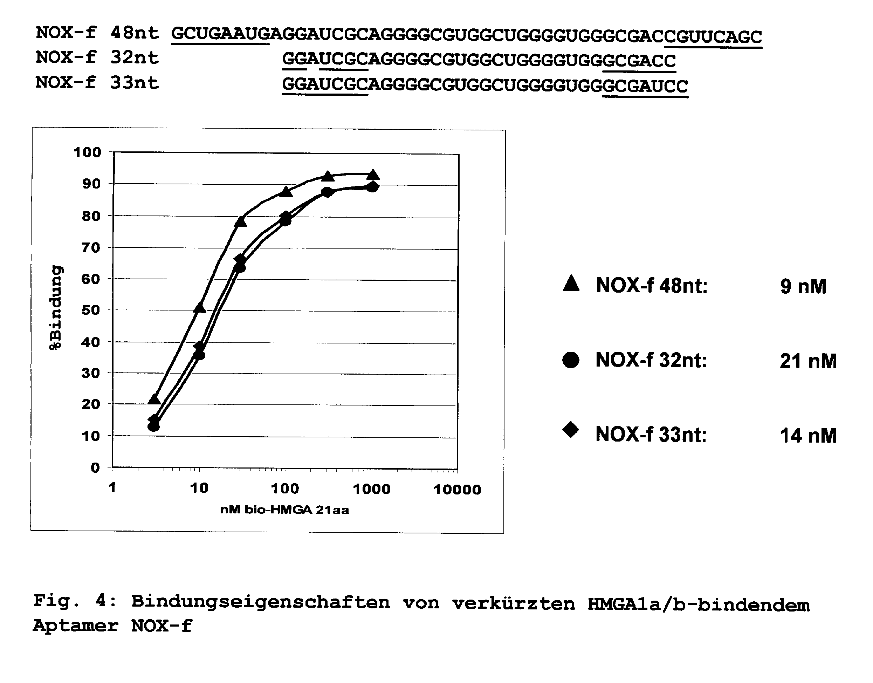

Eine Analyse der voraussichtlichen Struktur und Faltung der RNA-Aptamere nach Zuker (Nucleic Acids Res. 2003 Jul 1;31(13):3406-15) ergab, dass sich die vorgegebene Stem/ Helix-Struktur in einigen verlängert hat (132-C3, 132-B3, 132-C4, 132-E2, 132-A2, 132-H1, 132-F1, 122-G2, 122-E2, siehe Fig. 1A). Diese Stem-Helix-Struktur stellte die Grundlage für die weitere Verkürzung dieser Kandidaten dar. Diese weitere Verkürzung der Kandidaten erfolgte über die Identifizierung und Stabilisierung des minimalen Bindungsmotivs über Faltungs- und anschließende Deletionsanalyse der synthetischen D-RNAs im Hinblick auf die Bindung an das HMGA1a/b Fragment. Die Bestimmung dieser Bindungseigenschaften erfolgte per Gleichgewichtsanbindungsassay. Fig. 3 zeigt exemplarisch am Kandidaten NOX-f (132-B3) die Verkürzung des Aptamers auf Grundlage der stabilisierenden Stem-Struktur, die in verlängerter Form bei den Kandidaten 132-C3, 132-B3, 132-C4, 132-E2, 132-A2, 132-H1, 132-F1, 122-G2, 122-E2 (siehe Fig. 1A) zu finden ist. Eine Verkürzung auf eine 32 Nukleotide lange Aptamer-Variante von NOX-f mit einem 6 Nukleotide langen Stem (NOX-f 32nt) führte zu keinem Verlust der Bindungseigenschaften an das 21aa HMGA1a/b Fragment (Fig. 3 und 4). Das artifizielle Einfügen eines Adenosins an der dritten Position des 5'-ständigen Stem führte zu einer theoretischen Ausbildung eines 7 Nukleotid langen Stems ohne ausgeloopten Bereich und diente zur Komplettierung des Stems im 3'-Bereich (NOX-f 33nt, Fig. 3 und 4). Die Messung der Bindungseigenschaften (Affinität und Aktivität) mittels Gleichgewichtsanbindungsassay an die 21 Aminosäure lange Domäne von HMGA1a/b wurde nicht von diesen Veränderungen beeinflusst.

Die Sequenzen 132-G2, 122-A1, 122-C1, 122-B2 und 122-B4 weisen dagegen am 5'-Ende und 3'-Ende des repetitiv aufretenden Sequenzmotivs (GGGCG bzw. GGGUG bzw. GGGAG) eine deutlich kürzere Stem-Struktur auf. Eine Verkürzung derselben führte zu einem Verlust an Bindung. Eine mögliche Verkürzung des für diese Sequenzen längeren, zentralen Bereichs zwischen dem repetitiven Sequenzmotivs (GGGCG bzw. GGGUG bzw. GGGAG) wurde nicht vorgenommen.

Die Seq.-ID's der Aptamersequenzen der hierin offenbarten HMGA-bindenden Nukleinsäuren sind wie folgt:

| **Seq. ID** | **Interne Referenz** | **RNA/Peptid** |
|---|---|---|
| 34 | 132-C3, NOX-h | D-RNA (Aptamer) |
| 35 | 132-B3, NOX-f (48nt) | D-RNA (Aptamer) |
| 36 | 132-C4 | D-RNA (Aptamer) |
| 37 | 132-E2 | D-RNA (Aptamer) |
| 38 | 132-A2 | D-RNA (Aptamer) |
| 39 | 132-H1, NOX-i | D-RNA (Aptamer) |
| 40 | 132-F1 | D-RNA (Aptamer) |
| 41 | 132-G2, NOX-g | D-RNA (Aptamer) |
| 42 | 122-A1, NOX-A | D-RNA (Aptamer) |
| 43 | 122-C1, NOX-B | D-RNA (Aptamer) |
| 44 | 122-B2 | D-RNA (Aptamer) |
| 45 | 122-E2, NOX-C | D-RNA (Aptamer) |
| 46 | 122-G2, NOX-E | D-RNA (Aptamer) |
| 47 | 122-B4, NOX-D | D-RNA (Aptamer) |
| 48 | 132-B3 32nt, NOX-f 32nt | D-RNA (Aptamer) |
| 49 | 132-B3 33nt, NOX-f 33nt | D-RNA (Aptamer) |

Wie hierin bereits dargelegt und den Fachleuten auf dem Gebiet bekannt, bindet das aus L-Nukleotiden bestehende Enantiomer eines Aptamers, d.h. einer D-Nukleinsäure, das gegen ein D-Peptid erzeugt wurde, an das spiegelbildliche Enantiomer des D-Peptids, d.h. das L-Peptid, wie es natürlicherweise vorkommt. Diese L-Nukleinsäure wird hierin auch als Spiegelmer bezeichnet und weist ansonsten grundsätzlich die gleichen Bindungseigenschaften wie das Aptamer auf.

### 1.2 Charakteristische Eigenschaften von HMGA1a/b-bindenden Spigegelmeren

### 1.2.1 Repetitive Sequenzelemente: Box A1 und Box A2

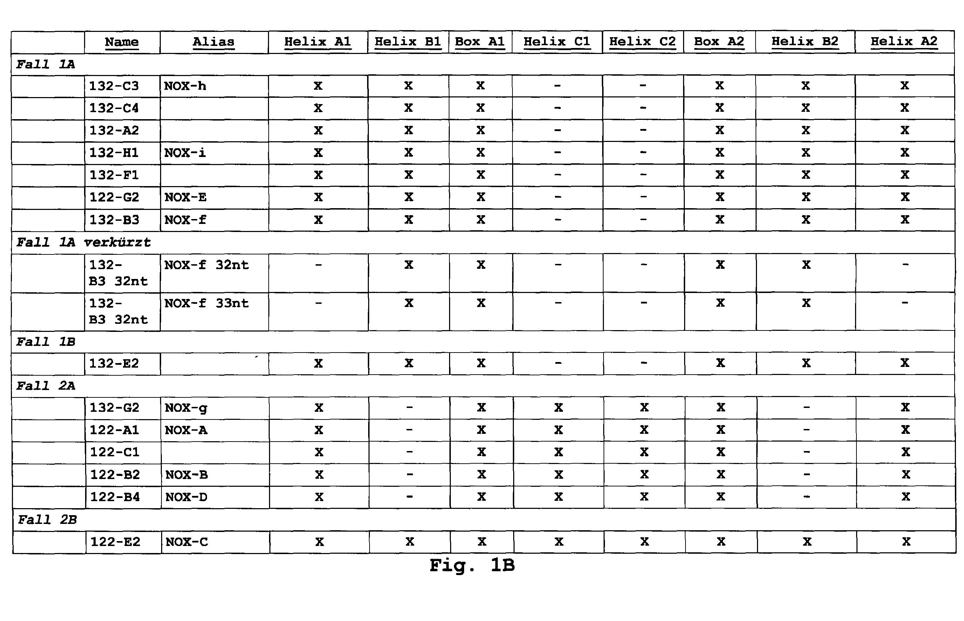

Charakteristisch für alle Spiegelmere, die gegen HMGA1a/b binden, ist ein repetitives Sequenzelement der Sequenz GGGCG bzw. GGGUG bzw. GGGAG. Dieses Sequenzelement taucht zweimal in HMGA1a/b-bindenden Spiegelmeren auf (Fig. 1A und 1B). Das dem 5'-Ende der Spiegelmere näher liegende Sequenzelement wird hierin als Box A1 bezeichnet. Das dem 3'-Ende der Spiegelmere näher liegende Sequenzelement wird dagegen hierin als Box A2 bezeichnet. Box A1 und Box A2 und ihre Anordnung zueinander stellen wahrscheinlich das entscheidende Merkmal HMGA1a/b-bindender Spiegelmere dar.

### 1.2.2 Sequenzabschnitt zwischen Box A1 und Box A2

Zwischen Box A1 und Box A2 liegt entweder ein Sequenzabschnitt mit einer Länge von sechs bis sieben Nukleotiden oder 12 bis 22 Nukleotiden (Fig. 1A und 1B). Da sich diese Sequenzabschnitte nicht nur in ihrer Länge unterscheiden, werden sie getrennt abgehandelt.

### Fall 1: Sequenzabschnitt umfasst sechs bis sieben Nukleotide

Hat der zwischen Box A1 und Box A2 liegende Sequenzabschnitt eine Länge von sechs Nukleotiden, so weist der Sequenzabschnitt die Sequenz UGGUUG, UGGCUG, CGGUUG, AGGUUG oder GUGUAA auf. Ein Einschub von einem Nukleotid (Uracil) in die Sequenz CGGUUG, was zu der Sequenz CGGUUUG führt, hat weder einen negativen noch einen positiven Einfluß auf die Bindungseigenschaften der Spiegelmere.

### Fall 2: Sequenzabschnitt umfasst 12-22 Nukleotide

Hat der zwischen Box A1 und Box A2 liegende Sequenzabschnitt eine Länge von 12 bis 22 Nukleotiden, so weist dieser Sequenzabschnitt zwei Sequenzbereiche gleicher Länge auf, die miteinander ggf. hybridisieren können (Helix C). Die Hybidisierung wird dabei von jeweils drei bis sechs Nukleotiden bewerkstelligt. Zwischen den die Helix C ausbildenden Nukleotiden liegen drei bis fünf ungepaarte Nukleotide vor. Zwischen dem 3'-Ende von Box A1 und dem 5'-Ende von Helix C liegen eins bis drei Nukleotide ungepaart vor. Zwischem dem 3'-Ende von Helix C und dem 5'-Ende von Box A2 können eins bis fünf Nukleotide ungepaart vorliegen.

### 1.2.3 Helikale Struktur am 5'- und am 3'-Ende der Spiegelmere

Alle HMGA1a/b-bindenden Spigelmere zeichnen an ihren 5'- und 3'-Enden durch Sequenzabschnitte aus, die miteinander hybridisieren können (Helix A1 und Helix A2, (Fig. 1A und 1B). Die Anzahl der jeweils der miteinander hybridisierenden Nukleotide kann dabei von vier bis acht variieren. Dabei kann dieser vermeintlich doppelsträngige Bereich an das 5'-Ende von Box A1 und das 3'-Ende von Box A2 heranreichen. Sollte dies nicht der Fall sein, so können Box A1 und Box A2 von zusätzlich miteinander hybridisierenden Nukleotiden (Helix B1 und Helix B2) flankiert sein. Dabei kann es sich um Bereiche von jeweils vier bis acht Nukleotiden handeln (Fig. 1A und 1B).

Im Rahmen der der vorliegenden Anmeldung zugrundeliegenden Erfindung wurden verschiedenen Klassen von Nukleinsäuren und insbesondere L-Nukleinsäuren identifiziert, die an das Zielmolekül binden. Die folgende Darstellung und Beschreibung dieser Klassen, die hierin auch als Fälle bezeichnet werden, stellt insoweit einen integralen Bestandteil der vorliegenden Anmeldung dar. Im folgenden werden für eine jede Klasse deren prinzipieller Aufbau und beispielhafte L-Nukleinsäuren für diese Klasse unter Verwendung der jeweiligen Kurzbezeichnungen der L-Nukleinsäuren angegeben.

### Fall 1: 132-C3, 132-B3, 132-C4, 132-E2, 132-A2, 132-H1, 132-F1, 122-G2, 132-B3 32nt, 132-B3 33nt

Helix A1-Nₓ-Helix B1-N₆N₇Box A1 N₁N₂GN₈N₃N₄N₅BOX A2 G-N_{Y}-Helix B2-N_{z}-Helix A2 (Fall 1A)

**bzw.** Helix A1-Nx-Helix B1-N6N7Box A1N1N2GN8N3N4N5BOX A2Helix B2-Nz-Helix A2 (Fall 1B)
N₁ = U, C, A, G;
N₂ = G, U;
N₃ = U, C;
N₄ = U, A;
N₅ = G, A;
N₆ = G, A, U;
N₇ = G, U;
N₈ = U oder kein Nukleotid;
Nₓ = null bis fünf Nukleotide;
N_{y} = null oder sechs Nukleotide;
N_{z} = null bis sechs Nukleotide;

Box A1 = Box A2 = GGGCG oder GGGUG oder GGGAG;

Helix A1 und Helix A2 = jeweils vier bis acht Nukleotide, die vollständig oder teilweise miteinander hybridisieren, wobei die Summe der jeweils miteinander hybridisierenden Nukleotide aus Helix A1 und Helix A2 und **Helix B1 und Helix B2** 10 bis 12 Nukleotide beträgt;

**Helix B1 und Helix B2** = jeweils vier bis acht Nukleotide, die miteinander hybridisieren,
wobei die Summe der jeweils miteinander hybridisierenden Nukleotide aus Helix A1 und Helix A2 und **Helix B1 und Helix B2** 10 bis 12 Nukleotide beträgt.

Die Moleküle sind auch nach der Verkürzung am 5'- und am 3'-Ende aktiv. Nach der Entfernung der Helix A1 und A2 sowie sowie der Bereiche N₆N7 und GNy behalten die verkürzten Moleküle ihre Bindungseigenschaften bei. Gezeigt wurde dies für die verkürzten Varianten 132-B3 32nt (NOX-f 32nt) und 132-B3 33nt (NOX-f 33nt) (siehe Fig. 3 und 4).

### Fall 2A: 132-G2, 122-A1, 122-C1, 122-B2, 122-B4

Helix A1-Nₐ-Box A1-N_{b}-Helix C1-N_{c}-Helix C2-N_{d}-Box A2-G-Nₑ-Helix A2

Nₐ = ein bis fünf Nukleotide
N_{b} = drei Nukleotide
N_{c} = drei bis fünf Nukleotide
N_{d} = zwei bis fünf Nukleotide
Nₑ = eins bis zwei Nukleotide, bevorzugterweise A oder UU Box A1 = Box A2 = GGGCG oder GGGUG oder GGGAG Helix A1 und Helix A2 = jeweils fünf bis sechs Nukleotide, die vollständig oder teilweise miteinander hybridisieren,
   Helix C1 und Helix C₂ = jeweils fünf bis sechs Nukleotide, die miteinander hybridisieren,

### Fall 2B: 122-E2

Helix A1-Nᵢ-Helix B1-Nⱼ-Box A1-A-Helix C1-N_{c}-Helix C2-G-Box A2-G-Helix B2-A-Helix A2

Nᵢ = zwei Nukleotide, bevorzugterweise CA
Nⱼ = zwei Nukleotide, bevorzugterweise AG
N_{c} = vier Nukleotide, bevorzugterweise GAUG Box A1 = Box A2 = GGGCG oder GGGUG oder GGGAG Helix A1 und Helix A2 = jeweils sechs Nukleotide, die miteinander hybridisieren,
   **Helix B1 und Helix B2** = jeweils fünf Nukleotide, die miteinander hybridisieren
   Helix C1 und Helix C2 = jeweils drei Nukleotide, die miteinander hybridisieren

### Beispiel 2: Domain-Approach

### 2.1 Bestimmung der Interaktion von HMGA1a/b-Spiegelmeren und rekombinantem HMGA1b im Kompetitionsassay

### Durchführung/Methode

### Klonierung von His6-markiertem HMGA1b

Der BD-Freedom^{™} ORF Klon GH00552L1.0 (high mobility group AT hookl) mit der für HMGA1b kodierenden Sequenz wurde von BioCat, Heidelberg gekauft. Die Sequenz ist darin bereits so verändert, dass das Stop-Codon in ein für Leucin kodierendes Codon umgewandelt ist, um C-terminale Fusionen zu ermöglichen. Die Sequenz des Klons entspricht am meisten der in der RefSeq-Datenbank mit der Nummer NM002131 gespeicherten Sequenz. Mit den primern HMG_fwd1 (TCGACACCATGGGTGAGTC, Seq.ID 34) und HMG_rev1 (GTCTAGAAAGCTTCCCAACTG, Seq.ID 35) wurde mit Hilfe einer Standard-PCR die für HMGA1b kodierende Sequenz amplifiziert. Dabei wurde die Base nach dem ATG von A nach G verändert, um damit eine Ncol-Schnittstelle einzuführen. Das PCR-Produkt wurde entsprechend der Herstellerangaben mit den Restriktionsenzymen NcoI und HindIII Beide von NEB, Frankfurt am Main; Deutschland) geschnitten und über ein Agarosegel gereinigt. Der Vektor pHO2d (Fasshauer et al. (1997) J.Biol.Chem. 272:28036 - 28041) ) wurde ebenfalls mit Ncol und HindIII geschnitten und über ein Agarosegel gereinigt. Der Vektor pHO2d erlaubt die Expression eines am C-terminalen Ende mit einer Sequenz aus 6 Histidinresten (His6-tag) fusionierten Proteins unter Kontrolle eines T7-Promotors (Fasshauer et al., 1997, JBC 272:28036).

Das gereinigte und geschnittene PCR-Produkt wurde über Nacht bei 15°C mit Hilfe einer T4-Ligase entsprechend der Herstellerangaben (MBI Fermentas, St. Leon-Roth, Deustchland) in den vorbereiteten Vektor ligiert. Mit dem Ligationsprodukt wurden Bakterien vom Stamm DH5□ transformiert. Die Korrektheit der Plasmide aus erhaltenen Kolonien wurde durch Sequenzierung sichergestellt. Das von pHO2d/HMGA1b kodierte Fusionsprotein HMGA1b-His6 hat gegenüber dem natürlichen HMGA1b-Protein ein Glycin (G) statt Serin (S) an Position 2 und nach dem C-terminalen Glutamin (Q) ein Leucin (L) (s.o.), gefolgt von 5 weiteren Aminosäuren (G S L N S) (vom Vektor kodiert), an die sich die sechs Histidine (H) anschließen.

### Expression und Reinigung von HMG1b-His6

Für die Expression des Fusionsproteins wurden Bakterien vom Stamm BL21 mit dem Plasmid pHO2d/HMGA1b transformiert. Die Expression des Fusionsproteins wurde mit Isopropylthio-β-D-Galaktosid (IPTG) induziert. Nach 4 h wurden die Bakterien für 15 min bei 10.000 x g abzentrifugiert und das Pellet bis zur weiteren Verwendung bei -20°C aufbewahrt.

Für die Extraktion des Fusionsproteins wurden zu einem gefrorenem Bakterienpellet von 500 ml Kultur 25 ml Extraktionspuffer (1% n-Octyl-β,D-Thioglucopyranosid (OTG) in 50 mM NaₓPO₄-Puffer, pH 8.0, 250 mM NaCl, 10 mM Imidazol und MiniProteaselnhibitoren-Tabletten (Roche, Mannheim, Deutschland) (5 St./50 ml)) gegeben, mit 5 µl Benzonase (grade 1; MERCK, Darmstadt, Deutschland) versetzt, durch Auf- und Abpipettieren homogenisiert und 5 min bei RT inkubiert. Danach wurde 15 min bei 10.000 x g (RT) zentrifugiert. Der Überstand wurde durch einen Faltenfilter geklärt und dann auf eine mit Waschpuffer (50 mM NaₓPO₄-Puffer, pH 8.0, 250 mM NaCl, 10 mM Imidazol, alle MERCK, Darmstadt, Deutschland) äquilibrierte HIS-Select-Säule (HIS-SELECT Cartridge, Sigma, Deisenhofen, Deutschland) aufgetragen. Nach Waschen der Säule mit 10 - 15 ml Waschpuffer wurde das Fusionsprotein mit Elutionspuffer (250 mM Imidazol in Waschpuffer) in 0,5 - 1 ml großen Fraktionen eluiert. Proteinhaltige Fraktionen wurden mittels Gelelektrophorese (16% Polyacrylamidgel nach Schäger & Jagow, 1987, Anal.Biochem. 166:368-379) auf Reinheit überprüft. Fraktionen mit Fusionsprotein wurden vereinigt, ggf. gegen einen geeigneten Puffer dialysiert und nach Proteinbestimmung nochmals auf Reinheit überprüft. Das gereinigte Fusionsprotein wurde in Aliquots bei -20°C gelagert.

### Bestimmung der Interaktion von HMGA1a/b-Spiegelmeren und HMGA1b-His6

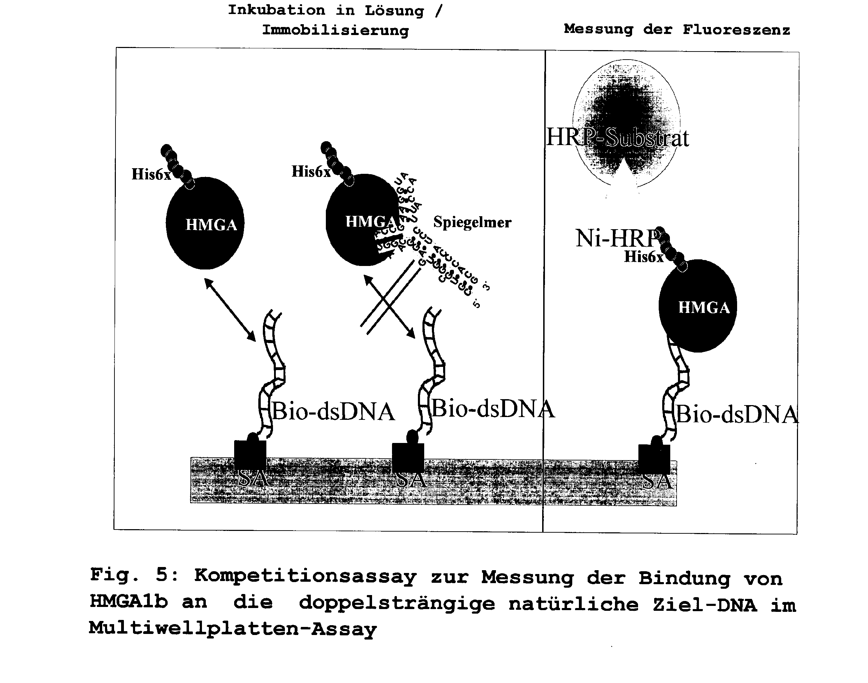

Zur genaueren Analyse der Affinität der HMGA1a/b-Spiegelmere zu HMGA1b wurde ein auf dem 96-well Format basierender Test verwendet. In diesem Test verhindert die Bindung der HMGA1a/b-Spiegelmere an HMGA1b-His6 dessen Interaktion mit einem DNA-Oligonukleotid, das eine Bindungsstelle für HMGA1a/b besitzt. Dieses DNA-Oligonukleotid (dsDNA AT-Hook) (Fashena et al., 1992) ist an einem Strang mit einem Biotin-Molekül markiert, über das es an mit Streptavidin beschichtete Platten gebunden werden kann. Der Nachweis von an DNA gebundenem HMGA1b-His6 erfolgt über mit Nickel modifizierter Meerrettichperoxidase (Nickel-HRP), welche ein fluorogenes Substrat umsetzt. Das Spiegelmer verdrängt in diesem Assay das rekombinante HMGA1b von seinem natürlichen Bindungspartner. Auf Grund der 1:1:1 Stöchometrie von Spiegelmer/ rHMGA1b/ dsDNA AT-Hook lassen sich direkt Aussagen über die Affinität der Spiegelmere gegenüber HMGA1b treffen. Das Prinzip des Assays ist in Fig. 5 dargestellt.

Zur Durchführung dieses Tests werden in einer Spitzbodenplatte Spiegelmere in verschiedenen Konzentrationen und HMGA1b-His6 (0,36µg/ml; ca. 30 nM) in einem Gesamtvolumen von 100 µl 10 min bei Raumtemperatur unter Schütteln inkubiert. Die Inkubationslösung enthält ausserdem: 25 mM Tris/HCl, pH 7.0 (Ambion, Austin, TX, USA), 140 mM KCl (Ambion, Austin, TX, USA), 12 mM NaCl (Ambion, Austin, TX, USA), 0,8 mM MgCl2 (Ambion, Austin, TX, USA), 0,25 mg/ml BSA (Roche, Mannheim, Deutschland), 1 mM DTT (Invitrogen, Karlsruhe, Deutschland), 18 - 20 µg/ml poly(dGdC) (Sigma, Deisenhofen, Deutschland)), 0,05 % Tween 20 (Roche, Mannheim, Deutschland). Dann werden 2 µl biotinyliertes DNA-Oligonukleotid dsDNA AT-Hook (äquimolare Mischung von 5'biotin-TCGAAAAAAGCAAAAAAAAAAAAAAAAAACTGGC (34nt) und 5'GCCAGTTTTTTTTTTTTTTTTTTGCTTTTTT (31 nt); 75 µM in 150 mM NaCl (Ambion, Austin, TX, USA)) zugegeben und weitere 10 min unter Schütteln bei RT inkubiert. Anschließend werden die Ansätze in eine mit Streptavidin beschichtete schwarze 96-well Platte (ReactiBind v. Pierce, Bonn, Deustchland)) überführt und 30 min unter leichtem Schütteln bei RT inkubiert. Danach werden die Vertiefungen der Platte drei mal mit je 200 µl TBSTCM (20 mM Tris/HCl, pH 7,6 (Ambion, Austin, TX, USA); 137 mM NaCl (Ambion, Austin, TX, USA), 1 mM MgCl2 (Ambion, Austin, TX, USA), 1 mM CaCl2 (Sigma, Deisenhofen, Deustchland), 0,05% Tween20 (Roche, Mannheim, Deustchland)) gewaschen.
In jede Vertiefung werden 50 µl einer verdünnten Nickel-HRP-Lösung gegeben (ExpressDetector Nickel-HRP, (Medac, Hamburg, Deutschland) 1:1000 in 10 mg/ml BSA (Roche, Mannheim, Deustchland)in TBSTCM) und 1 h unter leichtem Schütteln bei RT inkubiert. Danach werden die Vertiefungen wieder drei mal mit je 200 µl TBSTCM gewaschen. Zu jeder Vertiefung werden dann 100 µl des fluorogenen HRP Substrats (QuantaBlue, Pierce, Bonn, Deutschland) gegeben und nach 15 min die Fluoreszenz gemessen (ex: 340/em: 405 nm).

### Ergebnis

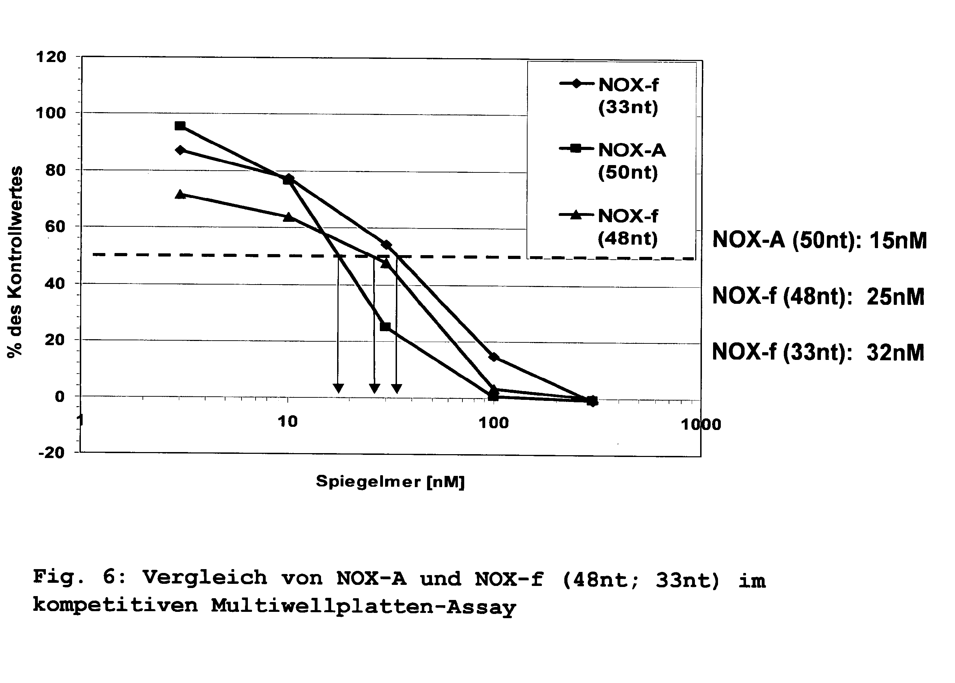

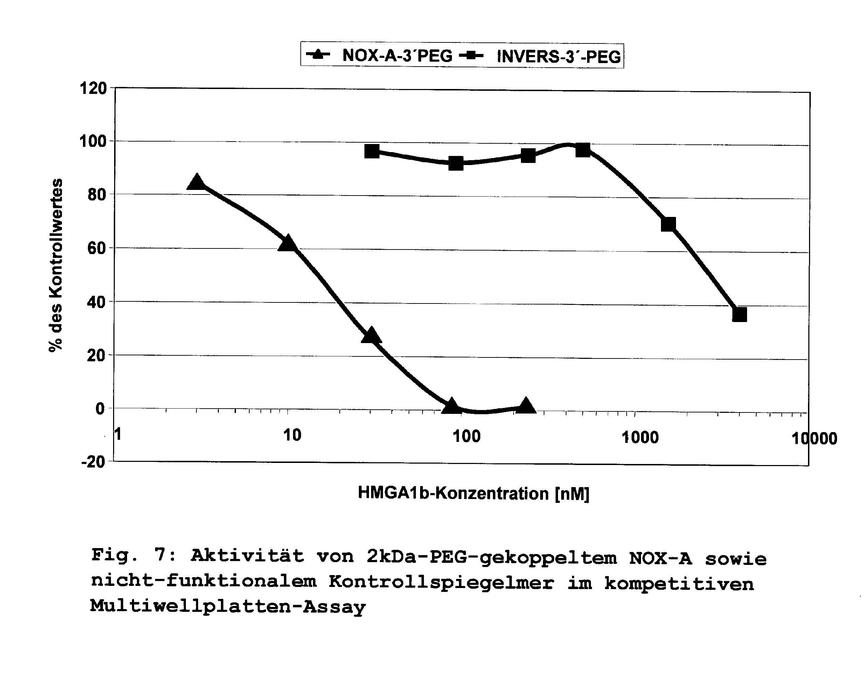

Es konnte gezeigt werden, dass die Spiegelmere NOX-A (50nt), NOX-f (33nt) und NOX-f (48nt) konzentrationsabhängig mit der Bindung von HMGA1b-His6 an das biotinylierte DNA-Oligonukleotid kompetieren (Fig. 6). Für Spiegelmer NOX-A ergibt sich eine IC50 von ca. 15 nM.
Im Gegensatz zu dem aktiven Spiegelmer zeigte ein Kontrollspiegelmer mit zu NOX-A inverser Sequenz bis zu einer Konzentration von 0,5 µM keinen Effekt auf die Bindung von HMGA1b-His6 an das DNA-Oligonukleotid hat und erst bei Konzentrationen über 1 µM unspezifische Interaktionen mit HMGA1b-His6 auftreten (Fig. 7).

### 2.2 Verwendung von Spiegelmeren zum Nachweis von HMGA1b durch Western Blot

### Durchführung/Methoden

Das rekombinant exprimierte HMGA1b wurde über Gelelektrophorese auf einem 16% PAA-Tricin-Gel aufgetrennt und mittels Elektroblottings auf Nitrozellulosemembranen transferiert. Die Membran wurde nachfolgend mit 5% Magermilch und 100nM unspezifischen Spiegelmer in 1xTBST (20 mM Tris/HCl ph 7.6, 137 mM NaCl, 0.1% Tween) für 1 Stunde geblockt und 3mal für 10 Minuten mit 1xTBST gewaschen. Die Detektion des rekombinaten HMGA1b erfolgte mit am 5'-Ende biotinyliertem Spiegelmer NOX-A (5'bioNOX-A). 5'bioNOX-A wurde in 1xTBST mit je 1mM Kalzium und Magnesium (TBST+Ca/Mg)und 100nM unspezifischem Spiegelmer verdünnt und für 1,5 Stunden inkubiert. Der Blot wurde anschließend 3mal für 10 Minuten mit 1x TBST+ Ca/ Mg gewaschen und das gebundene biotinylierte Spiegelmer mit einem anti-Biotin-Antikörper in TBST+ Ca/Mg für 45 Minuten inkubiert. Anschließend wurde der Blot 5mal für 10 Minuten mit IxTBST+Ca/Mg gewaschen und der mit gekoppelter Meerrettichperoxidase (HRP) Sekündarantikörper mittels LumiGLO Nachweisreagens (Cell Signaling Technology) detektiert.

### Ergebnis

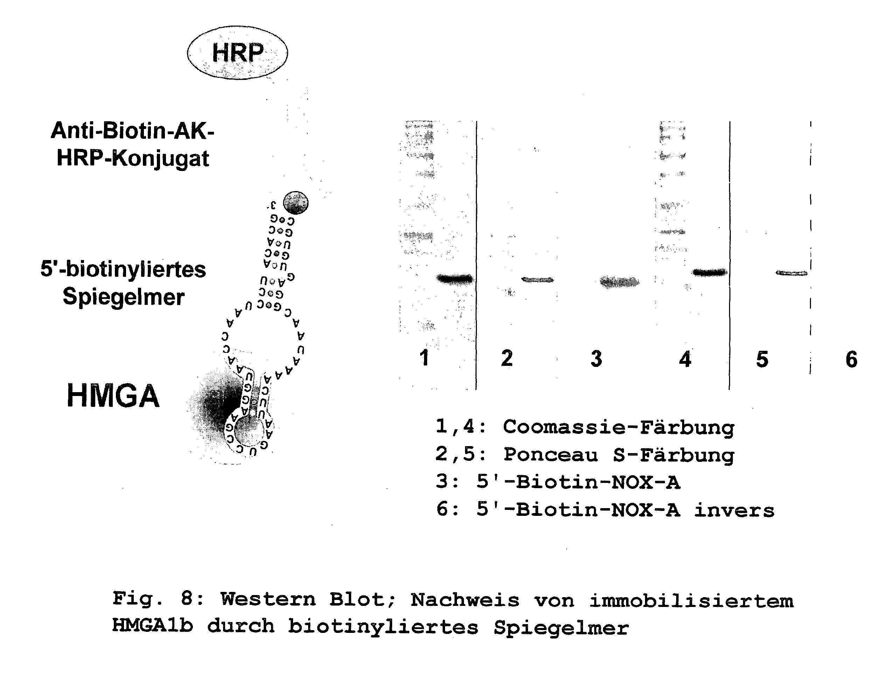

Mit Hilfe des beschriebenen Verfahrens konnte die Bindung eines 5'-terminal biotinylierten Spiegelmers an das rekombinant exprimierte HMGA1b gezeigt werden. Ähnlich einer auf Antikörper basierenden Detektion wurden 5µg HMGA1b nach Transfer auf eine Blotmembran mit 3nM bio-NOX-A detektiert. Das inverse Spiegelmer von NOX-A konnte HMGA1b nicht erkennen, was die spezifische Bindung von NOX-A belegt (Fig. 8).

### Beispiel 3: PEI-Spieglmer-Formulierung

### 3.1 Prinzip der Polyethylenimine vermittelten Transfektion von Spiegelmeren

Das Zielmolekül HMGA1a/b wird im Zytosol exprimiert und findet als Transkriptionsfaktor seinen natürlichen Bindungspartner, die doppelsträngige DNA im Zellkern. Durch Bindung des Spiegelmers an HMGA1a/b im Zytosol und Kompetition des durch die AT-Hooks auf der DNA gebundenen HMGA1a/b im Zellkern der Zelle sollen die HMGA1a/b-vermittelten zellulären Antworten antagonisiert werden. Aufgrund der negativen Ladung der Plasmamembran werden DNA- und RNA-Moleküle schlecht durch passiven Transport von einer Zelle aufgenommen. Einer der Ansätze des intrazellulären Transports vom Nukleinsäuren ist die Kondensation bzw. Verpackung mit geladenen Partikeln oder Reagenzien und damit verbundene Änderung der Ladung des Gesamtkomplexes. Dieser Komplex wird leicht über Endozytose aufgenommen und gelangt somit in das Zytosol der Zelle. Nachteile dieser Methode ist die Stabilität der DNA/ RNA bzw. das Freisetzen der Nukleinsäure aus dem endosomalen Kompartementen. Im Zytosol der Zelle entsteht aus dem eingeschnürten Endosom durch das Einbringen von Proteasen bzw. Nukleasen und durch Protonierung des Kompartiments schnell ein Lysosom. Nukleasen verdauen dort die Nukleinsäuren und zudem ist die Nukleinsäure im saurem Milieu nicht stabil. Über Exozytose bzw. Abbau im Golgi Apparat wird der gesamte Komplex schnell wieder aus der Zelle transportiert und somit gelangen nur wenige Nukleinsäuren in die Zelle. Eine der Herausforderung an ein geeignetes Transfektionssystem ist somit die Stabilisierung, sowie die Freisetzung der Nukleinsäure aus den Endosomen in das Zytosol. Hinsichtlich der Stabilität haben RNA-Spiegelmere ideale Eigenschaften für eine Transfektion von eukaryotischen Zellen, da sie als unnatürliche Enantiomere nicht von Enzymen gespalten werden.

Das ausgewählte Transfektionssystem basisiert auf der Bildung von Mizellen von Nukleinsäuren mit verzweigtem Polyethylenimin (PEI). Das Phosphatrückrat der Nukleinsäuren interagiert mit den freien Stickstoff-Positionen des PEI und bildet über Querverzweigung kleine Mizellen, welche aufgrund des PEI eine positive Ladung haben. Dabei wird PEI mit einem Molekulargewicht von 3 bis 800 kDa verwendet. Je kleiner das PEI gewählt wird desto kleiner sind die gebildeten Mizellen. Die Verwendung von 25 kDa querverzweigtem PEI (Sigma-Aldrich Cat. No. 40;872-7) führt bei der Zugabe von Nukleinsäuren zu Polyplexen von einer Größe von 100 bis zu 500nm, typischerweise jedoch zu Polyplexen einer Größe von 100-200nm. In der Regel wird eine Stickstoff/ Phosphat-ratio von 2:1 bis 5:1 verwendet; teilweise sogar bis zu 20:1. Die Verpackung der Nukleinsäure in Mizellen hat eine Änderung des zeta-Potential des Komplexes auf ∼ (+)21mV bei einer N/P 3 zur Folge. Es ist bekannt, dass mit zunehmenden, positiven zeta-Potential von Komplexen die Toxizität für kultivierte Zellen steigt. Diese Mizellen werden jedoch leicht durch Einschnürung der Plasmamembran als Endosomen von einer Zelle aufgenommen. Das PEI puffert nun einströmende Protonen ab, woraufhin viele Chlorid-Ionen im Inneren des Endosoms zu einem Anschwellen des Kompartiments aufgrund des osmotischen Drucks führen. Dieser Effekt des PEI wird als Protonen-Schwamm-Effekt in der Literatur beschrieben (Sonawane et al., JBC, 2003, Vol.278; No.45(7) pp.44826-44831) und führt letztlich zum Platzen des Endosoms und zur Freisetzung der Spiegelmere in das Zytosol.

Der Nukleinsäure-PEI Komplex neigt aufgrund einer stark positiven Ladung zur Interaktion und Aggregation mit Serumproteinen, sowie zur bereits beschriebenen Zelltoxizität. So wurde in der Literatur beschrieben, dass hohe Dosen an Nukleinsäure-PEI Mizellen nach subkutaner und intravenöser Injektion in Ratten schnell zu einer Akkumulation in der Lunge und somit zu Embolien/ Infarkten führen kann. Die Lösung für das Problem stellt die Derivatisierung der Nukleinsäure mit 2kDa Polyethylenglycol (PEG) dar. Diese Reste legen sich wie ein Schild um die Mizellen und verhindern die Bindung an Serumproteine ( Ogris et al., Gene Therapy, 1999, 6(595-605). Des Weiteren wird das zeta-Potential auf +/- OmV abgesenkt, was zu einer geringeren Zelltoxizität bei gleichbleibender Pufferkapazität des PEI hinsichtlich des protonsponge-effect führt.

### 3.2 Aktivität Spiegelmer mit PEG2000

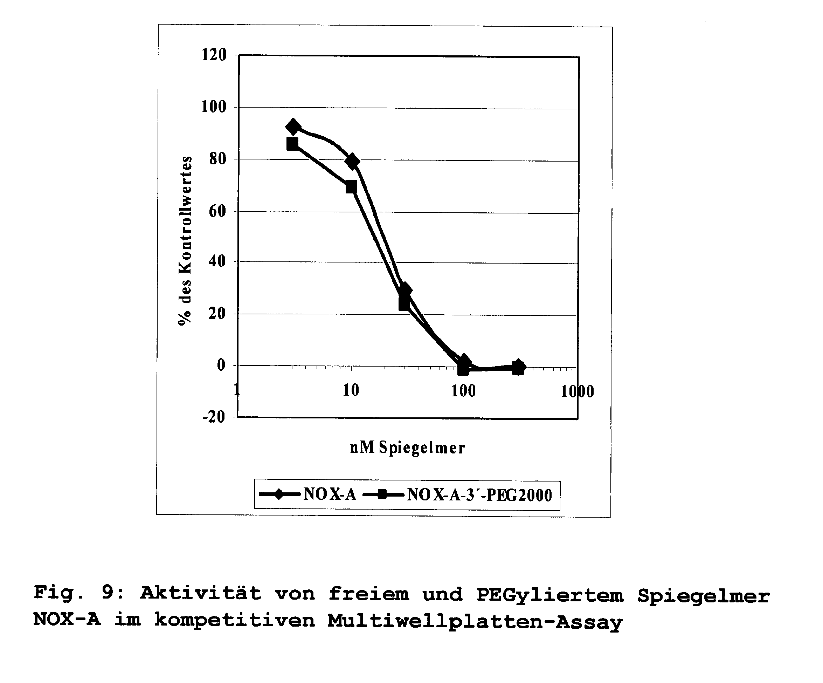

Die Leitkandidaten NOX-A und NOX-f wurden synthetisch als Aptamer und Spiegelmer mit 3'-terminaler Aminogruppe hergestellt und anschließend über den Amino-Rest PEGyliert. Mittels Gleichgewichtsanbindungsassays konnte gezeigt werden, dass die PEGylierung keinen Einfluss auf die Bindungseigenschaften der Aptamere an das HMGA1a/b Fragment hat. Des Weiteren konnte mittels Kompetitionsassays mit rekombinantem Volllänge- HMGA1a/b gezeigt werden, dass auch die Bindung von Spiegelmeren an das Volllänge- HMGA1a/b unabhängig von der 3'-terminalen PEGylierung ist (Fig. 9).

### 3.3 Verpackung Spiegelmer

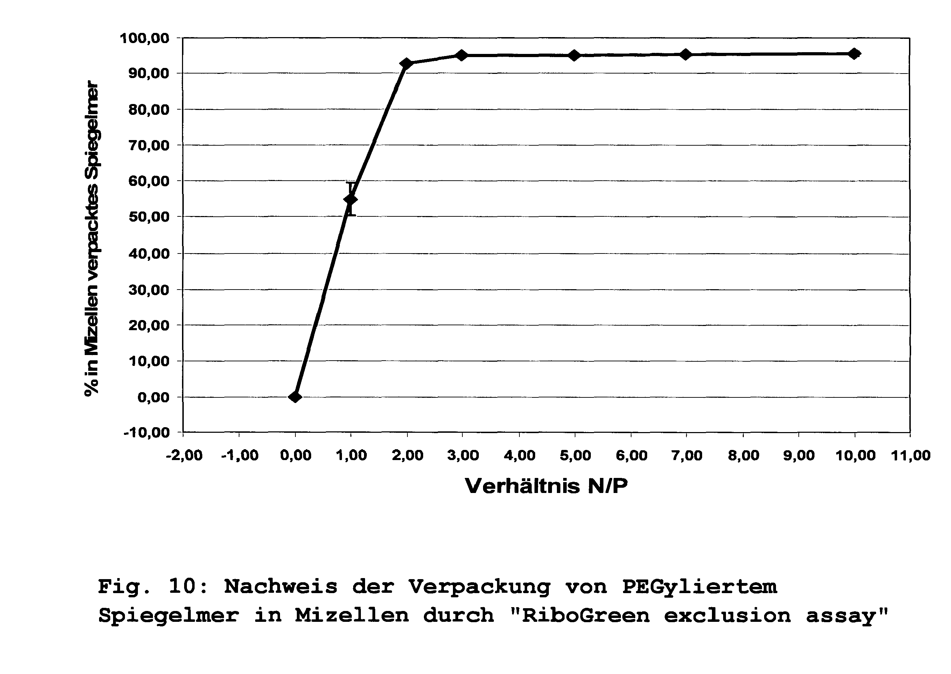

Die Verpackung von sterilem, PEGyliertem Spiegelmer erfolgte in PBS durch Zugabe von 25kDa querverzweigtem Polyethylenimine (PEI) (ALDRICH, Cat.: 40,872-7) in einem Verhältnis von 2,5:1 des absoluten Stickstoffanteils des PEI zum absoluten Phosphat des Ribonukleinsäurerückgrats (N/P 2,5). Die sterile, autoklavierte PEI Lösung hatte eine Konzentration von 200mM freien Stickstoffgruppen und wurde mit 1 M Salzsäure auf einen pH von 7,4 eingestellt. Das sterile filtrierte Spiegelmer wurde dabei mit einer Konzentration von bis zu 700 µM in 1xPBS mit Ca/Mg vorgelegt und nach der Zugabe von steril filtriertem PEI für 30-60 Minuten bei Raumtemperatur inkubiert. Idealerweise läuft die Komplexbildung bei möglichst geringer vorgelegter Konzentration an Spiegelmer ab, da hohe Konzentration an Spiegelmer zu unspezifisch großen Aggregaten führen. Die Bildung von Spiegelmer-Mizellen wurde mittels eines Farbstoff-Ausschluss-Assays gemessen. Dabei wurde bestimmt, wie viel Spiegelmer sich per Farbstoff vor und nach Verpackung in Mizellen nachweisen lässt. Als Farbstoff wurde RiboGreen (M. Probes) verwendet und die Fluoreszenz mit einem ELISA-Reader bestimmt. Je 1µM Spiegelmer wurde in 100µl 1xPBS vorlegt und steigende Mengen an PEI zugegeben. In einer fluoreszenz-geeigneten 96-well Mikrotiterplatte wurden 100µl 0,2µg/µl RiboGreen vorgelegt und nach einer Inkubationzeit des Mizellenansatzes von 30 Minuten bei Raumtemperatur je 10µl in die Mikrotiterplatte pipettiert. Ab einem N/P Verhältnis von 2 lagen mehr als 90% der Spiegelmere als Mizellen vor (Fig.10). PEI allein hatte dabei keinen Einfluss auf die Fluoreszenz des Farbstoffs

### 3.4 Stabilität Spiegelmer-Micellen

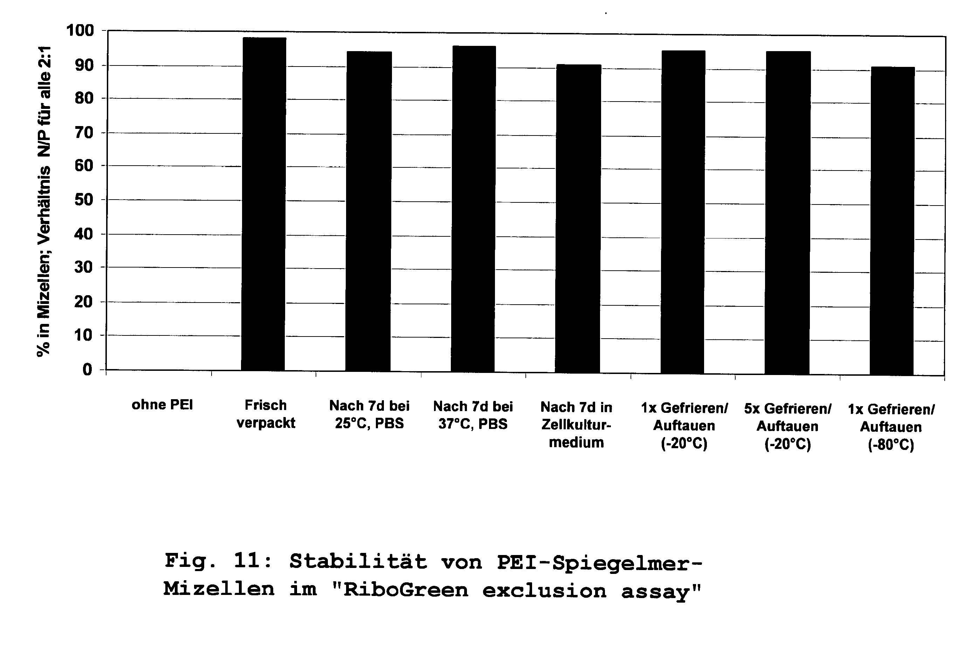

1µM Spiegelmermizellen wurden unter in Fig. 11 aufgeführten Bedingungen gelagert. Die Stabilität von Spiegelmer-Mizellen wurde mit unter Absatz 3.3 beschriebenen Farbstoff-Ausschluss-Assays gemessen. Eine Stabilitätsstudie der Mizellen zeigte, dass die Lagerung von Mizellen in unterschiedlichen Medien, sowie bei verschiedenen Temperaturen keinen Einfluss auf die Spiegelmer-Mizellen hat. In der Literatur beschieben ist auch die Gefriertrocknung von Ribozym/ PEI-Komplexen ohne Verlust der Eigenschaften des Ribozyms (Brus-C et al., J. Control Release, 2004, Feb, 20, 95(1), 199-31)

### 3.5 Aufnahme von Spiegelmer-Micellen

Die intrazelluläre Aufnahme von Spiegelmer Mizellen wurde am Zellkultursystem von HS578T Zellen etabliert. 1x10⁴ HS578T Zellen wurden auf sterile 20mm Deckgläschenwurden bis zu einer Konfluenz von 30-40% wachsen gelassen. 5'-markiertes Spiegelmer NOX-A-3'-PEG wurde mit einer N/P Ratio von 2,5:1 in Mizellen verpackt, in einer Konzentration von 1µM zu den Zellen gegeben und für 16 Stunden bei 37°C inkubiert. Als Kontrolle für die passive Aufnahme von Spiegelmeren wurde je 1µM reines Fluoreszenz-markiertes Spiegelmer mit den Zellen inkubiert. Die Zellen wurden anschließend 3x1ml 1xPBS gewaschen und für 30 Minuten mit 3% Paraformaldehyd fixiert. Die Präparate wurden wiederum mit 3x1ml 1xPBS gewaschen, zur Färbung des Chromatins im Zellkern für weitere 10-20 Sekunden mit einer DAPI-Lösung (1µl Stock auf 10ml 1xPBS) inkubiert, nochmals gewaschen und mit einer Paramountlösung eingedeckt. Die so vorbereiten Präparate wurden an einem Fluoreszenzmikroskop analysiert (Emission 488nm/ Extinktion 514-522nm).

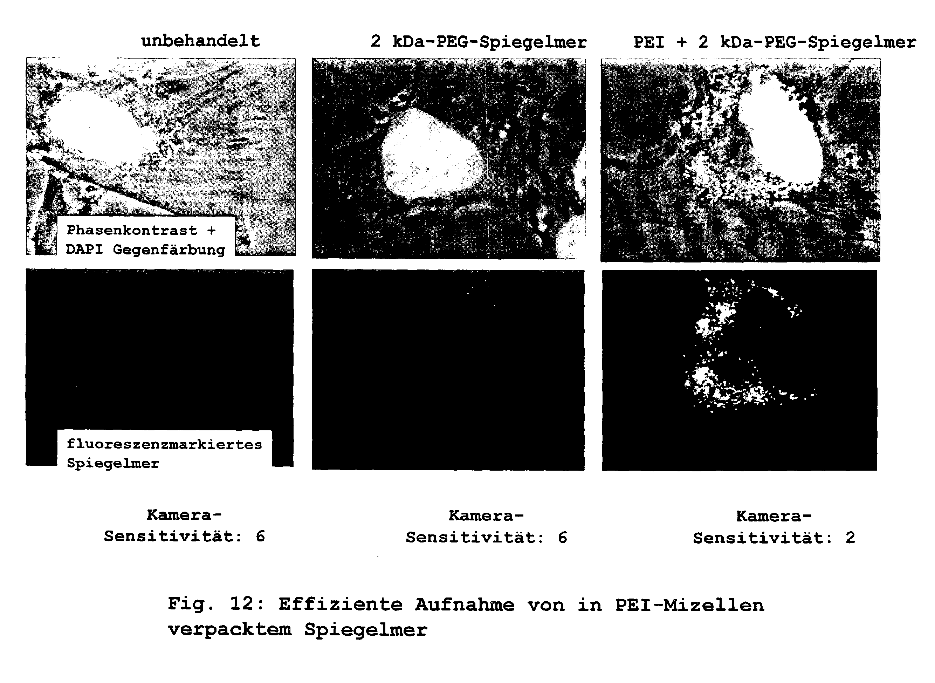

Es konnte gezeigt werden, dass Spiegelmer-Mizellen eine erhöhte Transfektionsquantität gegenüber "nackten", nicht verpacktem Spiegelmer haben (Fig. 12)

Die Transfektionseffizienz lag dabei >95% der Gesamtzellen und zeigte keinen Einfluss auf die Morphologie der Zellen. Das 5'-FITC-gekoppelte Spiegelmer war hauptsächlich im Zytosol und assoziiert an die Plasmamembran zu finden. Das punktuelle Verteilungsmuster weist auf einen Einschluss in Kompartimenten und das diffuse Muster auf freigesetztes Spiegelmer hin. Im Zellkern konnten nur ein geringes Signal an Spiegelmer detektiert werden.

### 3.6 Freisetzung von Spiegelmer

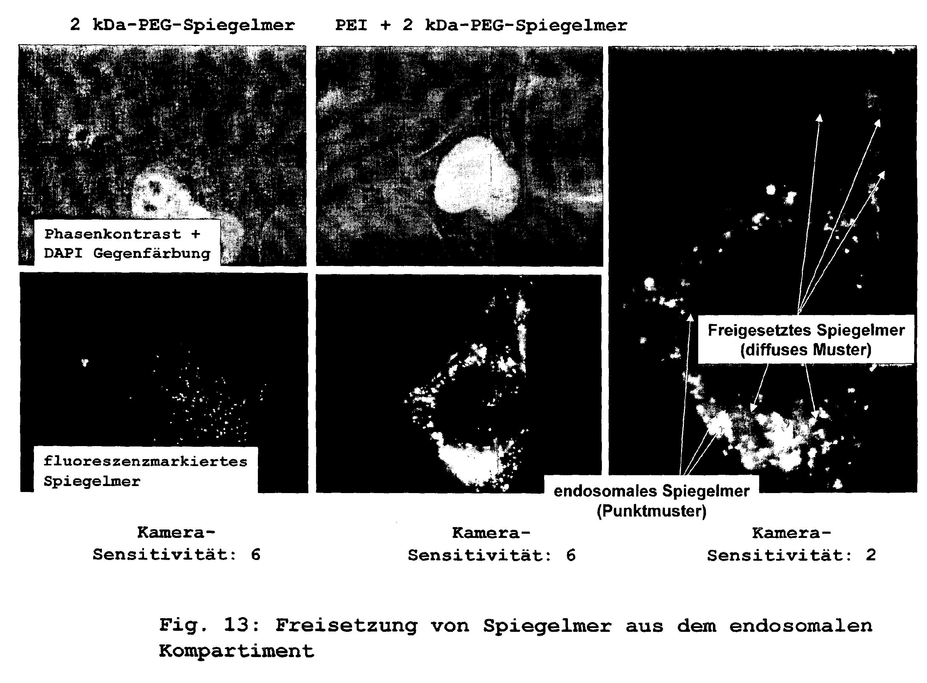

Die punktuelle Verteilung des Spiegelmere im Zytosol und perinukleären Raum der H578T Zellen deutet auf eine Anreicherung in Kompartementen der Zellen, z.B. Endosomen hin. Zur Überprüfung der Freisetzung der Spiegelmere aus diesen Kompartementen wurde das Verteilungsmuster einzelner stark vergrößerter Zellen analysiert (Fig.13). Es konnte zusätzlich zu der punktuellen Lokalisation der Spiegelmere ein diffuses Verteilungsmuster im Zytosol und an der Plasmamembran festgestellt werden, was ein Hinweis auf die endosomale Freisetzung der Spiegelmeren ist. Dieses Muster konnte bei "nackten" Spiegelmer nicht festgestellt werden.

### Beispiel 4: Bioaktivität in vivo

### 4.1 Proliferationsassay ohne PEI

### Wirkung auf die Proliferation von MCF-7 Zellen

Die potentielle Rolle von HMGA1a/b bei der Zellteilung wurde mittels Proliferations-Assays untersucht. Zunächst wurde Spiegelmer in einer hohen Dosis als "nackte" Nukleinsäure in das Zellkulturmedium gegeben und das Wachstum der Zellen über die Zeit verfolgt. Die Brustkrebszelllinie MCF-7 wurde als Modell ausgesucht, da in diesen Zellen eine geringe(zu antagonisierende) Expression von HMGA1a/b gefunden, bzw. die Rolle von HMGA1a/b in der Proliferation dieser Zellen bereits in der Literatur beschrieben wurde. Reeves et al. (Reeves-R et al., Molecular and Cellular Biology, Jan 2001, p575-594) konnten zeigen, dass die Überexpression von HMGA1a/b in MCF-7 Zellen zu einer verstärkten Proliferation führt und die Inhibition von HMGA1a/b mittels exprimierter antisense-Konstrukte die Proliferation von MCF-7 Zellen inhibiert.

### Durchführung/ Methode

0,5 x 10⁴ MCF-7 Zellen (ATCC) wurden in 96-well Platten (Costar) mit planem, durchsichtigem Boden ausgesät und für 16-24 Stunden in 100 µl RPMI 1640-Medium mit 10% fötalem Kälberserum (FCS) kultiviert. Anschließend wurden die Zellen mit PBS gewaschen und für weitere 48 Stunden mit Standardzellkulturmedium unter direkter Zugabe von steril filtriertem Spiegelmer kultiviert. Es folgte die Zugabe von 10µl einer Resazurin-Lösung (0,44 mM in PBS) zu den jeweiligen Ansätzen und die weitere Inkubation für 2 Stunden bei 37°C. Die Umsetzung von Resazurin durch den Stoffwechsel der Zelle korreliert direkt mit der Zellzahl. Die Farbänderung wurde in einem Fluostar Optima Multidetektions-Plattenlesegerät (BMG) gemessen (Emission 544 nm, Extinktion 590 nm). Jeder Wert wurde pro Experiment als Dreifachansatz bestimmt und auf die Werte von unbehandelten Kontrollzellen bezogen.

### Ergebnis

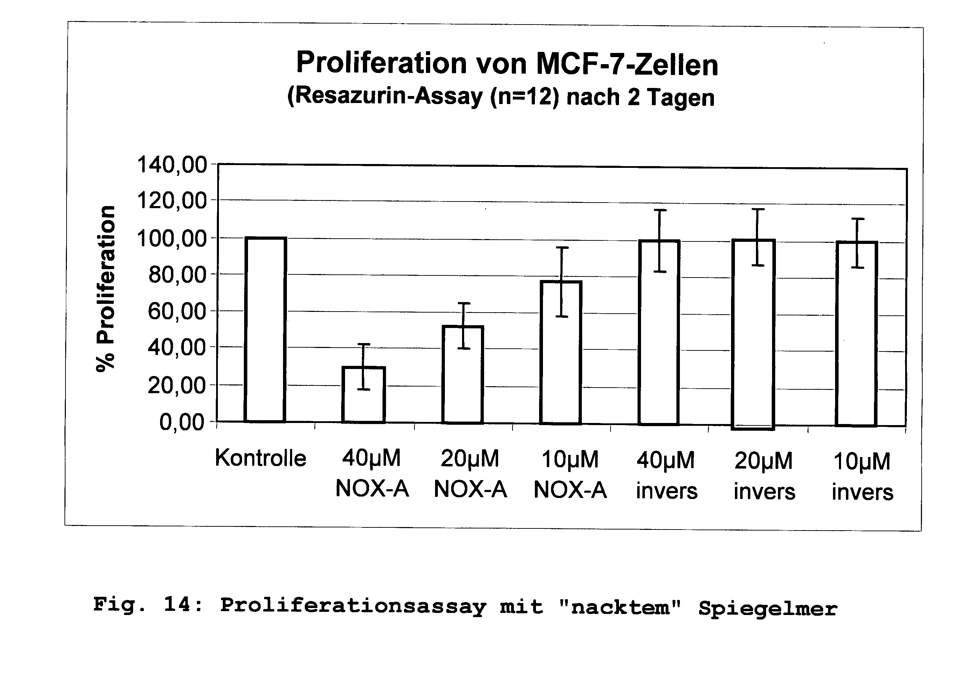

NOX-A inhibierte nach 2 Tagen Dosis-abhängig die Proliferation von MCF-7 Zellen (n=12) (Fig. **14**). Die maximale Hemmung der Proliferation auf ca. 30% des Wertes von unbehandelten Zellen wurde bei 40 µM gemessen. Bei Konzentrationen bis 40 µM konnte kein unspezifischer Effekt des inversen Kontroll-Spiegelmer festgestellt werden.

### 4.2 Proliferationsassay mit PEI

### Wirkung von Spiegelmer-Mizellen auf die Proliferation von H-1299 Zellen

### Durchführung/ Methode

1x10⁴ NCI-H-1299 Zellen (Lungenkarzinomzellen; ATCC) wurden in 24-well Platten (Costar) mit planem, durchsichtigem Boden ausgesät und für 16-24 Stunden in 1ml RPMI 1640 Medium mit 10% FCS kultiviert. Anschließend wurden die Zellen 2x mit PBS gewaschen und für weitere 3 Tage mit Zellkulturmedium mit 1% FCS und Spiegelmer-Mizellen kultiviert. Die Verpackung von sterilem, PEGyliertem Spiegelmer erfolgte zuvor in PBS durch Zugabe von 25kDa querverzweigtem Polyethylenimine (PEI) (Sigma) in einem Verhältnis von 2,5:1 des absoluten Stickstoffanteils des PEI zum absoluten Phosphat des Ribonukleinsäurerückgrats (N/P 2,5). Das sterile Spiegelmer wurde dabei mit einer Konzentration von 30 µM vorgelegt und nach der Zugabe des PEI für 30-60 Minuten bei Raumtemperatur inkubiert. Anschließend wurden die Spiegelmer-Mizellen mit Zellkulturmedium mit 1% FCS auf 1µM verdünnt, direkt zu den gewaschenen Zellen gegeben und für 3 Tage bei 37°C inkubiert
Es folgte die Zugabe von 100 µl Resazurin-Lösung zu den jeweiligen Ansätzen und die weitere Inkubation für 2 Stunden bei 37°C. Die Umsetzung von Resazurin durch den Stoffwechsel der Zelle korreliert direkt mit der Zellzahl. 100µl wurden den Ansätzen entnommen, in eine 96-well Platte überführt und die Farbänderung in einem Fluostar Optima Multidetektions-Plattenlesegerät (BMG) gemessen (Emission 544 nm, Extinktion 590 nm). Jeder Wert wurde pro Experiment als Doppelansatz bestimmt und auf die Werte von unbehandelten Kontrollzellen bezogen.

### Ergebnis

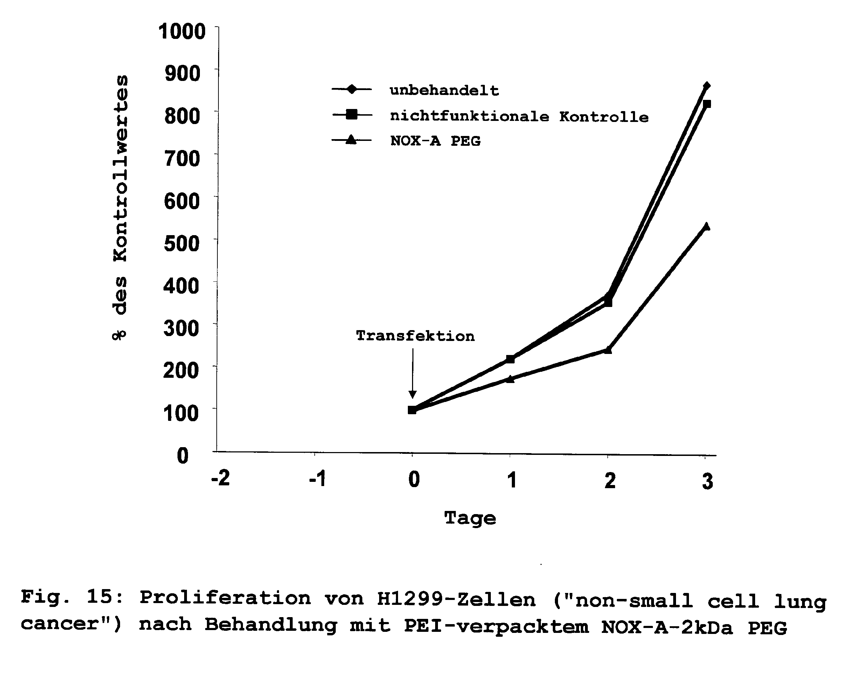

Die Verwendung von PEI (N/P 2,5) bei 1µM Spiegelmer zeigte zunächst keine Wirkung auf die Proliferation von Zellen. Durch Reduktion der Menge an FKS im Zellkulturmedium unter 1% konnte gezeigt werde, dass die Transfektion mit Spiegelmer-Mizellen einen Einfluss auf die Proliferation von H-1299 Zellen hat, welcher bei 10% FKS zuvor nicht sichtbar war (Fig 15). Möglicherweise stimuliert FKS die Proliferation so stark, dass der geringe Effekt nicht beobachtet werden konnte. Die Reduktion der FKS-Konzentration bei MCF-7 Zellen führte zum Absterben der Zellen über einem Zeitraum von 3 Tagen.

### 4.3 Inhibition Tumormarker cdc25a (mit PEI)

### Wirkung auf die HMGA1a/b vermittelte Regulation von Zellzyklusfaktoren am Beispiel des potentiellen Onkogens cdc25a

Reeves et al. (Molecular and Cellular Biology, Jan 2001, p575-594) konnten mittels cDNA Arrays durch Überexpression von HMGA1a/b in MCF-7 Zellen zeigen, dass HMGA1a/b die Expression einer Vielzahl von Genen induziert. Bis zu 100-fach überexprimiert werden dabei auch Zellzyklus- und Wachstumsfaktoren, wie etwa das als potentielles Onkogen identifizierte *cdc25a* (cell-division-cycle 25a-Phosphatase), welches für die Kontrolle des Übergangs von der G1 zur S-Phase des Zellzyklus eine entscheidende Rolle spielt. Die Aktivierung von solchen Kontrollpunkten führt nach Hemmung der Zellzyklus-Progression entweder zur Transkription von Genen, die an der DNA-Reparatur beteiligt sind oder, falls die DNA-Schäden irreparabel sind, zur Induktion der Apoptose. Als Zellkultur-Testsystem wurden hierfür die H-1299 Zellen ausgewählt, da diese bereits eine erhöhte Expression von HMGA1a/b zeigten.

### Durchführung/ Methode

1x10⁴ H-1299 Zellen wurden in 24-well Platten Platten (Costar) mit planem, durchsichtigem Boden ausgesät und für 16-24 Stunden in RPMI 1640-Medium mit 10% FCS kultiviert (Volumen 1 ml). Anschließend wurden die Zellen 2x mit PBS gewaschen und die Zellen für weitere 3 Tage mit Zellkulturmedium mit Spiegelmer-Mizellen mit 10% FCS kultiviert. Die Verpackung von sterilem, PEGyliertem Spiegelmer erfolgte zuvor in 1xPBS durch Zugabe von 25kDa querverzweigten Polyethylenimine (PEI) (Sigma) in einem Verhältnis von 2,5:1 des absoluten Stickstoffanteils des PEI zum absoluten Phosphat des Ribonukleinsäurerückgrats (N/P 2,5). Das sterile Spiegelmer wurde dabei mit einer Konzentration von 30µM vorgelegt und nach der Zugabe des PEI für 30-60 Minuten bei Raumtemperatur inkubiert. Anschließend wurden die Spiegelmer-Mizellen mit Zellkulturmedium mit 1%FCS auf die jeweilige Konzentration verdünnt, direkt zu den gewaschenen Zellen gegeben und für 3 Tage bei 37°C inkubiert. Die Zellen wurden 2mal mit PBS gewaschen und mittels eines Zellschabers geerntet.
Anschließend wurde die mRNA der Zellen mittels Roti-Quick-Kits (Roth, Cat.No.979.1) aus den Zellen isoliert und 0,2-1 µg Gesamt-RNA als Template für die PCR von *cdc25a* und GAPDH einsetzt.
Die Primer für die Amplifikation von GAPDH waren: forward primer: 5'-ACATGTTCCAATATGATTCC-3' und reverse primer: 5-TGGACTCCACGACGTACTCAG-3' bei einer Annealingtemperatur von 51°C , sowie für die Amplifikation von *cdc25a* : forward primer: 5'-GAGGAGTCTCACCTGGAAGTACA-3' und reverse primer 5'-GCCATTCAAAACCAGATGCCATAA-3' bei einer Annelaingtemperatur von 59°C Die PCR Bedingungen waren wie folgt: 0,2-0,75µM Primer, 1,5mM MgCl2 und 0,2mM dNTPs. Alle 2 PCR Zyklen wurde je ein Aliquot von 5µl über PicoGreen quantifiziert und in Korrelation zu GAPDH als Beladungskontrolle ausgewertet: dabei wird im ersten Schritt für jede untersuchte Probe der s.g. "crossing-point"-Wert (CP) des Referenzgens vom CP Wertdes zu untersuchenden Gens subtrahiert (dCP=CPZielgen-CP Referenzgen). Als CP ist die Anzahl der PCR Zyklen definiert, die nötig sind um ein konstant definiertes Fluoreszenzniveau zu erreichen. Am CP befindet sich in allen Reaktionsgefäßen die gleiche Menge an neu synthetisierter DNA. Nach dieser Normierung wird vom dCP Wert der experimentell behandelten Proben der dCP Wert einer Kontrolle (in diesem Fall GAPDH) abgezogen; man kommt zum s.g. "delta-delta CT" Berechnungsmodell. Der relative Expressionsunterschied einer Probe zwischen Behandlung und der Kontrolle (Ratio), normalisiert zum Referenzgen und bezogen auf eine Standardprobe, ergibt sich aus der arithmetischen Formel 2' ddCP
dCP=CP (*cdc25a)*- CP (GAPDH)
ddCP= dCP (Behandlung Spiegelmer NOX-A)- dCP( Kontrolle: PBS oder NOX-A invers) Ratio=2^{-ddCP}

### Ergebnis

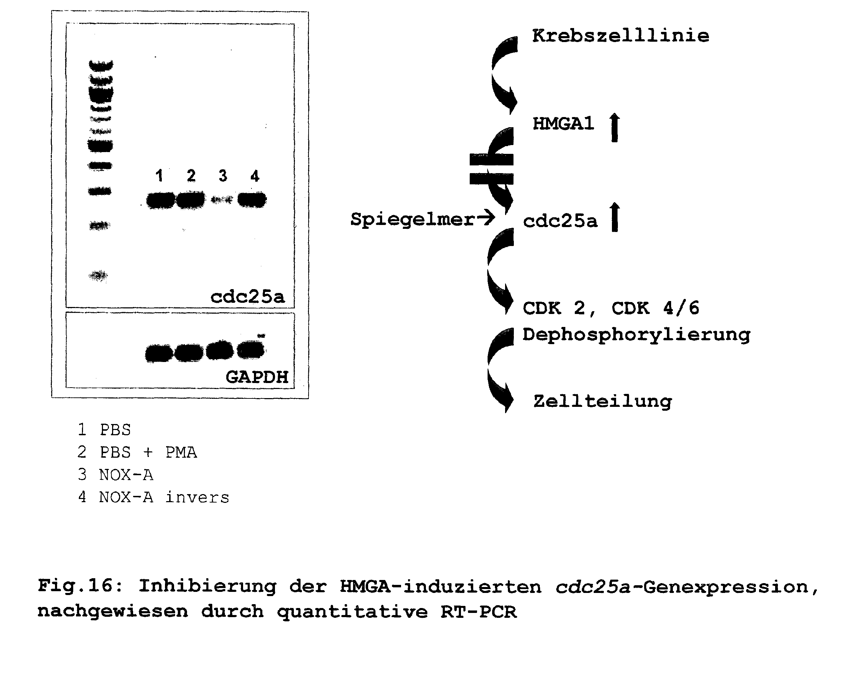

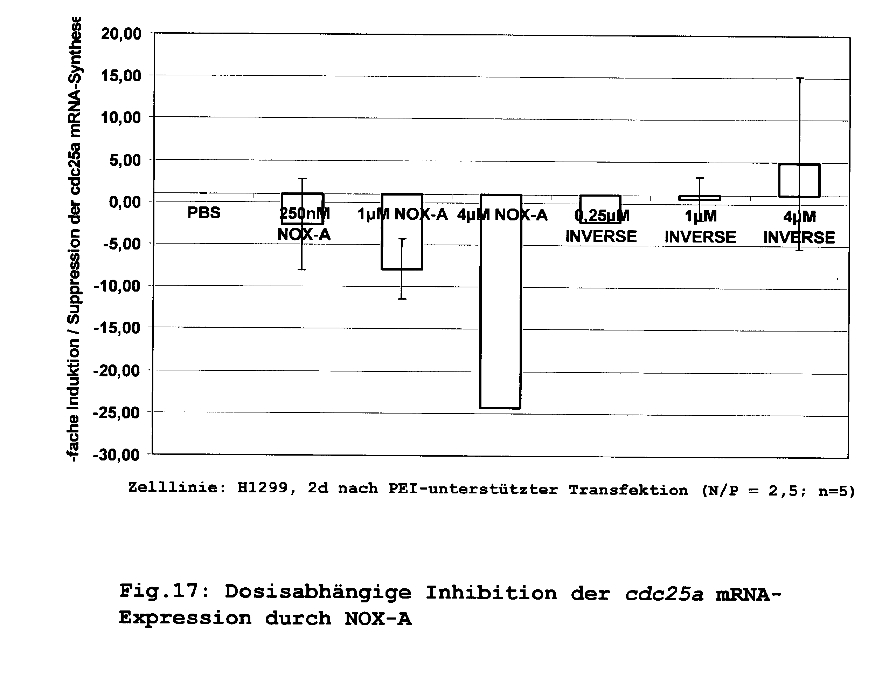

Mittels RT-PCR konnten *cdc25a* und HMGA1a/b in MCF-7 und H-1299 Zellen nachwiesen werden. MCF-7 Zellen zeigten bei einer geringen Expression von HMGA1a/b auch eine geringe Expression von *cdc25a,* wohingegen in H-1299 Zellen HMGA1a/b und *cdc25a* stark exprimiert wurden. Die Transfektion von H-1299 Zellen für 2 Tage mit HMGA1a/b bindenden Spiegelmeren führte zu einer deutlichen, dosisabhängigen Reduktion der Expression von *cdc25a* mRNA (Fig. 16 und Fig.17).

Ein Kontroll-Spiegelmer zeigte bis 4µM keinen unspezifischen Effekt, weder auf die GAPDH noch auf die *cdc25a* mRNA Expression. Daraus kann geschlussfolgert werden, dass mittels Spiegelmeren die HMGA1a/b induzierte Überexpression des potentiellen Onkogen *cdc25a* inhibiert werden kann.

### Beispiel 5 Wirksamkeitsstudie Xenograft-Modell

### Wirkung von Spiegelmeren auf das Tumorwachstum in vivo

Um die Hypothese zu überprüfen, dass HMGA1a/b bindende Spiegelmere das Wachstum von Tumoren in-vivo hemmen, wurden für die stark HMGA1a/b exprimierenden pankreaskarzinom Zellen PSN-1 ein Xenograft Modell entwickelt. Auf Grundlage dieses Modells wurde ein Therapieversuch mit 2mg/kg NOX-A Spiegelmer-Mizellen bei einer N/P 2,5 durchgeführt (siehe Beispiel 3, Absatz 3.3).

### Durchführung/ Methode

Männlichen Nacktmäusen (NMRI: nu/nu) (Gruppengröße n = 8) wurden jeweils 10⁷ PSN-1 Zellen (ECACC) subkutan in die Flanke injiziert und das Tumorwachstum über 22 Tage verfolgt. Die Tiere hatten ein mittleres Gewicht von 25-27 g und waren 6-8 Wochen alt. Das aktive Spiegelmer NOX-A-3'PEG und das inverse Kontroll-Spiegelmers INV-3'PEG wurden wie oben beschrieben durch Zugabe von PEI in einem Verhältnis von N/P 2,5 in Mizellen verpackt. 100µl der Spiegelmer-Mizellen-Suspension (entsprechend 3,46 nmol/Tier bzw 2 mg/kg) wurden jeweils täglich in Tumornähe subkutan injiziert. Die Messung des Tumorvolumens und Körpergewichts erfolgte 3x die Woche. Die Tiere wurden am Tag 22 getötet und die Verteilung von NOX-A im Plasma, Leber, Niere und Tumor quantifiziert.

Dazu wurden die Gewebe in Hybridisierungspuffer (0,5x SSC pH 7,0; 0,5% (w/v) SDSarcosinat) homogenisiert und 10 min bei 4000x g zentrifugiert. Die gewonnenen Überstände wurden bis zur weiteren Benutzung bei -20°C gelagert.

Die Menge an Spiegelmer in den Plasmaproben und in den Gewebhomogenaten wurde mittels eines Hybridisierungsassays untersucht (Drolet et al. (2000) Pharm.Res. 17:1503). Der Hybridisierungsassay basiert auf folgendem Prinzip: das nachzuweisende Spiegelmer (L-RNA-Molekül) wird an eine immobilisierte L-DNA-Oligonukleotidsonde ( = Capture-Sonde NOX-A; hier: 5'- CCCATATCCACCCACGTATCAGCCTTTTTTTT-NH2 -3'; komplementär zum 5'-Ende von HMGA1a/b-NOX-A) hybridisiert und mit einer biotinylierten Nachweis-L-DNA-Sonde (= Detektor-Sonde NOX-A; hier: 5'-Biotin-TTTTTTTTGGCTGAAACCACCCACATGG-3'; komplementär zum 3'-Ende von HMGA1a/b-NOX-A) detektiert. Dazu wird ein Streptavidin-Alkalische-Phosphatase-Konjugat in einem weiteren Schritt an den Komplex gebunden. Nach Zugabe eines Chemilumineszenz-Substrats wird Licht generiert und in einem Luminometer gemessen.

Immobilisierung der Oligonukleotid-Sonde: 100 µl der Capture-Sonde (0,75 pmol/ml in Kopplungspuffer: 500 mM Na₂HPO₄ pH 8,5, 0,5 mM EDTA) wurden pro well (Vertiefung einer Platte) in DNA BIND Platten (Corning Costar) überführt und über Nacht bei 4°C inkubiert. Anschließend wurde 3 x mit je 200 µl Kopplungspuffer gewaschen und für 1 h bei 37°C mit je 200 µl Blockierungspuffer (0,5% (w/v) BSA in Kopplungspuffer) inkubiert. Nach nochmaligem Waschen mit 200 µl Kopplungspuffer und 3× 200 µl Hybridisierungspuffer können die Platten zum Nachweis verwendet werden.

Hybridisierung und Nachweis: 10 µl EDTA Plasma oder Gewebehomogenat wurden mit 90 µl Detektionspuffer (2 pmol/µl Detektor-Sonde in Hybridisierungspuffer) gemischt und zentrifugiert. Je nach Bedarf erfolgten weitere Verdünnungen. Anschliessend wurden die Ansätze bei 95°C für 10 min denaturiert, in die entsprechend vorbereiteten DNA-BIND wells (s.o.) transferiert und für 45 min bei ca. 40°C inkubiert. Danach folgten Waschschritte: 2× 200 µl Hybridisierungspuffer und 3× 200 µl 1× TBS/Tween20 (20 mM Tris-Cl pH 7,6, 137 mM NaCl, 0,1 % (v/v) Tween 20). 1 µl Streptavidin-Alkalische-Phosphatase-Konjugat (Promega) wurden mit 5 ml 1× TBS/Tween 20 verdünnt. 100 µl des verdünnten Konjugats wurden pro well zugegeben und für eine Stunde bei Raumtemperatur inkubiert. Es folgten Waschschritte: 2× 200 µl 1× TBS/Tween 20 und 2× 200 µl 1× Assay-Puffer (20 mM Tris-Cl pH 9,8, 1 mM MgCl₂). Abschließend wurden 100 µl CSPD "Ready-To-Use Substrate" (Applied Biosystems) zugegeben, 30 min bei Raumtemperatur inkubiert und die Chemilumineszenz in einem Fluostar Optima Multidetektions-Plattenlesegerät (BMG) gemessen.

### Ergebnis

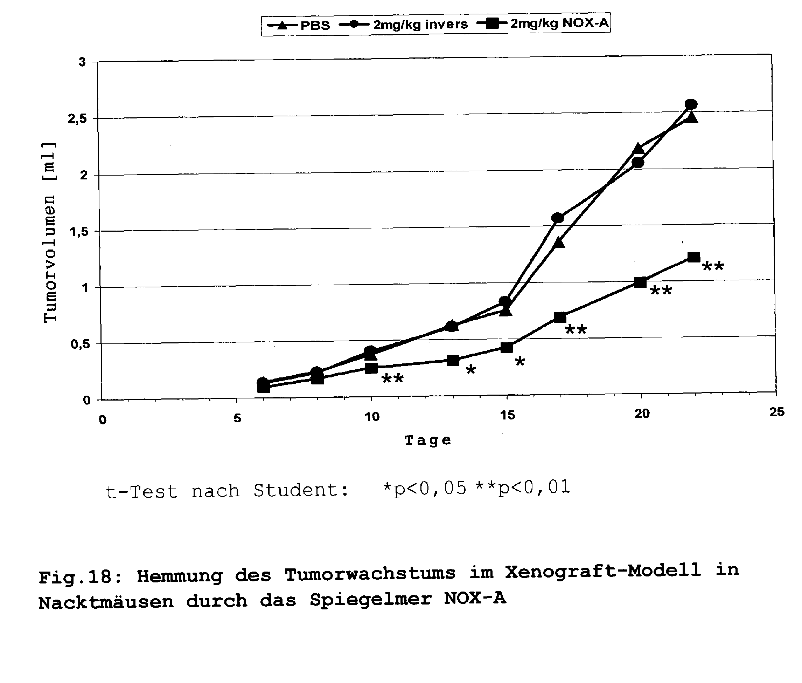

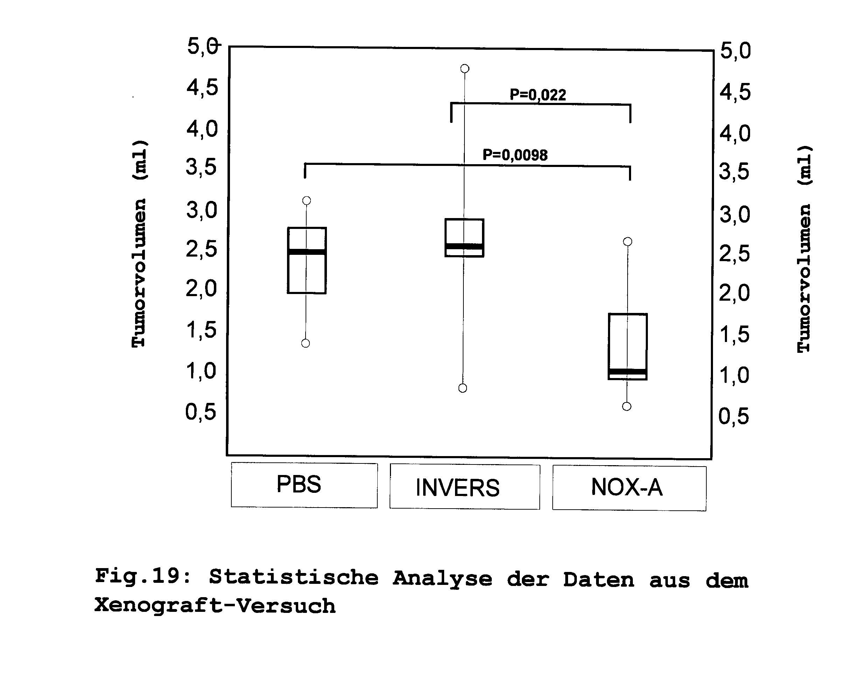

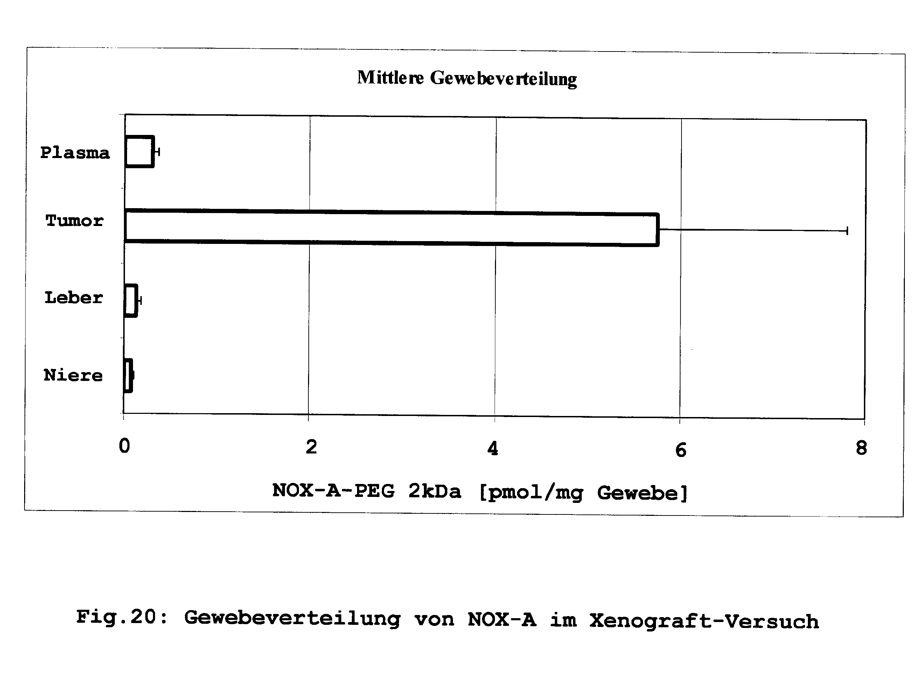

In einem Vorversuch hatte sich gezeigt, dass H-1299 Zellen nach Transplantation als Tumor deutlich langsamer wuchsen als PSN-1 und bei Vergleich der einzelnen Tiere ein inhomogenes Tumorwachstum zeigten und daher nicht als Xenograft-Modell für eine Therapiestudie geeignet erschienen. PSN-1 Zellen zeigten innerhalb von 22 Tagen ein aggressives Tumorwachstum. Es konnte gezeigt werden, dass NOX-A-Mizellen bei einer Dosis von 2mg/kg das Wachstum von PSN-1 Tumoren deutlich gegenüber der PBS Kontrolle verringern (Fig. **18** ). Das Gewicht der Tiere war von der Behandlung mit Spiegelmer-Mizellen unbeeinflusst. Das Kontroll-Spiegelmer zeigte keine unspezifische Inhibition des Tumorwachstums und hatte ebenfalls keine Wirkung auf das Gewicht der Tiere. Die Unterschiede der Tumorgrößen waren ab Tag 10 der Behandlung mit NOX-A3'PEG Mizellen gegenüber nicht behandelten Tieren (PBS Kontrolle) signifikant bzw. hoch signifikant (student's t-Test). Die EndpunktAnalyse nach 22 Tagen zeigte eine hoch signifikante, spezifische Verringerung des Tumorwachstums (p=0,0098 gegenüber PBS und p=0,022 gegenüber inversem Kontroll-Spiegelmer) (Fig. 19). PBS behandelte Mäuse zeigten einen durchschnittliches Tumorwachstum von 2,5 cm³, Tiere behandelt mit Kontroll-Spiegelmer ein durchschnittliches Tumorvolumen von 2,6 cm³ und NOX-A behandelte Tiere ein durchschnittliches Tumorvolumen von 1,2 cm³ nach 22 Tagen. (Box-andWhisker-Analyse). Dies entspricht einer Reduktion des Tumorwachstums um mehr als 50%.
Die Analyse der Gewebeverteilung von NOX-A zeigte eine hohe Konzentration im Tumor (Fig. 20).

### Beispiel 6: Vergleich der in vivo Gewebeverteilung von verpackten und unverpackten Spiegelmer

Zur Überprüfung der effizienten Einschleusung von Spiegelmer Mizellen wurde ein nicht funktionelles Spiegelmer (Proof Of Concept = POC) am 3'-Ende mit PEG 2kDa PEGyliert und mit einer Stickstoff/ Phosphat Ratio (N/P) von 2,5 in Mizellen verpackt (siehe Beispiel 3, Absatz 3.3). Analog zu dem in Beispiel 5 beschriebenen Versuchsaufbau wurde dieser Ansatz mit in Mizellen verpackten Spiegelmer sowie freiem, unverpackten Spiegelmer durchgeführt.

### Durchführung/ Methode

Männlichen Nacktmäusen (NMRI: nu/nu) (Gruppengröße n = 8) wurden jeweils 10⁷ PSN-1 Zellen (ECACC) subkutan in die Flanke injiziert und das Tumorwachstum über 25 Tage verfolgt. Die Tiere hatten ein mittleres Gewicht von 25-27 g und waren 6-8 Wochen alt. Das nicht funktionelles Spiegelmer POC-3'PEG wurde wie oben beschrieben durch Zugabe von PEI in einem Verhältnis von N/P 2,5 in Mizellen verpackt. Als Kontrolle für die nicht PEI vermittelte Einschleusung von Spiegelmer diente nicht in Mizellen verpacktes Spiegelmer POC-3'PEG. 100µl der Spiegelmer-Mizellen-Suspension bzw. Spiegelmer Lösung (entsprechend 1500 nmol/kg bzw. 2000 nmol/kg) wurden jeweils täglich in Tumornähe subkutan injiziert. Die Messung des Tumorvolumens und Körpergewichts erfolgte 3x die Woche. 24 bzw. 96 Stunden nach der letzten Injektion wurden je zwei Tiere pro Gruppe getötet und die Verteilung von POC-3'PEG (verpackt/unverpackt) im Plasma, Gehirn, Herz, Lunge, Leber, Niere, Gallenblase, Bauchspeicheldrüse und Tumor quantifiziert.

Dazu wurden die Gewebe in Hybridisierungspuffer (0,5x SSC pH 7,0; 0,5% (w/v) SDSarcosinat) homogenisiert und 10 min bei 4000x g zentrifugiert. Die gewonnenen Überstände wurden bis zur weiteren Benutzung bei -20°C gelagert.

Die Menge an Spiegelmer in den Plasmaproben und in den Gewebhomogenaten wurde mittels eines Hybridisierungsassays untersucht (Drolet et al. (2000) Pharm.Res. 17:1503). Der Hybridisierungsassay basiert auf folgendem Prinzip: das nachzuweisende Spiegelmer (L-RNA-Molekül) wird an eine immobilisierte L-DNA-Oligonukleotidsonde ( = Capture-Sonde POC ; hier: 5'- NH2(C7)-TTTTTTTTTAGCTCTGCACAGCGCT-3'; komplementär zum 3'-Ende von POC) hybridisiert und mit einer biotinylierten Nachweis-L-DNA-Sonde (= Detektor-Sonde POC; hier: 5'-CCGCATCAGACCGAGTTTCCTTATTTTTTTT-Biotin-3'; komplementär zum 5'-Ende von POC) detektiert. Dazu wird ein Streptavidin-Alkalische-Phosphatase-Konjugat in einem weiteren Schritt an den Komplex gebunden. Nach Zugabe eines Chemilumineszenz-Substrats wird Licht generiert und in einem Luminometer gemessen.

Immobilisierung der Oligonukleotid-Sonde: 100 µl der POC-Capture-Sonde (0,75 pmol/ml in Kopplungspuffer: 500 mM Na₂HPO₄ pH 8,5, 0,5 mM EDTA) wurden pro well (Vertiefung einer Platte) in DNA BIND Platten (Corning Costar) überführt und über Nacht bei 4°C inkubiert. Anschließend wurde 3 x mit je 200 µl Kopplungspuffer gewaschen und für 1 h bei 37°C mit je 200 µl Blockierungspuffer (0,5% (w/v) BSA in Kopplungspuffer) inkubiert. Nach nochmaligem Waschen mit 200 µl Kopplungspuffer und 3× 200 µl Hybridisierungspuffer können die Platten zum Nachweis verwendet werden.

Hybridisierung und Nachweis: 10 µl EDTA Plasma oder Gewebehomogenat wurden mit 90 µl Detektionspuffer (2 pmol/µl POC-Detektor-Sonde in Hybridisierungspuffer) gemischt und zentrifugiert. Je nach Bedarf erfolgten weitere Verdünnungen. Anschliessend wurden die Ansätze bei 95°C für 10 min denaturiert, in die entsprechend vorbereiteten DNA-BIND wells (s.o.) transferiert und für 45 min bei ca. 40°C inkubiert. Danach folgten Waschschritte: 2x 200 µl Hybridisierungspuffer und 3× 200 µl 1× TBS/Tween20 (20 mM Tris-C1 pH 7,6, 137 mM NaCl, 0,1 % (v/v) Tween 20). 1 µl Streptavidin-Alkalische-Phosphatase-Konjugat (Promega) wurden mit 5 ml 1× TBS/Tween 20 verdünnt. 100 µl des verdünnten Konjugats wurden pro well zugegeben und für eine Stunde bei Raumtemperatur inkubiert. Es folgten Waschschritte: 2× 200 µl 1× TBS/Tween 20 und 2× 200 µl 1× Assay-Puffer (20 mM Tris-Cl pH 9,8, 1 mM MgCl₂). Abschließend wurden 100 µl CSPD "Ready-To-Use Substrate" (Applied Biosystems) zugegeben, 30 min bei Raumtemperatur inkubiert und die Chemilumineszenz in einem Fluostar Optima Multidetektions-Plattenlesegerät (BMG) gemessen.

### Ergebnis

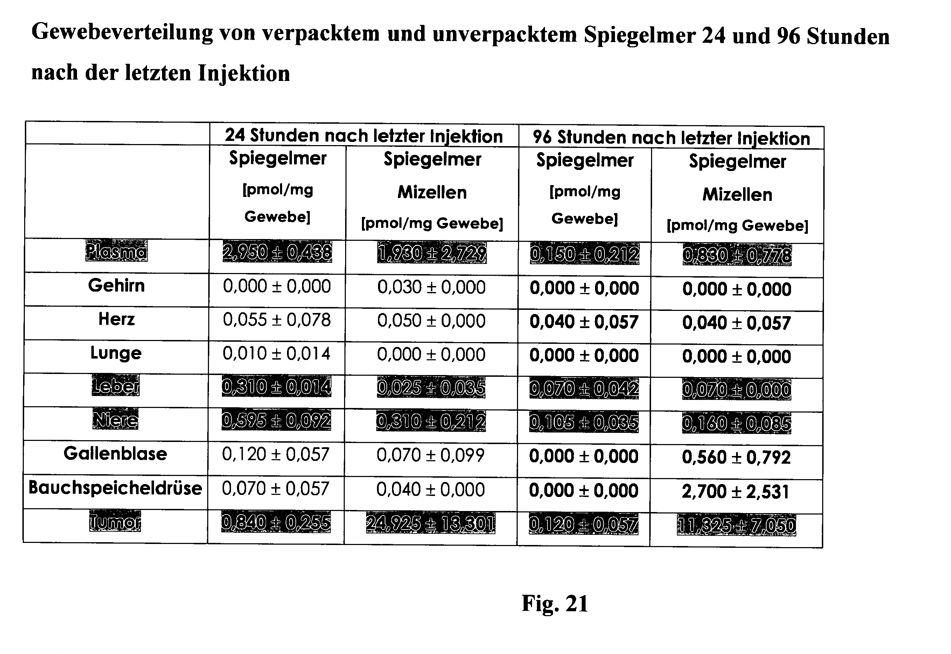

Die Analyse der Gewebeverteilung des nicht funktionellen Spiegelmers POC-3'PEG, welches in Mizellen verpackt wurde, zeigte nach 24 Stunden eine signifikant höhere Konzentration im Tumorgewebe (24,925 +/- 13,301 pmol/mg) als nicht verpacktes Spiegelmer (0,840 +/- 0,255 pmol/mg) (Fig. 21 A). Während die Konzentration des verpackten Spiegelmers sich nach weiteren drei Tagen (96 Stunden) halbiert hatte (11,325 +/- 7,050 pmol/mg), konnte vom unverpackten Spiegelmer nur noch eine sehr geringe Menge (0,120 +/- 0,057 pmol/mg) nachgewiesen werden.
Der Plasmaspiegel von nicht verpacktem Spiegelmer (2,950 +/- 0,438 pmol/ml) nach 24 Stunden war vergleichbar mit dem von PEI verpacktem Spiegelmer (1,930 +/- 2,729 pmol/ml). Nach 96 Stunden zeigten sich deutliche Unterschiede, wobei etwa die vierfache Menge des verpackten gegenüber dem unverpackten Spiegelmer nachgewiesen werden konnte.

Eine leichte Anreicherung in der Niere nach 24 Stunden konnte für beide Formulierungen beobachtet werden, wohingegen nur für nicht verpacktes Spiegelmer eine geringe Anreicherung in der Leber und Gallenblasen gefunden wurde. Nach 96 Stunden konnten in Leber und Niere für beide Formulierungen nur noch geringe Mengen an Spiegelmer nachgewiesen werden. Dahingegen konnte für verpacktes im Vergleich zum unverpacktem Spiegelmer leicht erhöhte Werte in der Gallenblase und Bauchspeicheldrüse (jedoch hohe Standardabweichung) beobachtet werden.

Zusammenfassend, konnte im Vergleich der Gewebeverteilung (24 bzw. 96 Stunden nach letzter Injektion) von Spiegelmeren in An- und Abwesenheit von PEI gezeigt werden, dass Spiegelmer-Mizellen eine deutlich verlängerte Verweildauer im Plasma und Tumor im Vergleich zu nicht verpacktem Material aufweisen (Abb. 21B) und somit einen vielversprechenden Ansatz zur Verwendung von Spiegelmeren gegen intrazelluläre Zielmoleküle darstellen.

Die folgenden Literaturstellen werden hierin durch Bezugnahme aufgenommen.
Abe et al. J Gastroenterol. 2003 ; 38, 1144-9
Abe N et al (1999). Cancer Res 59:1169-1174
Abe N et al (2000). Cancer Res 60:3117-3122
Abe N et al (2002). Pancreas 25:198-204
Anand A & Chada K (2000). Nat Genet 24 :377-380
Balcercak et al, Postepy Biochem, 2005; 51(3):261-9
Balcerczak et al Pathol Res Pract 2003; 199, 641-6
Baldassarre G et al (2003). Mol Cell Biol 23:2225-2238
Bandiera S et al (1998). Cancer Res 58:426-431
Battista S et al (1998) Oncogene 17:377-385
Belge G et al (1992). Cell Biol Int Rep 16 :339-347
Berlingieri MT et al. (1995). Mol Cell Biol 15:1545-1553
Birdsal SH et al (1992). Cancer Gen Cytogen 60:74-77
Bridge JA et al (1992). Cancer Detect Prevent 16:215-219
Briese et al. Int. J Gynevol Pathol 2006 Jan, 65-9
Bullerdiek J et al (1987). Cytogenet Cell Genet 45 :187-190
Bussemakers MJG et al (1991). Cancer Res 51 :606-611
Chada K et al (2004). US Patent 6,756,355
Chang et al. Dig Dis Sci, 2005 Oct, 1764-70
Chau KY et al (2000). J Neurosci 20 :7317-7324
Chau KY et al (2003). Mol Med 9 :154-165
Chen et al. Cancer Epidemiol Biomarkers Prev 2004 Jan, 30-3
Chiappetta et al. Clin Cancer Res. 2004 Nov, 7634-44
Chiappetta G et al (1995). Oncogene 10:1307-1314
Chiappetta G et al (1996). Oncogene 13:2439-2446
Chiappetta G et al (1998). Cancer Res 58:4193-4198
Chiappetta G et al (2001). Int J Cancer 91:147-151
Chin MT et al (1999). J Mol Cell Cardiol 31 :2199-2205
Chuma et al, Keio J Med 2004 Jun, 90-7
Cuff CA et al (2001). J Clin Invest 108 :1031-1040
Czyz et al. Langenbecks Arch Surg 2004, Jun, 193-7
Dal Cin P et al (1993). Genes Chromosomes Cancer 8:131-133
Dal Cin P et al (1995). Cancer Res 55:1565-1568
Diana F et al (2001). J Biol Chem 276 :11354-11361
Dolde CE et al (2002). Breast Cancer Res Treat 71 :181-191
Donato et al. Oncol Rep 2004 Jun, 1209-13
Du W et al (1993). Cell 74 :887-898
Evans A et al (2004). J Surg Oncol 88 :86-99
Fedele M et al (1996). Cancer Res 56:1896-1901
Fletcher AJ et al (1991). Am J Pathol 138:1199-1207
Fletcher AJ et al (1992). Cancer Res 52 :6224-6228
Fletcher AJ et al (1995). Genes Chromosomes Cancer 12:220-223
Flohr et al. Histol Histopathol 2003 Oct, 999-1004
Foster LC et al (1998). J Biol Chem 273 :20341-20346
Foster LC et al (2000). FASEB J 14 :368-378
French et al. Mol Cell Biol 1996, 5393-9
Friedman M et al (1993). Nucleic Acids Res 21:4259-4267
Giancotti V et al (1987). EMBO J 6:1981-1987
Giancotti V et al (1989). Exp Cell Res 184:538-545
Giancotti V et al. (1993). Eur J Biochem 213:825-832
Giannini G et al (1999). Cancer Res 59:2484-2492
Giannini G et al (2000). Br J Cancer 83:1503-1509
Grosschedl R et al (1994). Trends Genet 10 :94-100
Heim S et al (1988). Cancer Genet Cytogenet 32:13-17
Henderson et al J Virol 2000, 10523-34
Hindmarsh et al. J. Virol 1999, 2994-3003
Holth LT et al (1997). DNA Cell Biol 16:1299-1309
Huth JR et al (1997). Nat Struct Biol 4:657-665
Jain M et al (1996). J Clin Invest 97 :596-603
Johansson M et al (1992). Cancer Genet Cytogenet 60 :219-220
Johansson M et al (1993). Br J Cancer 67 :1236-1241
Johnson KR et al (1990). Exp Cell Res 187:69-76
Kazmierczak B et al (1995). Cancer Res 55 :2497-2499
Kettunen et al. Cancer Genet Cytogenet 2004 Mar, 98-106
Kim DH et al (1999). Int J Cancer 84 :376-380
Klotzbücher M et al (1999). Am J Pathol 155:1535-1542
Kottickal LV et al (1998). Biochem Biophys Res Commun 242 :452-456
Lee et al. Int J Oncol 2004, Apr, 847-51
Leger et al. Mol Cell Biol 1995, 3738-47
Leman ES et al (2003). J Cell Biochem 88 :599-608
Li et al J. Virol 1998, 2125-31
Li et al, Am J Dermatopathol 2004 Aug, 267-72
Mandahl N et al (1987). Int J Cancer 39 :685-688
Mandahl N et al (1989). Cancer 65 :242-248
Mandahl N et al (1993). Cancer 71 : 3009-3013
Mark J & Dahlenfors R (1986). Anticancer Res 6:299-308
Mark J et al (1980). Cancer Genet Cytogenet 2 :231-241
Mark J et al (1988). Anticancer Res 8:621-626
Masciullo et al Carcinogenesis 2003 Jul, 1191-8
Masciullo V et al (2003). Carcinogenesis 24:1191-1198
Melillo RM et al (2001). Mol Cell Biol 21:2485-2495
Nam et al Histopathology 2003 May, 466-71
Nestl A et al (2001). Cancer Res 61:1569-1577
Noguera R et al (1989). Virchows Arch A Pathol Anat Histopathol 415:377-382
Ogram SA et al (1997). J Biol Chem 270:14235-14242
Ozisik YY et al (1993). Cancer Genet Cytogenet 79 :136-138
Panagiotidis et al Virology 1999, 64-74
Pellacani A et al (1999). J Biol Chem 274:1525-1532
Peters et al. Cancer Epidemiol Biomarkers Prev 2005, Jul 17, 17-23
Pierantoni et al. Biochem J 2003 May, 145-50
Ram TG et al (1993). Cancer Res 53 :2655-2660
Reeves R & Beckerbauer K (2002). Progr Cell Cycle Res 5:279-286
Reeves R & Beckerbauer L (2001). Biochim Biophys Acta 1519:13-29
Reeves R & Nissen MS (1990). J Biol Chem 265:8573-8582
Reeves R et al (2001). Mol Cell Biol 21:575-594
Rogalla P et al (1996). Am J Pathol 149:775-779
Rohen C et al (1995). Cancer Genet Cytogenet 84:82-84
Sarhadi et al. J Pathol Mar 6 2006, Epup ahead of print
Sato et al. Pathol Res Pract, 2005; 201, 333-9
Scala S et al (2000). Proc Natl Acad Sci USA 97 :4256-4261
Schaefer et al. Mol Cell Biol. 1997, 873-86
Schlueter et al. Pathol Res Pract, 2005; 201, 101-7
Schoenmakers EFPM et al. (1995). Nat Genet 10:436-444
Sgarra R et al (2003). Biochemistry 42 :3575-3585
Sgarra R et al (2004). FEBS Lett 574 :1-8
Sreekantaiah C et al (1990). Cancer Genet Cytogenet 45 :81-84
Sreekantaiah C et al (1991). Cancer Res 5 :422-433
Staats B et al (1996). Breast Cancer Res Treat 38 :299-303
Tamimi Y et al (1996). Br J Cancer 74 :573-578
Tapasso F et al (2004). Cancer Gene Ther 11 :633-641
Tarbe N et al (2001). Anticancer Res 21:3221-3228
Thanos D & Maniatis T (1992). Cell 71:777-789
Turc-Carel C et al (1986). Cancer Genet Cytogenet 23 :283-289
Vallone D et al (1997). EMBO J 16:5310-5321
Van Maele et al. , Trends Biochem Sci 2006, 98-105
Vanni R et al (1988). Cancer Genet Cytogenet 32:33-34
Vanni R et al (1993). Cancer Genet Cytogenet 68:32-33
Walter TA et al (1989). Cancer Genet Cytogenet 41 :99-103
Wolffe AP (1994). Science 264:1100-1101
Wood LJ et al (2000a). Cancer Res 60:4256-4261
Wood LJ et al (2000b). Mol Cell Biol 20 :5490-5502
Xiang YY et al (1997). Int J Cancer 74 :1-6
Zhou X et al (1995). Nature 376 :771-774

### Ausführungsformen

Ausführungsform 1: Verwendung einer L-Nukleinsäure als intrazellulär aktives Agens.
Ausführungsform 2: Verwendung nach Ausführungsform 1, **dadurch gekennzeichnet, dass** die L-Nukleinsäure ein Spiegelmer ist.
Ausführungsform 3: Verwendung nach einer der Ausführungsformen 1 bis 2, **dadurch gekennzeichnet, dass** die L-Nukleinsäure in Wechselwirkung mit einem intrazellulären Rezeptor tritt.
Ausführungsform 4: Verwendung nach Ausführungsform 3, **dadurch gekennzeichnet, dass** der intrazelluläre Rezeptor ausgewählt ist aus der Gruppe umfassend molekulare Rezeptoren, Enzyme, Chaperon-Moleküle, Signalpeptide, intrazelluläre Strukturen und Stoffwechselintermediate.
Ausführungsform 5: Verwendung nach Ausführungsform 3, **dadurch gekennzeichnet, dass** der intrazelluläre Rezeptor ausgewählt ist aus der Gruppe umfassend Polypeptide, Kohlenhydrate, Nukleinsäuren, Lipide und Kombinationen davon.
Ausführungsform 6: Verwendung nach einer der Ausführungsformen 3 bis 5, **dadurch gekennzeichnet, dass** die L-Nukleinsäure mit einem intrazellulären Rezeptor innerhalb einer Zelle in Wechselwirkung tritt.
Ausführungsform 7: Verwendung nach einer der Ausführungsformen 3 bis 6, **dadurch gekennzeichnet, dass** der intrazelluläre Rezeptor ausgewählt ist aus der Gruppe umfassend Transkriptionsfaktoren und einen AT-Haken bindende DNA-bindende Proteine.
Ausführungsform 8: Verwendung nach Ausführungsform 7, **dadurch gekennzeichnet, dass** der intrazelluläre Rezeptor aus der Gruppe umfassend die HMG-Proteine ausgewählt ist, bevorzugterweise aus der Gruppe umfassend HMGA 1, HMGA1a, HMGA1b und HMGA2 ausgewählt ist.
Ausführungsform 9: Verfahren zum Binden eines intrazellulären Rezeptor umfassend:
   - Bereitstellen einer Zelle enthaltend mindestens den einen intrazellulären Rezeptor,
   - Bereitstellen einer L-Nukleinsäure, und
   - Inkubieren der Zelle mit der L-Nukleinsäure.
Ausführungsform 10: Verfahren nach Ausführungsform 9, **dadurch gekennzeichnet, dass** das Inkubieren unter Bedingungen erfolgt, so dass die L-Nukleinsäure an den intrazellulären Rezeptor in der Zelle bindet.
Ausführungsform 11: Verfahren nach Ausführungsform 9 und 10, **dadurch gekennzeichnet, dass** die L-Nukleinsäure ein Spiegelmer ist.
Ausführungsform 12: Verfahren nach einer der Ausführungsformen 9 bis 11, **dadurch gekennzeichnet, dass** nach der Inkubation der Zelle mit der L-Nukleinsäure bestimmt wird, ob eine Bindung, insbesondere eine intrazelluläre Bindung, der L-Nukleinsäure an dem intrazellulären Rezeptor erfolgt ist.
Ausführungsform 13: Verfahren nach einer der Ausführungsformen 9 bis 12, **dadurch gekennzeichnet, dass** der intrazelluläre Rezeptor ausgewählt ist aus der Gruppe umfassend molekulare Rezeptoren, Stoffwechselintermediate und Enzyme.
Ausführungsform 14: Verfahren nach einer der Ausführungsformen 9 bis 13, **dadurch gekennzeichnet, dass** der intrazelluläre Rezeptor ausgewählt ist aus der Gruppe umfassend Polypeptide, Kohlenhydrate, Nukleinsäure, Lipide und Kombinationen davon.
Ausführungsform 15: Verfahren nach einer der Ausführungsformen 9 bis 14, **dadurch gekennzeichnet, dass** der intrazelluläre Rezeptor ausgewählt ist aus der Gruppe umfassend Transkriptionsfaktoren und einen AT-Haken bindende DNA-bindende Proteine.
Ausführungsform 16: Verfahren nach Ausführungsform 15, **dadurch gekennzeichnet, dass** der intrazelluläre Rezeptor ausgewählt ist aus der Gruppe umfassend HMG-Proteine, bevorzugterweise ausgewählt ist aus der Gruppe umfassend HMGA1, HMGA1a, HMGA1 bund HMGA2.
Ausführungsform 17: Verwendung einer L-Nukleinsäure zur Herstellung eines Medikamentes zur Behandlung und/oder Prävention einer Erkrankung, wobei das Zielmolekül des Medikamentes ein intrazelluläres Zielmolekül ist.
Ausführungsform 18: Verwendung nach Ausführungsform 17, **dadurch gekennzeichnet, dass** der intrazelluläre Rezeptor ausgewählt ist aus der Gruppe umfassend molekulare Rezeptoren, Enzyme, Chaperon-Moleküle, Signalpeptide, intrazelluläre Strukturen und Stoffwechselintermediate.
Ausführungsform 19: Verwendung nach Ausführungsform 17, **dadurch gekennzeichnet, dass** der intrazelluläre Rezeptor ausgewählt ist aus der Gruppe umfassend Polypeptide, Kohlenhydrate, Nukleinsäuren, Lipide und Kombinationen davon.
Ausführungsform 20: Verwendung nach einer der Ausführungsformen 17 bis 19, **dadurch gekennzeichnet, dass** das Zielmolekül ausgewählt ist aus der Gruppe, die Transkriptionsfaktoren und einen AT-Haken bindende DNA-bindende Proteine umfasst.
Ausführungsform 21: Verwendung nach Ausführungsform 20, **dadurch gekennzeichnet, dass** das Zielmolekül ausgewählt ist aus der Gruppe umfassend HMG-Proteine, bevorzugterweise ausgewählt ist aus der Gruppe umfassend HMGA1, HMGA1a, HMGA1b und HMGA2.
Ausführungsform 22: Verwendung nach einer der Ausführungsformen 20 bis 21, **dadurch gekennzeichnet, dass** die Erkrankung ausgewählt ist aus der Gruppe umfassend Tumorerkrankungen, Virusinfektionen und Arteriosklerose.
Ausführungsform 23: Verwendung nach Ausführungsform 22, **dadurch gekennzeichnet, dass** die Tumorerkrankung ausgewählt ist aus der Gruppe umfassend mesenchymale Tumoren, epitheliale Tumoren, benigne Tumoren, maligne Tumoren und metastasierende Tumoren.
Ausführungsform 24: Verwendung nach einer der Ausführungsformen 20 bis 23, **dadurch gekennzeichnet, dass** das Zielmolekül HMGA ist und die Erkrankung ausgewählt ist aus der Gruppe umfassend Karzinome von Prostata, Pankreas, Schilddrüse, Cervix, Magen, Brust, Colon/Rektum, Ovarien; Neuroblastome; Lymphome, Uterus-leiomyome; Lipome; Endometriumpolypen; chondroide Hamartome der Lunge; pleomorphe Adenome der Kopfspeicheldrüsen; Hämangiopericytome; chondromatöse Tumore; aggressive Angiomyxome; diffuse Astrocytome; Osteoklastome; Hautkrebs; Burkitts Lymphom; Lewis-Lungenkrebs; Leukämie; nicht-kleinzelliges Lungenkarzinom; sowie jeweils Metastasen und/oder metastasierende Formen davon.
Ausführungsform 25: Verwendung nach Ausführungsform 22, **dadurch gekennzeichnet, dass** die Arteriosklerose durch HMGA1, HMGA1a, HMG1b und/oder HMGA2 vermittelte Bildung von artierosklerotischen Plaques ausgelöst oder bedingt ist.
Ausführungsform 26: Verwendung nach einer der Ausführungsformen 17 bis 25, **dadurch gekennzeichnet, dass** das intrazelluläre Zielmolekül intrazellulär vorliegt.
Ausführungsform 27: Verwendung einer L-Nukleinsäure zur Herstellung eines diagnostischen Mittels zur Diagnose, wobei das Zielmolekül des diagnostischen Mittels ein intrazelluläres Zielmolekül ist.
Ausführungsform 28: Verwendung nach Ausführungsform 27, **dadurch gekennzeichnet, dass** der intrazelluläre Rezeptor ausgewählt ist aus der Gruppe umfassend molekulare Rezeptoren, Enzyme, Chaperon-Moleküle, Signalpeptide, intrazelluläre Strukturen und Stoffwechselintermediate.
Ausführungsform 29: Verwendung nach Ausführungsform 27, **dadurch gekennzeichnet, dass** der intrazelluläre Rezeptor ausgewählt ist aus der Gruppe umfassend Polypeptide, Kohlenhydrate, Nukleinsäuren, Lipide und Kombinationen davon.
Ausführungsform 30: Verwendung nach einer der Ausführungsformen 27 bis 29, **dadurch gekennzeichnet, dass** das Zielmolekül ausgewählt ist aus der Gruppe, die Transkriptionsfaktoren und einen AT-Haken bindende DNA-bindende Proteine umfasst.
Ausführungsform 31: Verwendung nach Ausführungsform 30, **dadurch gekennzeichnet, dass** das Zielmolekül ausgewählt ist aus der Gruppe umfassend HMG-Proteine, bevorzugterweise ausgewählt ist aus der Gruppe umfassend HMGA, HMGA1a, HMGA1b bund HMGA2.
Ausführungsform 32: Verwendung nach einer der Ausführungsformen 30 bis 31, **dadurch gekennzeichnet, dass** die Erkrankung ausgewählt ist aus der Gruppe umfassend Tumorerkrankungen, Virusinfektionen und Arteriosklerose.
Ausführungsform 33: Verwendung nach Ausführungsform 32, **dadurch gekennzeichnet, dass** die Tumorerkrankung ausgewählt ist aus der Gruppe umfassend mesenchymale Tumoren, epitheliale Tumoren, benigne Tumoren, maligne Tumoren und metastatisierende Tumoren.
Ausführungsform 34: Verwendung nach einer der Ausführungsformen 30 bis 33, **dadurch gekennzeichnet, dass** das Zielmolekül HMGA ist und die Erkrankung ausgewählt ist aus der Gruppe umfassend Karzinome von Prostata, Pankreas, Schilddrüse, Cervix, Magen, Brust, Colon/Rektum, Ovarien; Neuroblastome; Lymphome, Uterus-leiomyome; Lipome; Endometriumpolypen; chondroide Hamartome der Lunge; pleomorphe Adenome der Kopfspeicheldrüsen; Hämangiopericytome; chondromatöse Tumore; aggressive Angiomyxome; diffuse Astrocytome; Osteoklastome; Hautkrebs; Burkitts Lymphom; Lewis-Lungenkrebs; Leukämie; nicht-kleinzelliges Lungenkarzinom; sowie jeweils Metastasen und/oder metastasierende Formen davon.
Ausführungsform 35: Verwendung nach Ausführungsform 32, **dadurch gekennzeichnet, dass** die Arteriosklerose durch HMGA1, HMGA1a, HMG1b und/oder HMGA2 vermittelte Bildung von artierosklerotischen Plaques ausgelöst wird.
Ausführungsform 36: Verwendung nach einer der Ausführungsformen 27 bis 34, **dadurch gekennzeichnet, dass** das intrazelluläre Zielmolekül intrazellulär vorliegt.
Ausführungsform 37: Zusammensetzung umfassend eine an ein intrazelluläres Zielmolekül bindende L-Nukleinsäure und ein Abgabevehikel.
Ausführungsform 38: Zusammensetzung nach Ausführungsform 37, **dadurch gekennzeichnet, dass** das Abgabevehikel ein für die intrazelluläre Abgabe der L-Nukleinsäure geeignetes Abgabevehikel ist.
Ausführungsform 39: Zusammensetzung nach Ausführungsform 37 oder 38, **dadurch gekennzeichnet, dass** das Abgabevehikel ausgewählt ist aus der Gruppe umfassend Vehikel, Konjugate und physikalische Maßnahmen.
Ausführungsform 40: Zusammensetzung nach Ausführungsform 39, **dadurch gekennzeichnet, dass** das Abgabevehikel ein Vehikel ist, wobei das Vehikel ausgewählt ist aus der Gruppe umfassend Liposome, Nanopartikel, Mikropartikel, Cyclodextrine oder Dendrimere, oder ein Vesikel ist, das aus Polypeptiden, Polyethylenimin und/oder amphipathischen Molekülen besteht.
Ausführungsform 41: Zusammensetzung nach Ausführungsform 39, **dadurch gekennzeichnet, dass** das Abgabevehikel ein Konjugat ist, wobei das Konjugat ein Konjugat für die Rezeptor-vermittelte Endozytose, ein Konjugat mit einem fusogenen Peptid, ein Konjugat mit einem Signalpeptid, ein Konjugat mit einer Nukleinsäure, bevorzugterweise ein Konjugat mit einem Spiegelmer oder ein lipophiles Konjugat ist.
Ausführungsform 42: Zusammensetzung nach Ausführungsform 39, **dadurch gekennzeichnet, dass** das Abgabevehikel eine physikalische Maßnahme ist, wobei die Maßnahme bevorzugterweise ausgewählt ist aus der Gruppe umfassend Elektroporation, lontophorese, Druck, Ultraschall und Schockwellen.
Ausführungsform 43: Zusammensetzung nach Ausführungsform 40, **dadurch gekennzeichnet, dass** das Abgabevehikel Polyethylenimin umfasst.
Ausführungsform 44: Zusammensetzung nach Ausführungsform 43, **dadurch gekennzeichnet, dass** das Polyethylenimin ein verzweigtes Polyethylenimin mit einem Molekulargewicht von etwa 25 kDa ist.
Ausführungsform 45: Zusammensetzung nach Ausführungsform 43 oder 44, **dadurch gekennzeichnet, dass** das Polyethylenimin eine Mizelle oder eine mizellenartige Struktur ausbildet.
Ausführungsform 46: Zusammensetzung nach einer der Ausführungsformen 37 bis 45,
   **dadurch gekennzeichnet, dass** die L-Nukleinsäure ein Spiegelmer ist.
Ausführungsform 47: Zusammensetzung nach Ausführungsform 46, **dadurch gekennzeichnet, dass** das Spiegelmer eine Modifikation trägt, wobei die Modifikation ausgewählt ist aus der Gruppe umfassend PEG-Reste.
Ausführungsform 48: Zusammensetzung nach Ausführungsform 47, **dadurch gekennzeichnet, dass** der PEG-Rest ein Molekulargewicht von etwa 1.000 bis 10.000 Da aufweist, bevorzugterweise ein Molekulargewicht von etwa 1.500 bis 2.500 Da und am bevorzugtesten ein Molekulargewicht von etwa 2.000 Da aufweist.
Ausführungsform 49: Zusammensetzung nach einer der Ausführungsformen 47 oder 48, **dadurch gekennzeichnet, dass** die Modifikation am 5'-Terminus oder am 3'-Terminus der L-Nukleinsäure gebunden ist.
Ausführungsform 50: Zusammensetzung nach einer der Ausführungsformen 46 bis 49,
   **dadurch gekennzeichnet, dass** in der Zusammensetzung das Verhältnis aus Gesamtanzahl der Stickstoffgruppen des Polyethylenimins und Gesamtanzahl der Phosphatgruppen der in der Zusammensetzung enthaltenen Nukleinsäure etwa 1 bis 20, bevorzugterweise etwa 1,5 bis 10, bevorzugtererweise etwa 2 bis 5 und am bevorzugtesten etwa 2 bis 3 beträgt.
Ausführungsform 51: Zusammensetzung nach einer der Ausführungsformen 37 bis 50,
   **dadurch gekennzeichnet, dass** die Zusammensetzung die L-Nukleinsäure intrazellulär bereitstellt.
Ausführungsform 52: Pharmazeutische Zusammensetzung umfassend eine Zusammensetzung nach einer der Ausführungsformen 37 bis 51, und einen pharmazeutisch akzeptablen Träger.
Ausführungsform 53: Verwendung nach einer der Ausführungsformen 1 bis 8, wobei die L-Nukleinsäure eine Zusammensetzung nach einer der Ausführungsformen 37 bis 52 ist.
Ausführungsform 54: Verfahren nach einer der Ausführungsformen 9 bis 16, wobei die L-Nukleinsäure eine Zusammensetzung nach einer der Ausführungsformen 37 bis 52 ist.
Ausführungsform 55: Verwendung nach einer der Ausführungsformen 17 bis 26, wobei die L-Nukleinsäure eine Zusammensetzung nach einer der Ausführungsformen 37 bis 52 ist.
Ausführungsform 56: Verwendung nach einer der Ausführungsformen 27 bis 36, wobei die L-Nukleinsäure eine Zusammensetzung nach einer der Ausführungsformen 37 bis 52 ist.
Ausführungsform 57: HMGA bindende Nukleinsäure, **dadurch gekennzeichnet, dass** die Nukleinsäure einen Abschnitt Box A1 und einen Abschnitt Box A2 umfasst, wobei der Abschnitt Box A1 und der Abschnitt Box A2 durch einen Zwischenabschnitt miteinander verbunden sind und wobei Box A1 und Box A2 einzeln und unabhängig voneinander aus der Gruppe ausgewählt sind, die GGGCG, GGGUG und GGGAG umfasst.
Ausführungsform 58: HMGA bindende Nukleinsäure nach Ausführungsform 57, **dadurch gekennzeichnet, dass** der Zwischenabschnitt entweder aus einem Zwischenabschnitt Z1 umfassend sechs oder sieben Nukleotide oder aus einem Zwischenabschnitt Z2 umfassend 12 bis 25 Nukleotide besteht.
Ausführungsform 59: HMGA bindende Nukleinsäure nach Ausführungsform 57 bis 58, **dadurch gekennzeichnet, dass** die Nukleinsäure am 5'-Ende des Abschnittes Box A1 einen ersten Abschnitt aufweist und am 3'-Ende des Abschnittes Box A2 einen zweiten Abschnitt aufweist, wobei bevorzugterweise beide Abschnitte unabhängig voneinander je vier bis acht Nukleotide umfassen.
Ausführungsform 60: HMGA bindende Nukleinsäure nach Ausführungsform 59, **dadurch gekennzeichnet, dass** die beiden Abschnitte wenigstens teilweise oder vollständig miteinander hybridisiert sind, wobei sich die Hybridisierung über vier bis acht Nukleotidpaare erstreckt.
Ausführungsform 61: HMGA bindende Nukleinsäure nach Ausführungsform 59 oder 60, **dadurch gekennzeichnet, dass** die Nukleinsäure am 5'-Ende des Abschnittes Box A1 einen Abschnitt Helix A1 und am 3'-Ende des Abschnittes Box A2 einen Abschnitt Helix A2 aufweist, wobei bevorzugterweise der Abschnitt Helix A1 vier bis acht Nukleotide umfasst und bevorzugterweise der Abschnitt Helix A2 vier bis acht Nukleotide umfasst, und wobei bevorzugterweise der Abschnitt Helix A1 den ersten Abschnitt am 5'-Ende des Abschnittes Box A1 oder einen Teil davon darstellt, und
   wobei bevorzugterweise der Abschnitt Helix A2 den zweiten Abschnitt am 3'-Ende des Abschnittes Box A2 oder einen Teil davon darstellt, wobei die Länge des Abschnittes Helix A1 unabhängig von der Länge des Abschnittes Helix A2 ist.
Ausführungsform 62: HMGA bindende Nukleinsäure nach Ausführungsform 61, **dadurch gekennzeichnet, dass** die Abschnitte Helix A1 und Helix A2 wenigstens teilweise oder vollständig miteinander hybridisiert sind, wobei sich die Hybridisierung über vier bis acht Nukleotidpaare erstreckt.
Ausführungsform 63: HMGA bindende Nukleinsäure nach Ausführungsform 61 oder 62, **dadurch gekennzeichnet, dass** zwischen dem 3'-Ende des Abschnittes Helix A1 und dem 5'-Ende des Abschnittes Box A1 ein Abschnitt Helix B1 angeordnet ist und zwischen dem 3'-Ende des Abschnittes Box A2 und dem 5'-Ende des Abschnittes Helix A2 ein Abschnitt Helix B2 angeordnet ist, wobei bevorzugterweise die Länge des Abschnittes Helix B1 und Helix B2 jeweils einzeln und unabhängig eine Länge von vier bis acht Nukleotiden umfasst.
Ausführungsform 64: HMGA bindende Nukleinsäure nach Ausführungsform 63, **dadurch gekennzeichnet, dass** die Abschnitte Helix B1 und Helix B2 wenigstens teilweise oder vollständig miteinander hybridisiert sind, wobei sich die Hybridisierung über vier bis acht Nukleotidpaare erstreckt.
Ausführungsform 65: HMGA bindende Nukleinsäure nach Ausführungsform 63 oder 64, **dadurch gekennzeichnet, dass** zwischen dem 3'-Ende des Abschnittes Helix A1 und dem 5'-Ende des Abschnittes Helix B1 null bis fünf Nukleotide angeordnet sind.
Ausführungsform 66: HMGA bindende Nukleinsäure nach Ausführungsform 65, **dadurch gekennzeichnet, dass** zwischen dem 3'-Ende des Abschnittes Helix A1 und dem 5'-Ende des Abschnittes Helix B1 zwei Nukleotide angeordnet sind.
Ausführungsform 67: HMGA bindende Nukleinsäure nach einer der Ausführungsformen 63 bis 66, **dadurch gekennzeichnet, dass** zwischen dem 3'-Ende des Abschnittes Helix B2 und dem 5'-Ende des Abschnittes Helix A2 null bis sechs Nukleotide angeordnet sind.
Ausführungsform 68: HMGA bindende Nukleinsäure nach Ausführungsform 67, bevorzugterweise insoweit sich diese auf Ausführungsform 66 bezieht, **dadurch gekennzeichnet, dass** zwischen dem 3'-Ende des Abschnittes Helix B2 und dem 5'-Ende des Abschnittes Helix A2 ein Nukleotid angeordnet ist.
Ausführungsform 69: HMGA bindende Nukleinsäure nach einer der Ausführungsformen 63 bis 68, wobei die Summe der Nukleotide von Abschnitt Helix A1 und von Abschnitt Helix B1 10 bis 12 Nukleotide -beträgt und die Summe der Nukleotide von Abschnitt Helix A2 und von Abschnitt Helix B2 10 bis 12 Nukleotide beträgt.
Ausführungsform 70: HMGA bindende Nukleinsäure nach Ausführungsform 69, wobei die Summe der hybridisierten Nukleotide aus der Hybridisierung von Abschnitt Helix A1 mit Abschnitt Helix A2 und von Abschnitt Helix B1 mit Abschnitt Helix B2 10 bis 12 Nukleotidpaare beträgt.
Ausführungsform 71: HMGA bindende Nukleinsäure nach einer der Ausführungsformen 63 bis 70, bevorzugterweise 63 oder 64, **dadurch gekennzeichnet, dass** die Nukleinsäure keinen Abschnitt Helix A1 und Helix A2 umfasst, wodurch am 5'-Ende der Nukleinsäure der Abschnitt Helix B1 und am 3'-Ende die Helix B2 angeordnet ist,
   wobei bevorzugterweise die Länge des Abschnittes Helix B1 und Helix B2 jeweils einzeln und unabhängig eine Länge von vier bis acht Nukleotiden umfasst.
Ausführungsform 72: HMGA bindende Nukleinsäure nach Ausführungsform 71, wobei die Abschnitte Helix B1 und Helix B2 wenigstens teilweise oder vollständig miteinander hybridisiert sind, wobei sich die Hybridisierung über vier bis acht Nukleotidpaare erstreckt.
Ausführungsform 73: HMGA bindende Nukleinsäure nach einer der Ausführungsformen 61 bis 62, **dadurch gekennzeichnet, dass** zwischen dem 3'-Ende des Abschnittes Helix A1 und dem 5'-Ende des Abschnittes Box A1 ein bis fünf Nukleotide angeordnet sind und zwischen dem 3'-Ende des Abschnittes Box A2 und dem 5'-Ende des Abschnittes Helix A2 ein bis drei Nukleotide angeordnet sind.
Ausführungsform 74: HMGA bindende Nukleinsäure nach einer der Ausführungsformmen 63 bis 72, **dadurch gekennzeichnet, dass** zwischen dem 3'-Ende des Abschnittes Helix B1 und dem 5'-Ende des Abschnittes Box A1 zwei Nukleotide angeordnet sind und zwischen dem 3'-Ende des Abschnittes Box A2 und dem 5'-Ende des Abschnittes Helix B2 eins bis sieben Nukleotide angeordnet sind.
Ausführungsform 75: HMGA bindende Nukleinsäure nach einer der Ausführungsformen 58 bis 65, 67, soweit sich diese auf die Ausführungsformen 63 bis 65 bezieht, 69 und 70, soweit sich diese auf die Ausführungsformen 63 bis 65 und 67 beziehen, 71 und 72 soweit sich diese auf die Ausführungsformen 63 bis 65 und 67 und 69 und 70 beziehen, oder 74, soweit sich diese auf die Ausführungsformen 63 bis 65, 67, 69 bis 72 bezieht, jeweils in dem hierin eingeschränkten Umfang, **dadurch gekennzeichnet, dass** der Zwischenabschnitt Z1 sechs oder sieben Nukleotide umfasst.
Ausführungsform 76: HMGA bindende Nukleinsäure nach Ausführungsform 75, **dadurch gekennzeichnet, dass** der Zwischenabschnitt Z1 die Sequenz N₁N₂GN₈N₃N₄N₅ aufweist, wobei
   N₁ = U, C, A oder G ist;
   N₂ = G oder U ist;
   N₃ = U oder C ist;
   N₄ = U oder A ist;
   N₅ = G oder A ist; und
   N₈ = U ist oder fehlt.
Ausführungsform 77: HMGA bindende Nukleinsäure nach Ausführungsform 76, **dadurch gekennzeichnet, dass** die Nukleinsäure einen Abschnitt Box A1 und einen Abschnitt Box A2 umfasst, wobei das 3'-Ende des Abschnittes Box A1 direkt mit dem 5'-Ende des Zwischenabschnittes Z1 verbunden ist, und das 3'-Ende des Zwischenabschnittes Z1 direkt mit dem 5`-Ende des Abschnittes Box A2 verbunden ist.
Ausführungsform 78: HMGA bindende Nukleinsäure nach einer der Ausführungsformen 75 bis 77, insbesondere 77, **dadurch gekennzeichnet, dass** die Nukleinsäure einen Abschnitt Helix B1 und einen Abschnitt Helix B2 umfasst.
Ausführungsform 79: HMGA bindende Nukleinsäure nach Ausführungsform 78, **dadurch gekennzeichnet, dass** die Abschnitte Helix B1 und Helix B2 jeweils einzeln und unabhängig voneinander vier bis acht Nukleotide umfassen, die bevorzugterweise vollständig oder teilweise miteinander hybridisiert sind.
Ausführungsform 80: HMGA bindende Nukleinsäure nach einer der Ausführungsformen 78 oder 79, **dadurch gekennzeichnet, dass** zwischen dem 3'-Ende des Abschnittes Helix B1 und dem 5'-Ende des Abschnittes Box A1 in 5'-3'-Richtung zwei Nukleotide N₆, N₇ angeordnet sind, wobei N₆ G, A oder U ist, und N₇ = G oder U ist.
Ausführungsform 81: HMGA bindende Nukleinsäure nach einer der Ausführungsformen 78 bis 80, **dadurch gekennzeichnet, dass** zwischen dem 3'-Ende des Abschnittes Box A2 und dem 5'-Ende des Abschnittes Helix B2 kein Nukleotid angeordnet ist, oder in 5'-3'-Richtung die Nukleotidsequenz GNy angeordnet ist, wobei N_{y} null bis sechs Nukleotide umfasst, bevorzugterweise 0 oder 6 Nukleotide.
Ausführungsform 82: HMGA bindende Nukleinsäure nach einer der Ausführungsformmen 75 bis 81, **dadurch gekennzeichnet, dass** die Nukleinsäure einen Abschnitt Helix A1 und Helix A2 umfasst.
Ausführungsform 83: HMGA bindende Nukleinsäure nach Ausführungsform 82, **dadurch gekennzeichnet, dass** die Abschnitte Helix A1 und Helix A2 jeweils und unabhängig voneinander vier bis acht Nukleotide umfassen, wobei bevorzugterweise_die Abschnitte Helix A1 und Helix A2 vollständig oder teilweise miteinander hybridisiert sind.
Ausführungsform 84: HMGA bindende Nukleinsäure nach einer der Ausführungsformen 82 oder 83, **dadurch gekennzeichnet, dass** zwischen dem 3'-Ende des Abschnittes Helix A1 und dem 5'-Ende des Abschnittes Helix B1 eine Nukleotidsequenz Nₓ angeordnet ist, wobei Nₓ null bis fünf Nukleotide umfasst.
Ausführungsform 85: HMGA bindende Nukleinsäure nach einer der Ausführungsformen 82 bis 84, **dadurch gekennzeichnet, dass** zwischen dem 3'-Ende des Abschnittes Helix B2 und dem 5'-Ende des Abschnittes Helix A2 eine Nukleotidsequenz N_{z} angeordnet ist, wobei N_{z} null bis sechs Nukleotide umfasst.
Ausführungsform 86: HMGA bindende Nukleinsäure nach einer der Ausführungsformen 78 bis 85, wobei die Summe der hybridisierten Nukleotide aus der Hybridisierung von Abschnitt Helix A1 mit Abschnitt Helix A2 und von Abschnitt Helix B1 mit Abschnitt Helix B2 10 bis 12 Nukleotidpaare beträgt.
Ausführungsform 87: HMGA bindende Nukleinsäure nach einer der Ausführungsformmen 81 bis 86, **dadurch gekennzeichnet, dass** zwischen dem 3'-Ende des Abschnittes Box A2 und dem 5'-Ende des Abschnittes Helix B2 in 5'-3'-Richtung die Nukleotidsequenz GN_{y} angeordnet ist, wobei N_{y} null bis sechs Nukleotide umfasst, bevorzugterweise 0 oder 6 Nukleotide.
Ausführungsform 88: HMGA bindende Nukleinsäure nach Ausführungsform 87 umfassend die folgende Struktur Helix A1-NX-Helix B1-N6N7Box A1N1N2GN8N3N4N5BOX A2G-NY-Helix B2-NZ-Helix A2 wobei
   N₁ = U, C, A oder G ist;
   N₂ = G oder U ist;
   N₃ = U oder C ist;
   N₄ = U oder A ist;
   N₅ = G oder A ist;
   N₆ = G, A oder U ist;
   N₇ = G oder U ist;
   N₈ = U oder kein Nukleotid ist;
   Nₓ = null bis fünf Nukleotide ist;
   N_{y} = null oder sechs Nukleotide ist; und
   N_{z} = null bis sechs Nukleotide ist;
   der Abschnitt Box A1 und Abschnitt Box A2 jeweils einzeln und unabhängig voneinander ausgewählt sind aus der Gruppe von Nukleotidsequenzen umfassend GGGCG, GGGUG und GGGAG;
   der Abschnitt Helix A1 und der Abschnitt Helix A2 jeweils einzeln und unabhängig voneinander vier bis acht Nukleotide umfassen, wobei bevorzugterweise die Abschnitte Helix A1 und Helix A2 vollständig oder teilweise miteinander hybridisiert sind, und
   die Abschnitte Helix B1 und Helix B2 jeweils einzeln und unabhängig voneinander vier bis acht Nukleotide umfassen, wobei bevorzugterweise die Abschnitte Helix B1 und Helix B2 vollständig oder teilweise miteinander hybridisiert sind und der hybridisierende Bereich vier bis acht Nukleotide umfasst, und wobei die Summe der hybridisierten Nukleotide aus der Hybridisierung von Abschnitt Helix A1 mit Abschnitt Helix A2 und von Abschnitt Helix B1 mit Abschnitt Helix B2 10 bis 12 Nukleotidpaare beträgt.
Ausführungsform 89: HMGA bindende Nukleinsäure nach Ausführungsform 87 oder 88, umfassend eine Sequenz ausgewählt aus der Gruppe umfassend SEQ. ID. No. 1, SEQ. ID. No. 2, SEQ. ID. No. 3, SEQ. ID. No. 5, SEQ. ID. No. 6, SEQ. ID. No. 7 und SEQ. ID. No. 13.
Ausführungsform 90: HMGA bindende Nukleinsäure nach einer der Ausführungsformen 81 bis 86, **dadurch gekennzeichnet, dass** sich das 3'-Ende des Abschnittes Box A2 unmittelbar an das 5'-Ende des Abschnittes Helix B2 anschließt.
Ausführungsform 91: HMGA bindende Nukleinsäure nach Ausführungsform 90 umfassend die folgende Struktur Helix A1-Nₓ-Helix B1-N₆N₇Box A1 N₁N₂GN₈N₃N₄N₅BOX A2 Helix B2-N_{z}-Helix A2 wobei
   N₁ = U, C, A oder G ist;
   N₂ = G oder U ist;
   N₃ = U oder C ist;
   N₄ = U oder A ist;
   N₅ = G oder A ist;
   N₆ = G, A oder U ist;
   N₇ = G oder U ist;
   N₈ = U oder kein Nukleotid ist;
   Nₓ = null bis fünf Nukleotide ist; und
   N_{z} = null bis sechs Nukleotide ist;
   der Abschnitt Box A1 und Abschnitt Box A2 jeweils einzeln und unabhängig voneinander ausgewählt sind aus der Gruppe von Nukleotidsequenzen umfassend GGGCG, GGGUG und GGGAG;
   der Abschnitt Helix A1 und der Abschnitt Helix A2 jeweils einzeln und unabhängig voneinander vier bis acht Nukleotide umfassen, wobei bevorzugterweise die Abschnitte Helix A1 und Helix A2 vollständig oder teilweise miteinander hybridisiert sind, und
   die Abschnitte Helix B1 und Helix B2 jeweils einzeln und unabhängig voneinander vier bis acht Nukleotide umfassen, wobei bevorzugterweise die Abschnitte Helix B1 und Helix B2 vollständig oder teilweise miteinander hybridisiert sind und der hybridisierende Bereich vier bis acht Nukleotide umfasst, und wobei die Summe der hybridisierten Nukleotide aus der Hybridisierung von Abschnitt Helix A1 mit Abschnitt Helix A2 und von Abschnitt Helix B1 mit Abschnitt Helix B2 10 bis 12 Nukleotidpaare beträgt.
Ausführungsform 92: HMGA bindende Nukleinsäure nach Ausführungsform 90 oder 91, umfassend eine Sequenz umfassend SEQ. ID. No. 3.
Ausführungsform 93: HMGA bindende Nukleinsäure nach Ausführungsform 88, umfassend die folgende Struktur Helix B1-N6N7Box A1N1N2GN8N3N4N5BOX A2G-NY-Helix B2
Ausführungsform 94: HMGA bindende Nukleinsäure nach Ausführungsform 91, umfassend die folgende Struktur. Helix B1-N6N7Box A1N1N2GN8N3N4N5BOX A2Helix B2
Ausführungsform 95: HMGA bindende Nukleinsäure nach Ausführungsform 93, wobei die Sequenz ausgewählt ist aus der Gruppe umfassend SEQ. ID. No. 15 und SEQ. ID. No. 16.
Ausführungsform 96: HMGA bindende Nukleinsäure nach einer der Ausführungsformen 58 bis 70 und 73 oder 74, **dadurch gekennzeichnet, dass** der Zwischenabschnitt Z₂ 12 bis 25 Nukleotide umfasst.
Ausführungsform 97: HMGA bindende Nukleinsäure nach Ausführungsform 96, **dadurch gekennzeichnet, dass** der Zwischenabschnitt Z2 einen Abschnitt Helix C1 und einen Abschnitt Helix C2 umfasst.
Ausführungsform 98: HMGA bindende Nukleinsäure nach Ausführungsform 97, **dadurch gekennzeichnet, dass** zwischen dem Abschnitt Helix C1 und dem Abschnitt Helix C2 ein zentraler Abschnitt N_{c} angeordnet ist.
Ausführungsform 99: HMGA bindende Nukleinsäure nach Ausführungsform 97 oder 98, **dadurch gekennzeichnet, dass** die Länge des Abschnittes Helix C1 und Helix C2 gleich lang ist.
Ausführungsform 100: HMGA bindende Nukleinsäure nach einer der Ausführungsformen 97 bis 99, **dadurch gekennzeichnet, dass** die Länge des Abschnittes Helix C1 und Helix C2 einzeln und unabhängig drei bis sechs Nukleotide ist.
Ausführungsform 101: HMGA bindende Nukleinsäure nach Ausführungsform 97 bis 100, **dadurch gekennzeichnet, dass** die Abschnitte Helix C1 und Helix C2 vollständig oder teilweise miteinander hybridisiert ist.
Ausführungsform 102: HMGA bindende Nukleinsäure nach einer der Ausführungsformen 96 bis 101, **dadurch gekennzeichnet, dass** der zentrale Abschnitt N_{c} drei bis fünf Nukleotide umfasst.
Ausführungsform 103: HMGA bindende Nukleinsäure nach einer der Ausführungsformen 96 bis 102, **dadurch gekennzeichnet, dass** die Nukleinsäure einen Abschnitt Box A1 und einen Abschnitt Box A2 umfasst, wobei zwischen dem 3'-Ende des Abschnittes Box A1 und dem 5'-Ende des Abschnittes Helix C1 eine Nukleotidsequenz N_{b} angeordnet ist und drei Nukleotide umfasst.
Ausführungsform 104: HMGA bindende Nukleinsäure nach einer der Ausführungsformen 96 bis 103, **dadurch gekennzeichnet, dass** die Nukleinsäure einen Abschnitt Box A1 und einen Abschnitt Box A2 umfasst, wobei zwischen dem 3'-Ende des Abschnittes Helix C2 und dem 5'-Ende des Abschnittes Box A2 eine Nukleotidsequenz N_{d} angeordnet ist und zwei bis fünf Nukleotide umfasst.
Ausführungsform 105: HMGA bindende Nukleinsäure nach einer der Ausführungsformen 96 bis 104, **dadurch gekennzeichnet, dass** die Nukleinsäure einen Abschnitt Helix A1 und einen Abschnitt Helix A2 umfasst.
Ausführungsform 106: HMGA bindende Nukleinsäure Ausführungsform 105, **dadurch gekennzeichnet, dass** die Abschnitte Helix A1 und Helix A2 jeweils einzeln und unabhängig voneinander fünf bis sechs Nukleotide umfassen, wobei bevorzugterweise der Abschnitt Helix A1 und der Abschnitt Helix A2 vollständig oder teilweise miteinander hybridisiert sind.
Ausführungsform 107: HMGA bindende Nukleinsäure einer der Ausführungsformen 105 bis 106, **dadurch gekennzeichnet, dass** das zwischen dem 3'-Ende des Abschnittes Helix A1 und dem 5'-Ende des Abschnittes Box A1 eine Nukleotidsequenz Nₐ angeordnet ist, wobei Nₐ ein bis fünf Nukleotide umfasst.
Ausführungsform 108: HMGA bindende Nukleinsäure nach einer der Ausführungsformen 105 bis 107, **dadurch gekennzeichnet, dass** zwischen dem 3'-Ende des Abschnittes Box A2 und dem 5'-Ende des Abschnittes Helix A2 in 5'-3'-Richtung eine Nukleotidsequenz GNₑ angeordnet ist, wobei Nₑ eins bis zwei Nukleotide, bevorzugterweise A oder UU umfasst.
Ausführungsform 109: HMGA bindende Nukleinsäure nach einer der Ausführungsformen 105 bis 108, **dadurch gekennzeichnet, dass** der Abschnitt Helix C1 und der Abschnitt Helix C2 jeweils einzeln und unabhängig voneinander eine Länge von fünf oder sechs Nukleotiden aufweisen, wobei bevorzugterweise die Abschnitte Helix C1 und Helix C2 vollständig oder teilweise miteinander hybridisiert sind.
Ausführungsform 110: HMGA bindende Nukleinsäure nach Ausführungsform 109, umfassend die folgende Struktur: Helix A1-Na-Box A1-Nb-Helix C1-Nc-Helix C2-Nd-Box A2-G-Ne-Helix A2 (III) wobei
   Nₐ = ein bis fünf Nukleotide ist;
   N_{b} = drei Nukleotide ist;
   Nₑ = drei bis fünf Nukleotide ist;
   N_{d} = zwei bis fünf Nukleotide ist; und
   Nₑ = eins bis zwei Nukleotide, bevorzugterweise A oder UU ist;
   der Abschnitt Box A1 und der Abschnitt Box A2 jeweils einzeln und unabhängig voneinander ausgewählt sind aus der Gruppe, die GGGCG, GGGUG und GGGAG umfasst,
   die Abschnitte Helix A1 und Helix A2 jeweils einzeln und unabhängig voneinander fünf oder sechs Nukleotide umfassen, und
   die Abschnitte Helix C1 und Helix C2 jeweils fünf oder sechs Nukleotide umfasst, die bevorzugterweise vollständig oder teilweise miteinander hybridisiert sind.
Ausführungsform 111: HMGA bindende Nukleinsäure nach Ausführungsform 110, umfassend eine Sequenz, die ausgewählt ist aus der Gruppe umfassend SEQ. ID. No. 8, SEQ. ID. No. 9, SEQ. ID. No. 10, SEQ. ID. No. 11, SEQ. ID. No. 14, SEQ. ID. No. 22 und SEQ. ID. No. 24.
Ausführungsform 112: HMGA bindende Nukleinsäure nach einer der Ausführungsformen 96 bis 101, **dadurch gekennzeichnet, dass** die Nukleinsäure einen Abschnitt Box A1 und einen Abschnitt Helix C1 umfasst, wobei zwischen dem 3'-Ende des Abschnittes Box A1 und dem 5'-Ende des Abschnittes Helix C1 ein Nukleotid A angeordnet ist.
Ausführungsform 113: HMGA bindende Nukleinsäure nach Ausführungsform 112,
   **dadurch gekennzeichnet, dass** die Nukleinsäure einen Abschnitt Helix C2 und einen Abschnitt Box A2 umfasst, wobei zwischen dem 3'-Ende des Abschnittes Helix C2 und dem 5'-Ende des Abschnittes Box A2 ein Nukleotid G angeordnet ist.
Ausführungsform 114: HMGA bindende Nukleinsäure nach Ausführungsform 112 oder 113, **dadurch gekennzeichnet, dass** der zentrale Abschnitt N_{c} vier Nukleotide umfasst, , wobei N_{c} bevorzugterweise GAUG ist.
Ausführungsform 115: HMGA bindende Nukleinsäure nach einer der Ausführungsformmen 112 bis 114, umfassend einen Abschnitt Helix B1 und einen Abschnitt Helix B2.
Ausführungsform 116: HMGA bindende Nukleinsäure nach Ausführungsform 115,
   **dadurch gekennzeichnet, dass** die Abschnitte Helix B1 und Helix B2 einzeln und unabhängig voneinander jeweils fünf Nukleotide umfassen, wobei bevorzugterweise der Abschnitt Helix B1 mit dem Abschnitt Helix B2 hybridisiert ist.
Ausführungsform 117: HMGA bindende Nukleinsäure nach Ausführungsform 115 oder 116, **dadurch gekennzeichnet, dass** zwischen dem 3'-Ende des Abschnittes Helix B1 und dem 5'-Ende des Abschnittes Box A1 eine Nukleotidsequenz umfassend zwei Nukleotide Nⱼ angeordnet sind, wobei Nⱼ bevorzugterweise AG ist.
Ausführungsform 118: HMGA bindende Nukleinsäure nach einer der Ausführungsformen 115 bis 117, **dadurch gekennzeichnet, dass** zwischen dem 3'-Ende des Abschnittes Box A2 und dem 5'-Ende von Helix B2 ein Nukleotid G angeordnet ist.
Ausfiihrungsform 119: HMGA bindende Nukleinsäure nach einer der Ausführungsformen 112 bis 118, umfassend einen Abschnitt Helix A1 und einen Abschnitt Helix A2.
Ausführungsform 120: HMGA bindende Nukleinsäure nach Ausführungsform 119,
   wobei die Abschnitte Helix A1 und Helix A2 einzeln und unabhängig voneinander jeweils sechs Nukleotide umfassen und bevorzugterweise der Abschnitt Helix A1 und der Abschnitt Helix A2 miteinander hybridisiert sind.
Ausführungsform 121: HMGA bindende Nukleinsäure nach Ausführungsform 119 oder 120, **dadurch gekennzeichnet, dass** zwischen dem 3'-Ende des Abschnittes Helix A1 und dem 5'-Ende des Abschnittes Helix B1 eine Nukleotidsequenz umfassend zwei Nukleotide Nᵢ angeordnet ist, wobei Nᵢ bevorzugterweise CA ist.
Ausführungsform 122: HMGA bindende Nukleinsäure nach einer der Ausführungsformen 119 bis 121, **dadurch gekennzeichnet, dass** zwischen dem 3'-Ende des Abschnittes Helix B2 und dem 5'-Ende des Abschnittes Helix A2 ein Nukleotid A ist.
Ausführungsform 123: HMGA bindende Nukleinsäure nach einer der Ausführungsformen 112 bis 122, **dadurch gekennzeichnet, dass** die Abschnitte Helix C1 und Helix C2 jeweils drei Nukleotide umfassen, wobei bevorzugterweise die Abschnitte Helix C1 und Helix C2 miteinander hybridisiert sind.
Ausführungsform 124: HMGA bindende Nukleinsäure nach Ausführungsform 123 umfassend die folgende Struktur: 
Helix A1-Nᵢ-Helix B1-Nⱼ-Box A1-A-Helix C1-N_{c}-Helix C2-G-Box A2-G-Helix B2-A -
Helix A2
 wobei
   Nᵢ = zwei Nukleotide umfasst, bevorzugterweise CA ist;
   Nj = zwei Nukleotide umfasst, bevorzugterweise AG ist;
   N_{c} = vier Nukleotide umfasst, bevorzugterweise GAUG ist;
   die Abschnitte Box A1 und Box A2 jeweils einzeln und unabhängig voneinander ausgewählt sind aus der Gruppe umfassend die Sequenzen GGGCG, GGGUG und GGGAG;
   die Abschnitte Helix A1 und Helix A2 jeweils einzeln und unabhängig sechs Nukleotide umfassen, die bevorzugterweise miteinander hybridisiert sind;
   die Abschnitte Helix B1 und Helix B2 jeweils einzeln und unabhängig fünf Nukleotide umfassen, wobei bevorzugterweise der Abschnitt Helix B1 und der Abschnitt Helix B2 miteinander hybridisiert ist; und
   die Abschnitte Helix C1 und Helix C2 jeweils einzeln und unabhängig drei Nukleotide umfassen, wobei bevorzugterweise die Abschnitte Helix C1 und Helix C2 miteinander hybridisiert sind.
Ausführungsform 125: HMGA bindende Nukleinsäure nach Ausführungsform 124, umfassend eine Sequenz, die ausgewählt ist aus der Gruppe umfassend SEQ. ID. No. 12.
Ausführungsform 126: Nukleinsäure nach einer der Ausführungsformen 57 bis 125,
   **dadurch gekennzeichnet, dass** sie an Transkriptionsfaktoren binden, insbesondere Transkriptionsfaktoren, die einen AT-Haken aufweisen.
Ausführungsform 127: Nukleinsäure bindend an einen Transkriptionsfaktor umfassend einen AT-Haken, wobei die Nukleinsäure einen Aufbau gemäß einer der Ausführungsformen 57 bis 126 umfasst.
Ausführungsform 128: Zusammensetzung nach einer der Ausführungsformen 37 bis 52,
   wobei die L-Nukleinsäure eine Nukleinsäure nach einer der Ausführungsformen 57 bis 127 ist.
Ausführungsform 129: Verwendung nach einer der Ausführungsformen 1 bis 8, wobei die L-Nukleinsäure eine Nukleinsäure nach einer der Ausführungsformen 57 bis 127 ist.
Ausführungsform 130: Verfahren nach einer der Ausführungsformen 9 bis 16, wobei die L-Nukleinsäure eine Nukleinsäure nach einer der Ausführungsformen 57 bis 127 ist.
Ausführungsform 131: Verwendung nach einer der Ausführungsformen 17 bis 26, wobei die L-Nukleinsäure eine Nukleinsäure nach einer der Ausführungsformen 57 bis 127 ist.
Ausführungsform 132: Verfahren nach einer der Ausführungsformen 27 bis 36, wobei die L-Nukleinsäure eine Nukleinsäure nach einer der Ausführungsformen 57 bis 127 ist.
Ausführungsform 133: Verfahren zum Screenen eines HMGA-Antagonisten oder HMGA-Agonisten umfassend die folgenden Schritte:
   - Bereitstellen eines Kandidaten-HMGA-Antagonisten und/oder eines Kandidaten-HMGA-Agonisten,
   - Bereitstellen einer Nukleinsäure nach einer der Ausführungsformen 57 bis 127,
   - Bereitstellen eines Testsystems, welches ein Signal in Gegenwart eines HMGA-Antagonisten und/oder eines HMGA-Agonisten liefert, und
   - Bestimmen, ob der Kandidaten-HMGA-Antagonist ein HMGA-Antagonist ist und/oder ob der Kandidaten-HMGA-Agonist ein HMGA-Agonist ist.
Ausführungsform 134: Verfahren zum Screenen eines HMGA-Agonisten und/oder eines HMGA-Antagonisten umfassend die folgenden Schritte:
   - Bereitstellen eines an einer Phase, bevorzugterweise einer Festphase, imobilisierten HMGA,
   - Bereitstellen einer Nukleinsäure gemäß einer der Ausführungsformen 57 bis 127, bevorzugterweise eine Nukleinsäure nach einer der Ausführungsformen 57 bis 127, die markiert ist,
   - Zugeben eines Kandidaten-HMGA-Agonisten und/oder eines Kandidaten-HMGA-Antagonisten, und
   - Bestimmen, ob der Kandidaten-HMGA-Agonist ein HMGA-Agonist ist und/oder ob der Kandidaten-HMGA-Antagonist ein HMGA-Antagonist ist.
Ausführungsform 135: Verfahren nach Ausführungsform 134, **dadurch gekennzeichnet, dass** die Bestimmung so durchgeführt wird, dass getestet wird, ob die Nukleinsäure durch den Kandidaten-HMGA-Agonisten oder durch den Kandidaten-HMGA-Antagonisten ersetzt wird.
Ausführungsform 136: Kit für den Nachweis von HMGA, umfassend eine Nukleinsäure nach einer der Ausführungsformen 57 bis 127.
Ausführungsform 137: HMGA-Antagonist, erhältlich durch ein Verfahren nach einer der Ausführungsformen 134 oder 135.
Ausführungsform 138: HMGA-Agonist, erhältlich durch ein Verfahren nach einer der Ausführungsformen 134 oder 135.
Ausführungsform 139: Komplex umfassend ein HMGA-Protein und eine Nukleinsäure nach einer der Ausführungsformen 57 bis 127.

Die in der vorangehenden Beschreibung, den Ansprüchen und den Zeichnungen offenbarten Merkmale der Erfindung können sowohl einzeln als auch in beliebiger Kombination zur Verwirklichung der Erfindung in ihren verschiedenen Ausführungsformen wesentlich sein.

## Patentansprüche

1. HMGA bindende Nukleinsäure, **dadurch gekennzeichnet, dass** die Nukleinsäure einen Abschnitt Box A1 und einen Abschnitt Box A2 umfasst, wobei der Abschnitt Box A1 und der Abschnitt Box A2 durch einen Zwischenabschnitt miteinander verbunden sind und wobei Box A1 und Box A2 einzeln und unabhängig voneinander aus der Gruppe ausgewählt sind, die GGGCG, GGGUG und GGGAG umfasst.

2. HMGA bindende Nukleinsäure, nach Anspruch 1, **dadurch gekennzeichnet, dass** HMGA aus der Gruppe umfassend HMGA1, HMGA1a, HMGA1b und HMGA2 ausgewählt ist.

3. HMGA bindende Nukleinsäure nach Anspruch 1 oder 2, **dadurch gekennzeichnet, dass** der Zwischenabschnitt entweder aus einem Zwischenabschnitt Z1 umfassend sechs oder sieben Nukleotide oder aus einem Zwischenabschnitt Z2 umfassend 12 bis 25 Nukleotide besteht.

4. HMGA bindende Nukleinsäure nach Anspruch 1 bis 3 , **dadurch gekennzeichnet, dass** die Nukleinsäure am 5'-Ende des Abschnittes Box A1 einen ersten Abschnitt aufweist und am 3'-Ende des Abschnittes Box A2 einen zweiten Abschnitt aufweist, wobei bevorzugterweise beide Abschnitte unabhängig voneinander je vier bis acht Nukleotide umfassen.

5. HMGA bindende Nukleinsäure nach Anspruch 1 bis 4, **dadurch gekennzeichnet, dass** die beiden Abschnitte wenigstens teilweise oder vollständig miteinander hybridisiert sind, wobei sich die Hybridisierung über vier bis acht Nukleotidpaare erstreckt.

6. HMGA bindende Nukleinsäure nach Anspruch 3 bis 5, **dadurch gekennzeichnet, dass** der Zwischenabschnitt Z1 die Sequenz N₁N₂GN₈N₃N₄N₅ aufweist, wobei
N₁ = U, C, A oder G ist;
N₂ = G oder U ist;
N₃ = U oder C ist;
N₄ = U oder A ist;
N₅ = G oder A ist; und
N₈ = U ist oder fehlt.

7. HMGA bindende Nukleinsäure nach Anspruch 1 bis 6 umfassend die folgende Struktur Helix A1-Nx-Helix B1-N6N7Box A1N1N2GN8N3N4N5BOX A2G-NY-Helix B2-Nz-Helix A2 wobei
N₁ = U, C, A oder G ist;
N₂ = G oder U ist;
N₃ = U oder C ist;
N₄ = U oder A ist;
N₅ = G oder A ist;
N₆ = G, A oder U ist;
N₇ = G oder U ist;
N₈ = U oder kein Nukleotid ist;
Nₓ = null bis fünf Nukleotide ist;
N_{y} = null oder sechs Nukleotide ist; und
N_{z} = null bis sechs Nukleotide ist;
der Abschnitt Box A1 und Abschnitt Box A2 jeweils einzeln und unabhängig voneinander ausgewählt sind aus der Gruppe von Nukleotidsequenzen umfassend GGGCG, GGGUG und GGGAG;
der Abschnitt Helix A1 und der Abschnitt Helix A2 jeweils einzeln und unabhängig voneinander vier bis acht Nukleotide umfassen, wobei bevorzugterweise die Abschnitte Helix A1 und Helix A2 vollständig oder teilweise miteinander hybridisiert sind, und
die Abschnitte Helix B1 und Helix B2 jeweils einzeln und unabhängig voneinander vier bis acht Nukleotide umfassen, wobei bevorzugterweise die Abschnitte Helix B1 und Helix B2 vollständig oder teilweise miteinander hybridisiert sind und der hybridisierende Bereich vier bis acht Nukleotide umfasst, und wobei die Summe der hybridisierten Nukleotide aus der Hybridisierung von Abschnitt Helix A1 mit Abschnitt Helix A2 und von Abschnitt Helix B1 mit Abschnitt Helix B2 10 bis 12 Nukleotidpaare beträgt.

8. HMGA bindende Nukleinsäure nach Anspruch 7, umfassend eine Sequenz ausgewählt aus der Gruppe umfassend SEQ. ID. No. 1, SEQ. ID. No. 2, SEQ. ID. No. 3, SEQ. ID. No. 5, SEQ. ID. No. 6, SEQ. ID. No. 7 und SEQ. ID. No. 13.

9. HMGA bindende Nukleinsäure nach Anspruch 7, umfassend die folgende Struktur

Helix B1-N6N7 Box A1 N1N2GN8N3N4N5 BOX A2 G-NY-Helix B2

10. HMGA bindende Nukleinsäure nach Anspruch 9, wobei die Sequenz ausgewählt ist aus der Gruppe umfassend SEQ. ID. No. 15 und SEQ. ID. No. 16.

11. HMGA bindende Nukleinsäure nach Anspruch 1 bis 6 umfassend die folgende Struktur Helix A1-Nx-Helix B1-N6N7Box A1N1N2GN8N3N4N5BOX A2Helix B2-Nz-Helix A2 wobei
N₁ = U, C, A oder G ist;
N₂ = G oder U ist;
N₃ = U oder C ist;
N₄ = U oder A ist;
N₅ = G oder A ist;
N₆ = G, A oder U ist;
N₇ = G oder U ist;
N₈ = U oder kein Nukleotid ist;
Nₓ = null bis fünf Nukleotide ist; und
N_{z} = null bis sechs Nukleotide ist;
der Abschnitt Box A1 und Abschnitt Box A2 jeweils einzeln und unabhängig voneinander ausgewählt sind aus der Gruppe von Nukleotidsequenzen umfassend GGGCG, GGGUG und GGGAG;
der Abschnitt Helix A1 und der Abschnitt Helix A2 jeweils einzeln und unabhängig voneinander vier bis acht Nukleotide umfassen, wobei bevorzugterweise die Abschnitte Helix A1 und Helix A2 vollständig oder teilweise miteinander hybridisiert sind, und
die Abschnitte Helix B1 und Helix B2 jeweils einzeln und unabhängig voneinander vier bis acht Nukleotide umfassen, wobei bevorzugterweise die Abschnitte Helix B1 und Helix B2 vollständig oder teilweise miteinander hybridisiert sind und der hybridisierende Bereich vier bis acht Nukleotide umfasst, und wobei die Summe der hybridisierten Nukleotide aus der Hybridisierung von Abschnitt Helix A1 mit Abschnitt Helix A2 und von Abschnitt Helix B1 mit Abschnitt Helix B2 10 bis 12 Nukleotidpaare beträgt.

12. HMGA bindende Nukleinsäure nach Anspruch 11, umfassend eine Sequenz umfassend SEQ. ID. No. 3.

13. HMGA bindende Nukleinsäure nach Anspruch 11, umfassend die folgende Struktur.

Helix B1-N6N7Box A1N1N2GN8N3N4N5BOX A2Helix B2

14. HMGA bindende Nukleinsäure nach Anspruch 1 bis 5, umfassend die folgende Struktur: Helix A1-Nₐ-Box A1-N_{b}-Helix C1-N_{c}-Helix C2-N_{d}-Box A2-G-Nₑ-Helix A2 (III) wobei
Nₐ = ein bis fünf Nukleotide ist;
N_{b} = drei Nukleotide ist;
N_{c} = drei bis fünf Nukleotide ist;
N_{d} = zwei bis fünf Nukleotide ist; und
Nₑ = eins bis zwei Nukleotide, bevorzugterweise A oder UU ist;
der Abschnitt Box A1 und der Abschnitt Box A2 jeweils einzeln und unabhängig voneinander ausgewählt sind aus der Gruppe, die GGGCG, GGGUG und GGGAG umfasst,
die Abschnitte Helix A1 und Helix A2 jeweils einzeln und unabhängig voneinander fünf oder sechs Nukleotide umfassen, und
die Abschnitte Helix C1 und Helix C2 jeweils fünf oder sechs Nukleotide umfasst, die bevorzugterweise vollständig oder teilweise miteinander hybridisiert sind.

15. HMGA bindende Nukleinsäure nach Anspruch 14, umfassend eine Sequenz, die ausgewählt ist aus der Gruppe umfassend SEQ. ID. No. 8, SEQ. ID. No. 9, SEQ. ID. No. 10, SEQ. ID. No. 11, SEQ. ID. No. 14, SEQ. ID. No. 22 und SEQ. ID. No. 24.

16. HMGA bindende Nukleinsäure nach Anspruch 1 bis 6 umfassend die folgende Struktur: 
Helix A1-Nᵢ-Helix B1-Nⱼ-Box A1-A-Helix C1-N_{c}-Helix C2-G-Box A2-G-Helix B2-A -
Helix A2
 wobei
Nᵢ = zwei Nukleotide umfasst, bevorzugterweise CA ist;
Nj = zwei Nukleotide umfasst, bevorzugterweise AG ist;
N_{c} = vier Nukleotide umfasst, bevorzugterweise GAUG ist;
die Abschnitte Box A1 und Box A2 jeweils einzeln und unabhängig voneinander ausgewählt sind aus der Gruppe umfassend die Sequenzen GGGCG, GGGUG und GGGAG;
die Abschnitte Helix A1 und Helix A2 jeweils einzeln und unabhängig sechs Nukleotide umfassen, die bevorzugterweise miteinander hybridisiert sind;
die Abschnitte Helix B1 und Helix B2 jeweils einzeln und unabhängig fünf Nukleotide umfassen, wobei bevorzugterweise der Abschnitt Helix B1 und der Abschnitt Helix B2 miteinander hybridisiert ist; und
die Abschnitte Helix C1 und Helix C2 jeweils einzeln und unabhängig drei Nukleotide umfassen, wobei bevorzugterweise die Abschnitte Helix C1 und Helix C2 miteinander hybridisiert sind.

17. HMGA bindende Nukleinsäure nach Anspruch 16, umfassend eine Sequenz, die ausgewählt ist aus der Gruppe umfassend SEQ. ID. No. 12.

18. Nukleinsäure nach einem der Ansprüche 1 bis 17, **dadurch gekennzeichnet, dass** sie an Transkriptionsfaktoren binden, insbesondere Transkriptionsfaktoren, die einen AT-Haken aufweisen.

19. Nukleinsäure bindend an einen Transkriptionsfaktor umfassend einen AT-Haken, wobei die Nukleinsäure einen Aufbau gemäß einem der Ansprüche 1 bis 17 umfasst.

20. Verfahren zum Screenen eines HMGA-Antagonisten oder HMGA-Agonisten umfassend die folgenden Schritte:
- Bereitstellen eines Kandidaten-HMGA-Antagonisten und/oder eines Kandidaten-HMGA-Agonisten,
- Bereitstellen einer Nukleinsäure nach einem der Ansprüche 1 bis 19,
- Bereitstellen eines Testsystems, welches ein Signal in Gegenwart eines HMGA-Antagonisten und/oder eines HMGA-Agonisten liefert, und
- Bestimmen, ob der Kandidaten-HMGA-Antagonist ein HMGA-Antagonist ist und/oder ob der Kandidaten-HMGA-Agonist ein HMGA-Agonist ist.

21. Verfahren zum Screenen eines HMGA-Agonisten und/oder eines HMGA-Antagonisten umfassend die folgenden Schritte:
- Bereitstellen eines an einer Phase, bevorzugterweise einer Festphase, imobilisierten HMGA,
- Bereitstellen einer Nukleinsäure gemäß einem der Ansprüche 1 bis 19, bevorzugterweise eine Nukleinsäure nach einem der Ansprüche 1 bis 19, die markiert ist,
- Zugeben eines Kandidaten-HMGA-Agonisten und/oder eines Kandidaten-HMGA-Antagonisten, und
- Bestimmen, ob der Kandidaten-HMGA-Agonist ein HMGA-Agonist ist und/oder ob der Kandidaten-HMGA-Antagonist ein HMGA-Antagonist ist.

22. Kit für den Nachweis von HMGA, umfassend eine Nukleinsäure nach einem der Ansprüche 1 bis 19.

23. HMGA-Antagonist, erhältlich durch ein Verfahren nach einem der Ansprüche 20 oder 21.

24. HMGA-Agonist, erhältlich durch ein Verfahren nach einem der Ansprüche 20 oder 21.

25. Komplex umfassend ein HMGA-Protein und eine Nukleinsäure nach einem der Ansprüche 1 bis 19.

26. HMGA bindende Nukleinsäure nach einem der Ansprüche 1 bis 19 zur Verwendung als Medikament zur Behandlung und/oder Prävention einer Erkrankung.

27. HMGA bindende Nukelinsäure nach einem der Ansprüche 1 bis 19 zur Verwendung als diagnostisches Mittel zur Diagnose einer Erkrankung.
